(19) **Europäisches Patentamt / European Patent Office / Office européen des brevets**

(11) **EP 2 138 097 A2**

(12) # EUROPEAN PATENT APPLICATION

(43) Date of publication:
**30.12.2009 Bulletin 2009/53**

(51) Int Cl.:
*A61B 5/15* (2006.01)   *A61M 5/172* (2006.01)
*A61B 5/00* (2006.01)   *G01N 33/487* (2006.01)
*A61M 5/142* (2006.01)

(21) Application number: **09169910.8**

(22) Date of filing: **13.02.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.02.2005 US 652660 P**
**02.03.2005 US 658001 P**
**21.04.2005 US 673551 P**
**06.10.2005 US 724199 P**
**21.12.2005 US 316407**

(62) Document number(s) of the earlier application(s) in accordance with Art. 76 EPC:
**06734872.2 / 1 855 591**

(71) Applicant: **Optiscan Biomedical Corporation**
**Hayward CA 94545 (US)**

(72) Inventors:
• **Hall, Dale W.**
**Oakland, CA 94618**
 **(US)**
• **Callicoat, David N.**
**Alameda, CA 94501 (US)**

• **Gable, Jennifer H.**
**Newark, CA 94560 (US)**
• **Braig, James R.**
**Piedmont, CA (US)**
• **Witte, Kenneth G.**
**San Jose, CA 95129 (US)**
• **Wechsler, Mark**
**San Mateo, CA 94403 (US)**
• **Rule, Peter**
**Los Altos Hills, CA 94024 (US)**
• **Keenan, Richard**
**Livermore, CA 94550 (US)**

(74) Representative: **HOFFMANN EITLE**
**Patent- und Rechtsanwälte**
**Arabellastrasse 4**
**81925 München (DE)**

Remarks:
This application was filed on 10-09-2009 as a divisional application to the application mentioned under INID code 62.

(54) **Apparatus and methods for analyzing body fluid samples**

(57)     An apparatus is provided for monitoring a predetermined parameter of a patient's body fluid while infusing an infusion fluid into the patient (6011). The apparatus comprises an infusion line (6017) and a catheter (6021) configured for insertion into a blood vessel of the patient, and a reversible infusion pump (6013) connected between a source (6015) of an infusion fluid and the infusion line and catheter. The apparatus further comprises a body fluid sensor assembly (6019) mounted in fluid communication with the infusion line and which includes a first sensor and a sample cell. The first sensor provides a signal indicative of a predetermined parameter of any fluid present in the infusion line. The apparatus further comprises a controller (6023) that is configured to operate the infusion pump in a forward direction so as to pump the infusion fluid through the infusion line and catheter for infusion into the patient. The controller is configured to intermittently interrupt its operating of the infusion pump in the forward direction to operate the infusion pump in a rearward direction so as to draw a body fluid sample from the patient through the catheter and infusion line. The controller, in response to detecting the arrival of the body fluid sample at the first sensor, is configured to cease its operating of the infusion pump in the rearward direction.

*FIG. 49*

**Description**

BACKGROUND

Field

Certain embodiments disclosed herein relate to methods and apparatus for determining the concentration of an analyte in a sample, such as an analyte in a sample of bodily fluid, as well as methods and apparatus which can be used to support the making of such determinations.

Description of the Related Art

It is a common practice to measure the levels of certain analytes, such as glucose, in a bodily fluid, such as blood. Often this is done in a hospital or clinical setting when there is a risk that the levels of certain analytes may move outside a desired range, which in turn can jeopardize the health of a patient. Certain currently known systems for analyte monitoring in a hospital or clinical setting suffer from various drawbacks.

SUMMARY

In one embodiment, an apparatus for monitoring a predetermined parameter of a patient's body fluid and infusing the patient is disclosed. The apparatus comprises an infusion line having a patient end configured for fluid communication with a blood vessel of the patient, a source of an infusion fluid, the source being in fluid communication with the infusion line, and a reversible infusion pump coupled to the infusion line. The apparatus further comprises a body fluid sensor assembly mounted in fluid communication with the infusion line and including a first sensor. The first sensor comprises an optical sensor configured to provide a signal indicative of a predetermined parameter of fluid present in the infusion line near the first sensor. The apparatus further comprises a controller that is configured to operate the infusion pump in a forward direction, to pump the infusion fluid through the infusion line toward the patient end. The controller is configured to intermittently interrupt its operation of the infusion pump in the forward direction to operate the infusion pump in a rearward direction, to draw a body fluid sample from the patient through the patient end of the infusion line.

An embodiment of a method of extracting and analyzing bodily fluids from a patient at the point of care for the patient comprises establishing fluid communication between an analyte detection system, a sensor assembly, and a bodily fluid in the patient. The sensor assembly comprises a first sensor. The method further comprises drawing from the patient a portion of the bodily fluid and separating from the drawn portion a first component of the bodily fluid, while the analyte detection system and the sensor assembly remain in fluid communication with the patient. The method additionally comprises analyzing, with the analyte detection system, the first component to measure a concentration of an analyte. In certain other embodiments, the first sensor comprises an optical sensor.

One embodiment of an apparatus for extracting and analyzing bodily fluids from a patient at the point of care for the patient comprises an infusion line having a patient end configured for fluid communication with a blood vessel of the patient, a source of an infusion fluid, the source in fluid communication with the infusion line, and a reversible infusion pump coupled to the infusion line. The apparatus further comprises a body fluid sensor assembly mounted in fluid communication with the infusion line and including a first sensor. The first sensor is configured to provide a signal indicative of a predetermined parameter of fluid present in the infusion line near the first sensor. Additionally, the apparatus includes a controller that is configured to operate the infusion pump in a forward direction, to pump the infusion fluid through the infusion line toward the patient end. The controller is configured to intermittently interrupt its operation of the infusion pump in the forward direction to operate the infusion pump in a rearward direction, to draw a body fluid sample from the patient through the patient end of the infusion line. The apparatus also comprises a fluid component separator mounted in fluid communication with the infusion line and configured to separate from the body fluid sample a first component of the body fluid sample. In certain other embodiments, the first sensor comprises an optical sensor.

In one embodiment, an apparatus for monitoring a predetermined parameter of a patient's blood while infusing an infusion fluid into the patient is disclosed. The apparatus comprises an infusion line having a patient end configured for insertion into a blood vessel of the patient, a source of an infusion fluid, the source being in fluid communication with the infusion line, and a reversible infusion pump coupled to the infusion line. The apparatus also includes a blood chemistry sensor assembly mounted in fluid communication with the infusion line and including a first sensor that provides a signal indicative of a predetermined parameter of fluid present in the infusion line. The apparatus further comprises a device operatively connected to the apparatus to provide one or more anti-clotting agents to at least a portion of the infusion line. The apparatus further comprises a controller configured to operate the infusion pump in a forward direction, to pump the infusion fluid through the patient end of the infusion line for infusion into the patient. The controller is configured to intermittently interrupt its operation of the infusion pump in the forward direction to operate the infusion pump in a rearward direction, to draw a blood sample from the patient through the patient end of the infusion line into sensing contact with the first sensor of the blood chemistry sensor assembly. In certain other embodiments, the first sensor comprises an optical sensor.

In another embodiment, a patient status monitoring system is disclosed. The system comprises a first body fluid analyzer and a body fluid sensor assembly. The body fluid sensor assembly includes a first sensor that provides a signal indicative of a predetermined parameter of fluid present in the body fluid sensor assembly. The system includes a controller configured to monitor the signal from the body fluid sensor assembly and to detect a change in the signal indicative of an arrival of the fluid present in the body fluid sensor assembly at the first sensor. The system further comprises a fluid passageway configured to provide fluid communication among the first body fluid analyzer, the body fluid sensor assembly, and a body fluid in a patient. The first body fluid analyzer is configured to be in communication with a data network, which includes at least one data file. The first body fluid analyzer is configured to access the at least one data file via the data network. In certain other embodiments, the first sensor comprises an optical sensor.

An embodiment of an analyte detection system comprises a body fluid sensor assembly including a first sensor. The first sensor is configured to provide information relating to a measurement of at least one analyte in a body fluid sample that is in sensing contact with the first sensor. The system further comprises a processor and stored program instructions executable by the processor such that the system: (a) identifies, based on the measurement, one or more possible interferents to the measurement of the at least one analyte in the body fluid sample; (b) calculates a calibration which reduces error attributable to the one or more possible interferents; (c) applies the calibration to the measurement; and (d) estimates, based on the calibrated measurement, a concentration of the at least one analyte in the body fluid sample. In certain other embodiments, the first sensor comprises an optical sensor.

One embodiment of an apparatus for analyzing the composition of bodily fluid comprises a first fluid passageway having a patient end, which is configured to provide fluid communication with a bodily fluid within a patient, and at least one pump coupled to the first fluid passageway. The at least one pump is configured to have an infusion mode in which the pump is operable to deliver infusion fluid to the patient through the patient end and a sample draw mode in which the pump is operable to draw a sample of the bodily fluid from the patient through the patient end. The apparatus further comprises a controller that is configured to operate the at least one pump in the infusion mode and in the sample draw mode. The apparatus also comprises a spectroscopic analyte detection system accessible via the first fluid passageway such that the analyte detection system can receive at least one component of the drawn sample of bodily fluid and can determine a concentration of at least one analyte. The analyte detection system is spaced from the patient end of the first fluid passageway. The apparatus also includes a body fluid sensor assembly mounted in fluid communication with the first fluid passageway at or near the patient end and spaced from the analyte detection system. The body fluid sensor assembly includes a first sensor and a second sensor. The first sensor is configured to sense a first property indicative of the fluid within the first fluid passageway and to provide a first signal indicative of the first property. The second sensor is configured to sense a second property indicative of the fluid within the first fluid passageway and to provide a second signal indicative of the second property. The controller is configured to monitor the first signal provided by the first sensor and to detect a change in the first signal indicative of an arrival of the drawn sample of body fluid at the first sensor.

In some embodiments of the apparatus for analyzing the composition of bodily fluid, the first sensor is selected from the group consisting of a colorimetric sensor, a hemoglobin sensor, a hematocrit sensor, a pressure sensor, a dilution sensor, and a bubble sensor. In other embodiments, the second sensor comprises an electrochemical sensor or an optical sensor.

One embodiment of a fluid handling and analysis system comprises a fluid transport network comprising at least a first fluid passageway. The fluid transport network includes a patient end configured to maintain fluid communication with a bodily fluid in a patient and a sample analysis chamber and waste container, each accessible by the fluid transport network. The system also includes a bodily fluid sensor assembly in fluid communication with the fluid transport network. The bodily fluid sensor assembly includes a first sensor that provides a signal indicative of a predetermined parameter of fluid present in the first fluid passageway. Additionally, the system includes a pump unit in operative engagement with the fluid transport network and configured to have an infusion mode in which the pump unit delivers an infusion fluid to the patient through the patient end and a sample draw mode in which the pump unit draws a volume of the bodily fluid from the patient through the patient end, toward the sample analysis chamber. The fluid transport network and the pump unit are configured to draw a volume of the bodily fluid from the patient, isolate a fraction of the bodily fluid from the volume, and pass the fraction to the sample analysis chamber and then to the waste container. In certain other embodiments, the first sensor comprises an optical sensor.

An embodiment of an apparatus for monitoring a predetermined parameter of a patient's body fluid while infusing an infusion fluid into the patient is disclosed. The apparatus comprises an infusion line having a patient end configured for insertion into a blood vessel of the patient, a source of an infusion fluid, the source being in fluid communication with the infusion line, and a reversible infusion pump coupled to the infusion line. The apparatus also includes a body fluid sensor assembly mounted in fluid communication with the infusion line. The sensor assembly includes a temperature sensor that provides temperature information relating to fluid present in the body fluid sensor assembly. The sensor assembly also includes a first sensor that provides a signal indicative of a parameter of fluid present in the body fluid sensor assembly. The apparatus further comprises a controller configured to operate the infusion pump in a forward direction, to pump the infusion fluid through the infusion line for infusion into the patient. The controller is configured to intermittently interrupt its operation of the infusion pump in the forward direction to operate the infusion pump in a rearward direction, to draw a body fluid sample from the patient through the patient end of the infusion line into sensing contact with the first sensor and the temperature sensor of the body fluid sensor assembly. The controller further is configured to monitor the signal provided by the first sensor of the body fluid sensor assembly and to detect a change in the first signal indicative of an arrival of the body fluid sample at the first sensor. The signal produced by the first sensor provides an indication of a predetermined parameter of the patient's body fluid when the body fluid sample is in sensing contact with the first sensor. The controller is configured to acquire the temperature information and to use the temperature information when determining the predetermined parameter of the patient's body fluid. In certain other embodiments, the first sensor comprises an optical sensor.

In one embodiment, an apparatus is provided for monitoring a predetermined parameter of a patient's body fluid while infusing an infusion fluid into the patient. The apparatus comprises an infusion line and a catheter configured for insertion into a blood vessel of the patient, and a reversible infusion pump connected between a source of an infusion fluid and the infusion line and catheter. The apparatus further comprises a body fluid sensor assembly mounted in fluid communication with the infusion line and which includes a first sensor and a sample cell. The first sensor provides a signal indicative of a predetermined parameter of any fluid present in the infusion line. The sample cell is substantially transmissive to light comprising a wavelength $\lambda$. The apparatus further comprises a controller configured to operate the infusion pump in a forward direction so as to pump the infusion fluid through the infusion line and catheter for infusion into the patient. The controller is configured to intermittently interrupt its operating of the infusion pump in the forward direction to operate the infusion pump in a rearward direction so as to draw a body fluid sample from the patient through the catheter and infusion line. The body fluid sample drawn from the patient is disposed such that a first portion of the body fluid sample is in sensing contact with the first sensor of the body fluid sensor assembly, and a second portion of the body fluid sample is disposed within the sample cell of the body fluid sensor assembly. The controller further is configured to monitor the signal provided by the first sensor of the body fluid sensor assembly and to detect a change in the signal indicative of the arrival of the body fluid sample at the first sensor. The controller, in response to detecting the arrival of the body fluid sample at the first sensor, is configured to cease its operating of the infusion pump in the rearward direction. The signal produced by the first sensor provides an indication of a predetermined parameter of the patient's body fluid when the body fluid sample is in sensing contact with the first sensor.

In another embodiment, the apparatus further comprises a light source configured to produce light comprising the wavelength $\lambda$ and a light detector configured to be responsive to light comprising the wavelength $\lambda$. An optical path is defined from the light source to the sample cell and from the sample cell to the light detector such that the light source, the light detector, and the sample cell are configured to be in optical communication along the optical path.

One embodiment of a method of extracting and analyzing bodily fluids from a patient at the point of care for the patient is disclosed. The method comprises establishing fluid communication between an analyte detection system, a sensor assembly, and a bodily fluid in the patient. The sensor assembly comprises a first sensor. The method further comprises drawing from the patient a portion of the bodily fluid and monitoring a signal produced by the first sensor of the sensor assembly and detecting a change in the signal indicative of an arrival of the bodily fluid at the first sensor. The method additionally comprises ceasing to draw the bodily fluid from the patient in response to detecting the arrival of the bodily fluid at the first sensor and separating from the drawn portion a first component of the bodily fluid, while the analyte detection system and the sensor assembly remain in fluid communication with the patient. Additionally, the analyte detection system is used for analyzing the first component to measure a concentration of an analyte.

An embodiment of an apparatus for monitoring a predetermined parameter of a patient's blood while infusing an infusion fluid into the patient is disclosed. The apparatus comprises an infusion line and a catheter configured for insertion into a blood vessel of the patient and a reversible infusion pump connected between a source of an infusion fluid and the infusion line and catheter. The apparatus also includes a blood chemistry sensor assembly mounted in fluid communication with the infusion line and which includes a first sensor that provides a signal indicative of a predetermined parameter of any fluid present in the infusion line. A device is operatively connected to provide one or more anti-clotting agents to at least a portion of the infusion line. The apparatus further comprises a controller that operates the infusion pump in a forward direction so as to pump the infusion fluid through the infusion line and catheter for infusion into the patient. The controller intermittently interrupts its operating of the infusion pump in the forward direction to operate the infusion pump in a rearward direction so as to draw a blood sample from the patient through the catheter and infusion line into sensing contact with the first sensor of the blood chemistry sensor assembly. The controller further is configured to monitor the signal provided by the first sensor of the blood chemistry sensor assembly and to detect a change in the signal indicative of the arrival of the blood sample at the first sensor. The controller, in response to detecting the arrival of the blood sample at the first sensor, ceases its operating of the infusion pump in the rearward direction. The signal that is produced by the first sensor provides an indication of a predetermined parameter of the patient's blood when the blood sample is in sensing contact with the first sensor.

In another embodiment of this apparatus, the one or more anti-clotting agents comprise a detergent, and the device provides the detergent within at least a portion of the infusion line. In yet another embodiment of the apparatus, the anti-clotting device comprises an ultrasound generator positionable to transmit an ultrasonic energy to the infusion line.

An embodiment of a patient status monitoring system comprises a body fluid analyzer, a body fluid sensor assembly including a first sensor that provides a signal indicative of a predetermined parameter of any fluid present in the body fluid sensor assembly, and a controller configured to monitor the signal from the body fluid sensor assembly and to detect a change in the signal indicative of an arrival of the body fluid sample at the first sensor. The system also comprises a fluid passageway configured to provide fluid communication among the body fluid analyzer, the body fluid sensor assembly, and a body fluid in a patient. The controller is configured to be in communication with a data network that is configured to include at least one data file. The controller is configured to access the at least one data file via the data network. In another embodiment, the controller is configured to update the at least one data file.

In another embodiment of the patient status monitoring system, the system further comprises an infusion line and a catheter configured for insertion into a blood vessel of the patient and a reversible infusion pump connected between a source of an infusion fluid and the infusion line and catheter. The controller is configured to operate the infusion pump in a forward direction so as to pump the infusion fluid through the infusion line and catheter for infusion into the patient. The controller is configured to intermittently interrupt its operating of the infusion pump in the forward direction to operate the infusion pump in a rearward direction so as to draw a body fluid sample from the patient through the catheter and infusion line into sensing contact with the first sensor of the body fluid sensor assembly. The controller, in response to detecting the arrival of the body fluid sample at the first sensor, ceases its operating of the infusion pump in the rearward direction. The signal produced by the first sensor provides an indication of a predetermined parameter of the patient's body fluid when the body fluid sample is in sensing contact with the first sensor.

An embodiment of an analyte detection system comprises a body fluid sensor assembly including a first sensor that is configured to provide information relating to a measurement of at least one analyte in a body fluid sample that is in sensing contact with the first sensor. The system includes a processor and stored program instructions executable by the processor. The stored program instructions are such that the system can identify, based on the measurement, one or more possible interferents to the measurement of the at least one analyte in the body fluid sample, calculate a calibration which reduces error attributable to the one or more possible interferents, apply the calibration to the measurement, and estimate, based on the calibrated measurement, a concentration of the at least one analyte in the body fluid sample.

Another embodiment of this analyte detection system further comprises an infusion line and a catheter configured for insertion into a blood vessel of a patient and a reversible infusion pump fluidly connected between a source of an infusion fluid and the infusion line and catheter. The infusion pump and the body fluid sensor assembly are configured to be in fluid communication with the infusion line. A controller operates the infusion pump in a forward direction so as to pump the infusion fluid through the infusion line and catheter for infusion into the patient. The controller intermittently interrupts its operating of the infusion pump in the forward direction to operate the infusion pump in a rearward direction so as to draw the body fluid sample from the patient through the catheter and infusion line into sensing contact with the first sensor of body fluid sensor assembly. The controller further is configured to monitor the signal provided by the first sensor of the body fluid sensor assembly and to detect a change in the signal indicative of the arrival of the body fluid sample at the first sensor. The controller, in response to detecting the arrival of the body fluid sample at the first sensor, ceases its operating of the infusion pump in the rearward direction.

An embodiment of an apparatus for analyzing the composition of bodily fluid comprises a first fluid passageway having a patient end which is configured to provide fluid communication with a bodily fluid within a patient and at least one pump coupled to the first fluid passageway. The at least one pump has an infusion mode in which the pump is operable to deliver infusion fluid to the patient through the patient end and a sample draw mode in which the pump is operable to draw a sample of the bodily fluid from the patient through the patient end. A controller is configured to operate the at least one pump in the infusion mode and in the sample draw mode. The apparatus includes a spectroscopic analyte detection system accessible via the first fluid passageway such that the analyte detection system can receive at least one component of the drawn sample of bodily fluid and can determine a concentration of at least one analyte. The analyte detection system is spaced from the patient end of the first fluid passageway. The apparatus also includes a body fluid sensor assembly mounted in fluid communication with the first fluid passageway at or near the patient end and spaced from the analyte detection system. The body fluid sensor assembly includes a first sensor configured to sense a property of the fluid within the first fluid passageway and configured to provide a signal indicative of the property. The controller is configured to monitor the signal provided by the first sensor of the body fluid sensor assembly and to detect a change in the signal indicative of an arrival of the body fluid at the first sensor.

In another embodiment of the apparatus for analyzing the composition of bodily fluid, the first sensor is selected from the group consisting of a colorimetric sensor, a hemoglobin sensor, a hematocrit sensor, a pressure sensor, a dilution sensor, and a bubble sensor

An embodiment of a fluid handling and analysis system is disclosed. The system comprises a fluid transport network, which comprises at least a first fluid passageway. The fluid transport network also includes a patient end configured to maintain fluid communication with a bodily fluid in a patient. The system includes a sample analysis chamber and waste container, which are each accessible by the fluid transport network. A bodily fluid sensor assembly is configured to be in fluid communication with the fluid transport network and includes a first sensor that provides a signal indicative of a predetermined parameter of any fluid present in the first fluid passageway. A pump unit is in operative engagement with the fluid transport network. The pump unit has an infusion mode in which the pump unit delivers an infusion fluid to the patient through the patient end and a sample draw mode in which the pump unit draws a volume of the bodily fluid from the patient through the patient end and toward the sample analysis chamber. A spectroscopic fluid analyzer is configured to analyze a sample of the bodily fluid while the sample is in the sample analysis chamber and to determine a concentration of at least one analyte. The fluid transport network and the pump unit are configured to draw a volume of the bodily fluid from the patient, isolate a fraction of the bodily fluid from the volume, and pass the fraction to the sample analysis chamber and then to the waste container.

An embodiment of an apparatus for monitoring a predetermined parameter of a patient's body fluid while infusing an infusion fluid into the patient is disclosed. The apparatus comprises an infusion line and a catheter configured for insertion into a blood vessel of the patient and a reversible infusion pump connected between a source of an infusion fluid and the infusion line and catheter. A body fluid sensor assembly is mounted in fluid communication with the infusion line and includes a first sensor that provides a signal indicative of a parameter of any fluid present in the body fluid sensor assembly. The body fluid sensor assembly further includes a thermal device. A controller operates the infusion pump in a forward direction so as to pump the infusion fluid through the infusion line and catheter for infusion into the patient. The controller intermittently interrupts its operating of the infusion pump in the forward direction to operate the infusion pump in a rearward direction so as to draw a body fluid sample from the patient through the catheter and infusion line into sensing contact with the first sensor and the thermal device of the body fluid sensor assembly. The controller further is configured to monitor the signal provided by the first sensor of the body fluid sensor assembly and to detect a change in the first signal indicative of an arrival of the body fluid sample at the first sensor. The controller, in response to detecting the arrival of the body fluid sample at the first sensor, ceases its operating of the infusion pump in the rearward direction. The signal that is produced by the first sensor provides an indication of a predetermined parameter of the patient's body fluid when the body fluid sample is in sensing contact with the first sensor. The controller is configured to communicate with the thermal device so as to regulate a temperature of the body fluid sample.

In other embodiments of the apparatus for monitoring a predetermined parameter of a patient's body fluid, the thermal device may comprise a thermoelectric device. In one embodiment, the thermoelectric device comprises a Peltier thermoelectric device.

Certain objects and advantages of the invention(s) are described herein. Of course, it is to be understood that not necessarily all such objects or advantages may be achieved in accordance with any particular embodiment. Thus, for example, those skilled in the art will recognize that the invention(s) may be embodied or carried out in a manner that achieves or optimizes one advantage or group of advantages as taught herein without necessarily achieving other objects or advantages as may be taught or suggested herein.

Certain embodiments are summarized above. However, despite the foregoing discussion of certain embodiments, only the appended claims (and not the present summary) are intended to define the invention(s). The summarized embodiments, and other embodiments, will become readily apparent to those skilled in the art from the following detailed description of the preferred embodiments having reference to the attached figures, the invention(s) not being limited to any particular embodiment(s) disclosed.

BRIEF DESCRIPTION OF THE DRAWINGS

FIGURE 1 is a schematic of a fluid handling system in accordance with one embodiment;

FIGURE 1A is a schematic of a fluid handling system, wherein a fluid handling and analysis apparatus of the fluid handling system is shown in a cutaway view;

FIGURE 1B is a cross-sectional view of a bundle of the fluid handling system of FIGURE 1A taken along the line 1B-1B;

FIGURE 2 is a schematic of an embodiment of a sampling apparatus;

FIGURE 3 is a schematic showing details of an embodiment of a sampling apparatus;

FIGURE 4 is a schematic of an embodiment of a sampling unit;

FIGURE 5 is a schematic of an embodiment of a sampling apparatus;

FIGURE 6A is a schematic of an embodiment of gas injector manifold;

FIGURE 6B is a schematic of an embodiment of gas injector manifold;

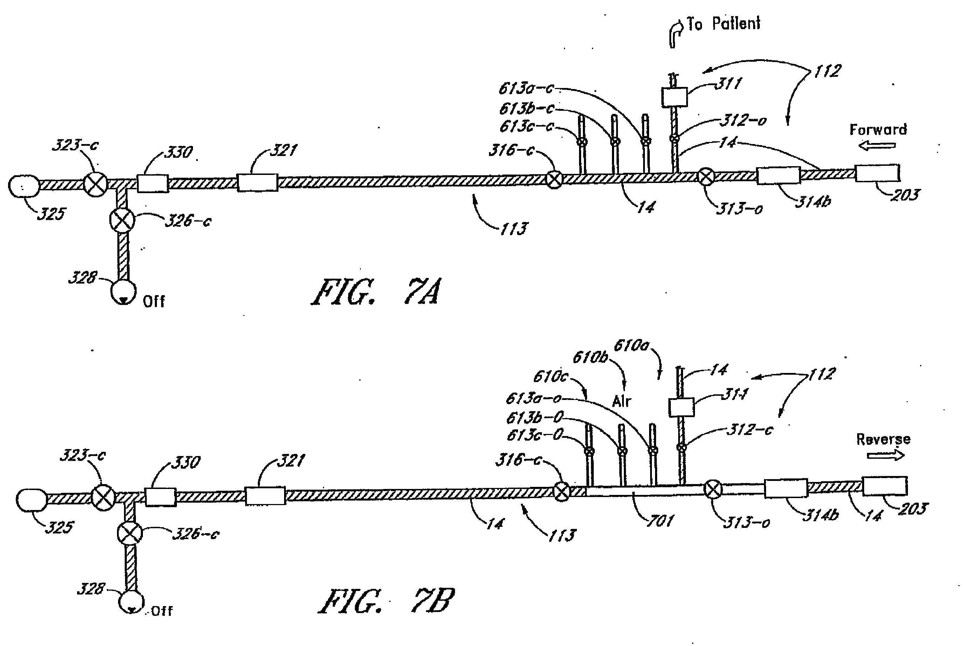

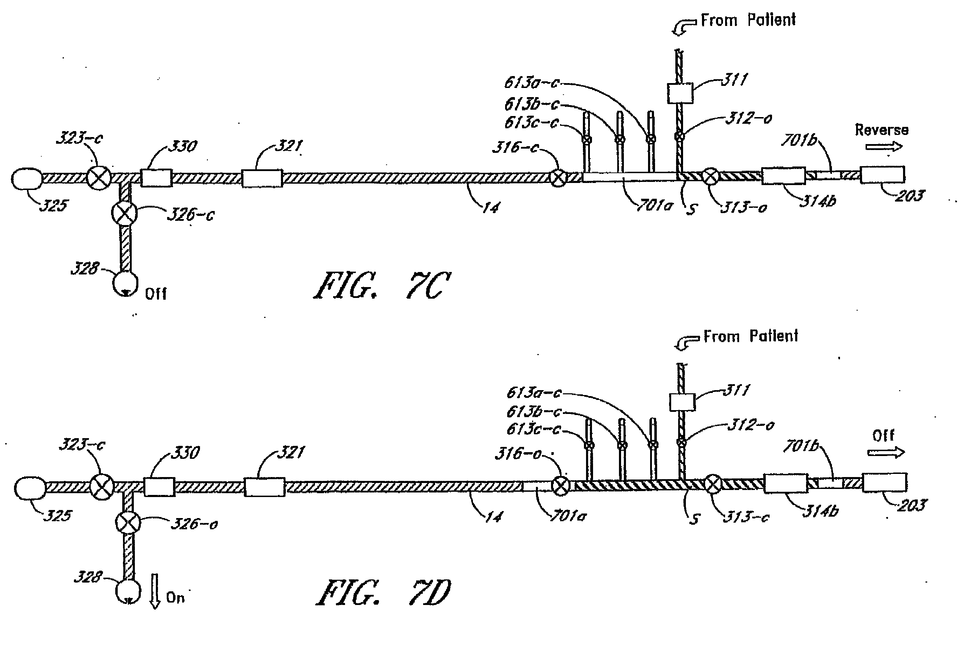

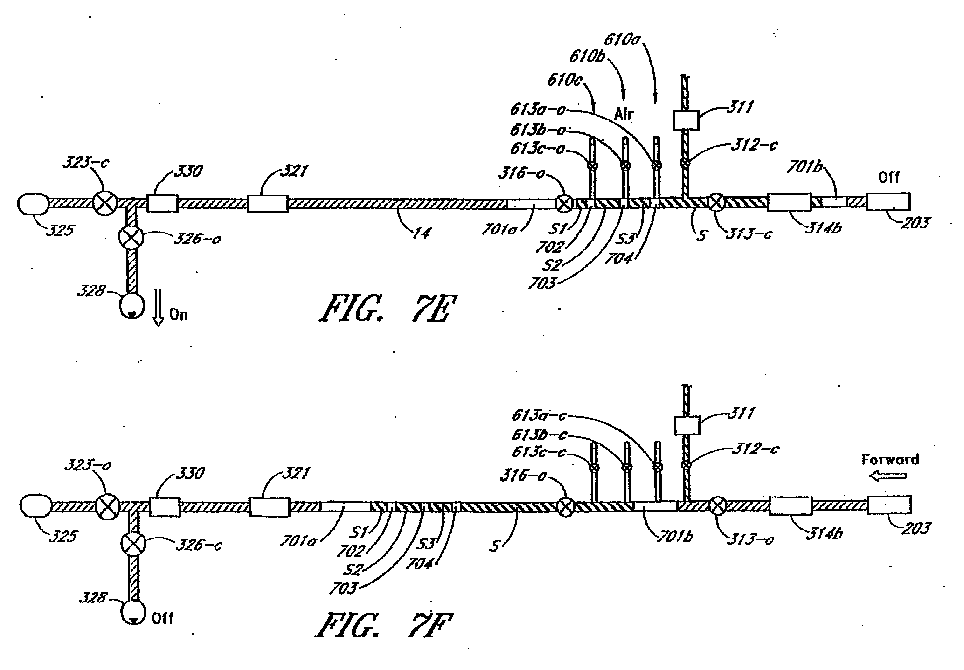

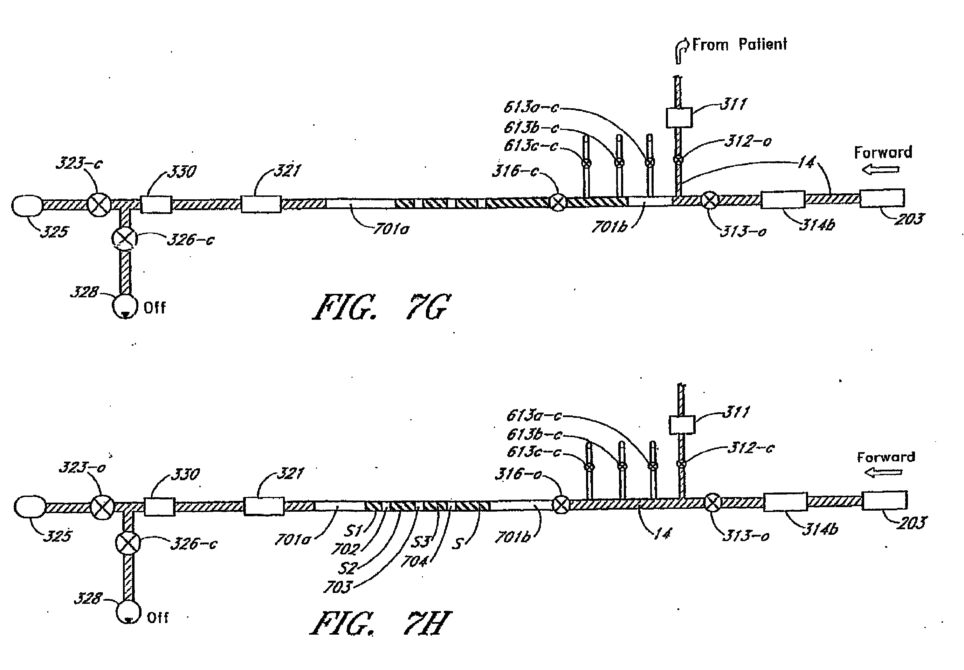

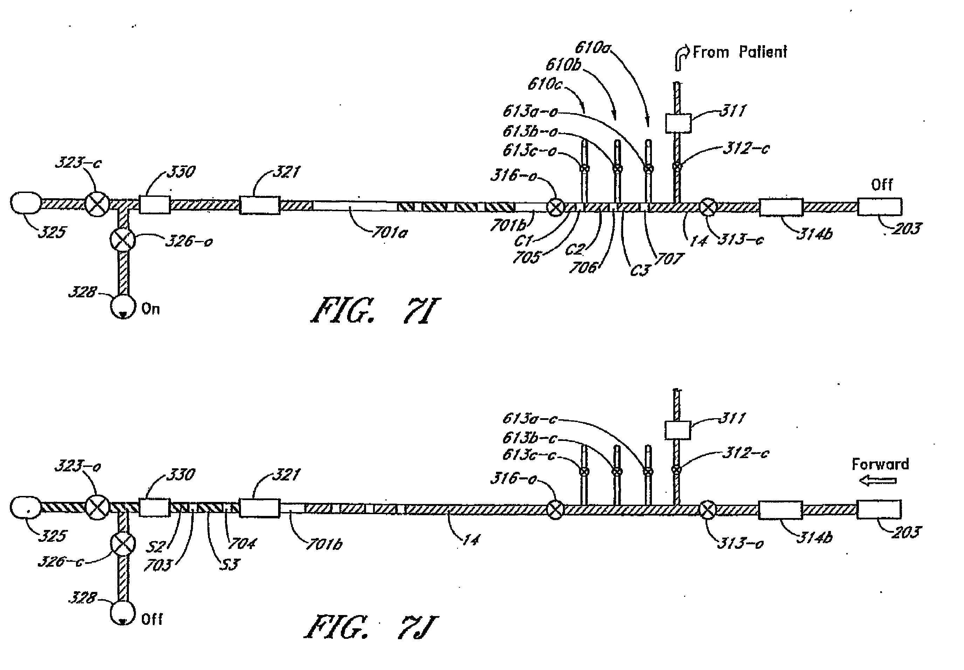

FIGURES 7A-7J are schematics illustrating methods of using the infusion and blood analysis system, where FIGURE 7A shows one embodiment of a method of infusing a patient, and FIGURES 7B-7J illustrate steps in a method of sampling from a patient, where FIGURE 7B shows fluid being cleared from a portion of the first and second passageways; FIGURE 7C shows a sample being drawn into the first passageway; FIGURE 7D shows a sample being drawn into second passageway; FIGURE 7E shows air being injected into the sample; FIGURE 7F shows bubbles being cleared from the second passageway; FIGURES 7H and 7I show the sample being pushed part way into the second passageway followed by fluid and more bubbles; and FIGURE 7J shows the sample being pushed to analyzer;

FIGURE 8 is a perspective front view of an embodiment of a sampling apparatus;

FIGURE 9 is a schematic front view of one embodiment of a sampling apparatus cassette;

FIGURE 10 is a schematic front view of one embodiment of a sampling apparatus instrument;

FIGURE 11 is an illustration of one embodiment of an arterial patient connection;

FIGURE 12 is an illustration of one embodiment of a venous patient connection;

FIGURES 13A, 13B, and 13C are various views of one embodiment of a pinch valve, where FIGURE 13A is a front view, FIGURE 13B is a sectional view, and FIGURE 13C is a sectional view showing one valve in a closed position;

FIGURES 14A and 14B are various views of one embodiment of a pinch valve, where FIGURE 14A is a front view and FIGURE 14B is a sectional view showing one valve in a closed position;

FIGURE 15 is a side view of one embodiment of a separator;

FIGURE 16 is an exploded perspective view of the separator of FIGURE 15;

FIGURE 17 is one embodiment of a fluid analysis apparatus;

FIGURE 18 is a top view of a cuvette for use in the apparatus of FIGURE 17;

FIGURE 19 is a side view of the cuvette of FIGURE 18;

FIGURE 20 is an exploded perspective view of the cuvette of FIGURE 18;

FIGURE 21 is a schematic of an embodiment of a sample preparation unit;

FIGURE 22A is a perspective view of another embodiment of a fluid handling and analysis apparatus having a main instrument and removable cassette;

FIGURE 22B is a partial cutaway, side elevational view of the fluid handling and analysis apparatus with the cassette spaced from the main instrument;

FIGURE 22C is a cross-sectional view of the fluid handling and analysis apparatus of FIGURE 22A wherein the cassette is installed onto the main instrument;

FIGURE 23A is a cross-sectional view of the cassette of the fluid handling and analysis apparatus of FIGURE 22A taken along the line 23A-23A;

FIGURE 23B is a cross-sectional view of the cassette of FIGURE 23A taken along the line 23B-23B of FIGURE 23A;

FIGURE 23C is a cross-sectional view of the fluid handling and analysis apparatus having a fluid handling network, wherein a rotor of the cassette is in a generally vertical orientation;

FIGURE 23D is a cross-sectional view of the fluid handling and analysis apparatus, wherein the rotor of the cassette is in a generally horizontal orientation;

FIGURE 23E is a front elevational view of the main instrument of the fluid handling and analysis apparatus of FIGURE 23C;

FIGURE 24A is a cross-sectional view of the fluid handling and analysis apparatus having a fluid handling network in accordance with another embodiment;

FIGURE 24B is a front elevational view of the main instrument of the fluid handling and analysis apparatus of FIGURE 24A;

FIGURE 25A is a front elevational view of a rotor having a sample element for holding sample fluid;

FIGURE 25B is a rear elevational view of the rotor of FIGURE 25A;

FIGURE 25C is a front elevational view of the rotor of FIGURE 25A with the sample element filled with a sample fluid;

FIGURE 25D is a front elevational view of the rotor of FIGURE 25C after the sample fluid has been separated;

FIGURE 25E is a cross-sectional view of the rotor taken along the line 25E-25E of FIGURE 25A;

FIGURE 25F is an enlarged sectional view of the rotor of FIGURE 25E;

FIGURE 26A is an exploded perspective view of a sample element for use with a rotor of a fluid handling and analysis apparatus;

FIGURE 26B is a perspective view of an assembled sample element;

FIGURE 27A is a front elevational view of a fluid interface for use with a cassette;

FIGURE 27B is a top elevational view of the fluid interface of FIGURE 27A;

FIGURE 27C is an enlarged side view of a fluid interface engaging a rotor;

FIGURE 28 is a cross-sectional view of the main instrument of the fluid handling and analysis apparatus of FIGURE 22A taken along the line 28-28;

FIGURE 29 is a graph illustrating the absorption spectra of various components that may be present in a blood sample;

FIGURE 30 is a graph illustrating the change in the absorption spectra of blood having the indicated additional components of FIGURE 29 relative to a Sample Population blood and glucose concentration, where the contribution due to water has been numerically subtracted from the spectra;

FIGURE 31 is an embodiment of an analysis method for determining the concentration of an analyte in the presence of possible interferents;

FIGURE 32 is one embodiment of a method for identifying interferents in a sample for use with the embodiment of FIGURE 31;

FIGURE 33A is a graph illustrating one embodiment of the method of FIGURE 32, and FIGURE 33B is a graph further illustrating the method of FIGURE 32;

FIGURE 34 is a one embodiment of a method for generating a model for identifying possible interferents in a sample for use with an embodiment of FIGURE 31;

FIGURE 35 is a schematic of one embodiment of a method for generating randomly-scaled interferent spectra;

FIGURE 36 is one embodiment of a distribution of interferent concentrations for use with the embodiment of FIGURE 35;

FIGURE 37 is a schematic of one embodiment of a method for generating combination interferent spectra;

FIGURE 38 is a schematic of one embodiment of a method for generating an interferent-enhanced spectral database;

FIGURE 39 is a graph illustrating the effect of interferents on the error of glucose estimation;

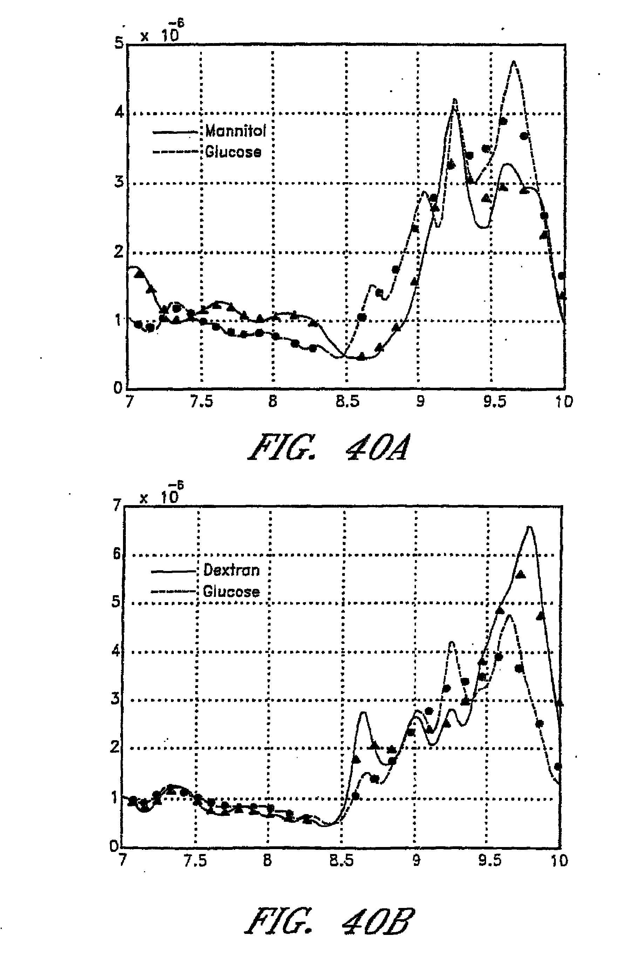

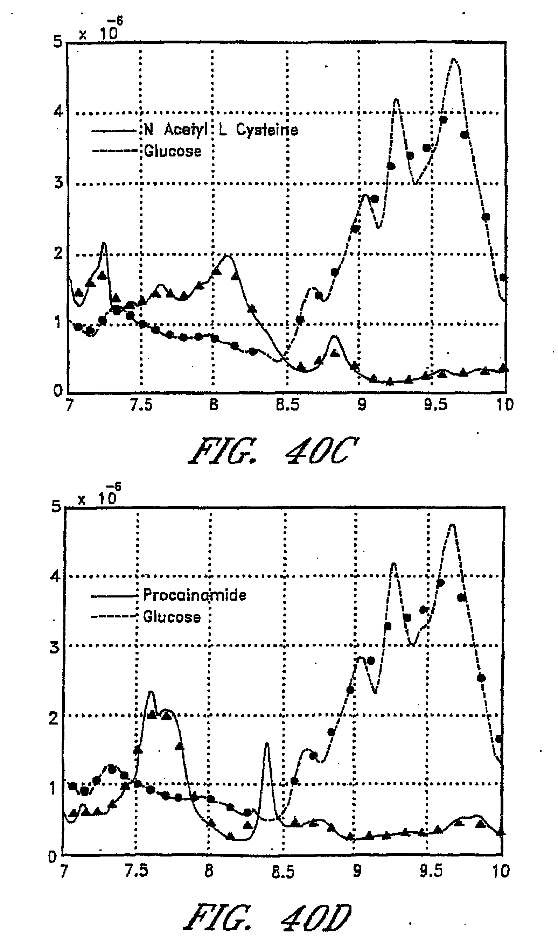

FIGURES 40A, 40B, 40C, and 40D each have a graph showing a comparison of the absorption spectrum of glucose with different interferents taken using two different techniques: a Fourier Transform Infrared (FTIR) spectrometer having an interpolated resolution of 1 cm$^{-1}$ (solid lines with triangles); and by 25 finite-bandwidth IR filters having a Gaussian profile and full-width half-maximum (FWHM) bandwidth of 28 cm$^{-1}$ corresponding to a bandwidth that varies from 140 nm at 7.08 $\mu$m, up to 279 nm at 10 $\mu$m (dashed lines with circles). The Figures show a comparison of glucose with mannitol (FIGURE 40A), dextran (FIGURE 40B), n-acetyl L cysteine (FIGURE 40C), and procainamide (FIGURE 40D), at a concentration level of 1 mg/dL and path length of 1 $\mu$m;

FIGURE 41 shows a graph of the blood plasma spectra for 6 blood sample taken from three donors in arbitrary units for a wavelength range from 7 $\mu$m to 10 $\mu$m, where the symbols on the curves indicate the central wavelengths of the 25 filters;

FIGURES 42A, 42B, 42C, and 42D contain spectra of the Sample Population of 6 samples having random amounts of mannitol (FIGURE 42A), dextran (FIGURE 42B), n-acetyl L cysteine (FIGURE 42C), and procainamide (FIGURE 42D), at a concentration levels of 1 mg/dL and path lengths of 1 $\mu$m;

FIGURES 43A-43D are graphs comparing calibration vectors obtained by training in the presence of an interferent, to the calibration vector obtained by training on clean plasma spectra for mannitol (FIGURE 43A), dextran (FIGURE 43B), n-acetyl L cysteine (FIGURE 43C), and procainamide (FIGURE 43D) for water-free spectra;

FIGURE 44 is a schematic illustration of another embodiment of the analyte detection system;

FIGURE 45 is a plan view of one embodiment of a filter wheel suitable for use in the analyte detection system depicted in FIGURE 44;

FIGURE 46 is a partial sectional view of another embodiment of an analyte detection system;

FIGURE 47 is a detailed sectional view of a sample detector of the analyte detection system illustrated in FIGURE 46;

FIGURE 48 is a detailed sectional view of a reference detector of the analyte detection system illustrated in FIGURE 46;

FIGURE 49 is a diagrammatic illustration of an automated infusion and blood testing system;

FIGURE 50 is a plan view of a first blood chemistry sensor assembly, this sensor assembly including sensors indicative of the concentrations of carbon dioxide, oxygen, potassium, calcium, and sodium, as well as sensors indicative of hematocrit, temperature, and pH;

FIGURE 50A is a top view of the sensor assembly shown in FIGURE 50;

FIGURE 51 is a plan view of a second blood chemistry sensor assembly, this sensor assembly including a sensor indicative of glucose concentration;

FIGURE 52A is a diagrammatic illustration of an automated infusion and blood testing system that includes an anti-clotting device comprising a cleaning solution;

FIGURE 52B is a diagrammatic illustration of an automated infusion and blood testing system wherein the anti-clotting cleaning solution is disposed within a sensor assembly;

FIGURE 52C is a diagrammatic illustration of an automated infusion and blood testing system wherein an anti-clotting device comprises an ultrasonic transducer;

FIGURE 53A is a schematic illustration of one embodiment of a body fluid analyzing system in communication with a data system and in communication with a patient;

FIGURE 53B is a schematic illustration of one embodiment of a body fluid analyzing system in communication with a data system and in communication with a patient and that further comprises a second body fluid analyzer separate from the patient.

FIGURE 54A is a plan view of an embodiment of a blood chemistry sensor assembly including a color sensor;

FIGURE 54B is a plan view of an embodiment of a blood chemistry sensor assembly providing blood separation;

FIGURE 54C is a plan view of an embodiment of a blood chemistry sensor assembly providing blood separation and in fluid communication with an analyzer; and

FIGURE 54D is a plan view of another embodiment of a blood chemistry sensor assembly providing blood separation and in fluid communication with an analyzer.

Reference symbols are used in the Figures to indicate certain components, aspects or features shown therein, with reference symbols common to more than one Figure indicating like components, aspects or features shown therein.

DETAILED DESCRIPTION OF THE PREFERRED EMBODIMENTS

Although certain preferred embodiments and examples are disclosed below, it will be understood by those skilled in the art that the inventive subject matter extends beyond the specifically disclosed embodiments to other alternative embodiments and/or uses of the invention, and to obvious modifications and equivalents thereof. Thus it is intended that the scope of the inventions herein disclosed should not be limited by the particular disclosed embodiments described below. Thus, for example, in any method or process disclosed herein, the acts or operations making up the method/process may be performed in any suitable sequence, and are not necessarily limited to any particular disclosed sequence. For purposes of contrasting various embodiments with the prior art, certain aspects and advantages of these embodiments are described where appropriate herein. Of course, it is to be understood that not necessarily all such aspects or advantages may be achieved in accordance with any particular embodiment. Thus, for example, it should be recognized that the various embodiments may be carried out in a manner that achieves or optimizes one advantage or group of advantages as taught herein without necessarily achieving other aspects or advantages as may be taught or suggested herein. While the systems and methods discussed herein can be used for invasive techniques, the systems and methods can also be used for non-invasive techniques or other suitable techniques, and can be used in hospitals, healthcare facilities, ICUs, or residences.

OVERVIEW OF EMBODIMENTS OF FLUID HANDLING SYSTEMS

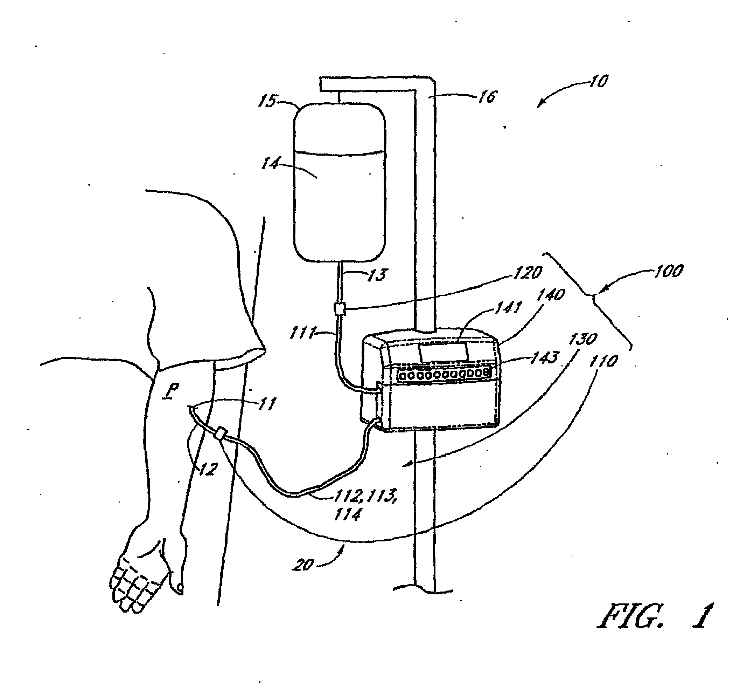

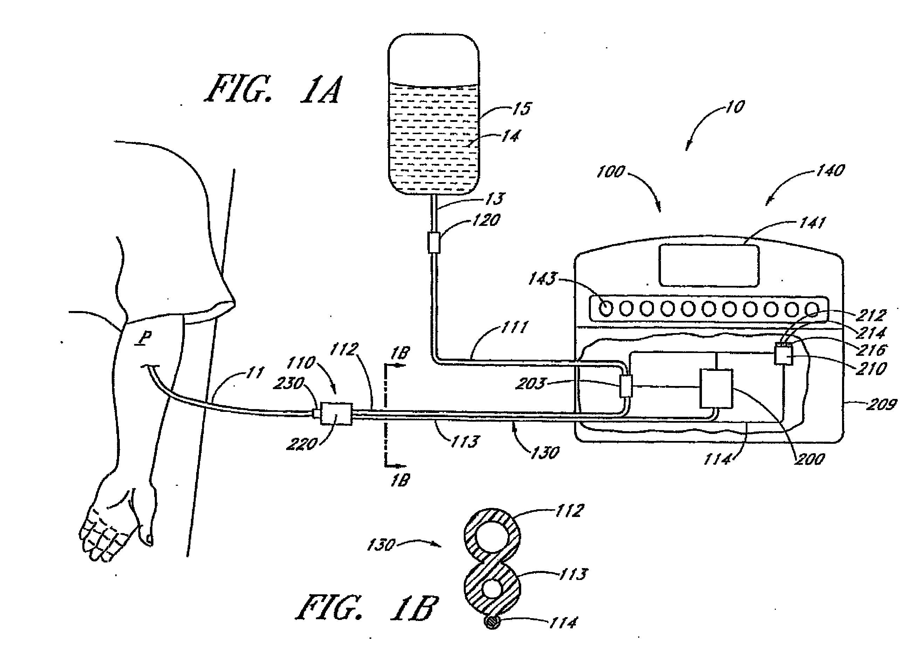

Disclosed herein are fluid handling systems and various methods of analyzing sample fluids. FIGURE 1 illustrates an embodiment of a fluid handling system 10 which can determine the concentration of one or more substances in a sample fluid, such as a whole blood sample from a patient P. The fluid handling system 10 can also deliver an infusion fluid 14 to the patient P.

The fluid handling system 10 is located bedside and generally comprises a container 15 holding the infusion fluid 14 and a sampling system 100 which is in communication with both the container 15 and the patient P. In some embodiments, the fluid handling system 10 can be in fluid communication with an extracorporeal fluid conduit containing a volume of a bodily fluid. A tube 13 extends from the container 15 to the sampling system 100. A tube 12 extends from the sampling system 100 to the patient P. In some embodiments, in lieu of the depicted tube 12, any suitable extracorporeal fluid conduit, such as a catheter, IV tube or an IV network, can be connected to the sampling system 100 with a connector such as the depicted connector 110. The extracorporeal fluid conduit need not be attached to the patient P; for example, the extracorporeal fluid conduit can be in fluid communication with a container of the bodily fluid of interest (e.g. blood), or the extracorporeal fluid conduit can serve as a stand-alone volume of the bodily fluid of interest. In some embodiments, one or more components of the fluid handling system 10 can be located at another facility, room, or other suitable remote location. One or more components of the fluid handling system 10 can communicate with one or more other components of the fluid handling system 10 (or with other devices) by any suitable communication means, such as communication interfaces including, but not limited to, optical interfaces, electrical interfaces, and wireless interfaces. These interfaces can be part of a local network, internet, wireless network, or other suitable networks.

The infusion fluid 14 can comprise water, saline, dextrose, lactated Ringer's solution, drugs, insulin, mixtures thereof, or other suitable substances. The illustrated sampling system 100 allows the infusion fluid to pass to the patient P and/or uses the infusion fluid in the analysis. In some embodiments, the fluid handling system 10 may not employ infusion fluid. The fluid handling system 10 may thus draw samples without delivering any fluid to the patient P.

The sampling system 100 can be removably or permanently coupled to the tube 13 and tube 12 via connectors 110, 120. The patient connector 110 can selectively control the flow of fluid through a bundle 130, which includes a patient connection passageway 112 and a sampling passageway 113, as shown in FIGURE 1B. The sampling system 100 can also draw one or more samples from the patient P by any suitable means. The sampling system 100 can perform one or more analyses on the sample, and then returns the sample to the patient or a waste container. In some embodiments, the sampling system 100 is a modular unit that can be removed and replaced as desired. The sampling system 100 can include, but is not limited to, fluid handling and analysis apparatuses, connectors, passageways, catheters, tubing, fluid control elements, valves, pumps, fluid sensors, pressure sensors, temperature sensors, hematocrit sensors, hemoglobin sensors, colorimetric sensors, and gas (or "bubble") sensors, fluid conditioning elements, gas injectors, gas filters, blood plasma separators, and/or communication devices (e.g., wireless devices) to permit the transfer of information within the sampling system or between sampling system 100 and a network. The illustrated sampling system 100 has a patient connector 110 and a fluid handling and analysis apparatus 140, which analyzes a sample drawn from the patient P. The fluid handling and analysis apparatus 140 and patient connector 110 cooperate to control the flow of infusion fluid into, and/or samples withdrawn from, the patient P. Samples can also be withdrawn and transferred in other suitable manners.

FIGURE 1A is a close up view of the fluid handling and analysis apparatus 140 which is partially cutaway to reveal some of its internal components. The fluid handling and analysis apparatus 140 preferably includes a pump 203 that controls the flow of fluid from the container 15 to the patient P and/or the flow of fluid drawn from the patient P. The pump 203 can selectively control fluid flow rates, direction(s) of fluid flow(s), and other fluid flow parameters as desired. As used herein, the term "pump" is a broad term and means, without limitation, a pressurization/pressure device, vacuum device, or any other suitable means for causing fluid flow. The pump 203 can include, but is not limited to, a reversible peristaltic pump, two unidirectional pumps that work in concert with valves to provide flow in two directions, a unidirectional pump, a displacement pump, a syringe, a diaphragm pump, roller pump, or other suitable pressurization device.

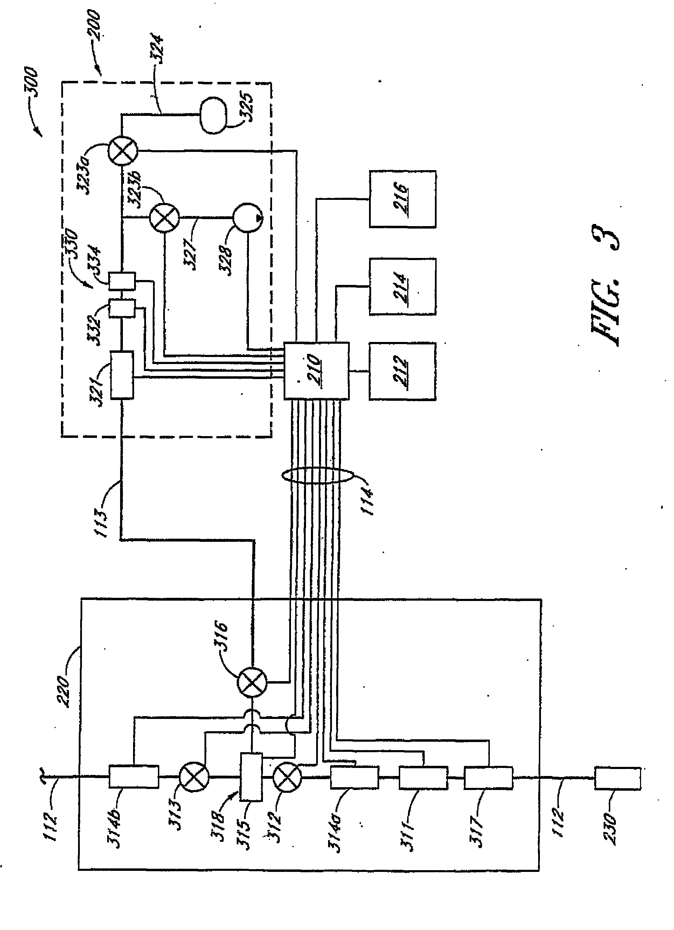

The illustrated fluid handling and analysis apparatus 140 has a display 141 and input devices 143. The illustrated fluid handling and analysis apparatus 140 can also have a sampling unit 200 configured to analyze the drawn fluid sample. The sampling unit 200 can thus receive a sample, prepare the sample, and/or subject the sample (prepared or unprepared) to one or more tests. The sampling unit 200 can then analyze results from the tests. The sampling unit 200 can include, but is not limited to, separators, filters, centrifuges, sample elements, and/or detection systems, as described in detail below. The sampling unit 200 (see FIGURE 3) can include an analyte detection system for detecting the concentration of one or more analytes in the body fluid sample. In some embodiments, the sampling unit 200 can prepare a sample for analysis. If the fluid handling and analysis apparatus 140 performs an analysis on plasma contained in whole blood taken from the patient P, filters, separators, centrifuges, or other types of sample preparation devices can be used to separate plasma from other components of the blood. After the separation process, the sampling unit 200 can analyze the plasma to determine, for example, the patient P's glucose level. The sampling unit 200 can employ spectroscopic methods, colorimetric methods, electrochemical methods, or other suitable methods for analyzing samples.

With continued reference to FIGURES 1 and 1A, the fluid 14 in the container 15 can flow through the tube 13 and into a fluid source passageway 111. The fluid can further flow through the passageway 111 to the pump 203, which can pressurize the fluid. The fluid 14 can then flow from the pump 203 through the patient connection passageway 112 and catheter 11 into the patient P. To analyze the patient's P body fluid (e.g., whole blood, blood plasma, interstitial fluid, bile, sweat, excretions, etc.), the fluid handling and analysis apparatus 140 can draw a sample from the patient P through the catheter 11 to a patient connector 110. The patient connector 110 directs the fluid sample into the sampling passageway 113 which leads to the sampling unit 200. The sampling unit 200 can perform one or more analyses on the sample. The fluid handling and analysis apparatus 140 can then output the results obtained by the sampling unit 200 on the display 141.

In some embodiments, the fluid handling system 10 can draw and analyze body fluid sample(s) from the patient P to provide real-time or near-real-time measurement of glucose levels. Body fluid samples can be drawn from the patient P continuously, at regular intervals (e.g., every 5, 10, 15, 20, 30 or 60 minutes), at irregular intervals, or at any time or sequence for desired measurements. These measurements can be displayed bedside with the display 141 for convenient monitoring of the patient P.

The illustrated fluid handling system 10 is mounted to a stand 16 and can be used in hospitals, ICUs, residences, healthcare facilities, and the like. In some embodiments, the fluid handling system 10 can be transportable or portable for an ambulatory patient. The ambulatory fluid handling system 10 can be coupled (e.g., strapped, adhered, etc.) to a patient, and may be smaller than the bedside fluid handling system 10 illustrated in FIGURE 1. In some embodiments, the fluid handling system 10 is an implantable system sized for subcutaneous implantation and can be used for continuous monitoring. In some embodiments, the fluid handling system 10 is miniaturized so that the entire fluid handling system can be implanted. In other embodiments, only a portion of the fluid handling system 10 is sized for implantation.

In some embodiments, the fluid handling system 10 is a disposable fluid handling system and/or has one or more disposable components. As used herein, the term "disposable" when applied to a system or component (or combination of components), such as a cassette or sample element, is a broad term and means, without limitation, that the component in question is used a finite number of times and then discarded. Some disposable components are used only once and then discarded. Other disposable components are used more than once and then discarded. For example, the fluid handling and analysis apparatus 140 can have a main instrument and a disposable cassette that can be installed onto the main instrument, as discussed below. The disposable cassette can be used for predetermined length of time, to prepare a predetermined amount of sample fluid for analysis, etc. In some embodiments, the cassette can be used to prepare a plurality of samples for subsequent analyses by the main instrument. The reusable main instrument can be used with any number of cassettes as desired. Additionally or alternatively, the cassette can be a portable, handheld cassette for convenient transport. In these embodiments, the cassette can be manually mounted to or removed from the main instrument. In some embodiments, the cassette may be a non disposable cassette which can be permanently coupled to the main instrument, as discussed below.

Disclosed herein are a number of embodiments of fluid handling systems, sampling systems, fluid handling and analysis apparatuses, analyte detection systems, and methods of using the same. Section I below discloses various embodiments of the fluid handling system that may be used to transport fluid from a patient for analysis. Section II below discloses several embodiments of fluid handling methods that may be used with the apparatus discussed in Section I: Section III below discloses several embodiments of a sampling system that may be used with the apparatus of Section I or the methods of Section II. Section IV below discloses various embodiments of a sample analysis system that may be used to detect the concentration of one or more analytes in a material sample. Section V below discloses methods for determining analyte concentrations from sample spectra.

SECTION I - FLUID HANDLING SYSTEM

FIGURE 1 is a schematic of the fluid handling system 10 which includes the container 15 supported by the stand 16 and having an interior that is fillable with the fluid 14, the catheter 11, and the sampling system 100. Fluid handling system 10 includes one or more passageways 20 that form conduits between the container, the sampling system, and the catheter. Generally, sampling system 100 is adapted to accept a fluid supply, such as fluid 14, and to be connected to a.patient, including, but not limited to catheter 11 which is used to catheterize a patient P. Fluid 14 includes, but is not limited to, fluids for infusing a patient such as saline, lactated Ringer's solution, or water. Sampling system 100, when so connected, is then capable of providing fluid to the patient. In addition, sampling system 100 is also capable of drawing samples, such as blood, from the patient through catheter 11 and passageways 20, and analyzing at least a portion of the drawn sample. Sampling system 100 measures characteristics of the drawn sample including, but not limited to, one or more of the blood plasma glucose, blood urea nitrogen (BUN), hematocrit, hemoglobin, or lactate levels. Optionally, sampling system 100 includes other devices or sensors to measure other patient or apparatus related information including, but not limited to, patient blood pressure, pressure changes within the sampling system, or sample draw rate.

More specifically, FIGURE 1 shows sampling system 100 as including the patient connector 110, the fluid handling and analysis apparatus 140, and the connector 120. Sampling system 100 may include combinations of passageways, fluid control and measurement devices, and analysis devices to direct, sample, and analyze fluid. Passageways 20 of sampling system 100 include the fluid source passageway 111 from connector 120 to fluid handling and analysis apparatus 140, the patient connection passageway 112 from the fluid handling and analysis apparatus to patient connector 110, and the sampling passageway 113 from the patient connector to the fluid handling and analysis apparatus. The reference of passageways 20 as including one or more passageway, for example passageways 111, 112, and 113 are provided to facilitate discussion of the system. It is understood that passageways may include one or more separate components and may include other intervening components including, but not limited to, pumps, valves, manifolds, and analytic equipment.

As used herein, the term "passageway" is a broad term and is used in its ordinary sense and includes, without limitation except as explicitly stated, as any opening through a material through which a fluid, such as a liquid or a gas, may pass so as to act as a conduit. Passageways include, but are not limited to, flexible, inflexible or partially flexible tubes, laminated structures having openings, bores through materials, or any other structure that can act as a conduit and any combination or connections thereof. The internal surfaces of passageways that provide fluid to a patient or that are used to transport blood are preferably biocompatible materials, including but not limited to silicone, polyetheretherketone (PEEK), or polyethylene (PE). One type of preferred passageway is a flexible tube having a fluid contacting surface formed from a biocompatible material. A passageway, as used herein, also includes separable portions that, when connected, form a passageway.

The inner passageway surfaces may include coatings of various sorts to enhance certain properties of the conduit, such as coatings that affect the ability of blood to clot or to reduce friction resulting from fluid flow. Coatings include, but are not limited to, molecular or ionic treatments.

As used herein, the term "connected" is a broad term and is used in its ordinary sense and includes, without limitation except as explicitly stated, with respect to two or more things (e.g., elements, devices, patients, etc.): a condition of physical contact or attachment, whether direct, indirect (via, e.g., intervening member(s)), continuous, selective, or intermittent; and/or a condition of being in fluid, electrical, or optical-signal communication, whether direct, indirect, continuous, selective (e.g., where there exist one or more intervening valves, fluid handling components, switches, loads, or the like), or intermittent. A condition of fluid communication is considered to exist whether or not there exists a continuous or contiguous liquid or fluid column extending between or among the two or more things in question. Various types of connectors can connect components of the fluid handling system described herein. As used herein, the term "connector" is a broad term and is used in its ordinary sense and includes, without limitation except as explicitly stated, as a device that connects passageways or electrical wires to provide communication (whether direct, indirect, continuous, selective, or intermittent) on either side of the connector. Connectors contemplated herein include a device for connecting any opening through which a fluid may pass. These connectors may have intervening valves, switches, fluid handling devices, and the like for affecting fluid flow. In some embodiments, a connector may also house devices for the measurement, control, and preparation of fluid, as described in several of the embodiments.

Fluid handling and analysis apparatus 140 may control the flow of fluids through passageways 20 and the analysis of samples drawn from a patient P, as described subsequently. Fluid handling and analysis apparatus 140 includes the display 141 and input devices, such as buttons 143. Display 141 provides information on the operation or results of an analysis performed by fluid handling and analysis apparatus 140. In one embodiment, display 141 indicates the function of buttons 143, which are used to input information into fluid handling and analysis apparatus 140. Information that may be input into or obtained by fluid handling and analysis apparatus 140 includes, but is not limited to, a required infusion or dosage rate, sampling rate, or patient specific information which may include, but is not limited to, a patient identification number or medical information. In an other alternative embodiment, fluid handling and analysis apparatus 140 obtains information on patient P over a communications network, for example an hospital communication network having patient specific information which may include, but is not limited to, medical conditions, medications being administered, laboratory blood reports, gender, and weight. As one example of the use of fluid handling system 10, which is not meant to limit the scope of the present invention, FIGURE 1 shows catheter 11 connected to patient P.

As discussed subsequently, fluid handling system 10 may catheterize a patient's vein or artery. Sampling system 100 is releasably connectable to container 15 and catheter 11. Thus, for example, FIGURE 1 shows container 15 as including the tube 13 to provide for the passage of fluid to, or from, the container, and catheter 11 as including the tube 12 external to the patient. Connector 120 is adapted to join tube 13 and passageway 111. Patient connector 110 is adapted to join tube 12 and to provide for a connection between passageways 112 and 113.

Patient connector 110 may also include one or more devices that control, direct, process, or otherwise affect the flow through passageways 112 and 113. In some embodiments, one or more lines 114 are provided to exchange signals between patient connector 110 and fluid handling and analysis apparatus 140. The lines 114 can be electrical lines, optical communicators, wireless communication channels, or other means for communication. As shown in FIGURE 1, sampling system 100 may also include passageways 112 and 113, and lines 114. The passageways and electrical lines between apparatus 140 and patient connector 110 are referred to, with out limitation, as the bundle 130.

In various embodiments, fluid handling and analysis apparatus 140 and/or patient connector 110, includes other elements (not shown in FIGURE 1) that include, but are not limited to: fluid control elements, including but not limited to valves and pumps; fluid sensors, including but not limited to pressure sensors, temperature sensors, hematocrit sensors, hemoglobin sensors, colorimetric sensors, and gas (or "bubble") sensors; fluid conditioning elements, including but not limited to gas injectors, gas filters, and blood plasma separators; and wireless communication devices to permit the transfer of information within the sampling system or between sampling system 100 and a wireless network.

In one embodiment, patient connector 110 includes devices to determine when blood has displaced fluid 14 at the connector end, and thus provides an indication of when a sample is available for being drawn through passageway 113 for sampling. The presence of such a device at patient connector 110 allows for the operation of fluid handling system 10 for analyzing samples without regard to the actual length of tube 12. Accordingly, bundle 130 may include elements to provide fluids, including air, or information communication between patient connector 110 and fluid handling and analysis apparatus 140 including, but not limited to, one or more other passageways and/or wires.

In one embodiment of sampling system 100, the passageways and lines of bundle 130 are sufficiently long to permit locating patient connector 110 near patient P, for example with tube 12 having a length of less than 0.1 to 0.5 meters, or preferably approximately 0.15 meters and with fluid handling and analysis apparatus 140 located at a convenient distance, for example on a nearby stand 16. Thus, for example, bundle 130 is from 0.3 to 3 meters, or more preferably from 1.5 to 2.0 meters in length. It is preferred, though not required, that patient connector 110 and connector 120 include removable connectors adapted for fitting to tubes 12 and 13, respectively. Thus, in one embodiment, container 15/tube 13 and catheter 11/tube 12 are both standard medical components, and sampling system 100 allows for the easy connection and disconnection of one or both of the container and catheter from fluid handling system 10.

In another embodiment of sampling system 100, tubes 12 and 13 and a substantial portion of passageways 111 and 112 have approximately the same internal cross-sectional area. It is preferred, though not required, that the internal cross-sectional area of passageway 113 is less than that of passageways 111 and 112 (see FIGURE 1B). As described subsequently, the difference in areas permits fluid handling system 10 to transfer a small sample volume of blood from patient connector 110 into fluid handling and analysis apparatus 140.

Thus, for example, in one embodiment passageways 111 and 112 are formed from a tube having an inner diameter from 0.3 millimeter to 1.50 millimeter, or more preferably having a diameter from 0.60 millimeter to 1.2 millimeter.

Passageway 113 is formed from a tube having an inner diameter from 0.3 millimeter to 1.5 millimeter, or more preferably having an inner diameter of from 0.6 millimeter to 1.2 millimeter.

While FIGURE 1 shows sampling system 100 connecting a patient to a fluid source, the scope of the present disclosure is not meant to be limited to this embodiment. Alternative embodiments include, but are not limited to, a greater or fewer number of connectors or passageways, or the connectors may be located at different locations within fluid handling system 10, and alternate fluid paths. Thus, for example, passageways 111 and 112 may be formed from one tube, or may be formed from two or more coupled tubes including, for example, branches to other tubes within sampling system 100, and/or there may be additional branches for infusing or obtaining samples from a patient. In addition, patient connector 110 and connector 120 and sampling system 100 alternatively include additional pumps and/or valves to control the flow of fluid as described below.

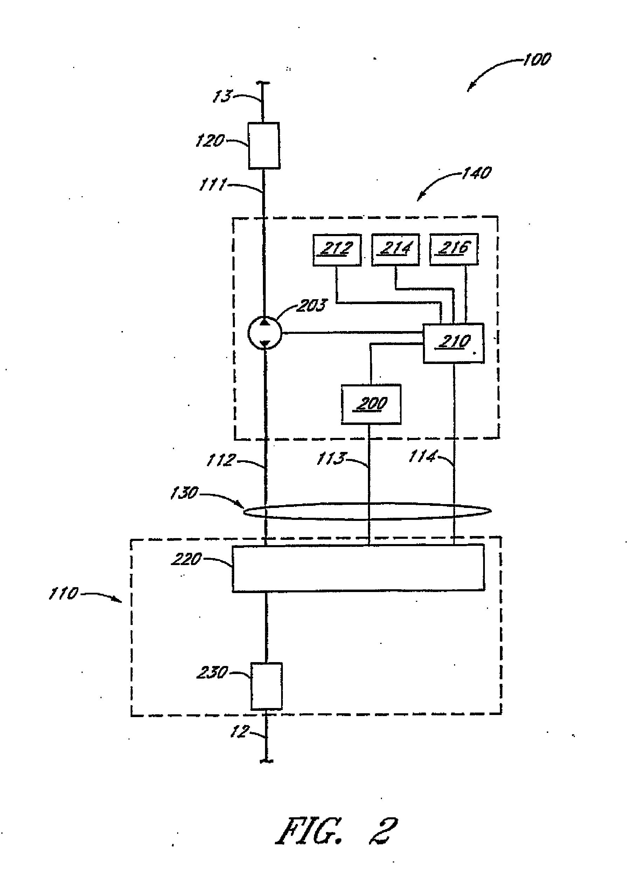

FIGURES 1A and 2 illustrate a sampling system 100 configured to analyze blood from patient P which may be generally similar to the embodiment of the sampling system illustrated in FIGURE 1, except as further detailed below. Where possible, similar elements are identified with identical reference numerals in the depiction of the embodiments of FIGURES 1 to 2. FIGURES 1A and 2 show patient connector 110 as including a sampling assembly 220 and a connector 230, portions of passageways 111 and 113, and lines 114, and fluid handling and analysis apparatus 140 as including the pump 203, the sampling unit 200, and a controller 210. The pump 203, sampling unit 200, and controller 210 are contained within a housing 209 of the fluid handling and analysis apparatus 140. The passageway 111 extends from the connector 120 through the housing 209 to the pump 203. The bundle 130 extends from the pump 203, sampling unit 200, and controller 210 to the patient connector 110.

In FIGURES 1A and 2, the passageway 111 provides fluid communication between connector 120 and pump 203 and passageway 113 provides fluid communication between pump 203 and connector 110. Controller 210 is in communication with pump 203, sampling unit 200, and sampling assembly 220 through lines 114. Controller 210 has access to memory 212, and optionally has access to a media reader 214, including but not limited to a DVD or CD-ROM reader, and communications link 216, which can comprise a wired or wireless communications network, including but not limited to a dedicated line, an intranet, or an Internet connection.

As described subsequently in several embodiments, sampling unit 200 may include one or more passageways, pumps and/or valves, and sampling assembly 220 may include passageways, sensors, valves, and/or sample detection devices. Controller 210 collects information from sensors and devices within sampling assembly 220, from sensors and analytical equipment within sampling unit 200, and provides coordinated signals to control pump 203 and pumps and valves, if present, in sampling assembly 220.

Fluid handling and analysis apparatus 140 includes the ability to pump in a forward direction (towards the patient) and in a reverse direction (away from the patient). Thus, for example, pump 203 may direct fluid 14 into patient P or draw a sample, such as a blood sample from patient P, from catheter 11 to sampling assembly 220, where it is further directed through passageway 113 to sampling unit 200 for analysis. Preferably, pump 203 provides a forward flow rate at least sufficient to keep the patient vascular line open. In one embodiment, the forward flow rate is from 1 to 5 ml/hr. In some embodiments, the flow rate of fluid is about 0.05 ml/hr, 0.1 ml/hr, 0.2 ml/hr, 0.4 ml/hr, 0.6 ml/hr, 0.8 ml/hr, 1.0 ml/hr, and ranges encompassing such flow rates. In some embodiments, for example, the flow rate of fluid is less than about 1.0 ml/hr. In certain embodiments, the flow rate of fluid may be about 0.1 ml/hr or less. When operated in a reverse direction, fluid handling and analysis apparatus 140 includes the ability to draw a sample from the patient to sampling assembly 220 and through passageway 113. In one embodiment, pump 203 provides a reverse flow to draw blood to sampling assembly 220, preferably by a sufficient distance past the sampling assembly to ensure that the sampling assembly contains an undiluted blood sample. In one embodiment, passageway 113 has an inside diameter of from 25 to 200 microns, or more preferably from 50 to 100 microns. Sampling unit 200 extracts a small sample, for example from 10 to 100 microliters of blood, or more preferably approximately 40 microliters volume of blood, from sampling assembly 220.

In one embodiment, pump 203 is a directionally controllable pump that acts on a flexible portion of passageway 111. Examples of a single, directionally controllable pump include, but are not limited to a reversible peristaltic pump or two unidirectional pumps that work in concert with valves to provide flow in two directions. In an alternative embodiment, pump 203 includes a combination of pumps, including but not limited to displacement pumps, such as a syringe, and/or valve to provide bi-directional flow control through passageway 111.

Controller 210 includes one or more processors for controlling the operation of fluid handling system 10 and for analyzing sample measurements from fluid handling and analysis apparatus 140. Controller 210 also accepts input from buttons 143 and provides information on display 141. Optionally, controller 210 is in bi-directional communication with a wired or wireless communication system, for example a hospital network for patient information. The one or more processors comprising controller 210 may include one or more processors that are located either within fluid handling and analysis apparatus 140 or that are networked to the unit.

The control of fluid handling system 10 by controller 210 may include, but is not limited to, controlling fluid flow to infuse a patient and to sample, prepare, and analyze samples. The analysis of measurements obtained by fluid handling and analysis apparatus 140 of may include, but is not limited to, analyzing samples based on inputted patient specific information, from information obtained from a database regarding patient specific information, or from information provided over a network to controller 210 used in the analysis of measurements by apparatus 140.

Fluid handling system 10 provides for the infusion and sampling of a patient blood as follows. With fluid handling system 10 connected to bag 15 having fluid 14 and to a patient P, controller 210 infuses a patient by operating pump 203 to direct the fluid into the patient. Thus, for example, in one embodiment, the controller directs that samples be obtained from a patient by operating pump 203 to draw a sample. In one embodiment, pump 203 draws a predetermined sample volume, sufficient to provide a sample to sampling assembly 220. In another embodiment, pump 203 draws a sample until a device within sampling assembly 220 indicates that the sample has reached the patient connector 110. As an example which is not meant to limit the scope of the present invention, one such indication is provided by a sensor that detects changes in the color of the sample. Another example is the use of a device that indicates changes in the material within passageway 111 including, but not limited to, a decrease in the amount of fluid 14, a change with time in the amount of fluid, a measure of the amount of hemoglobin, or an indication of a change from fluid to blood in the passageway.

When the sample reaches sampling assembly 220, controller 210 provides an operating signal to valves and/or pumps in sampling system 100 (not shown) to draw the sample from sampling assembly 220 into sampling unit 200. After a sample is drawn towards sampling unit 200, controller 210 then provides signals to pump 203 to resume infusing the patient. In one embodiment, controller 210 provides signals to pump 203 to resume infusing the patient while the sample is being drawn from sampling assembly 220. In an alternative embodiment, controller 210 provides signals to pump 203 to stop infusing the patient while the sample is being drawn from sampling assembly 220. In another alternative embodiment, controller 210 provides signals to pump 203 to slow the drawing of blood from the patient while the sample is being drawn from sampling assembly 220.

In another alternative embodiment, controller 210 monitors indications of obstructions in passageways or catheterized blood vessels during reverse pumping and moderates the pumping rate and/or direction of pump 203 accordingly. Thus, for example, obstructed flow from an obstructed or kinked passageway or of a collapsing or collapsed catheterized blood vessel that is being pumped will result in a lower pressure than an unobstructed flow. In one embodiment, obstructions are monitored using a pressure sensor in sampling assembly 220 or along passageways 20. If the pressure begins to decrease during pumping, or reaches a value that is lower than a predetermined value then controller 210 directs pump 203 to decrease the reverse pumping rate, stop pumping, or pump in the forward direction in an effort to reestablish unobstructed pumping.

FIGURE 3 is a schematic showing details of a sampling system 300 which may be generally similar to the embodiments of sampling system 100 as illustrated in FIGURES 1 and 2, except as further detailed below. Sampling system 300 includes sampling assembly 220 having, along passageway 112: connector 230 for connecting to tube 12, a pressure sensor 317, a colorimetric sensor 311, a first bubble sensor 314a, a first valve 312, a second valve 313, and a second bubble sensor 314b. Passageway 113 forms a "T" with passageway 111 at a junction 318 that is positioned between the first valve 312 and second valve 313, and includes a gas injector manifold 315 and a third valve 316. The lines 114 comprise control and/or signal lines extending from colorimetric sensor 311, first, second, and third valves (312, 313, 316), first and second bubble sensors (314a, 314b), gas injector manifold 315, and pressure sensor 317. Sampling system 300 also includes sampling unit 200 which has a bubble sensor 321, a sample analysis device 330, a first valve 323a, a waste receptacle 325, a second valve 323b, and a pump 328. Passageway 113 forms a "T" to form a waste line 324 and a pump line 327.

It is preferred, though not necessary, that the sensors of sampling system 100 are adapted to accept a passageway through which a sample may flow and that sense through the walls of the passageway. As described subsequently, this arrangement allows for the sensors to be reusable and for the passageways to be disposable. It is also preferred, though not necessary, that the passageway is smooth and without abrupt dimensional changes which may damage blood or prevent smooth flow of blood. In addition, is also preferred that the passageways that deliver blood from the patient to the analyzer not contain gaps or size changes that permit fluid to stagnate and not be transported through the passageway.

In one embodiment, the respective passageways on which valves 312, 313, 316, and 323 are situated along passageways that are flexible tubes, and valves 312, 313, 316, and 323 are "pinch valves," in which one or more movable surfaces compress the tube to restrict or stop flow therethrough. In one embodiment, the pinch valves include one or more moving surfaces that are actuated to move together and "pinch" a flexible passageway to stop flow therethrough. Examples of a pinch valve include, for example, Model PV256 Low Power Pinch Valve (Instech Laboratories, Inc., Plymouth Meeting, PA). Alternatively, one or more of valves 312, 313, 316, and 323 may be other valves for controlling the flow through their respective passageways.

Colorimetric sensor 311 accepts or forms a portion of passageway 111 and provides an indication of the presence or absence of blood within the passageway. In one embodiment, colorimetric sensor 311 permits controller 210 to differentiate between fluid 14 and blood. Preferably, colorimetric sensor 311 is adapted to receive a tube or other passageway for detecting blood. This permits, for example, a disposable tube to be placed into or through a reusable colorimetric sensor. In an alternative embodiment, colorimetric sensor 311 is located adjacent to bubble sensor 314b. Examples of a colorimetric sensor include, for example, an Optical Blood Leak/Blood vs. Saline Detector available from Introtek International (Edgewood, NJ).

As described subsequently, sampling system 300 injects a gas - referred to herein and without limitation as a "bubble" - into passageway 113. Sampling system 300 includes gas injector manifold 315 at or near junction 318 to inject one or more bubbles, each separated by liquid, into passageway 113. The use of bubbles is useful in preventing longitudinal mixing of liquids as they flow through passageways both in the delivery of a sample for analysis with dilution and for cleaning passageways between samples. Thus, for example the fluid in passageway 113 includes, in one embodiment of the invention, two volumes of liquids, such as sample S or fluid 14 separated by a bubble, for multiple volumes of liquid each separated by a bubble therebetween.

Bubble sensors 314a, 314b and 321 each accept or form a portion of passageway 112 or 113 and provide an indication of the presence of air, or the change between the flow of a fluid and the flow of air, through the passageway. Examples of bubble sensors include, but are not limited to ultrasonic or optical sensors, that can detect the difference between small bubbles or foam from liquid in the passageway. Once such bubble detector is an MEC Series Air Bubble/ Liquid Detection Sensor (Introtek International, Edgewood, NY). Preferably, bubble sensor 314a, 314b, and 321 are each adapted to receive a tube or other passageway for detecting bubbles. This permits, for example, a disposable tube to be placed through a reusable bubble sensor.

Pressure sensor 317 accepts or forms a portion of passageway 111 and provides an indication or measurement of a fluid within the passageway. When all valves between pressure sensor 317 and catheter 11 are open, pressure sensor 317 provides an indication or measurement of the pressure within the patient's catheterized blood vessel. In one embodiment, the output of pressure sensor 317 is provided to controller 210 to regulate the operation of pump 203. Thus, for example, a pressure measured by pressure sensor 317 above a predetermined value is taken as indicative of a properly working system, and a pressure below the predetermined value is taken as indicative of excessive pumping due to, for example, a blocked passageway or blood vessel. Thus, for example, with pump 203 operating to draw blood from patient P, if the pressure as measured by pressure sensor 317 is within a range of normal blood pressures, it may be assumed that blood is being drawn from the patient and pumping continues. However, if the pressure as measured by pressure sensor 317 falls below some level, then controller 210 instructs pump 203 to slow or to be operated in a forward direction to reopen the blood vessel. One such pressure sensor is a Deltran IV part number DPT-412 (Utah Medical Products, Midvale, UT).

Sample analysis device 330 receives a sample and performs an analysis. In several embodiments, device 330 is configured to prepare of the sample for analysis. Thus, for example, device 330 may include a sample preparation unit 332 and an analyte detection system 334, where the sample preparation unit is located between the patient and the analyte detection system. In general, sample preparation occurs between sampling and analysis. Thus, for example, sample preparation unit 332 may take place removed from analyte detection, for example within sampling assembly 220, or may take place adjacent or within analyte detection system 334.

As used herein, the term "analyte" is a broad term and is used in its ordinary sense and includes, without limitation, any chemical species the presence or concentration of which is sought in the material sample by an analyte detection system. For example, the analyte(s) include, but not are limited to, glucose, ethanol, insulin, water, carbon dioxide, blood oxygen, cholesterol, bilirubin, ketones, fatty acids, lipoproteins, albumin, urea, creatinine, white blood cells, red blood cells, hemoglobin, oxygenated hemoglobin, carboxyhemoglobin, organic molecules, inorganic molecules, pharmaceuticals, cytochrome, various proteins and chromophores, microcalcifications, electrolytes, sodium, potassium, chloride, bicarbonate, and hormones. As used herein, the term "material sample" (or, alternatively, "sample") is a broad term and is used in its ordinary sense and includes, without limitation, any collection of material which is suitable for analysis. For example, a material sample may comprise whole blood, blood components (e.g., plasma or serum), interstitial fluid, intercellular fluid, saliva, urine, sweat and/or other organic or inorganic materials, or derivatives of any of these materials. In one embodiment, whole blood or blood components may be drawn from a patient's capillaries.

In one embodiment, sample preparation unit 332 separates blood plasma from a whole blood sample or removes contaminants from a blood sample and thus comprises one or more devices including, but not limited to, a filter, membrane, centrifuge, or some combination thereof. In alternative embodiments, analyte detection system 334 is adapted to analyze the sample directly and sample preparation unit 332 is not required.

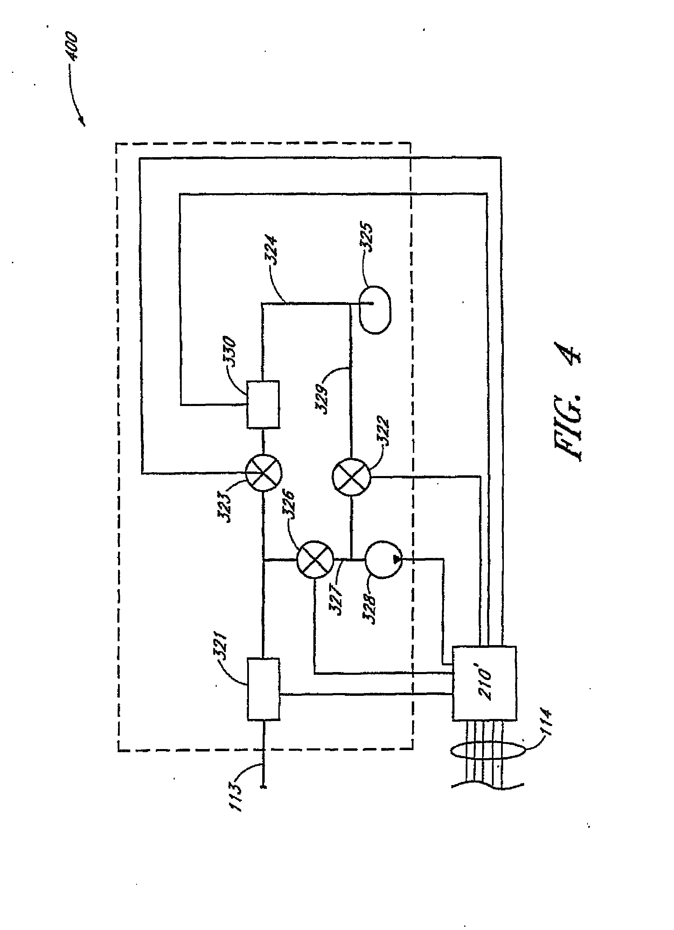

Generally, sampling assembly 220 and sampling unit 200 direct the fluid drawn from sampling assembly 220 into passageway 113 into sample analysis device 330. FIGURE 4 is a schematic of an embodiment of a sampling unit 400 that permits some of the sample to bypass sample analysis device 330. Sampling unit 400 may be generally similar to sampling unit 200, except as further detailed below. Sampling unit 400 includes bubble sensor 321, valve 323, sample analysis device 330, waste line 324, waste receptacle 325, valve 326, pump line 327, pump 328, a valve 322, and a waste line 329. Waste line 329 includes valve 322 and forms a "T" at pump line 337 and waste line 329. Valves 316, 322, 323, and 326 permit a flow through passageway 113 to be routed through sample analysis device 330, to be routed to waste receptacle 325, or to be routed through waste line 324 to waste receptacle 325.

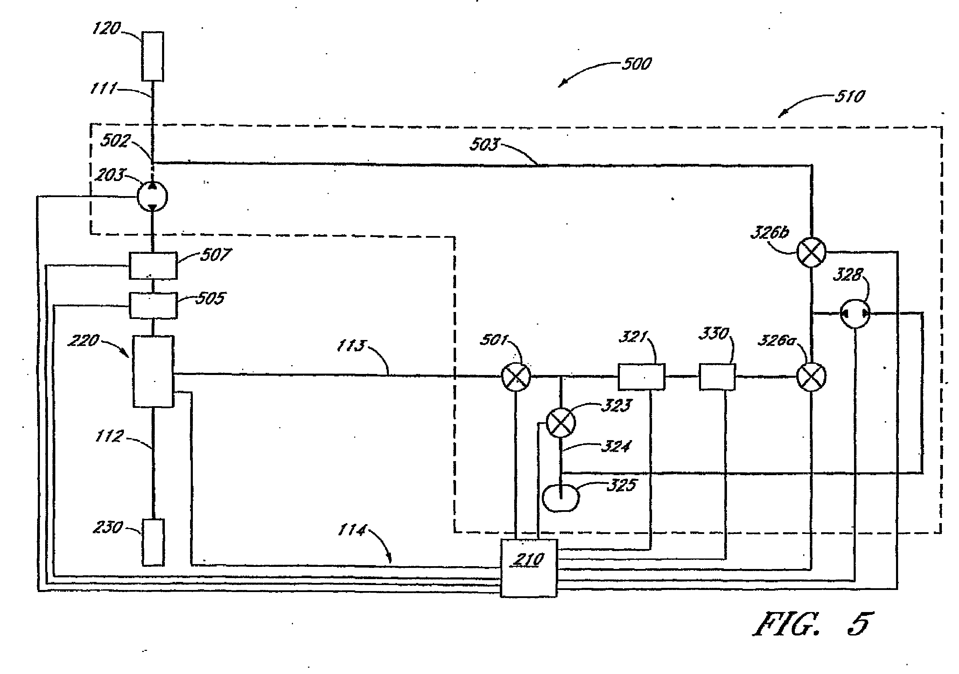

FIGURE 5 is a schematic of one embodiment of a sampling system 500 which may be generally similar to the embodiments of sampling system 100 or 300 as illustrated in FIGURES 1 through 4, except as further detailed below. Sampling system 500 includes an embodiment of a sampling unit 510 and differs from sampling system 300 in part, in that liquid drawn from passageway 111 may be returned to passageway 111 at a junction 502 between pump 203 and connector 120.

With reference to FIGURE 5, sampling unit 510 includes a return line 503 that intersects passageway 111 on the opposite side of pump 203 from passageway 113, a bubble sensor 505 and a pressure sensor 507, both of which are controlled by controller 210. Bubble sensor 505 is generally similar to bubble sensors 314a, 314b and 321 and pressure sensor 507 is generally similar to pressure sensor 317. Pressure sensor 507 is useful in determining the correct operation of sampling system 500 by monitoring pressure in passageway 111. Thus, for example, the pressure in passageway 111 is related to the pressure at catheter 11 when pressure sensor 507 is in fluid communication with catheter 11 (that is, when any intervening valve(s) are open). The output of pressure sensor 507 is used in a manner similar to that of pressure sensor 317 described previously in controlling pumps of sampling system 500.

Sampling unit 510 includes valves 501, 326a, and 326b under the control of controller 210. Valve 501 provides additional liquid flow control between sampling unit 200 and sampling unit 510. Pump 328 is preferably a bi-directional pump that can draw fluid from and into passageway 113. Fluid may either be drawn from and returned to passageway 501, or may be routed to waste receptacle 325. Valves 326a and 326b are situated on either side of pump 328. Fluid can be drawn through passageway 113 and into return line 503 by the coordinated control of pump 328 and valves 326a and 326b. Directing flow from return line 503 can be used to prime sampling system 500 with fluid. Thus, for example, liquid may be pulled into sampling unit 510 by operating pump 328 to pull liquid from passageway 113 while valve 326a is open and valve 326b is closed. Liquid may then be pumped back into passageway 113 by operating pump 328 to push liquid into passageway 113 while valve 326a is closed and valve 326b is open.

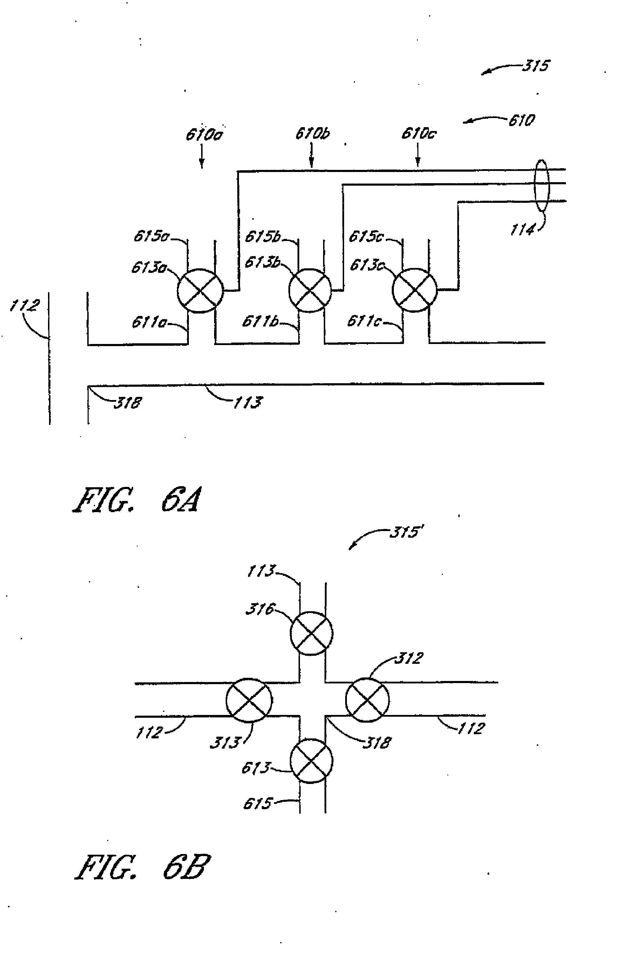

FIGURE 6A is a schematic of an embodiment of gas injector manifold 315 which may be generally similar or included within the embodiments illustrated in FIGURES 1 through 5, except as further detailed below. Gas injector manifold 315 is a device that injects one or more bubbles in a liquid within passageway 113 by opening valves to the atmosphere and lowering the liquid pressure within the manifold to draw in air. As described subsequently, gas injector manifold 315 facilitates the injection of air or other gas bubbles into a liquid within passageway 113. Gas injector manifold 315 has three gas injectors 610 including a first injector 610a, a second injector 610b, and a third injector 610c. Each injector 610 includes a corresponding passageway 611 that begins at one of several laterally spaced locations along passageway 113 and extends through a corresponding valve 613 and terminates at a corresponding end 615 that is open to the atmosphere. In an alternative embodiment, a filter is placed in end 615 to filter out dust or particles in the atmosphere. As described subsequently, each injector 610 is capable of injecting a bubble into a liquid within passageway 113 by opening the corresponding valve 613, closing a valve on one end of passageway 113 and operating a pump on the opposite side of the passageway to lower the pressure and pull atmospheric air into the fluid. In one embodiment of gas injector manifold 315, passageways 113 and 611 are formed within a single piece of material (e.g., as bores formed in or through a plastic or metal housing (not shown)). In an alternative embodiment, gas injector manifold 315 includes fewer than three injectors, for example one or two injectors, or includes more than three injectors. In another alternative embodiment, gas injector manifold 315 includes a controllable high pressure source of gas for injection into a liquid in passageway 113. It is preferred that valves 613 are located close to passageway 113 to minimize trapping of fluid in passageways 611.

Importantly, gas injected into passageways 20 should be prevented from reaching catheter 11. As a safety precaution, one embodiment prevents gas from flowing towards catheter 11 by the use of bubble sensor 314a as shown, for example, in FIGURE 3. If bubble sensor 314a detects gas within passageway 111, then one of several alternative embodiments prevents unwanted gas flow. In one embodiment, flow in the vicinity of sampling assembly 220 is directed into line 113 or through line 113 into waste receptacle 325. With further reference to FIGURE 3, upon the detection of gas by bubble sensor 314a, valves 316 and 323a are opened, valve 313 and the valves 613a, 613b and 613c of gas injector manifold 315 are closed, and pump 328 is turned on to direct flow away from the portion of passageway 111 between sampling assembly 220 and patient P into passageway 113. Bubble sensor 321 is monitored to provide an indication of when passageway 113 clears out. Valve 313 is then opened, valve 312 is closed, and the remaining portion of passageway 111 is then cleared. Alternatively, all flow is immediately halted in the direction of catheter 11, for example by closing all valves and stopping all pumps. In an alternative embodiment of sampling assembly 220, a gas-permeable membrane is located within passageway 113 or within gas injector manifold 315 to remove unwanted gas from fluid handling system 10, e.g., by venting such gas through the membrane to the atmosphere or a waste receptacle.

FIGURE 6B is a schematic of an embodiment of gas injector manifold 315' which may be generally similar to, or included within, the embodiments illustrated in FIGURES 1 through 6A, except as further detailed below. In gas injector manifold 315', air line 615 and passageway 113 intersect at junction 318. Bubbles are injected by opening valve 316 and 613 while drawing fluid into passageway 113. Gas injector manifold 315' is thus more compact that gas injector manifold 315, resulting in a more controllable and reliable gas generator.

SECTION II - FLUID HANDLING METHODS

One embodiment of a method of using fluid handling system 10, including sampling assembly 220 and sampling unit 200 of FIGURES 2, 3 and 6A, is illustrated in Table 1 and in the schematic fluidic diagrams of FIGURES 7A-7J. In general, the pumps and valves are controlled to infuse a patient, to extract a sample from the patient up passageway 111 to passageway 113, and to direct the sample along passageway 113 to device 330. In addition, the pumps and valves are controlled to inject bubbles into the fluid to isolate the fluid from the diluting effect of previous fluid and to clean the lines between sampling. The valves in FIGURES 7A-7J are labeled with suffices to indicate whether the valve is open or closed. Thus a valve "x," for example, is shown as valve "x-o" if the valve is open and "x-c" if the valve is closed.

| Mode | Step | Pump 203 | Pump 328 | Valve 312 | Valve 313 | Valve 613a | Valve 613b | Valve 613c | Valve 316 | Valve 323a | Valve 323b |
|---|---|---|---|---|---|---|---|---|---|---|---|
| Infuse patient | (FIGURE 7A) Infuse patient | F | Off | O | O | C | C | C | C | C | C |
| Sample patient | (FIGURE 7B) Clear fluid from passageways | R | Off | C | O | one or more are open: O | O | O | C | C | C |
|  | (FIGURE 7C) Draw sample until after colorimetric sensor 311 senses blood | R | Off | O | O | C | C | C | C | C | C |
|  | (FIGURE 7D) Inject sample into bubble manifold | Off | On | O | C | C | C | C | O | C | O |
|  | Alternative to FIGURE 7D | R | On | O | O | C | C | C | O | C | O |
|  | (FIGURE 7E) Inject bubbles | Off | On | C | C | sequentially: O | O | O | O | C | O |
|  | (FIGURE 7F) Clear bubbles from patient line | F | Off | C | O | C | C | C | O | O | C |
|  | (FIGURE 7G) Clear blood from patient line | F | Off | O | O | C | C | C | C | C | C |
|  | (FIGURE 7H) Move bubbles out of bubbler | F | Off | C | O | C | C | C | O | O | C |
|  | (FIGURE 7I) Add cleaning bubbles | Off | On | C | C | sequentially: O | O | O | O | C | O |
|  | (FIGURE 7J) Push sample to analyzer until bubble sensor 321 detects bubble | F | Off | C | O | C | C | C | O | O | C |

F = Forward (fluid into patient), R = Reverse (fluid from patient), O = Open, C = Closed

Table 1. Methods of operating system 10 as illustrated in FIGURES 7A-7J

FIGURE 7A illustrates one embodiment of a method of infusing a patient. In the step of FIGURE 7A, pump 203 is operated forward (pumping towards the patient) pump 328 is off, or stopped, valves 313 and 312 are open, and valves 613a, 613b, 613c, 316, 323a, and 323b are closed. With these operating conditions, fluid 14 is provided to patient P. In a preferred embodiment, all of the other passageways at the time of the step of FIGURE 7A substantially contain fluid 14.

The next nine figures (FIGURES 7B-7J) illustrate steps in a method of sampling from a patient. The following steps are not meant to be inclusive of all of the steps of sampling from a patient, and it is understood that alternative embodiments may include more steps, fewer steps, or a different ordering of steps. FIGURE 7B illustrates a first sampling step, where liquid is cleared from a portion of patient connection passageway and sampling passageways 112 and 113. In the step of FIGURE 7B, pump 203 is operated in reverse (pumping away from the patient), pump 328 is off, valve 313 is open, one or more of valves 613a, 613b, and 613c are open, and valves 312, 316, 323a, and 326b are closed. With these operating conditions, air 701 is drawn into sampling passageway 113 and back into patient connection passageway 112 until bubble sensor 314b detects the presence of the air.

FIGURE 7C illustrates a second sampling step, where a sample is drawn from patient P into patient connection passageway 112. In the step of FIGURE 7C, pump 203 is operated in reverse, pump 328 is off, valves 312 and 313 are open, and valves 316, 613a, 613b, 613c, 323a, and 323b are closed. Under these operating conditions, a sample S is drawn into passageway 112, dividing air 701 into air 701a within sampling passageway 113 and air 701b within the patient connection passageway 112. Preferably this step proceeds until sample S extends just past the junction of passageways 112 and 113. In one embodiment, the step of FIGURE 7C proceeds until variations in the output of colorimetric sensor 311 indicate the presence of a blood (for example by leveling off to a constant value), and then proceeds for an additional set amount of time to ensure the presence of a sufficient volume of sample S.

FIGURE 7D illustrates a third sampling step, where a sample is drawn into sampling passageway 113. In the step of FIGURE 7D, pump 203 is off, or stopped, pump 328 is on, valves 312, 316, and 326b are open, and valves 313, 613a, 613b, 613c and 323a are closed. Under these operating conditions, blood is drawn into passageway 113. Preferably, pump 328 is operated to pull a sufficient amount of sample S into passageway 113. In one embodiment, pump 328 draws a sample S having a volume from 30 to 50 microliters. In an alternative embodiment, the sample is drawn into both passageways 112 and 113. Pump 203 is operated in reverse, pump 328 is on, valves 312, 313, 316, and 323b are open, and valves 613a, 613b, 613c and 323a are closed to ensure fresh blood in sample S.

FIGURE 7E illustrates a fourth sampling step, where air is injected into the sample. Bubbles which span the cross-sectional area of sampling passageway 113 are useful in preventing contamination of the sample as it is pumped along passageway 113. In the step of FIGURE 7E, pump 203 is off, or stopped, pump 328 is on, valves 316, and 323b are open , valves 312, 313 and 323a are closed, and valves 613a, 613b, 613c are each opened and closed sequentially to draw in three separated bubbles. With these operating conditions, the pressure in passageway 113 falls below atmospheric pressure and air is drawn into passageway 113. Alternatively, valves 613a, 613b, 613c may be opened simultaneously for a short period of time, generating three spaced bubbles. As shown in FIGURE 7E, injectors 610a, 610b, and 610c inject bubbles 704, 703, and 702, respectively, dividing sample S into a forward sample S1, a middle sample S2, and a rear sample S3.

FIGURE 7F illustrates a fifth sampling step, where bubbles are cleared from patient connection passageway 112. In the step of FIGURE 7F, pump 203 is operated in a forward direction, pump 328 is off, valves 313, 316, and 323a are open, and valves 312, 613a, 613b, 613c, and 323b are closed. With these operating conditions, the previously injected air 701 b is drawn out of first passageway 111 and into second passageway 113. This step proceeds until air 701b is in passageway 113.

FIGURE 7G illustrates a sixth sampling step, where blood in passageway 112 is returned to the patient. In the step of FIGURE 7G, pump 203 is operated in a forward direction, pump 328 is off, valves 312 and 313 are open, and valves 316, 323a, 613a, 613b, 613c and 323b are closed. With these operating conditions, the previously injected air remains in passageway 113 and passageway 111 is filled with fluid 14.

FIGURES 7H and 7I illustrates a seventh and eighth sampling steps, where the sample is pushed part way into passageway 113 followed by fluid 14 and more bubbles. In the step of FIGURE 7H, pump 203 is operated in a forward direction, pump 328 is off, valves 313, 316, and 323a are open, and valves 312, 613a, 613b, 613c, and 323b are closed. With these operating conditions, sample S is moved partway into passageway 113 with bubbles injected, either sequentially or simultaneously, into fluid 14 from injectors 610a, 610b, and 610c. In the step of FIGURE 7I, the pumps and valves are operated as in the step of FIGURE 7E, and fluid 14 is divided into a forward solution C1, a middle solution C2, and a rear solution C3 separated by bubbles 705, 706, and 707.

The last step shown in FIGURE 7 is FIGURE 7J, where middle sample S2 is pushed to sample analysis device 330. In the step of FIGURE 7J, pump 203 is operated in a forward direction, pump 328 is off, valves 313, 316, and 323a are open, and valves 312, 613a, 613b, 613c, and 323b are closed. In this configuration, the sample is pushed into passageway 113. When bubble sensor 321 detects bubble 702, pump 203 continues pumping until sample S2 is taken into device sample analysis 330. Additional pumping using the settings of the step of FIGURE 7J permits the sample S2 to be analyzed and for additional bubbles and solutions to be pushed into waste receptacle 325, cleansing passageway 113 prior to accepting a next sample.

SECTION III - SAMPLING SYSTEM

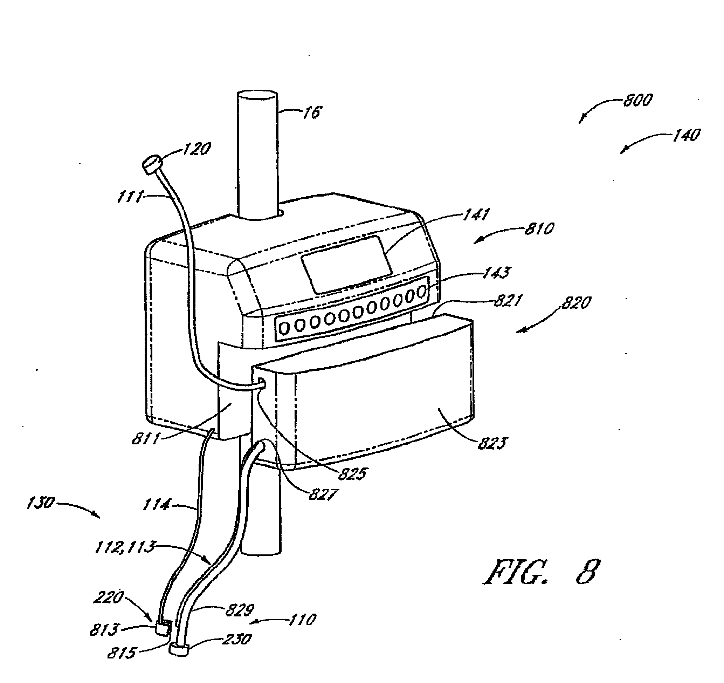

FIGURE 8 is a perspective front view of a third embodiment of a sampling system 800 which may be generally similar to sampling system 100, 300 or 500 and the embodiments illustrated in FIGURES 1 through 7, except as further detailed below. The fluid handling and analysis apparatus 140 of sampling system 800 includes the combination of an instrument 810 and a sampling system cassette 820. FIGURE 8 illustrates instrument 810 and cassette 820 partially removed from each other. Instrument 810 includes controller 210 (not shown), display 141 and input devices 143, a cassette interface 811, and lines 114. Cassette 820 includes passageway 111 which extends from connector 120 to connector 230, and further includes passageway 113, a junction 829 of passageways 111 and 113, an instrument interface 821, a front surface 823, an inlet 825 for passageway 111, and an inlet 827 for passageways 111 and 113. In addition, sampling assembly 220 is formed from a sampling assembly instrument portion 813 having an opening 815 for accepting junction 829. The interfaces 811 and 821 engage the components of instrument 810 and cassette 820 to facilitate pumping fluid and analyzing samples from a patient, and sampling assembly instrument portion 813 accepts junction 829 in opening 815 to provide for sampling from passageway 111.

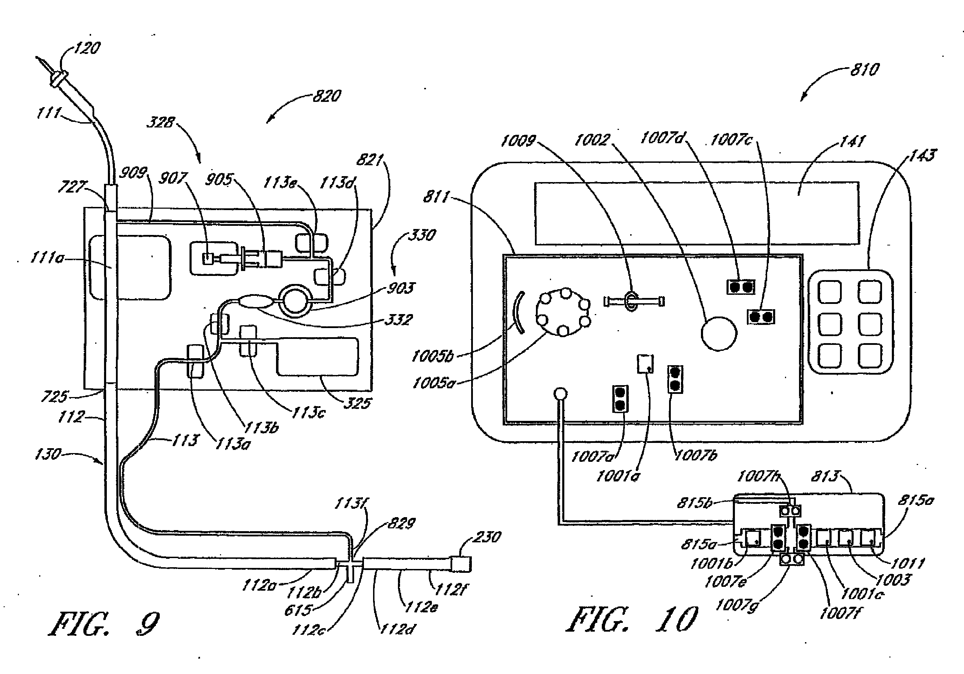

FIGURES 9 and 10 are front views of a sampling system cassette 820 and instrument 810, respectively, of a sampling system 800. Cassette 820 and instrument 810, when assembled, form various components of FIGURES 9 and 10 that cooperate to form an apparatus consisting of sampling unit 510 of FIGURE 5, sampling assembly 220 of FIGURE 3, and gas injection manifold 315' of FIGURE 6B.

More specifically, as shown in FIGURE 9, cassette 820 includes passageways 20 including: passageway 111 having portions 111 a, 112a, 112b, 112c, 112d, 112e, and 112f; passageway 113 having portions 113a, 113b, 113c, 113d, 113e, and 113f; passageway 615; waste receptacle 325; disposable components of sample analysis device 330 including, for example, a sample preparation unit 332 adapted to allow only blood plasma to pass therethrough and a sample chamber 903 for placement within analyte detection system 334 for measuring properties of the blood plasma; and a displacement pump 905 having a piston control 907.

As shown in FIGURE 10, instrument 810 includes bubble sensor units 1001 a, 1001b, and 1001c, colorimetric sensor, which is a hemoglobin sensor unit 1003, a peristaltic pump roller 1005a and a roller support 1005b, pincher pairs 1007a, 1007b, 1007c, 1007d, 1007e, 1007f, 1007g, and 1007h, an actuator 1009, and a pressure sensor unit 1011. In addition, instrument 810 includes portions of sample analysis device 330 which are adapted to measure a sample contained within sample chamber 903 when located near or within a probe region 1002 of an optical analyte detection system 334.

Passageway portions of cassette 820 contact various components of instrument 810 to form sampling system 800. With reference to FIGURE 5 for example, pump 203 is formed from portion 111 a placed between peristaltic pump roller 1005a and roller support 1005b to move fluid through passageway 111 when the roller is actuated; valves 501, 323, 326a, and 326b are formed with pincher pairs 1007a, 1007b, 1007c, and 1007d surrounding portions 113a, 113c, 113d, and 1113e, respectively, to permit or block fluid flow therethrough. Pump 328 is formed from actuator 1009 positioned to move piston control 907. It is preferred that the interconnections between the components of cassette 820 and instrument 810 described in this paragraph are made with one motion. Thus for example the placement of interfaces 811 and 821 places the passageways against and/or between the sensors, actuators, and other components.

In addition to placement of interface 811 against interface 821, the assembly of apparatus 800 includes assembling sampling assembly 220. More specifically, an opening 815a and 815b are adapted to receive passageways 111 and 113, respectively, with junction 829 within sampling assembly instrument portion 813. Thus, for example, with reference to FIGURE 3, valves 313 and 312 are formed when portions 112b and 112c are placed within pinchers of pinch valves 1007e and 1007f, respectively, bubble sensors 314b and 314a are formed when bubble sensor units 1001b, and 1001c are in sufficient contact with portions 112a and 112d, respectively, to determine the presence of bubbles therein; hemoglobin detector is formed when hemoglobin sensor 1003 is in sufficient contact with portion 112e, and pressure sensor 317 is formed when portion 112f is in sufficient contact with pressure sensor unit 1011 to measure the pressure of a fluid therein. With reference to FIGURE 6B, valves 316 and 613 are formed when portions 113f and 615 are placed within pinchers of pinch valves 1007h and 1007g, respectively.

In operation, the assembled main instrument 810 and cassette 820 of FIGURES 9-10 can function as follows. The system can be considered to begin in an idle state or infusion mode in which the roller pump 1005 operates in a forward direction (with the impeller 1005a turning counterclockwise as shown in FIGURE 10) to pump infusion fluid from the container 15 through the passageway 111 and the passageway 112, toward and into the patient P. In this infusion mode the pump 1005 delivers infusion fluid to the patient at a suitable infusion rate as discussed elsewhere herein.

When it is time to conduct a measurement, air is first drawn into the system to clear liquid from a portion of the passageways 112, 113, in a manner similar to that shown in FIGURE 7B. Here, the single air injector of FIGURE 9 (extending from the junction 829 to end 615, opposite the passageway 813) functions in place of the manifold shown in FIGURES 7A-7J. Next, to draw a sample, the pump 1005 operates in a sample draw mode, by operating in a reverse direction and pulling a sample of bodily fluid (e.g. blood) from the patient into the passageway 112 through the connector 230. The sample is drawn up to the hemoglobin sensor 1003, and is preferably drawn until the output of the sensor 1003 reaches a desired plateau level indicating the presence of an undiluted blood sample in the passageway 112 adjacent the sensor 1003.

From this point the pumps 905, 1005, valves 1007e, 1007f, 1007g, 1007h, bubble sensors 1001b, 1001c and/or hemoglobin sensor 1003 can be operated to move a series of air bubbles and sample-fluid columns into the passageway 113, in a manner similar to that shown in FIGURES 7D-7F. The pump 905, in place of the pump 328, is operable by moving the piston control 907 of the pump 905 in the appropriate direction (to the left or right as shown in FIGURES 9-10) with the actuator 1009.

Once a portion of the bodily fluid sample and any desired bubbles have moved into the passageway 113, the valve 1007h can be closed, and the remainder of the initial drawn sample or volume of bodily fluid in the passageway 112 can be returned to the patient, by operating the pump 1005 in the forward or infusion direction until the passageway 112 is again filled with infusion fluid.

With appropriate operation of the valves 1007a-1007h, and the pump(s) 905 and/or 1005, at least a portion of the bodily fluid sample in the passageway 113 (which is 10-100 microliters in volume, or 20, 30, 40, 50 or 60 microliters, in various embodiments) is moved through the sample preparation unit 332 (in the depicted embodiment a filter or membrane; alternatively a centrifuge as discussed in greater detail below). Thus, only one or more components of the bodily fluid (e.g., only the plasma of a blood sample) passes through the unit 332 or filter/membrane and enters the sample chamber or cell 903. Alternatively, where the unit 332 is omitted, the "whole" fluid moves into the sample chamber 903 for analysis.

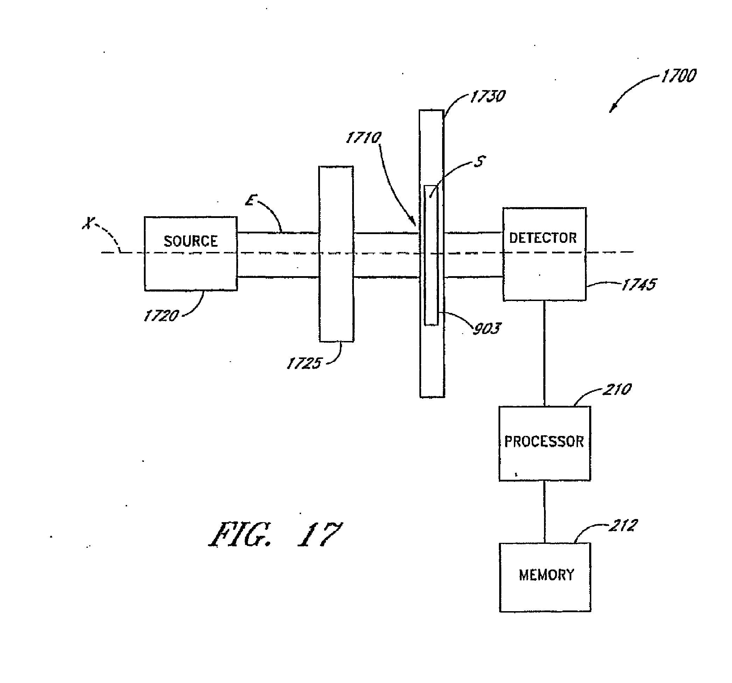

Once the component(s) or whole fluid is in the sample chamber 903, the analysis is conducted to determine a level or concentration of one or more analytes, such as glucose, lactate, carbon dioxide, blood urea nitrogen, hemoglobin, and/or any other suitable analytes as discussed elsewhere herein. Where the analyte detection system 1700 is spectroscopic (e.g. the system 1700 of FIGURES 17 or 44-46), a spectroscopic analysis of the component(s) or whole fluid is conducted.

After the analysis, the body fluid sample within the passageway 113 is moved into the waste receptacle 325. Preferably, the pump 905 is operated via the actuator 1009 to push the body fluid, behind a column of saline or infusion fluid obtained via the passageway 909, back through the sample chamber 903 and sample preparation unit 332, and into the receptacle 325. Thus, the chamber 903 and unit 332 are back-flushed and filled with saline or infusion fluid while the bodily fluid is delivered to the waste receptacle. Following this flush a second analysis can be made on the saline or infusion fluid now in the chamber 903, to provide a "zero" or background reading. At this point, the fluid handling network of FIGURE 9, other than the waste receptacle 325, is empty of bodily fluid, and the system is ready to draw another bodily fluid sample for analysis.

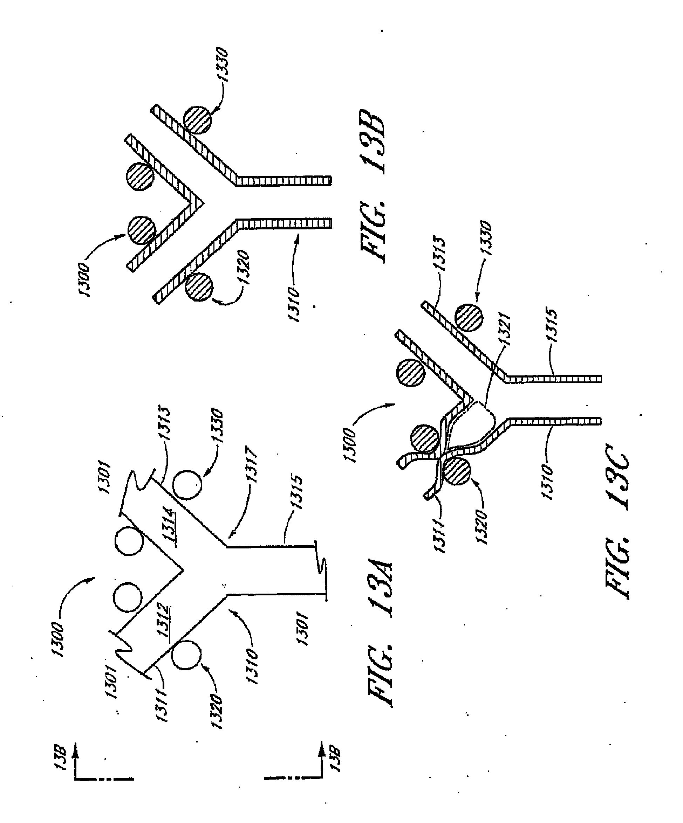

In some embodiments of the apparatus 140, a pair of pinch valve pinchers acts to switch flow between one of two branches of a passageway. FIGURES 13A and 13B are front view and sectional view, respectively, of a first embodiment pinch valve 1300 in an open configuration that can direct flow either one or both of two branches, or legs, of a passageway. Pinch valve 1300 includes two separately controllable pinch valves acting on a "Y" shaped passageway 1310 to allow switch of fluid between various legs. In particular, the internal surface of passageway 1310 forms a first leg 1311 having a flexible pinch region 1312, a second leg 1313 having a flexible pinch region 1314, and a third leg 1315 that joins the first and second legs at an intersection 1317. A first pair of pinch valve pinchers 1320 is positioned about pinch region 1312 and a second pair of pinch valve pinchers 1330 is positioned about pinch region 1314. Each pair of pinch valve pinchers 1320 and 1330 is positioned on opposite sides of their corresponding pinch regions 1312, 1314 and perpendicular to passageway 1310, and are individually controllable by controller 210 to open and close, that is allow or prohibit fluid communication across the pinch regions. Thus, for example, when pinch valve pinchers 1320 (or 1330) are brought sufficiently close, each part of pinch region 1312 (or 1314) touches another part of the pinch region and fluid may not flow across the pinch region.

As an example of the use of pinch valve 1300, FIGURE 13B shows the first and second pair of pinch valve pinchers 1320, 1330 in an open configuration. FIGURE 13C is a sectional view showing the pair of pinch valve pinchers 1320 brought together, thus closing off a portion of first leg 1311 from the second and third legs 1313, 1315. In part as a result of the distance between pinchers 1320 and intersection 1317 there is a volume 1321 associated with first leg 1311 that is not isolated ("dead space"). It is preferred that dead space is minimized so that fluids of different types can be switched between the various legs of the pinch valve. In one embodiment, the dead space is reduced by placing the placing the pinch valves close to the intersection of the legs. In another embodiment, the dead space is reduced by having passageway walls of varying thickness. Thus, for example, excess material between the pinch valves and the intersection will more effectively isolate a valved leg by displacing a portion of volume 1321.

As an example of the use of pinch valve 1300 in sampling system 300, pinchers 1320 and 1330 are positioned to act as valve 323 and 326, respectively.

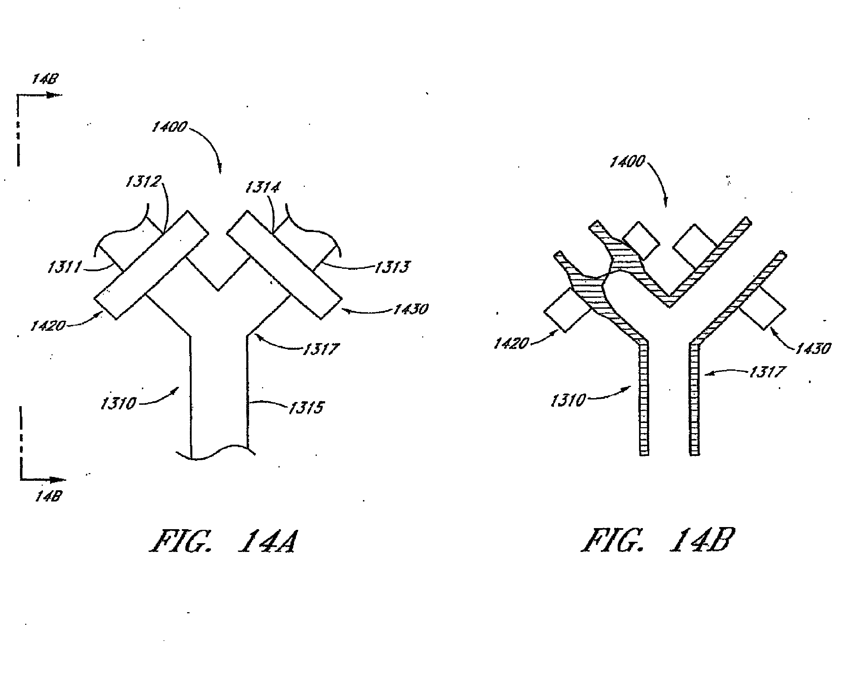

FIGURES 14A and 14B are various views of a second embodiment pinch valve 1400, where FIGURE 14A is a front view and FIGURE 14B is a sectional view showing one valve in a closed position. Pinch valve 1400 differs from pinch valve 1300 in that the pairs of pinch valve pinchers 1320 and 1330 are replaced by pinchers 1420 and 1430, respectively, that are aligned with passageway 1310.

Alternative embodiment of pinch valves includes 2, 3, 4, or more passageway segments that meet at a common junction, with pinchers located at one or more passageways near the junction.

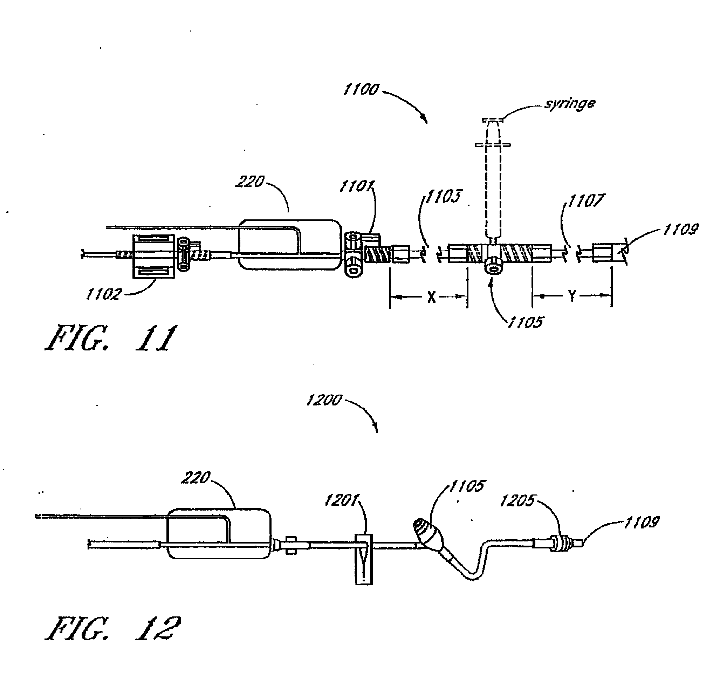

FIGURES 11 and 12 illustrate various embodiment of connector 230 which may also form or be attached to disposable portions of cassette 820 as one embodiment of an arterial patient connector 1100 and one embodiment a venous patient connector 1200. Connectors 1100 and 1200 may be generally similar to the embodiment illustrated in FIGURES 1-10, except as further detailed below.

As shown in FIGURE 11, arterial patient connector 1100 includes a stopcock 1101, a first tube portion 1103 having a length X, a blood sampling port 1105 to acquire blood samples for laboratory analysis, and fluid handling and analysis apparatus 140, a second tube 1107 having a length Y, and a tube connector 1109. Arterial patient connector 1100 also includes a pressure sensor unit 1102 that is generally similar to pressure sensor unit 1011, on the opposite side of sampling assembly 220. Length X is preferably from to 6 inches (0.15 meters) to 50 inches (1.27 meters) or approximately 48 inches (1.2 meters) in length. Length Y is preferably from 1 inch (25 millimeters) to 20 inches (0.5 meters), or approximately 12 inches (0.3 meters) in length. As shown in FIGURE 12, venous patient connector 1200 includes a clamp 1201, injection port 1105, and tube connector 1109.

## SECTION IV - SAMPLE ANALYSIS SYSTEM

In several embodiments, analysis is performed on blood plasma. For such embodiments, the blood plasma must be separated from the whole blood obtained from the patient. In general, blood plasma may be obtained from whole blood at any point in fluid handling system 10 between when the blood is drawn, for example at patient connector 110 or along passageway 113, and when it is analyzed. For systems where measurements are preformed on whole blood, it may not be necessary to separate the blood at the point of or before the measurements is performed.

For illustrative purposes, this section describes several embodiments of separators and analyte detection systems which may form part of system 10. The separators discussed in the present specification can, in certain embodiments, comprise fluid component separators. As used herein, the term "fluid component separator" is a broad term and is used in its ordinary sense and includes, without limitation, any device that is operable to separate one or more components of a fluid to generate two or more unlike substances. For example, a fluid component separator can be operable to separate a sample of whole blood into plasma and nonplasma components, and/or to separate a solid-liquid mix (e.g. a solids-contaminated liquid) into solid and liquid components. A fluid component separator need not achieve complete separation between or among the generated unlike substances. Examples of fluid component separators include filters, membranes, centrifuges, electrolytic devices, or components of any of the foregoing. Fluid component separators can be "active" in that they are operable to separate a fluid more quickly than is possible through the action of gravity on a static, "standing" fluid. Section IV.A below discloses a filter which can be used as a blood separator in certain embodiments of the apparatus disclosed herein. Section IV.B below discloses an analyte detection system which can be used in certain embodiments of the apparatus disclosed herein. Section IV.C below discloses a sample element which can be used in certain embodiments of the apparatus disclosed herein. Section IV.D below discloses a centrifuge and sample chamber which can be used in certain embodiments of the apparatus disclosed herein.

## SECTION IV.A - BLOOD FILTER

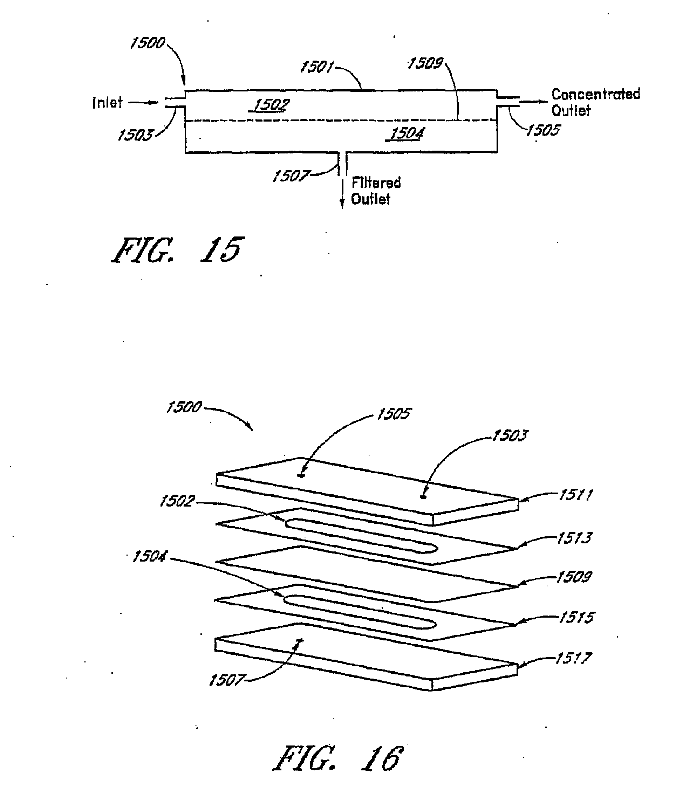

Without limitation as to the scope of the present invention, one embodiment of sample preparation unit 332 is shown as a blood filter 1500, as illustrated in FIGURES 15 and 16, where FIGURE 15 is a side view of one embodiment of a filter, and FIGURE 16 is an exploded perspective view of the filter.

As shown in the embodiment of FIGURE 15, filter 1500 that includes a housing 1501 with an inlet 1503, a first outlet 1505 and a second outlet 1507. Housing 1501 contains a membrane 1509 that divides the internal volume of housing 1501 into a first volume 1502 that include inlet 1503 and first outlet 1505 and a second volume 1504. FIGURE 16 shows one embodiment of filter 1500 as including a first plate 1511 having inlet 1503 and outlet 1505, a first spacer 1513 having an opening forming first volume 1502, a second spacer 1515 having an opening forming second volume 1504, and a second plate 1517 having outlet 1507.

Filter 1500 provides for a continuous filtering of blood plasma from whole blood. Thus, for example, when a flow of whole blood is provided at inlet 1503 and a slight vacuum is applied to the second volume 1504 side of membrane 1509, the membrane filters blood cells and blood plasma passes through second outlet 1507. Preferably, there is transverse blood flow across the surface of membrane 1509 to prevent blood cells from clogging filter 1500. Accordingly, in one embodiment of the inlet 1503 and first outlet 1505 may be configured to provide the transverse flow across membrane 1509.

In one embodiment, membrane 1509 is a thin and strong polymer film. For example, the membrane filter may be a 10 micron thick polyester or polycarbonate film. Preferably, the membrane filter has a smooth glass-like surface, and the holes are uniform, precisely sized, and clearly defined. The material of the film may be chemically inert and have low protein binding characteristics.

One way to manufacture membrane 1509 is with a Track Etching process. Preferably, the "raw" film is exposed to charged particles in a nuclear reactor, which leaves "tracks" in the film. The tracks may then be etched through the film, which results in holes that are precisely sized and uniformly cylindrical. For example, GE Osmonics, Inc. (4636 Somerton Rd. Trevose, PA 19053-6783) utilizes a similar process to manufacture a material that adequately serves as the membrane filter. The surface the membrane filter depicted above is a GE Osmonics Polycarbonate TE film.

As one example of the use of filter 1500, the plasma from 3 cc of blood may be extracted using a polycarbonate track etch film ("PCTE") as the membrane filter. The PCTE may have a pore size of 2 $\mu$m and an effective area of 170 millimeter$^2$. Preferably, the tubing connected to the supply, exhaust and plasma ports has an internal diameter of 1 millimeter. In one embodiment of a method employed with this configuration, 100 $\mu$l of plasma can be initially extracted from the blood. After saline is used to rinse the supply side of the cell, another 100 $\mu$l of clear plasma can be extracted. The rate of plasma extraction in this method and configuration can be about 15-25 $\mu$l/min.

Using a continuous flow mechanism to extract plasma may provide several benefits. In one preferred embodiment, the continuous flow mechanism is reusable with multiple samples, and there is negligible sample carryover to contaminate subsequent samples. One embodiment may also eliminate most situations in which plugging may occur. Additionally, a preferred configuration provides for a low internal volume.

Additional information on filters, methods of use thereof, and related technologies may be found in U.S. Patent Application Publication No. 2005/0038357, published on February 17, 2005, titled SAMPLE ELEMENT WITH BARRIER MATERIAL; and U.S. Patent Application No. 11/122,794, filed on May 5, 2005, titled SAMPLE ELEMENT WITH SEPARATOR. The entire contents of the above noted publication and patent application are hereby incorporated by reference herein and made a part of this specification.

SECTION IV.B - ANALYTE DETECTION SYSTEM

One embodiment of analyte detection system 334, which is not meant to limit the scope of the present invention, is shown in FIGURE 17 as an optical analyte detection system 1700. Analyte detection system 1700 is adapted to measure spectra of blood plasma. The blood plasma provided to analyte detection system 334 may be provided by sample preparation unit 332, including but not limited to a filter 1500.

Analyte detection system 1700 comprises an energy source 1720 disposed along a major axis X of system 1700. When activated, the energy source 1720 generates an energy beam E which advances from the energy source 1720 along the major axis X. In one embodiment, the energy source 1720 comprises an infrared source and the energy beam E comprises an infrared energy beam.

The energy beam E passes through an optical filter 1725 also situated on the major axis X, before reaching a probe region 1710. Probe region 1710 is portion of apparatus 322 in the path of an energized beam E that is adapted to accept a material sample S. In one embodiment, as shown in FIGURE 17, probe region 1710 is adapted to accept a sample element or cuvette 1730, which supports or contains the material sample S. In one embodiment of the present invention, sample element 1730 is a portion of passageway 113, such as a tube or an optical cell. After passing through the sample element 1730 and the sample S, the energy beam E reaches a detector 1745.

As used herein, "sample element" is a broad term and is used in its ordinary sense and includes, without limitation, structures that have a sample chamber and at least one sample chamber wall, but more generally includes any of a number of structures that can hold, support or contain a material sample and that allow electromagnetic radiation to pass through a sample held, supported or contained thereby; e.g., a cuvette, test strip, etc.

In one embodiment of the present invention, sample element 1730 forms a disposable portion of cassette 820, and the remaining portions of system 1700 form portions of instrument 810, and probe region 1710 is probe region 1002.

With further reference to FIGURE 17, the detector 1745 responds to radiation incident thereon by generating an electrical signal and passing the signal to processor 210 for analysis. Based on the signal(s) passed to it by the detector 1745, the processor computes the concentration of the analyte(s) of interest in the sample S, and/or the absorbance/transmittance characteristics of the sample S at one or more wavelengths or wavelength bands employed to analyze the sample. The processor 210 computes the concentration(s), absorbance(s), transmittance(s), etc. by executing a data processing algorithm or program instructions residing within memory 212 accessible by the processor 210.

In the embodiment shown in FIGURE 17, the filter 1725 may comprise a varying-passband filter, to facilitate changing, over time and/or during a measurement taken with apparatus 322, the wavelength or wavelength band of the energy beam E that may pass the filter 1725 for use in analyzing the sample S. (In various other embodiments, the filter 1725 may be omitted altogether.) Some examples of a varying-passband filter usable with apparatus 322 include, but are not limited to, a filter wheel (discussed in further detail below), an electronically tunable filter, such as those manufactured by Aegis Semiconductor (Woburn, MA), a custom filter using an "Active Thin Films platform," a Fabry-Perot interferometer, such as those manufactured by Scientific Solutions, Inc. (North Chelmsford, MA), a custom liquid crystal Fabry-Perot (LCFP) Tunable Filter, or a tunable monochrometer, such as a HORIBA (Jobin Yvon, Inc. (Edison, NJ) H1034 type with 7-10 $\mu$m grating, or a custom designed system.

In one embodiment detection system 1700, filter 1725 comprises a varying-passband filter, to facilitate changing, over time and/or during a measurement taken with the detection system 1700, the wavelength or wavelength band of the energy beam E that may pass the filter 25 for use in analyzing the sample S. When the energy beam E is filtered with a varying-passband filter, the absorption/transmittance characteristics of the sample S can be analyzed at a number of wavelengths or wavelength bands in a separate, sequential manner. As an example, assume that it is desired to analyze the sample S at N separate wavelengths (Wavelength 1 through Wavelength N). The varying-passband filter is first operated or tuned to permit the energy beam E to pass at Wavelength 1, while substantially blocking the beam E at most or all other wavelengths to which the detector 1745 is sensitive (including Wavelengths 2-N). The absorption/ transmittance properties of the sample S are then measured at Wavelength 1, based on the beam E that passes through the sample S and reaches the detector 1745. The varying-passband filter is then operated or tuned to permit the energy beam E to pass at Wavelength 2, while substantially blocking other wavelengths as discussed above; the sample S is then analyzed at Wavelength 2 as was done at Wavelength 1. This process is repeated until all of the wavelengths of interest have been employed to analyze the sample S. The collected absorption/transmittance data can then be analyzed by the processor 210 to determine the concentration of the analyte(s) of interest in the material sample S. The measured spectra of sample S is referred to herein in general as $C_s(\lambda_i)$, that is, a wavelength dependent spectra in which $C_s$ is, for example, a transmittance, an absorbance, an optical density, or some other measure of the optical properties of sample S having values at or about a number of wavelengths $\lambda_i$, where i ranges over the number of measurements taken. The measurement $C_s(\lambda_i)$ is a linear array of measurements that is alternatively written as $Cs_i$.

The spectral region of system 1700 depends on the analysis technique and the analyte and mixtures of interest. For example, one useful spectral region for the measurement of glucose in blood using absorption spectroscopy is the mid-IR (for example, about 4 microns to about 11 microns). In one embodiment system 1700, energy source 1720 produces a beam E having an output in the range of about 4 microns to about 11 microns. Although water is the main contributor to the total absorption across this spectral region, the peaks and other structures present in the blood spectrum from about 6.8 microns to 10.5 microns are due to the absorption spectra of other blood components. The 4 to 11 micron region has been found advantageous because glucose has a strong absorption peak structure from about 8.5 to 10 microns, whereas most other blood constituents have a low and flat absorption spectrum in the 8.5 to 10 micron range. The main exceptions are water and hemoglobin, both of which are interferents in this region.

The amount of spectral detail provided by system 1700 depends on the analysis technique and the analyte and mixture of interest. For example, the measurement of glucose in blood by mid-IR absorption spectroscopy is accomplished with from 11 to 25 filters within a spectral region. In one embodiment system 1700, energy source 1720 produces a beam E having an output in the range of about 4 microns to about 11 microns, and filter 1725 include a number of narrow band filters within this range, each allowing only energy of a certain wavelength or wavelength band to pass therethrough. Thus, for example, one embodiment filter 1725 includes a filter wheel having 11 filters with a nominal wavelength approximately equal to one of the following: 3 $\mu$m, 4.06 $\mu$m, 4.6 $\mu$m, 4.9 $\mu$m, 5.25 $\mu$m, 6.12 $\mu$m, 6.47 $\mu$m, 7.98 $\mu$m, 8.35 $\mu$m, 9.65 $\mu$m, and 12.2 $\mu$m.

In one embodiment, individual infrared filters of the filter wheel are multicavity, narrow band dielectric stacks on germanium or sapphire substrates, manufactured by either OCLI (JDS Uniphase, San Jose, CA) or Spectrogon US, Inc. (Parsippany, NJ). Thus, for example, each filter may nominally be 1 millimeter thick and 10 millimeter square. The peak transmission of the filter stack is typically between 50% and 70%, and the bandwidths are typically between 150 nm and 350 nm with center wavelengths between 4 and 10$\mu$m. Alternatively, a second blocking IR filter is also provided in front of the individual filters. The temperature sensitivity is preferably <0.01% per degree C to assist in maintaining nearly constant measurements over environmental conditions.

In one embodiment, the detection system 1700 computes an analyte concentration reading by first measuring the electromagnetic radiation detected by the detector 1745 at each center wavelength, or wavelength band, without the sample element 1730 present on the major axis X (this is known as an "air" reading). Second, the system 1700 measures the electromagnetic radiation detected by the detector 1745 for each center wavelength, or wavelength band, with the material sample S present in the sample element 1730, and the sample element and sample S in position on the major axis X (i.e., a "wet" reading). Finally, the processor 210 computes the concentration(s), absorbance(s) and/or transmittances relating to the sample S based on these compiled readings.

In one embodiment, the plurality of air and wet readings are used to generate a pathlength corrected spectrum as follows. First, the measurements are normalized to give the transmission of the sample at each wavelength. Using both a signal and reference measurement at each wavelength, and letting $S_i$ represent the signal of detector 1745 at wavelength i and $R_i$ represent the signal of the detector at wavelength i, the transmittance, $T_i$ at wavelength i may computed as $T_i = S_i(wet) / S_i(air)$. Optionally, the spectra may be calculated as the optical density, $OD_i$, as - Log($T_i$). Next, the transmission over the wavelength range of approximately 4.5 $\mu$m to approximately 5.5 $\mu$m is analyzed to

determine the pathlength. Specifically, since water is the primary absorbing species of blood over this wavelength region, and since the optical density is the product of the optical pathlength and the known absorption coefficient of water (OD = L σ, where L is the optical pathlength and σ is the absorption coefficient), any one of a number of standard curve fitting procedures may be used to determine the optical pathlength, L from the measured OD. The pathlength may then be used to determine the absorption coefficient of the sample at each wavelength. Alternatively, the optical pathlength may be used in further calculations to convert absorption coefficients to optical density.

Blood samples may be prepared and analyzed by system 1700 in a variety of configurations. In one embodiment, sample S is obtained by drawing blood, either using a syringe or as part of a blood flow system, and transferring the blood into sample chamber 903. In another embodiment, sample S is drawn into a sample container that is a sample chamber 903 adapted for insertion into system 1700.

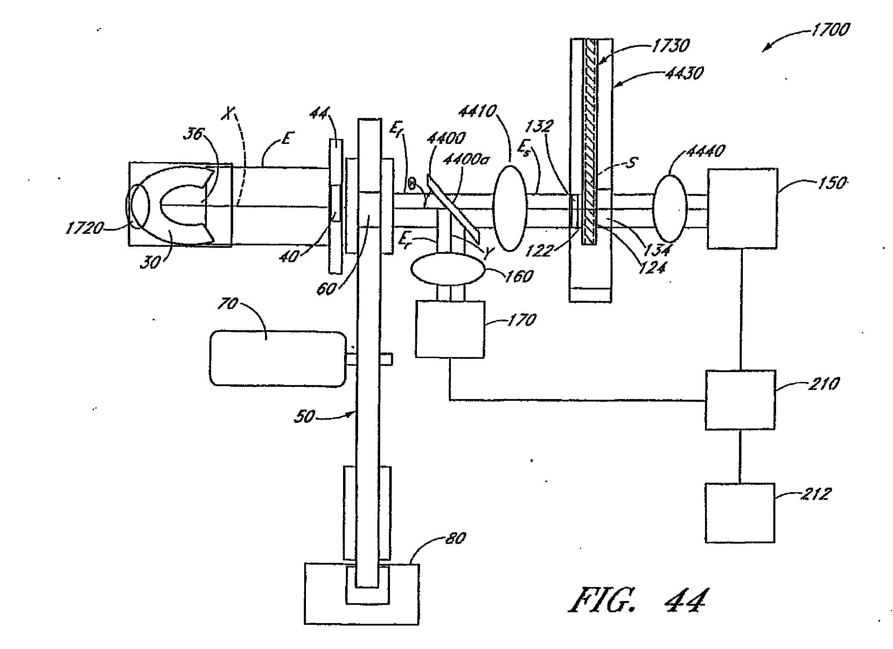

FIGURE 44 depicts another embodiment of the analyte detection system 1700, which may be generally similar to the embodiment illustrated in FIGURE 17, except as further detailed below. Where possible, similar elements are identified with identical reference numerals in the depiction of the embodiments of FIGURES 17 and 44.

The detection system 1700 shown in FIGURE 44 includes a collimator 30 located between source 1720 and filter 1725 and a beam sampling optics 90 between the filter and sample element 1730. Filter 1725 includes a primary filter 40 and a filter wheel assembly 4420 which can insert one of a plurality of optical filters into energy beam E. System 1700 also includes a sample detector 150 may be generally similar to sample detector 1725, except as further detailed below.

As shown in FIGURE 44, energy beam E from source 1720 passes through collimator 30 through which the before reaching a primary optical filter 40 which is disposed downstream of a wide end 36 of the collimator 30. Filter 1725 is aligned with the source 1720 and collimator 30 on the major axis X and is preferably configured to operate as a broadband filter, allowing only a selected band, e.g. between about 2.5 μm and about 12.5 μm, of wavelengths emitted by the source 1720 to pass therethrough, as discussed below. In one embodiment, the energy source 1720 comprises an infrared source and the energy beam E comprises an infrared energy beam. One suitable energy source 1720 is the TOMA TECH™ IR-50 available from HawkEye Technologies of Milford, Connecticut.

With further reference to FIGURE 44, primary filter 40 is mounted in a mask 44 so that only those portions of the energy beam E which are incident on the primary filter 40 can pass the plane of the mask-primary filter assembly. The primary filter 40 is generally centered on and oriented orthogonal to the major axis X and is preferably circular (in a plane orthogonal to the major axis X) with a diameter of about 8 mm. Of course, any other suitable size or shape may be employed. As discussed above, the primary filter 40 preferably operates as a broadband filter. In the illustrated embodiment, the primary filter 40 preferably allows only energy wavelengths between about 4 μm and about 11 μm to pass therethrough. However, other ranges of wavelengths can be selected. The primary filter 40 advantageously reduces the filtering burden of secondary optical filter(s) 60 disposed downstream of the primary filter 40 and improves the rejection of electromagnetic radiation having a wavelength outside of the desired wavelength band. Additionally, the primary filter 40 can help minimize the heating of the secondary filter(s) 60 by the energy beam E passing therethrough. Despite these advantages, the primary filter 40 and/or mask 44 may be omitted in alternative embodiments of the system 1700 shown in FIGURE 44.

The primary filter 40 is preferably configured to substantially maintain its operating characteristics (center wavelength, passband width) where some or all of the energy beam E deviates from normal incidence by a cone angle of up to about twelve degrees relative to the major axis X. In further embodiments, this cone angle may be up to about 15 to 35 degrees, or from about 15 degrees or 20 degrees. The primary filter 40 may be said to "substantially maintain" its operating characteristics where any changes therein are insufficient to affect the performance or operation of the detection system 1700 in a manner that would raise significant concerns for the user(s) of the system in the context in which the system 1700 is employed.

In the embodiment illustrated in FIGURE 44, filter wheel assembly 4420 includes an optical filter wheel 50 and a stepper motor 70 connected to the filter wheel and configured to generate a force to rotate the filter wheel 50. Additionally, a position sensor 80 is disposed over a portion of the circumference of the filter wheel 50 and may be configured to detect the angular position of the filter wheel 50 and to generate a corresponding filter wheel position signal, thereby indicating which filter is in position on the major axis X. Alternatively, the stepper motor 70 may be configured to track or count its own rotation(s), thereby tracking the angular position of the filter wheel, and pass a corresponding position signal to the processor 210. Two suitable position sensors are models EE-SPX302-W2A and EE-SPX402-W2A available from Omron Corporation of Kyoto, Japan.

Optical filter wheel 50 is employed as a varying-passband filter, to selectively position the secondary filter(s) 60 on the major axis X and/or in the energy beam E. The filter wheel 50 can therefore selectively tune the wavelength (s) of the energy beam E downstream of the wheel 50. These wavelength(s) vary according to the characteristics of the secondary filter(s) 60 mounted in the filter wheel 50. The filter wheel 50 positions the secondary filter(s) 60 in the energy beam E in a "one-at-a-time" fashion to sequentially vary, as discussed above, the wavelengths or wavelength bands employed to analyze the material sample S. An alternative to filter wheel 50 is a linear filter translated by a motor (not shown). The linear filter may be, for example, a linear array of separate filters or a single filter with filter properties that change in a linear dimension.

In alternative arrangements, the single primary filter 40 depicted in FIGURE 44 may be replaced or supplemented with additional primary filters mounted on the filter wheel 50 upstream of each of the secondary filters 60. As yet another alternative, the primary filter 40 could be implemented as a primary filter wheel (not shown) to position different primary filters on the major axis X at different times during operation of the detection system 1700, or as a tunable filter.

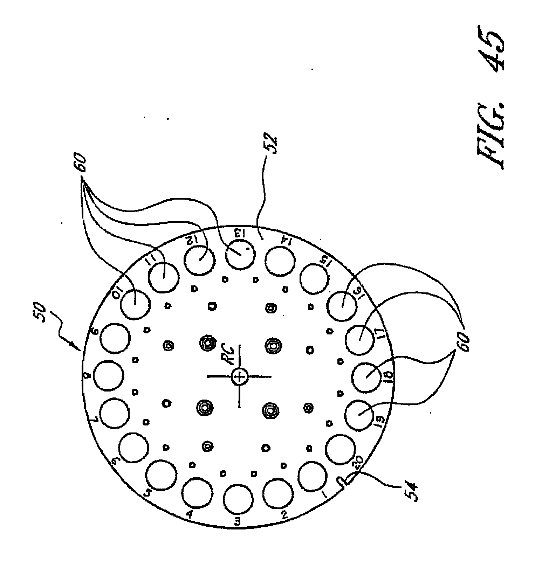

The filter wheel 50, in the embodiment depicted in FIGURE 45, can comprise a wheel body 52 and a plurality of secondary filters 60 disposed on the body 52, the center of each filter being equidistant from a rotational center RC of the wheel body. The filter wheel 50 is configured to rotate about an axis which is (i) parallel to the major axis X and (ii) spaced from the major axis X by an orthogonal distance approximately equal to the distance between the rotational center RC and any of the center(s) of the secondary filter(s) 60. Under this arrangement, rotation of the wheel body 52 advances each of the filters sequentially through the major axis X, so as to act upon the energy beam E. However, depending on the analyte(s) of interest or desired measurement speed, only a subset of the filters on the wheel 50 may be employed in a given measurement run. A home position notch 54 may be provided to indicate the home position of the wheel 50 to a position sensor 80.

In one embodiment, the wheel body 52 can be formed from molded plastic, with each of the secondary filters 60 having, for example a thickness of 1 mm and a 10 mm x 10 mm or a 5 mm x 5 mm square configuration. Each of the filters 60, in this embodiment of the wheel body, is axially aligned with a circular aperture of 4 mm diameter, and the aperture centers define a circle of about 1.70 inches diameter, which circle is concentric with the wheel body 52. The body 52 itself is circular, with an outside diameter of 2.00 inches.

Each of the secondary filter(s) 60 is preferably configured to operate as a narrow band filter, allowing only a selected energy wavelength or wavelength band (i.e., a filtered energy beam ($E_f$) to pass therethrough. As the filter wheel 50 rotates about its rotational center RC, each of the secondary filter(s) 60 is, in turn, disposed along the major axis X for a selected dwell time corresponding to each of the secondary filter(s) 60.

The "dwell time" for a given secondary filter 60 is the time interval, in an individual measurement run of the system 1700, during which both of the following conditions are true: (i) the filter is disposed on the major axis X; and (ii) the source 1720 is energized. The dwell time for a given filter may be greater than or equal to the time during which the filter is disposed on the major axis X during an individual measurement run. In one embodiment of the analyte detection system 1700, the dwell time corresponding to each of the secondary filter(s) 60 is less than about 1 second. However, the secondary filter(s) 60 can have other dwell times, and each of the filter(s) 60 may have a different dwell time during a given measurement run.

From the secondary filter 60, the filtered energy beam (Ef) passes through a beam sampling optics 90, which includes a beam splitter 4400 disposed along the major axis X and having a face 4400a disposed at an included angle θ relative to the major axis X. The splitter 4400 preferably separates the filtered energy beam (Ef) into a sample beam (Es) and a reference beam (Er).

With further reference to FIGURE 44, the sample beam (Es) passes next through a first lens 4410 aligned with the splitter 4400 along the major axis X. The first lens 4410 is configured to focus the sample beam (Es) generally along the axis X onto the material sample S. The sample S is preferably disposed in a sample element 1730 between a first window 122 and a second window 124 of the sample element 1730. The sample element 1730 is further preferably removably disposed in a holder 4430, and the holder 4430 has a first opening 132 and a second opening 134 configured for alignment with the first window 122 and second window 124, respectively. Alternatively, the sample element 1730 and sample S may be disposed on the major axis X without use of the holder 4430.

At least a fraction of the sample beam (Es) is transmitted through the sample S and continues onto a second lens 4440 disposed along the major axis X. The second lens 4440 is configured to focus the sample beam (Es) onto a sample detector 150, thus increasing the flux density of the sample beam (Es) incident upon the sample detector 150. The sample detector 150 is configured to generate a signal corresponding to the detected sample beam (Es) and to pass the signal to a processor 210, as discussed in more detail below.

Beam sampling optics 90 further includes a third lens 160 and a reference detector 170. The reference beam (Er) is directed by beam sampling optics 90 from the beam splitter 4400 to third lens 160 disposed along a minor axis Y generally orthogonal to the major axis X. The third lens 160 is configured to focus the reference beam (Er) onto reference detector 170, thus increasing the flux density of the reference beam (Er) incident upon the reference detector 170. In one embodiment, the lenses 4410, 4440, 160 may be formed from a material which is highly transmissive of infrared radiation, for example germanium or silicon. In addition, any of the lenses 4410, 4440 and 160 may be implemented as a system of lenses, depending on the desired optical performance. The reference detector 170 is also configured to generate a signal corresponding to the detected reference beam (Er) and to pass the signal to the processor 210, as discussed in more detail below. Except as noted below, the sample and reference detectors 150, 170 may be generally similar to the detector 1745 illustrated in FIGURE 17. Based on signals received from the sample and reference detectors 150, 170, the processor 210 computes the concentration(s), absorbance(s), transmittance(s), etc. relating to the sample S by executing a data processing algorithm or program instructions residing within the memory 212 accessible by the processor 210.

In further variations of the detection system 1700 depicted in FIGURE 44, beam sampling optics 90, including the beam splitter 4400, reference detector 170 and other structures on the minor axis Y may be omitted, especially where the output intensity of the source 1720 is sufficiently stable to obviate any need to reference the source intensity in operation of the detection system 1700. Thus, for example, sufficient signals may be generated by detectors 170 and 150 with one or more of lenses 4410, 4440, 160 omitted. Furthermore, in any of the embodiments of the analyte detection system 1700 disclosed herein, the processor 210 and/or memory 212 may reside partially or wholly in a standard personal computer ("PC") coupled to the detection system 1700.

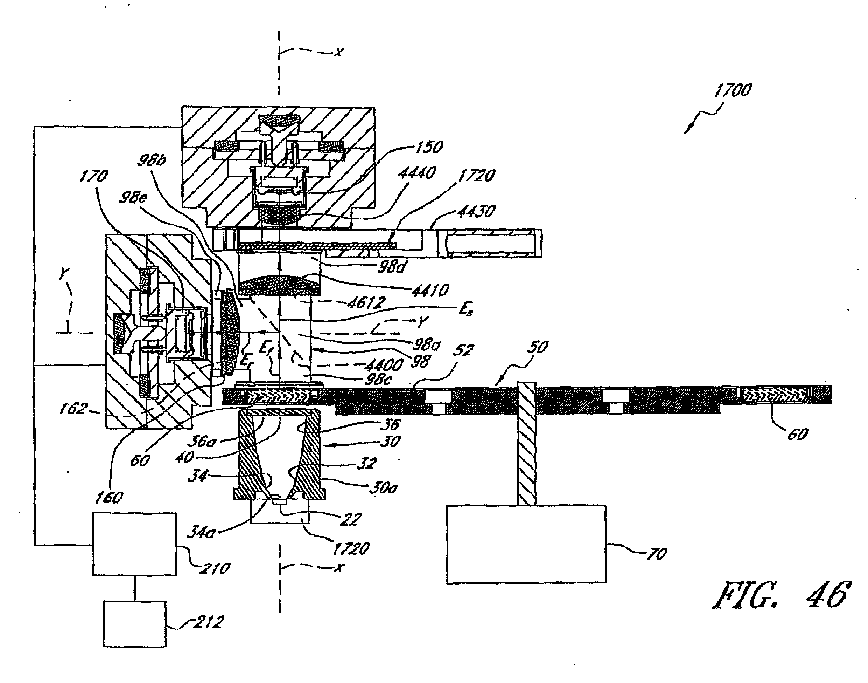

FIGURE 46 depicts a partial cross-sectional view of another embodiment of an analyte detection system 1700, which may be generally similar to any of the embodiments illustrated in FIGURES 17, 44, and 45, except as further detailed below. Where possible, similar elements are identified with identical reference numerals in the depiction of the embodiments of FIGURES 17, 44, and 45.

The energy source 1720 of the embodiment of FIGURE 46 preferably comprises an emitter area 22 which is substantially centered on the major axis X. In one embodiment, the emitter area 22 may be square in shape. However the emitter area 22 can have other suitable shapes, such as rectangular, circular, elliptical, etc. One suitable emitter area 22 is a square of about 1.5 mm on a side; of course, any other suitable shape or dimensions may be employed.

The energy source 1720 is preferably configured to selectably operate at a modulation frequency between about 1 Hz and 30 Hz and have a peak operating temperature of between about 1070 degrees Kelvin and 1170 degrees Kelvin. Additionally, the source 1720 preferably operates with a modulation depth greater than about 80% at all modulation frequencies. The energy source 1720 preferably emits electromagnetic radiation in any of a number of spectral ranges, e.g., within infrared wavelengths; in the mid-infrared wavelengths; above about 0.8 $\mu$m; between about 5.0 $\mu$m and about 20.0 $\mu$m; and/or between about 5.25 $\mu$m and about 12.0 $\mu$m. However, in other embodiments, the detection system 1700 may employ an energy source 1720 which is unmodulated and/or which emits in wavelengths found anywhere from the visible spectrum through the microwave spectrum, for example anywhere from about 0.4 $\mu$m to greater than about 100 $\mu$m. In still other embodiments, the energy source 1720 can emit electromagnetic radiation in wavelengths between about 3.5 $\mu$m and about 14 $\mu$m, or between about 0.8 $\mu$m and about 2.5 $\mu$m, or between about 2.5 $\mu$m and 20 $\mu$m, or between about 20 $\mu$m and about 100 $\mu$m, or between about 6.85 $\mu$m and about 10.10 $\mu$m. In yet other embodiments, the energy source 1720 can emit electromagnetic radiation within the radio frequency (RF) range or the terahertz range. All of the above-recited operating characteristics are merely exemplary, and the source 1720 may have any operating characteristics suitable for use with the analyte detection system 1700.

A power supply (not shown) for the energy source 1720 is preferably configured to selectably operate with a duty cycle of between about 30% and about 70%. Additionally, the power supply is preferably configured to selectably operate at a modulation frequency of about 10Hz, or between about 1 Hz and about 30 Hz. The operation of the power supply can be in the form of a square wave, a sine wave, or any other waveform defined by a user.

With further reference to FIGURE 46, the collimator 30 comprises a tube 30a with one or more highly-reflective inner surfaces 32 which diverge from a relatively narrow upstream end 34 to a relatively wide downstream end 36 as they extend downstream, away from the energy source 1720. The narrow end 34 defines an upstream aperture 34a which is situated adjacent the emitter area 22 and permits radiation generated by the emitter area to propagate downstream into the collimator. The wide end 36 defines a downstream aperture 36a. Like the emitter area 22, each of the inner surface(s) 32, upstream aperture 34a and downstream aperture 36a is preferably substantially centered on the major axis X.

As illustrated in FIGURE 46, the inner surface(s) 32 of the collimator may have a generally curved shape, such as a parabolic, hyperbolic, elliptical or spherical shape. One suitable collimator 30 is a compound parabolic concentrator (CPC). In one embodiment, the collimator 30 can be up to about 20 mm in length. In another embodiment, the collimator 30 can be up to about 30 mm in length. However, the collimator 30 can have any length, and the inner surface(s) 32 may have any shape, suitable for use with the analyte detection system 1700.

The inner surfaces 32 of the collimator 30 cause the rays making up the energy beam E to straighten (i.e., propagate at angles increasingly parallel to the major axis X) as the beam E advances downstream, so that the energy beam E becomes increasingly or substantially cylindrical and oriented substantially parallel to the major axis X. Accordingly, the inner surfaces 32 are highly reflective and minimally absorptive in the wavelengths of interest, such as infrared wavelengths.

The tube 30a itself may be fabricated from a rigid material such as aluminum, steel, or any other suitable material, as long as the inner surfaces 32 are coated or otherwise treated to be highly reflective in the wavelengths of interest. For example, a polished gold coating may be employed. Preferably, the inner surface(s) 32 of the collimator 30 define a circular cross-section when viewed orthogonal to the major axis X; however, other cross-sectional shapes, such as a square or other polygonal shapes, parabolic or elliptical shapes may be employed in alternative embodiments.

As noted above, the filter wheel 50 shown in FIGURE 46 comprises a plurality of secondary filters 60 which preferably operate as narrow band filters, each filter allowing only energy of a certain wavelength or wavelength band to pass therethrough. In one configuration suitable for detection of glucose in a sample S, the filter wheel 50 comprises twenty or twenty-two secondary filters 60, each of which is configured to allow a filtered energy beam (Ef) to travel therethrough with a nominal wavelength approximately equal to one of the following: 3 $\mu$m, 4.06 $\mu$m, 4.6 $\mu$m, 4.9 $\mu$m, 5.25 $\mu$m, 6.12 $\mu$m, 6.47 $\mu$m, 7.98 $\mu$m, 8.35 $\mu$m, 9.65 $\mu$m, and 12.2 $\mu$m. (Moreover, this set of wavelengths may be employed with or in any of the embodiments of the analyte detection system 1700 disclosed herein.) Each secondary filter's 60 center wavelength is preferably equal to the desired nominal wavelength plus or minus about 2%. Additionally, the secondary filters 60 are preferably configured to have a bandwidth of about 0.2 $\mu$m, or alternatively equal to the nominal wavelength plus or minus about 2%-10%.

In another embodiment, the filter wheel 50 comprises twenty secondary filters 60, each of which is configured to allow a filtered energy beam (Ef) to travel therethrough with a nominal center wavelengths of: 4.275 $\mu$m, 4.5 $\mu$m, 4.7 $\mu$m, 5.0 $\mu$m, 5.3 $\mu$m, 6.056 $\mu$m, 7.15 $\mu$m, 7.3 $\mu$m, 7.55 $\mu$m, 7.67 $\mu$m, 8.06 $\mu$m, 8.4 $\mu$m, 8.56 $\mu$m, 8.87 $\mu$m, 9.15 $\mu$m, 9.27 $\mu$m, 9.48 $\mu$m, 9.68 $\mu$m, 9.82 $\mu$m, and 10.06 $\mu$m. (This set of wavelengths may also be employed with or in any of the embodiments of the analyte detection system 1700 disclosed herein.) In still another embodiment, the secondary filters 60 may conform to any one or combination of the following specifications: center wavelength tolerance of $\pm$ 0.01 $\mu$m; half-power bandwidth tolerance of $\pm$ 0.01 $\mu$m; peak transmission greater than or equal to 75%; cut-on/cut-off slope less than 2%; center-wavelength temperature coefficient less than .01% per degree Celsius; out of band attenuation greater than OD 5 from 3 $\mu$m to 12 $\mu$m; flatness less than 1.0 waves at 0.6328 $\mu$m; surface quality of E-E per Mil-F-48616; and overall thickness of about 1 mm.

In still another embodiment, the secondary filters mentioned above may conform to any one or combination of the following half-power bandwidth ("HPBW") specifications:

| Center Wavelength ($\mu$m) | HPBW ($\mu$m) | Center Wavelength ($\mu$m) | HPBW ($\mu$m) |
|---|---|---|---|
| 4.275 | 0.05 | 8.06 | 0.3 |
| 4.5 | 0.18 | 8.4 | 0.2 |
| 4.7 | 0.13 | 8.56 | 0.18 |
| 5.0 | 0.1 | 8.87 | 0.2 |
| 5.3 | 0.13 | 9.15 | 0.15 |
| 6.056 | 0.135 | 9.27 | 0.14 |
| 7.15 | 0.19 | 9.48 | 0.23 |
| 7.3 | 0.19 | 9.68 | 0.3 |
| 7.55 | 0.18 | 9.82 | 0.34 |
| 7.67 | 0.197 | 10.06 | 0.2 |

In still further embodiments, the secondary filters may have a center wavelength tolerance of $\pm$ 0.5 % and a half-power bandwidth tolerance of $\pm$ 0.02 $\mu$m.

Of course, the number of secondary filters employed, and the center wavelengths and other characteristics thereof, may vary in further embodiments of the system 1700, whether such further embodiments are employed to detect glucose, or other analytes instead of or in addition to glucose. For example, in another embodiment, the filter wheel 50 can have fewer than fifty secondary filters 60. In still another embodiment, the filter wheel 50 can have fewer than twenty secondary filters 60. In yet another embodiment, the filter wheel 50 can have fewer than ten secondary filters 60.

In one embodiment, the secondary filters 60 each measure about 10 mm long by 10 mm wide in a plane orthogonal to the major axis X, with a thickness of about 1 mm. However, the secondary filters 60 can have any other (e.g., smaller) dimensions suitable for operation of the analyte detection system 1700. Additionally, the secondary filters 60 are preferably configured to operate at a temperature of between about 5 °C and about 35 °C and to allow transmission of more than about 75% of the energy beam E therethrough in the wavelength(s) which the filter is configured to pass.

According to the embodiment illustrated in FIGURE 46, the primary filter 40 operates as a broadband filter and the secondary filters 60 disposed on the filter wheel 50 operate as narrow band filters. However, one of ordinary skill in the art will realize that other structures can be used to filter energy wavelengths according to the embodiments described herein. For example, the primary filter 40 may be omitted and/or an electronically tunable filter or Fabry-Perot interferometer (not shown) can be used in place of the filter wheel 50 and secondary filters 60. Such a tunable filter or interferometer can be configured to permit, in a sequential, "one-at-a-time" fashion, each of a set of wavelengths or wavelength bands of electromagnetic radiation to pass therethrough for use in analyzing the material sample S.

A reflector tube 98 is preferably positioned to receive the filtered energy beam (Ef) as it advances from the secondary filter(s) 60. The reflector tube 98 is preferably secured with respect to the secondary filter(s) 60 to substantially prevent introduction of stray electromagnetic radiation, such as stray light, into the reflector tube 98 from outside of the detection system 1700. The inner surfaces of the reflector tube 98 are highly reflective in the relevant wavelengths and preferably have a cylindrical shape with a generally circular cross-section orthogonal to the major and/or minor axis X, Y. However, the inner surface of the tube 98 can have a cross-section of any suitable shape, such as oval, square, rectangular, etc. Like the collimator 30, the reflector tube 98 may be formed from a rigid material such as aluminum, steel, etc., as long as the inner surfaces are coated or otherwise treated to be highly reflective in the wavelengths of interest. For example, a polished gold coating may be employed.

According to the embodiment illustrated in FIGURE 46, the reflector tube 98 preferably comprises a major section 98a and a minor section 98b. As depicted, the reflector tube 98 can be T-shaped with the major section 98a having a greater length than the minor section 98b. In another example, the major section 98a and the minor section 98b can have the same length. The major section 98a extends between a first end 98c and a second end 98d along the major axis X. The minor section 98b extends between the major section 98a and a third end 98e along the minor axis Y.

The major section 98a conducts the filtered energy beam (Ef) from the first end 98c to the beam splitter 4400, which is housed in the major section 98a at the intersection of the major and minor axes X, Y. The major section 98a also conducts the sample beam (Es) from the beam splitter 4400, through the first lens 4410 and to the second end 98d. From the second end 98d the sample beam (Es) proceeds through the sample element 1730, holder 4430 and second lens 4440, and to the sample detector 150. Similarly, the minor section 98b conducts the reference beam (Er) through beam sampling optics 90 from the beam splitter 4400, through the third lens 160 and to the third end 98e. From the third end 98e the reference beam (Er) proceeds to the reference detector 170.

The sample beam (Es) preferably comprises from about 75% to about 85% of the energy of the filtered energy beam (Ef). More preferably, the sample beam (Es) comprises about 80% of the energy of the filtered energy beam (Es). The reference beam (Er) preferably comprises from about 10% and about 50% of the energy of the filtered energy beam (Es). More preferably, the reference beam (Er) comprises about 20% of the energy of the filtered energy beam (Ef). Of course, the sample and reference beams may take on any suitable proportions of the energy beam E.

The reflector tube 98 also houses the first lens 4410 and the third lens 160. As illustrated in FIGURE 46, the reflector tube 98 houses the first lens 4410 between the beam splitter 4400 and the second end 98d. The first lens 4410 is preferably disposed so that a plane 4612 of the lens 4410 is generally orthogonal to the major axis X. Similarly, the tube 98 houses the third lens 160 between the beam splitter 4400 and the third end 98e. The third lens 160 is preferably disposed so that a plane 162 of the third lens 160 is generally orthogonal to the minor axis Y. The first lens 4410 and the third lens 160 each has a focal length configured to substantially focus the sample beam (Es) and reference beam (Er), respectively, as the beams (Es, Er) pass through the lenses 4410, 160. In particular, the first lens 4410 is configured, and disposed relative to the holder 4430, to focus the sample beam (Es) so that substantially the entire sample beam (Es) passes through the material sample S, residing in the sample element 1730. Likewise, the third lens 160 is configured to focus the reference beam (Er) so that substantially the entire reference beam (Er) impinges onto the reference detector 170.

The sample element 1730 is retained within the holder 4430, which is preferably oriented along a plane generally orthogonal to the major axis X. The holder 4430 is configured to be slidably displaced between a loading position and a measurement position within the analyte detection system 1700. In the measurement position, the holder 4430 contacts a stop edge 136 which is located to orient the sample element 1730 and the sample S contained therein on the major axis X.

The structural details of the holder 4430 depicted in FIGURE 46 are unimportant, so long as the holder positions the sample element 1730 and sample S on and substantially orthogonal to the major axis X, while permitting the energy beam E to pass through the sample element and sample. As with the embodiment depicted in FIGURE 44, the holder 4430 may be omitted and the sample element 1730 positioned alone in the depicted location on the major axis X. However, the holder 4430 is useful where the sample element 1730 (discussed in further detail below) is constructed from a highly brittle or fragile material, such as barium fluoride, or is manufactured to be extremely thin.

As with the embodiment depicted in FIGURE 44, the sample and reference detectors 150, 170 shown in FIGURE 46 respond to radiation incident thereon by generating signals and passing them to the processor 210. Based these signals received from the sample and reference detectors 150, 170, the processor 210 computes the concentration(s), absorbance(s), transmittance(s), etc. relating to the sample S by executing a data processing algorithm or program instructions residing within the memory 212 accessible by the processor 210. In further variations of the detection system 1700 depicted in FIGURE 46, the beam splitter 4400, reference detector 170 and other structures on the minor axis Y may be omitted, especially where the output intensity of the source 1720 is sufficiently stable to obviate any need to reference the source intensity in operation of the detection system 1700.

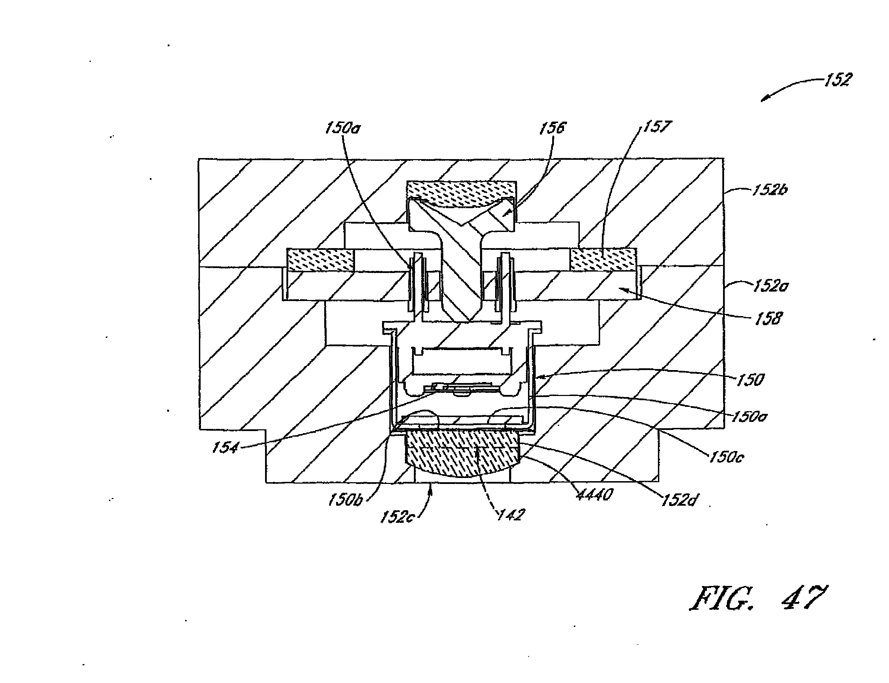

FIGURE 47 depicts a sectional view of the sample detector 150 in accordance with one embodiment. Sample detector 150 is mounted in a detector housing 152 having a receiving portion 152a and a cover 152b. However, any suitable structure may be used as the sample detector 150 and housing 152. The receiving portion 152a preferably defines an aperture 152c and a lens chamber 152d, which are generally aligned with the major axis X when the housing 152 is mounted in the analyte detection system 1700. The aperture 152c is configured to allow at least a fraction of the sample beam (Es) passing through the sample S and the sample element 1730 to advance through the aperture 152c and into the lens chamber 152d.

The receiving portion 152a houses the second lens 4440 in the lens chamber 152d proximal to the aperture 152c. The sample detector 150 is also disposed in the lens chamber 152d downstream of the second lens 4440 such that a detection plane 154 of the detector 150 is substantially orthogonal to the major axis X. The second lens 4440 is positioned such that a plane 142 of the lens 4440 is substantially orthogonal to the major axis X. The second lens 4440 is configured, and is preferably disposed relative to the holder 4430 and the sample detector 150, to focus substantially all of the sample beam (Es) onto the detection plane 154, thereby increasing the flux density of the sample beam (Es) incident upon the detection plane 154.

With further reference to FIGURE 47, a support member 156 preferably holds the sample detector 150 in place in the receiving portion 152a. In the illustrated embodiment, the support member 156 is a spring 156 disposed between the sample detector 150 and the cover 152b. The spring 156 is configured to maintain the detection plane 154 of the sample detector 150 substantially orthogonal to the major axis X. A gasket 157 is preferably disposed between the cover 152b and the receiving portion 152a and surrounds the support member 156.

The receiving portion 152a preferably also houses a printed circuit board 158 disposed between the gasket 157 and the sample detector 150. The board 158 connects to the sample detector 150 through at least one connecting member 150a. The sample detector 150 is configured to generate a detection signal corresponding to the sample beam (Es) incident on the detection plane 154. The sample detector 150 communicates the detection signal to the circuit board 158 through the connecting member 150a, and the board 158 transmits the detection signal to the processor 210.

In one embodiment, the sample detector 150 comprises a generally cylindrical housing 150a, e.g. a type TO-39 "metal can" package, which defines a generally circular housing aperture 150b at its "upstream" end. In one embodiment, the housing 150a has a diameter of about 0.323 inches and a depth of about 0.248 inches, and the aperture 150b may have a diameter of about 0.197 inches.

A detector window 150c is disposed adjacent the aperture 150b, with its upstream surface preferably about 0.078 inches (+/- 0.004 inches) from the detection plane 154. (The detection plane 154 is located about 0.088 inches (+/- 0.004 inches) from the upstream edge of the housing 150a, where the housing has a thickness of about 0.010 inches.) The detector window 150c is preferably transmissive of infrared energy in at least a 3-12 micron passband; accordingly, one suitable material for the window 150c is germanium. The endpoints of the passband may be "spread" further to less than 2.5 microns, and/or greater than 12.5 microns, to avoid unnecessary absorbance in the wavelengths of interest. Preferably, the transmittance of the detector window 150c does not vary by more than 2% across its passband. The window 150c is preferably about 0.020 inches in thickness. The sample detector 150 preferably substantially retains its operating characteristics across a temperature range of -20 to +60 degrees Celsius.

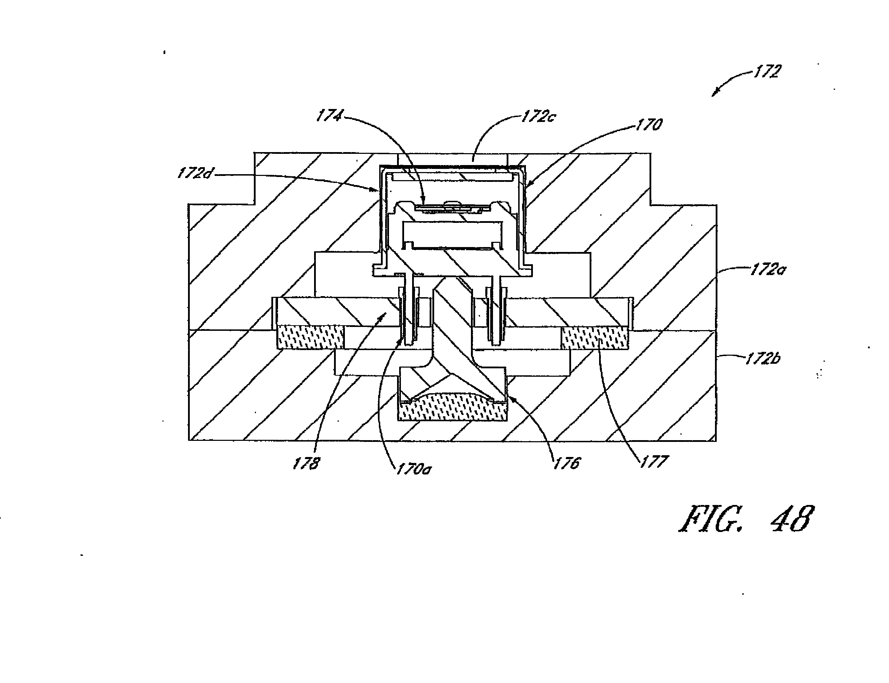

FIGURE 48 depicts a sectional view of the reference detector 170 in accordance with one embodiment. The reference detector 170 is mounted in a detector housing 172 having a receiving portion 172a and a cover 172b. However, any suitable structure may be used as the sample detector 150 and housing 152. The receiving portion 172a preferably defines an aperture 172c and a chamber 172d which are generally aligned with the minor axis Y, when the housing 172 is mounted in the analyte detection system 1700. The aperture 172c is configured to allow the reference beam (Er) to advance through the aperture 172c and into the chamber 172d.

The receiving portion 172a houses the reference detector 170 in the chamber 172d proximal to the aperture 172c. The reference detector 170 is disposed in the chamber 172d such that a detection plane 174 of the reference detector 170 is substantially orthogonal to the minor axis Y. The third lens 160 is configured to substantially focus the reference beam (Er) so that substantially the entire reference beam (Er) impinges onto the detection plane 174, thus increasing the flux density of the reference beam (Er) incident upon the detection plane 174.

With further reference to FIGURE 48, a support member 176 preferably holds the reference detector 170 in place in the receiving portion 172a. In the illustrated embodiment, the support member 176 is a spring 176 disposed between the reference detector 170 and the cover 172b. The spring 176 is configured to maintain the detection plane 174 of the reference detector 170 substantially orthogonal to the minor axis Y. A gasket 177 is preferably disposed between the cover 172b and the receiving portion 172a and surrounds the support member 176.

The receiving portion 172a preferably also houses a printed circuit board 178 disposed between the gasket 177 and the reference detector 170. The board 178 connects to the reference detector 170 through at least one connecting member 170a. The reference detector 170 is configured to generate a detection signal corresponding to the reference beam (Er) incident on the detection plane 174. The reference detector 170 communicates the detection signal to the circuit board 178 through the connecting member 170a, and the board 178 transmits the detection signal to the processor 210.

In one embodiment, the construction of the reference detector 170 is generally similar to that described above with regard to the sample detector 150.

In one embodiment, the sample and reference detectors 150, 170 are both configured to detect electromagnetic radiation in a spectral wavelength range of between about 0.8 $\mu$m and about 25 $\mu$m. However, any suitable subset of the foregoing set of wavelengths can be selected. In another embodiment, the detectors 150, 170 are configured to detect electromagnetic radiation in the wavelength range of between about 4$\mu$m and about 12 $\mu$m. The detection planes 154, 174 of the detectors 150, 170 may each define an active area about 2 mm by 2 mm or from about 1 mm by 1 mm to about 5 mm by 5 mm; of course, any other suitable dimensions and proportions may be employed. Additionally, the detectors 150, 170 may be configured to detect electromagnetic radiation directed thereto within a cone angle of about 45 degrees from the major axis X.

In one embodiment, the sample and reference detector subsystems 150, 170 may further comprise a system (not shown) for regulating the temperature of the detectors. Such a temperature-regulation system may comprise a suitable electrical heat source, thermistor, and a proportional-plus-integral-plus-derivative (PID) control. These components may be used to regulate the temperature of the detectors 150, 170 at about 35 °C. The detectors 150, 170 can also optionally be operated at other desired temperatures. Additionally, the PID control preferably has a control rate of about 60 Hz and, along with the heat source and thermistor, maintains the temperature of the detectors 150, 170 within about 0.1 °C of the desired temperature.

The detectors 150, 170 can operate in either a voltage mode or a current mode, wherein either mode of operation preferably includes the use of a pre-amp module. Suitable voltage mode detectors for use with the analyte detection system 1700 disclosed herein include: models LIE 302 and 312 by InfraTec of Dresden, Germany; model L2002 by BAE Systems of Rockville, Maryland; and model LTS-1 by Dias of Dresden, Germany. Suitable current mode detectors include: InfraTec models LIE 301, 315, 345 and 355; and 2x2 current-mode detectors available from Dias.

In one embodiment, one or both of the detectors 150, 170 may meet the following specifications, when assuming an incident radiation intensity of about $9.26 \times 10^{-4}$ watts (rms) per cm$^2$, at 10 Hz modulation and within a cone angle of about 15 degrees: detector area of 0.040 cm$^2$ (2 mm x 2 mm square); detector input of $3.70 \times 10^{-5}$ watts (rms) at 10 Hz; detector sensitivity of 360 volts per watt at 10 Hz; detector output of $1.333 \times 10^{-2}$ volts (rms) at 10 Hz; noise of $8.00 \times 10^{-8}$ volts/sqrtHz at 10 Hz; and signal-to-noise ratios of $1.67 \times 10^5$ rms/sqrtHz and 104.4 dB/sqrtHz; and detectivity of $1.00 \times 10^9$ cm sqrtHz/watt.

In alternative embodiments, the detectors 150, 170 may comprise microphones and/or other sensors suitable for operation of the detection system 1700 in a photoacoustic mode.

The components of any of the embodiments of the analyte detection system 1700 may be partially or completely contained in an enclosure or casing (not shown) to prevent stray electromagnetic radiation, such as stray light, from contaminating the energy beam E. Any suitable casing may be used. Similarly, the components of the detection system 1700 may be mounted on any suitable frame or chassis (not shown) to maintain their operative alignment as depicted in FIGURES 17, 44, and 46. The frame and the casing may be formed together as a single unit, member or collection of members.

In one method of operation, the analyte detection system 1700 shown in FIGURES 44 or 46 measures the concentration of one or more analytes in the material sample S, in part, by comparing the electromagnetic radiation detected by the sample and reference detectors 150, 170. During operation of the detection system 1700, each of the secondary filter(s) 60 is sequentially aligned with the major axis X for a dwell time corresponding to the secondary filter 60. (Of course, where an electronically tunable filter or Fabry-Perot interferometer is used in place of the filter wheel 50, the tunable filter or interferometer is sequentially tuned to each of a set of desired wavelengths or wavelength bands in lieu of the sequential alignment of each of the secondary filters with the major axis X.) The energy source 1720 is then operated at (any) modulation frequency, as discussed above, during the dwell time period. The dwell time may be different for each secondary filter 60 (or each wavelength or band to which the tunable filter or interferometer is tuned). In one embodiment of the detection system 1700, the dwell time for each secondary filter 60 is less than about 1 second. Use of a dwell time specific to each secondary filter 60 advantageously allows the detection system 1700 to operate for a longer period of time at wavelengths where errors can have a greater effect on the computation of the analyte concentration in the material sample S. Correspondingly, the detection system 1700 can operate for a shorter period of time at wavelengths where errors have less effect on the computed analyte concentration. The dwell times may otherwise be nonuniform among the filters/wavelengths/bands employed in the detection system.

For each secondary filter 60 selectively aligned with the major axis X, the sample detector 150 detects the portion of the sample beam (Es), at the wavelength or wavelength band corresponding to the secondary filter 60, that is transmitted through the material sample S. The sample detector 150 generates a detection signal corresponding to the detected electromagnetic radiation and passes the signal to the processor 210. Simultaneously, the reference detector 170 detects the reference beam (Er) transmitted at the wavelength or wavelength band corresponding to the secondary filter 60. The reference detector 170 generates a detection signal corresponding to the detected electromagnetic radiation and passes the signal to the processor 210. Based on the signals passed to it by the detectors 150, 170, the processor 210 computes the concentration of the analyte(s) of interest in the sample S, and/or the absorbance/transmittance characteristics of the sample S at one or more wavelengths or wavelength bands employed to analyze the sample. The processor 210 computes the concentration(s), absorbance(s), transmittance(s), etc. by executing a data processing algorithm or program instructions residing within the memory 212 accessible by the processor 210.

The signal generated by the reference detector may be used to monitor fluctuations in the intensity of the energy beam emitted by the source 1720, which fluctuations often arise due to drift effects, aging, wear or other imperfections in the source itself. This enables the processor 210 to identify changes in intensity of the sample beam (Es) that are attributable to changes in the emission intensity of the source 1720, and not to the composition of the sample S. By so doing, a potential source of error in computations of concentration, absorbance, etc. is minimized or eliminated.

In one embodiment, the detection system 1700 computes an analyte concentration reading by first measuring the electromagnetic radiation detected by the detectors 150, 170 at each center wavelength, or wavelength band, without the sample element 1730 present on the major axis X (this is known as an "air" reading). Second, the system 1700 measures the electromagnetic radiation detected by the detectors 150, 170 for each center wavelength, or wavelength band, with the material sample S present in the sample element 1730, and the sample element 1730 and sample S in position on the major axis X (i.e., a "wet" reading). Finally, the processor 180 computes the concentration(s), absorbance (s) and/or transmittances relating to the sample S based on these compiled readings.

In one embodiment, the plurality of air and wet readings are used to generate a pathlength corrected spectrum as follows. First, the measurements are normalized to give the transmission of the sample at each wavelength. Using both a signal and reference measurement at each wavelength, and letting $S_i$ represent the signal of detector 150 at wavelength i and $R_i$ represent the signal of detector 170 at wavelength i, the transmission, $\tau_i$ is computed as $\tau_i = S_i(wet)/R_i(wet) / S_i(air)/R_i(air)$. Optionally, the spectra may be calculated as the optical density, $OD_i$, as - $Log(T_i)$.

Next, the transmission over the wavelength range of approximately 4.5 $\mu$m to approximately 5.5 $\mu$m is analyzed to determine the pathlength. Specifically, since water is the primary absorbing species of blood over this wavelength region, and since the optical density is the product of the optical pathlength and the known absorption coefficient of water (OD = L $\sigma$, where L is the optical pathlength and $\sigma$ is the absorption coefficient), any one of a number of standard curve fitting procedures may be used to determine the optical pathlength, L from the measured OD. The pathlength may then be used to determine the absorption coefficient of the sample at each wavelength. Alternatively, the optical pathlength may be used in further calculations to convert absorption coefficients to optical density.

Additional information on analyte detection systems, methods of use thereof, and related technologies may be found in the above-mentioned and incorporated U.S. Patent Application Publication No. 2005/0038357, published on February 17, 2005, titled SAMPLE ELEMENT WITH BARRIER MATERIAL.

SECTION IV.C - SAMPLE ELEMENT

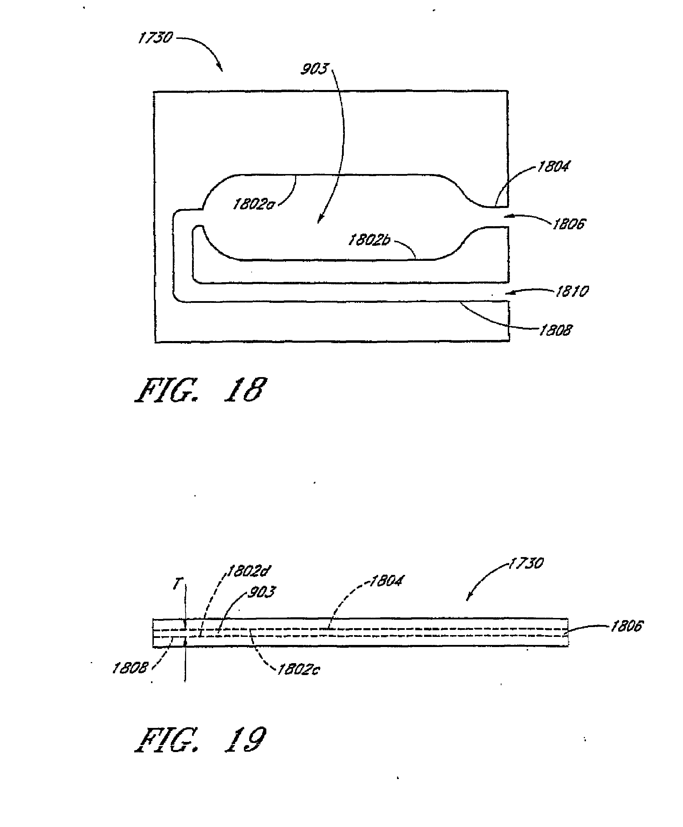

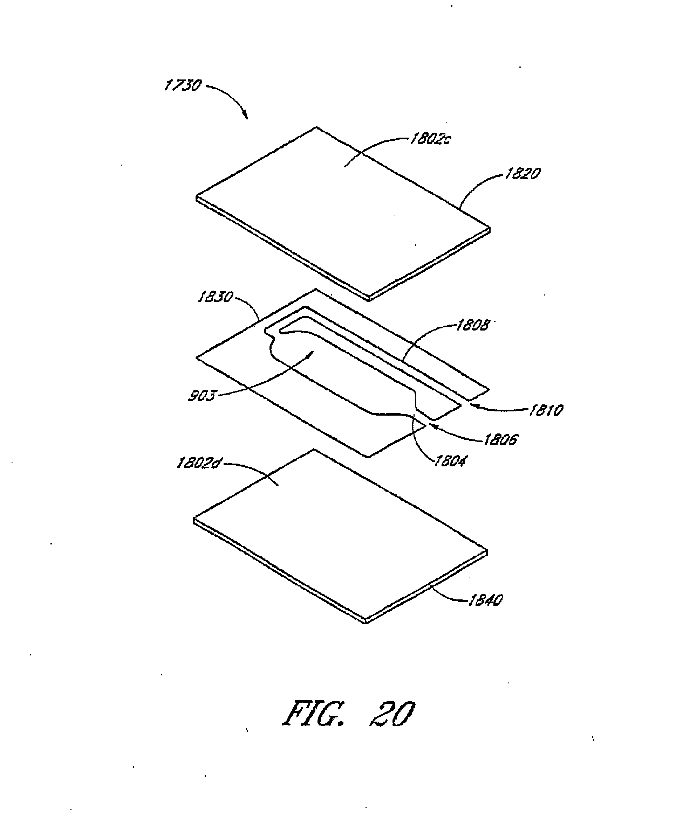

FIGURE 18 is a top view of a sample element 1730, FIGURE 19 is a side view of the sample element, and FIGURE 20 is an exploded perspective view of the sample element. In one embodiment of the present invention, sample element 1730 includes sample chamber 903 that is in fluid communication with and accepts filtered blood from sample preparation unit 332. The sample element 1730 comprises a sample chamber 903 defined by sample chamber walls 1802. The sample chamber 903 is configured to hold a material sample which may be drawn from a patient, for analysis by the detection system with which the sample element 1730 is employed.

In the embodiment illustrated in FIGURES 18-19, the sample chamber 903 is defined by first and second lateral chamber walls 1802a, 1802b and upper and lower chamber walls 1802c, 1802d; however, any suitable number and configuration of chamber walls may be employed. At least one of the upper and lower chamber walls 1802c, 1802d is formed from a material which is sufficiently transmissive of the wavelength(s) of electromagnetic radiation that are employed by the sample analysis apparatus 322 (or any other system with which the sample element is to be used). A chamber wall which is so transmissive may thus be termed a "window;" in one embodiment, the upper and lower chamber walls 1802c, 1802d comprise first and second windows so as to permit the relevant wavelength(s) of electromagnetic radiation to pass through the sample chamber 903. In another embodiment, only one of the upper and lower chamber walls 1802c, 1802d comprises a window; in such an embodiment, the other of the upper and lower chamber walls may comprise a reflective surface configured to back-reflect any electromagnetic energy emitted into the sample chamber 903 by the analyte detection system with which the sample element 1730 is employed. Accordingly, this embodiment is well suited for use with an analyte detection system in which a source and a detector of electromagnetic energy are located on the same side as the sample element.

In various embodiments, the material that makes up the window(s) of the sample element 1730 is completely transmissive, i.e., it does not absorb any of the electromagnetic radiation from the source 1720 and filters 1725 that is incident upon it. In another embodiment, the material of the window(s) has some absorption in the electromagnetic range of interest, but its absorption is negligible. In yet another embodiment, the absorption of the material of the window(s) is not negligible, but it is stable for a relatively long period of time. In another embodiment, the absorption of the window(s) is stable for only a relatively short period of time, but sample analysis apparatus 322 is configured to observe the absorption of the material and eliminate it from the analyte measurement before the material properties can change measurably. Materials suitable for forming the window(s) of the sample element 1730 include, but are not limited to, calcium fluoride, barium fluoride, germanium, silicon, polypropylene, polyethylene, or any polymer with suitable transmissivity (i.e., transmittance per unit thickness) in the relevant wavelength(s). Where the window(s) are formed from a polymer, the selected polymer can be isotactic, atactic or syndiotactic in structure, so as to enhance the flow of the sample between the window(s). One type of polyethylene suitable for constructing the sample element 1730 is type 220, extruded or blow molded, available from KUBE Ltd. of Staefa, Switzerland.

In one embodiment, the sample element 1730 is configured to allow sufficient transmission of electromagnetic energy having a wavelength of between about 4 $\mu$m and about 10.5 $\mu$m through the window(s) thereof. However, the sample element 1730 can be configured to allow transmission of wavelengths in any spectral range emitted by the energy source 1720. In another embodiment, the sample element 1730 is configured to receive an optical power of more than about 1.0 MW/cm$^2$ from the sample beam (Es) incident thereon for any electromagnetic radiation wavelength transmitted through the filter 1725. Preferably, the sample chamber 903 of the sample element 1730 is configured to allow a sample beam (Es) advancing toward the material sample S within a cone angle of 45 degrees from the major axis X (see FIGURE 17) to pass therethrough.

In the embodiment illustrated in FIGURES 18-19, the sample element further comprises a supply passage 1804 extending from the sample chamber 903 to a supply opening 1806 and a vent passage 1808 extending from the sample chamber 903 to a vent opening 1810. While the vent and supply openings 1806, 1810 are shown at one end of the sample element 1730, in other embodiments the openings may be positioned on other sides of the sample element 1730, so long as it is in fluid communication with the passages 1804 and 1808, respectively.

In operation, the supply opening 1806 of the sample element 1730 is placed in contact with the material sample S, such as a fluid flowing from a patient. The fluid is then transported through the sample supply passage 1804 and into the sample chamber 903 via an external pump or by capillary action.

Where the upper and lower chamber walls 1802c, 1802d comprise windows, the distance T (measured along an axis substantially orthogonal to the sample chamber 903 and/or windows 1802a, 1802b, or, alternatively, measured along an axis of an energy beam (such as but not limited to the energy beam E discussed above) passed through the sample chamber 903) between them comprises an optical pathlength. In various embodiments, the pathlength is between about 1 $\mu$m and about 300 $\mu$m, between about 1 $\mu$m and about 100 $\mu$m, between about 25 $\mu$m and about 40 $\mu$m, between about 10 $\mu$m and about 40 $\mu$m, between about 25 $\mu$m and about 60 $\mu$m, or between about 30 $\mu$m and about 50 $\mu$m. In still other embodiments, the optical pathlength is about 50 $\mu$m, or about 25 $\mu$m. In some instances, it is desirable to hold the pathlength T to within about plus or minus 1 $\mu$m from any pathlength specified by the analyte detection system with which the sample element 1730 is to be employed. Likewise, it may be desirable to orient the walls 1802c, 1802d with respect to each other within plus or minus 1 $\mu$m of parallel, and/or to maintain each of the walls 1802c, 1802d to within plus or minus 1 $\mu$m of planar (flat), depending on the analyte detection system with which the sample element 1730 is to be used. In alternative embodiments, walls 1802c, 1802d are flat, textured, angled, or some combination thereof.

In one embodiment, the transverse size of the sample chamber 903 (i.e., the size defined by the lateral chamber walls 1802a, 1802b) is about equal to the size of the active surface of the sample detector 1745. Accordingly, in a further embodiment the sample chamber 903 is round with a diameter of about 4 millimeter to about 12 millimeter, and more preferably from about 6 millimeter to about 8 millimeter.

The sample element 1730 shown in FIGURES 18-19 has, in one embodiment, sizes and dimensions specified as follows. The supply passage 1804 preferably has a length of about 15 millimeter, a width of about 1.0 millimeter, and a height equal to the pathlength T. Additionally, the supply opening 1806 is preferably about 1.5 millimeter wide and smoothly transitions to the width of the sample supply passage 1804. The sample element 1730 is about 0.5 inches (12 millimeters) wide and about one inch (25 millimeters) long with an overall thickness of between about 1.0 millimeter and about 4.0 millimeter. The vent passage 1808 preferably has a length of about 1.0 millimeter to 5.0 millimeter and a width of about 1.0 millimeter, with a thickness substantially equal to the pathlength between the walls 1802c, 1802d. The vent aperture 1810 is of substantially the same height and width as the vent passage 1808. Of course, other dimensions may be employed in other embodiments while still achieving the advantages of the sample element 1730.

The sample element 1730 is preferably sized to receive a material sample S having a volume less than or equal to about 15 $\mu$L (or less than or equal to about 10 $\mu$L, or less than or equal to about 5 $\mu$L) and more preferably a material sample S having a volume less than or equal to about 2 $\mu$L. Of course, the volume of the sample element 1730, the volume of the sample chamber 903, etc. can vary, depending on many variables, such as the size and sensitivity of the sample detector 1745, the intensity of the radiation emitted by the energy source 1720, the expected flow properties of the sample, and whether flow enhancers are incorporated into the sample element 1730. The transport of fluid to the sample chamber 903 is achieved preferably through capillary action, but may also be achieved through wicking or vacuum action, or a combination of wicking, capillary action, peristaltic, pumping, and/or vacuum action.

FIGURE 20 depicts one approach to constructing the sample element 1730. In this approach, the sample element 1730 comprises a first layer 1820, a second layer 1830, and a third layer 1840. The second layer 1830 is preferably positioned between the first layer 1820 and the third layer 1840. The first layer 1820 forms the upper chamber wall 1802c, and the third layer 1840 forms the lower chamber wall 1802d. Where either of the chamber walls 1802c, 1802d comprises a window, the window(s)/wall(s) 1802c/1802d in question may be formed from a different material as is employed to form the balance of the layer(s) 1820/1840 in which the wall(s) are located. Alternatively, the entirety of the layer(s) 1820/1840 may be formed of the material selected to form the window(s)/wall(s) 1802c, 1802d. In this case, the window(s)/wall(s) 1802c, 1802d are integrally formed with the layer(s) 1820, 1840 and simply comprise the regions of the respective layer(s) 1820, 1840 which overlie the sample chamber 903.

With further reference to FIGURE 20, second layer 1830 may be formed entirely of an adhesive that joins the first and third layers 1820, 1840. In other embodiments, the second layer 1830 may be formed from similar materials as the first and third layers, or any other suitable material. The second layer 1830 may also be formed as a carrier with an adhesive deposited on both sides thereof. The second layer 1830 includes voids which at least partially form the sample chamber 903, sample supply passage 1804, supply opening 1806, vent passage 1808, and vent opening 1810. The thickness of the second layer 1830 can be the same as any of the pathlengths disclosed above as suitable for the sample element 1730. The first and third layers can be formed from any of the materials disclosed above as suitable for forming the window(s) of the sample element 1730. In one embodiment, layers 1820, 1840 are formed from material having sufficient structural integrity to maintain its shape when filled with a sample S. Layers 1820, 1830 may be, for example, calcium fluoride having a thickness of 0.5 millimeter. In another embodiment, the second layer 1830 comprises the adhesive portion of Adhesive Transfer Tape no. 9471LE available from 3M Corporation. In another embodiment, the second layer 1830 comprises an epoxy, available, for example, from TechFilm (31 Dunham Road, Billerica, MA 01821), that is bound to layers 1820, 1840 as a result of the application of pressure and heat to the layers.

The sample chamber 903 preferably comprises a reagentless chamber. In other words, the internal volume of the sample chamber 903 and/or the wall(s) 1802 defining the chamber 903 are preferably inert with respect to the sample to be drawn into the chamber for analysis. As used herein, "inert" is a broad term and is used in its ordinary sense and includes, without limitation, substances which will not react with the sample in a manner which will significantly affect any measurement made of the concentration of analyte(s) in the sample with sample analysis apparatus 322 or any other suitable system, for a sufficient time (e.g., about 1-30 minutes) following entry of the sample into the chamber 903, to permit measurement of the concentration of such analyte(s). Alternatively, the sample chamber 903 may contain one or more reagents to facilitate use of the sample element in sample assay techniques which involve reaction of the sample with a reagent.

In one embodiment of the present invention, sample element 1730 is used for a limited number of measurements and is disposable. Thus, for example, with reference to FIGURES 8-10, sample element 1730 forms a disposable portion of cassette 820 adapted to place sample chamber 903 within probe region 1002.

Additional information on sample elements, methods of use thereof, and related technologies may be found in the above-mentioned and incorporated U.S. Patent Application Publication No. 2005/0038357, published on February 17, 2005, titled SAMPLE ELEMENT WITH BARRIER MATERIAL; and in the above-mentioned and incorporated U.S. Patent Application No. 11/122,794, filed on May 5, 2005, titled SAMPLE ELEMENT WITH SEPARATOR.

SECTION IV.D - CENTRIFUGE

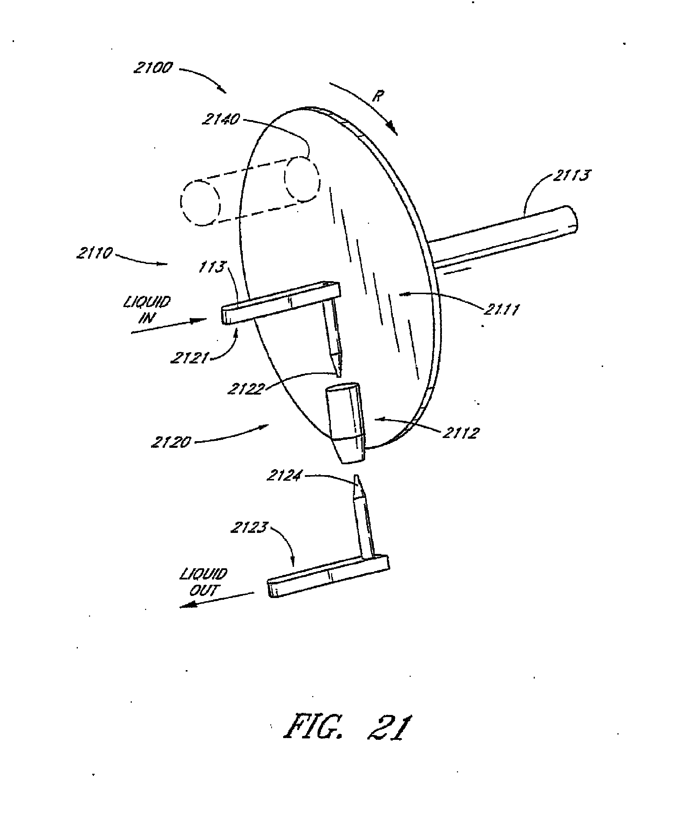

FIGURE 21 is a schematic of one embodiment of a sample preparation unit 2100 utilizing a centrifuge and which may be generally similar to the sample preparation unit 332, except as further detailed below. In general, the sample preparation unit 332 includes a centrifuge in place of, or in addition to a filter, such as the filter 1500. Sample preparation unit 2100 includes a fluid handling element in the form of a centrifuge 2110 having a sample element 2112 and a fluid interface 2120. Sample element 2112 is illustrated in FIGURE 21 as a somewhat cylindrical element. This embodiment is illustrative, and the sample element may be cylindrical, planar, or any other shape or configuration that is compatible with the function of holding a material (preferably a liquid) in the centrifuge 2110. The centrifuge 2110 can be used to rotate the sample element 2112 such that the material held in the sample element 2112 is separated.

In some embodiments, the fluid interface 2120 selectively controls the transfer of a sample from the passageway 113 and into the sample element 2112 to permit centrifuging of the sample. In another embodiment, the fluid interface 2120 also permits a fluid to flow though the sample element 2112 to cleanse or otherwise prepare the sample element for obtaining an analyte measurement. Thus, the fluid interface 2120 can be used to flush and fill the sample element 2112.

As shown in FIGURE 21, the centrifuge 2110 comprises a rotor 2111 that includes the sample element 2112 and an axle 2113 attached to a motor, not shown, which is controlled by the controller 210. The sample element 2112 is preferably generally similar to the sample element 1730 except as described subsequently.

As is further shown in FIGURE 21, fluid interface 2120 includes a fluid injection probe 2121 having a first needle 2122 and a fluid removal probe 2123. The fluid removal probe 2123 has a second needle 2124. When sample element 2112 is properly oriented relative to fluid interface 2120, a sample, fluid, or other liquid is dispensed into or passes through the sample element 2112. More specifically, fluid injection probe 2121 includes a passageway to receive a sample, such as a bodily fluid from the patient connector 110. The bodily fluid can be passed through the fluid injection probe 2121 and the first needle 2122 into the sample element 2112. To remove material from the sample element 2112, the sample 2112 can be aligned with the second needle 2124, as illustrated. Material can be passed through the second needle 2124 into the fluid removal probe 2123. The material can then pass through a passageway of the removal probe 2123 away from the sample element 2112.

One position that the sample element 2112 may be rotated through or to is a sample measurement location 2140. The location 2140 may coincide with a region of an analysis system, such as an optical analyte detection system. For example, the location 2140 may coincide with a probe region 1002, or with a measurement location of another apparatus.

The rotor 2111 may be driven in a direction indicated by arrow R, resulting in a centrifugal force on sample(s) within sample element 2112. The rotation of a sample(s) located a distance from the center of rotation creates centrifugal force. In some embodiments, the sample element 2112 holds whole blood. The centrifugal force may cause the denser parts of the whole blood sample to move further out from the center of rotation than lighter parts of the blood sample. As such, one or more components of the whole blood can be separated from each other. Other fluids or samples can also be removed by centrifugal forces. In one embodiment, the sample element 2112 is a disposable container that is mounted on to a disposable rotor 2111. Preferably, the container is plastic, reusable and flushable. In other embodiments, the sample element 2112 is a non-disposable container that is permanently attached to the rotor 2111.

The illustrated rotor 2111 is a generally circular plate that is fixedly coupled to the axle 2113. The rotor 2111 can alternatively have other shapes. The rotor 2111 preferably comprises a material that has a low density to keep the rotational inertia low and that is sufficiently strong and stable to maintain shape under operating loads to maintain close optical alignment. For example, the rotor 2111 can be comprised of GE brand ULTEM (trademark) polyetherimide (PEI). This material is available in a plate form that is stable but can be readily machined. Other materials having similar properties can also be used.

The size of the rotor 2111 can be selected to achieve the desired centrifugal force. In some embodiments, the diameter of rotor 2111 is from about 75 millimeters to about 125 millimeters, or more preferably from about 100 millimeters to about 125 millimeters. The thickness of rotor 2111 is preferably just thick enough to support the centrifugal forces and can be, for example, from about 1.0 to 2.0 millimeter thick.

In an alternative embodiment, the fluid interface 2120 selectively removes blood plasma from the sample element 2112 after centrifuging. The blood plasma is then delivered to an analyte detection system for analysis. In one embodiment, the separated fluids are removed from the sample element 2112 through the bottom connector. Preferably, the location and orientation of the bottom connector and the container allow the red blood cells to be removed first. One embodiment may be configured with a red blood cell detector. The red blood cell detector may detect when most of the red blood cells have exited the container by determining the haemostatic level. The plasma remaining in the container may then be diverted into the analysis chamber. After the fluids have been removed from the container, the top connector may inject fluid (e.g., saline) into the container to flush the system and prepare it for the next sample.

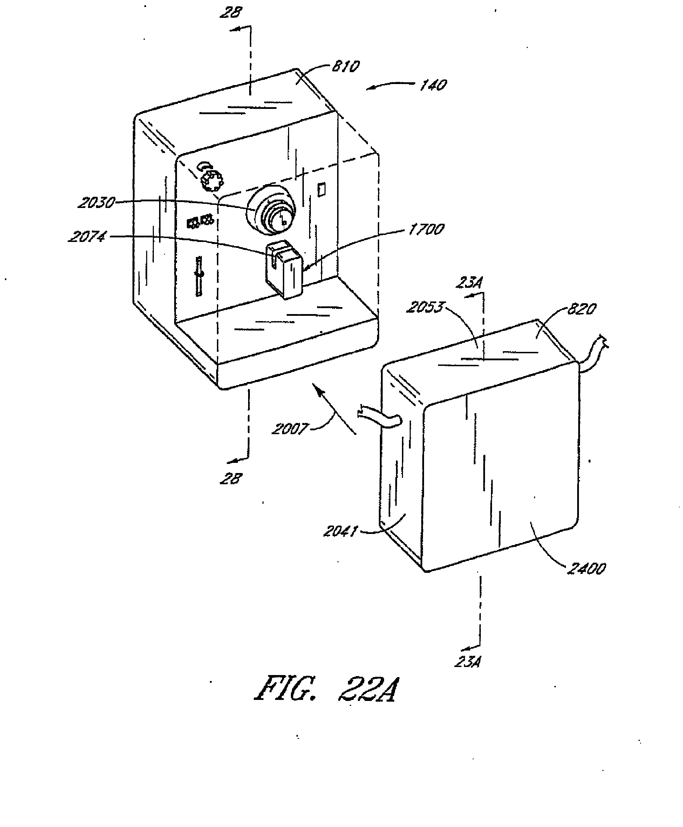

FIGURES 22A to 23C illustrate another embodiment of a fluid handling and analysis apparatus 140, which employs a removable, disposable fluid handling cassette 820. The cassette 820 is equipped with a centrifuge rotor assembly 2016 to facilitate preparation and analysis of a sample. Except as further described below, the apparatus 140 of FIGURES 22A-22C can in certain embodiments be similar to any of the other embodiments of the apparatus 140 discussed herein, and the cassette 820 can in certain embodiments be similar to any of the embodiments of the cassettes 820 disclosed herein.

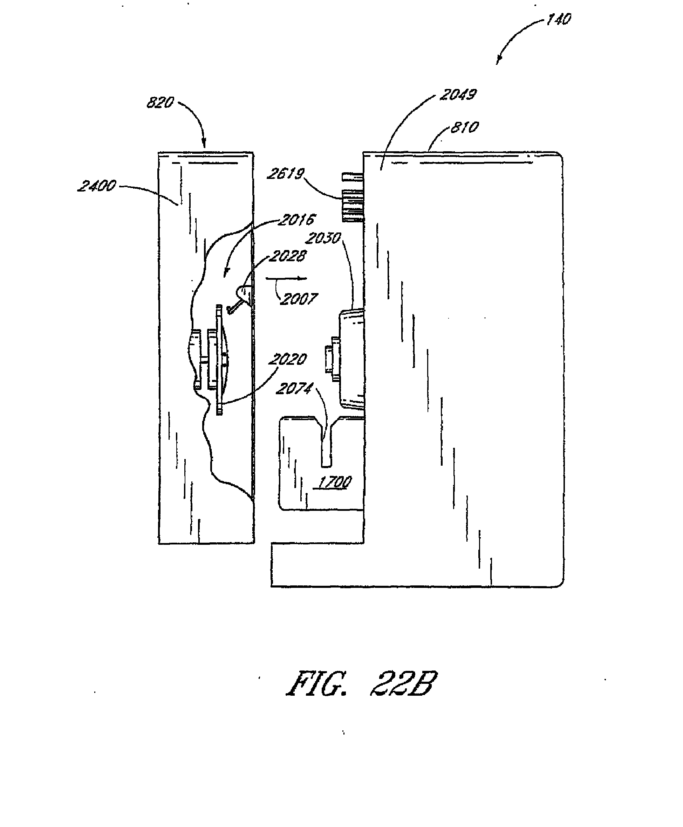

The removable fluid handling cassette 820 can be removably engaged with a main analysis instrument 810. When the fluid handling cassette 820 is coupled to the main instrument 810, a drive system 2030 of the main instrument 810 mates with the rotor assembly 2016 of the cassette 820 (FIGURE 22B). Once the cassette 820 is coupled to the main instrument 810, the drive system 2030 engages and can rotate the rotor assembly 2016 to apply a centrifugal force to a body fluid sample carried by the rotor assembly 2016.

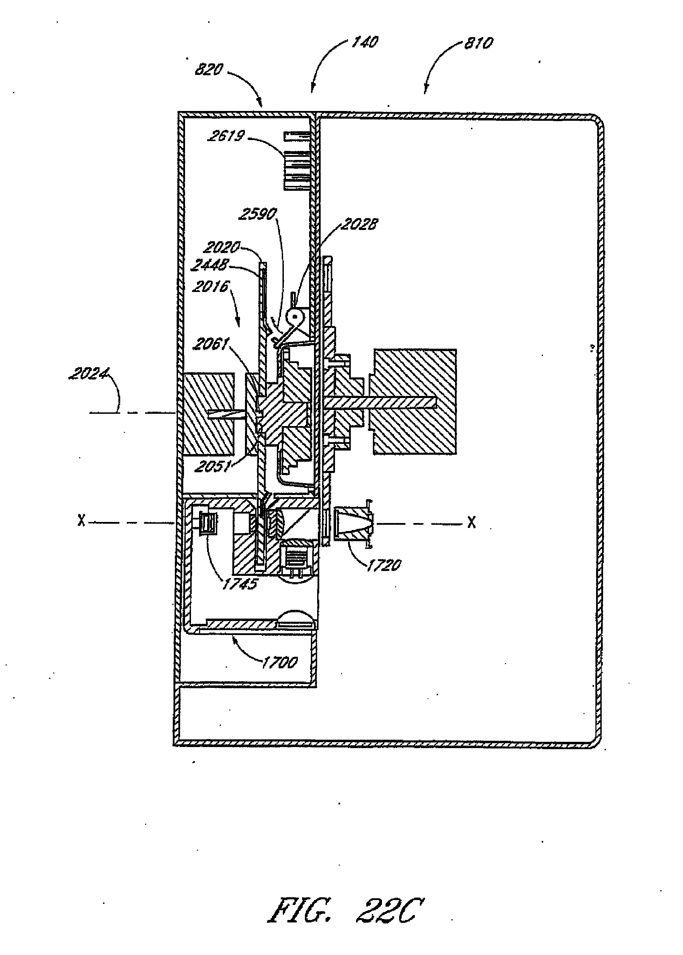

In some embodiments, the rotor assembly 2016 includes a rotor 2020 sample element 2448 (FIGURE 22C) for holding a sample for centrifuging. When the rotor 2020 is rotated, a centrifugal force is applied to the sample contained within the sample element 2448. The centrifugal force causes separation of one or more components of the sample (e.g., separation of plasma from whole blood). The separated component(s) can then be analyzed by the apparatus 140, as will be discussed in further detail below.

The main instrument 810 includes both the centrifuge drive system 2030 and an analyte detection system 1700, a portion of which protrudes from a housing 2049 of the main instrument 810. The drive system 2030 is configured to releasably couple with the rotor assembly 2016, and can impart rotary motion to the rotor assembly 2016 to rotate the rotor 2020 at a desired speed. After the centrifuging process, the analyte detection system 1700 can analyze one or more components separated from the sample carried by the rotor 2020. The projecting portion of the illustrated detection system 1700 forms a slot 2074 for receiving a portion of the rotor 2020 carrying the sample element 2448 so that the detection system 1700 can analyze the sample or component(s) carried in the sample element 2448.

To assemble the fluid handling and analysis apparatus 140 as shown in FIGURE 22C, the cassette 820 is placed on the main instrument 810, as indicated by the arrow 2007 of FIGURES 22A and 22B. The rotor assembly 2016 is accessible to the drive system 2030, so that once the cassette 820 is properly mounted on the main instrument 810, the drive system 2030 is in operative engagement with the rotor assembly 2016. The drive system 2030 is then energized to spin the rotor 2020 at a desired speed. The spinning rotor 2020 can pass repeatedly through the slot 2074 of the detection system 1700.

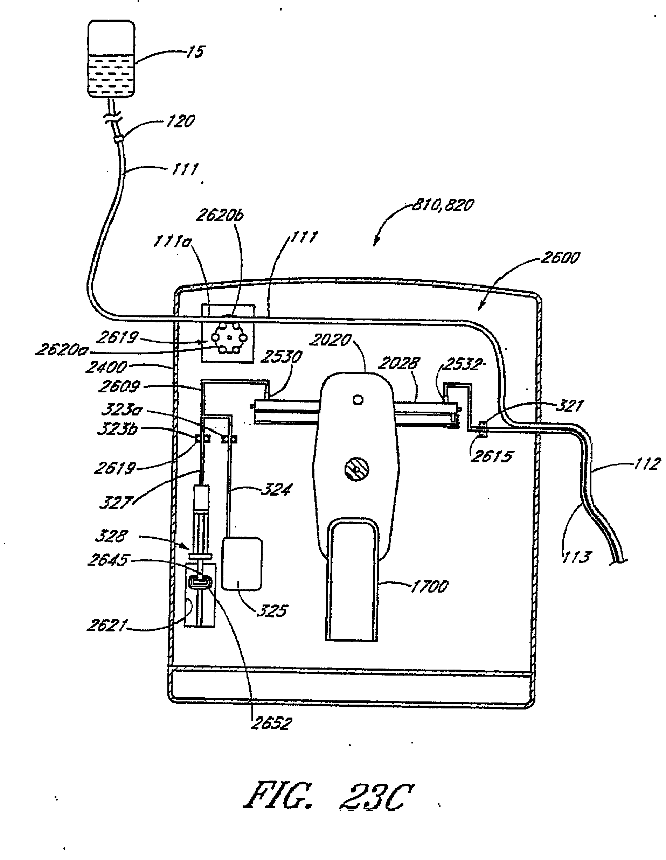

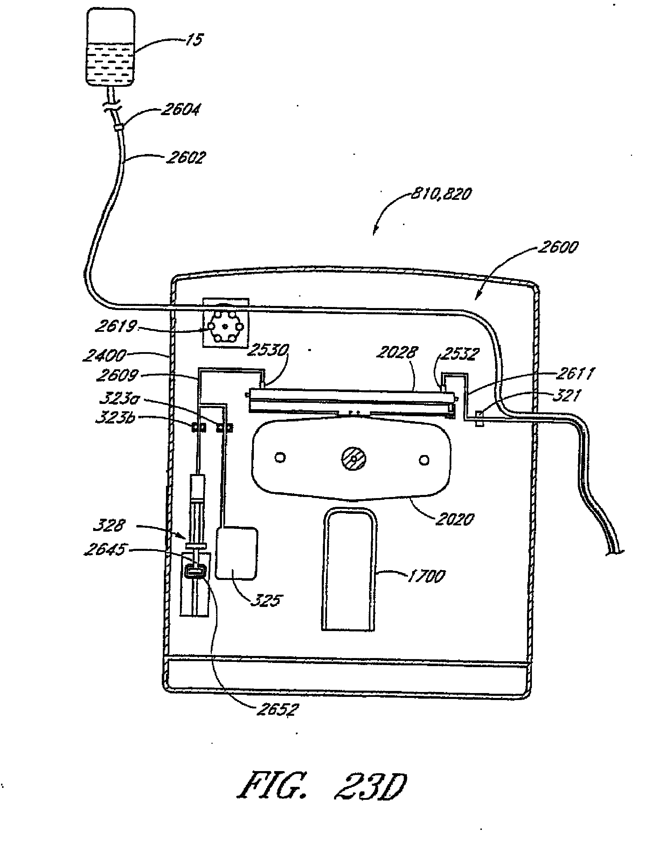

After the centrifuging process, the rotor 2020 is rotated to an analysis position (see FIGURES 22B and 23C) wherein the sample element 2448 is positioned within the slot 2074. With the rotor 2020 and sample element 2448 in the analysis position, the analyte detection system 1700 can analyze one or more of the components of the sample carried in the sample element 2448. For example, the detection system 1700 can analyze at least one of the components that is separated out during the centrifuging process. After using the cassette 820, the cassette 820 can be removed from the main instrument 810 and discarded. Another cassette 820 can then be mounted to the main instrument 810.

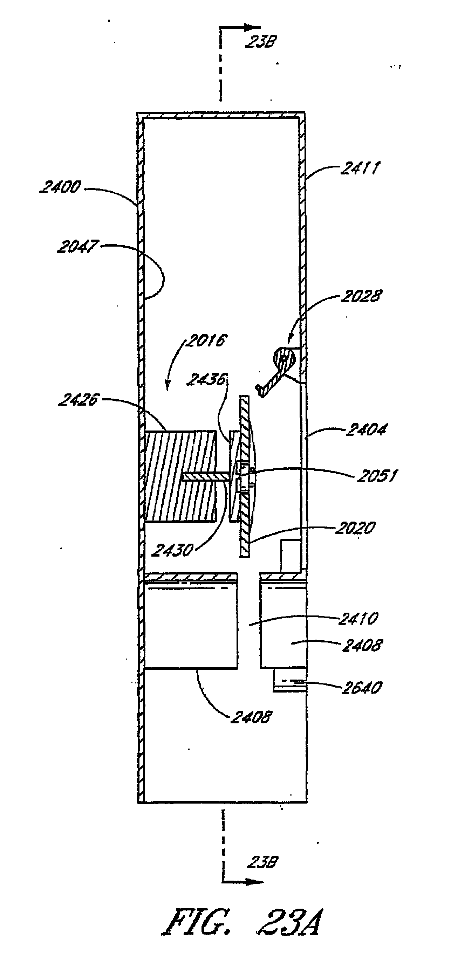

With reference to FIGURE 23A, the illustrated cassette 820 includes the housing 2400 that surrounds the rotor assembly 2016, and the rotor 2020 is pivotally connected to the housing 2400 by the rotor assembly 2016. The rotor 2020 includes a rotor interface 2051 for driving engagement with the drive system 2030 upon placement of the cassette 820 on the main instrument 810.

In some embodiments, the cassette 820 is a disposable fluid handling cassette. The reusable main instrument 810 can be used with any number of cassettes 820 as desired. Additionally or alternatively, the cassette 820 can be a portable, handheld cassette for convenient transport. In these embodiments, the cassette 820 can be manually mounted to or removed from the main instrument 810. In some embodiments, the cassette 820 may be a non disposable cassette which can be permanently coupled to the main instrument 810.

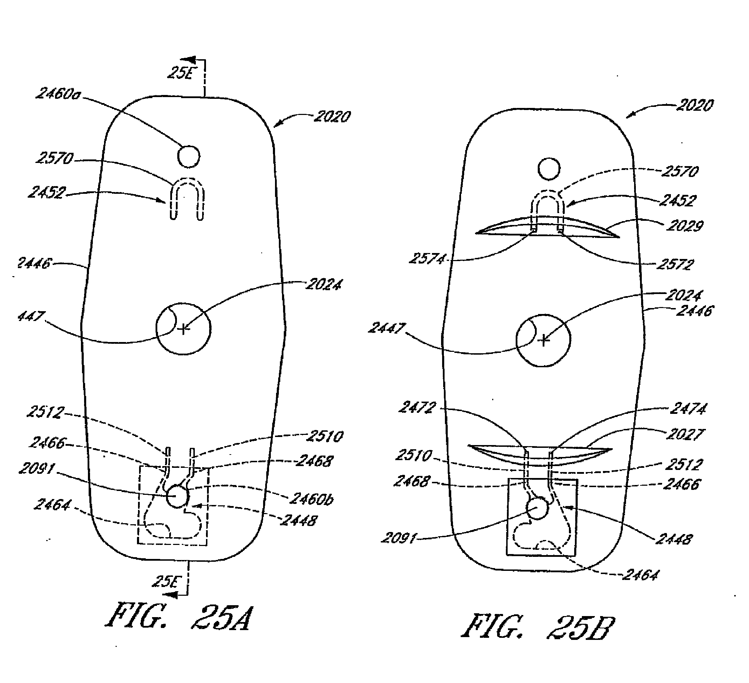

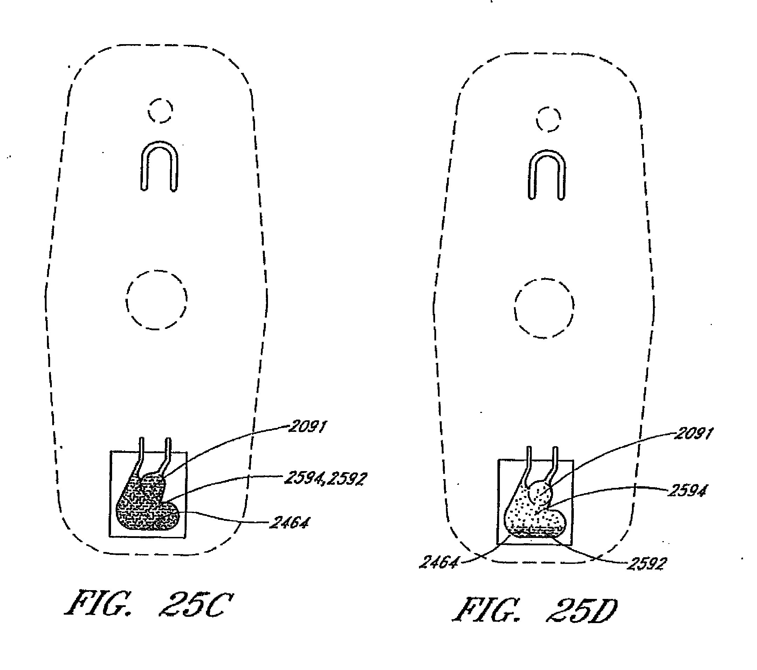

FIGURES 25A and 25B illustrate the centrifugal rotor 2020, which is capable of carrying a sample, such as bodily fluid. Thus, the illustrated centrifugal rotor 2020 can be considered a fluid handling element that can prepare a sample for analysis, as well as hold the sample during a spectroscopic analysis. The rotor 2020 preferably comprises an elongate body 2446, at least one sample element 2448, and at least one bypass element 2452. The sample element 2448 and bypass element 2452 can be located at opposing ends of the rotor 2020. The bypass element 2452 provides a bypass flow path that can be used to clean or flush fluid passageways of the fluid handling and analysis apparatus 140 without passing fluid through the sample element 2448.

The illustrated rotor body 2446 can be a generally planar member that defines a mounting aperture 2447 for coupling to the drive system 2030. The illustrated rotor 2020 has a somewhat rectangular shape. In alternative embodiments, the rotor 2020 is generally circular, polygonal, elliptical, or can have any other shape as desired. The illustrated shape can facilitate loading when positioned horizontally to accommodate the analyte detection system 1700.

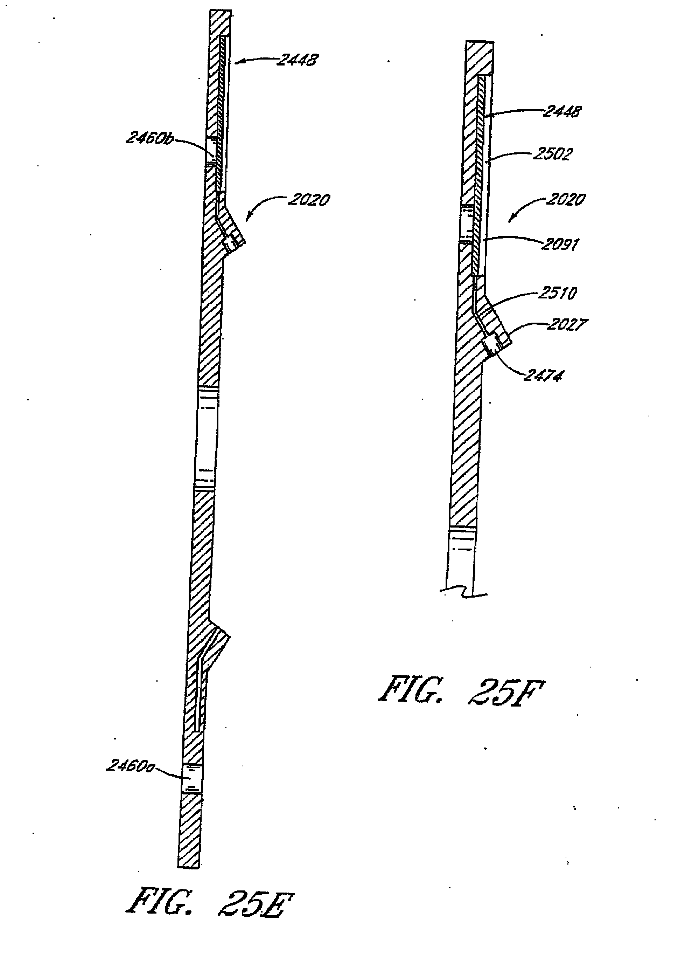

With reference to FIGURE 25B, a pair of opposing first and second fluid connectors 2027, 2029 extends outwardly from a front face of the rotor 2020, to facilitate fluid flow through the rotor body 2446 to the sample element 2448 and bypass element 2452, respectively. The first fluid connector 2027 defines an outlet port 2472 and an inlet port 2474 that are in fluid communication with the sample element 2448. In the illustrated embodiment, fluid channels 2510, 2512 extend from the outlet port 2472 and inlet port 2474, respectively, to the sample element 2448. (See FIGURES 25E and 25F.) As such, the ports 2472, 2474 and channels 2510, 2512 define input and return flow paths through the rotor 2020 to the sample element 2448 and back.

With continued reference to FIGURE 25B, the rotor 2020 includes the bypass element 2452 which permits fluid flow therethrough from an outlet port 2572 to the inlet port 2574. A channel 2570 extends between the outlet port 2572 and the inlet port 2574 to facilitate this fluid flow. The channel 2570 thus defines a closed flow path through the rotor 2020 from one port 2572 to the other port 2574. In the illustrated embodiment, the outlet port 2572 and inlet port 2574 of the bypass element 2452 have generally the same spacing therebetween on the rotor 2020 as the outlet port 2472 and the inlet port 2474.

One or more windows 2460a, 2460b can be provided for optical access through the rotor 2020. A window 2460a proximate the bypass element 2452 can be a through-hole (see FIGURE 25E) that permits the passage of electromagnetic radiation through the rotor 2020. A window 2460b proximate the sample element 2448 can also be a similar through-hole which permits the passage of electromagnetic radiation. Alternatively, one or both of the windows 2460a, 2460b can be a sheet constructed of calcium fluoride, barium fluoride, germanium, silicon, polypropylene, polyethylene, combinations thereof, or any material with suitable transmissivity (i.e., transmittance per unit thickness) in the relevant wavelength(s). The windows 2460a, 2460b are positioned so that one of the windows 2460a, 2460b is positioned in the slot 2074 when the rotor 2020 is in a vertically orientated position.

Various fabrication techniques can be used to form the rotor 2020. In some embodiments, the rotor 2020 can be formed by molding (e.g., compression or injection molding), machining, or a similar production process or combination of production processes. In some embodiments, the rotor 2020 is comprised of plastic. The compliance of the plastic material can be selected to create the seal with the ends of pins 2542, 2544 of a fluid interface 2028 (discussed in further detail below). Non-limiting exemplary plastics for forming the ports (e.g., ports 2572, 2574, 2472, 2474) can be relatively chemically inert and can be injection molded or machined. These plastics include, but are not limited to, PEEK and polyphenylenesulfide (PPS). Although both of these plastics have high modulus, a fluidic seal can be made if sealing surfaces are produced with smooth finish and the sealing zone is a small area where high contact pressure is created in a very small zone. Accordingly, the materials used to form the rotor 2020 and pins 2542, 2544 can be selected to achieve the desired interaction between the rotor 2020 and the pins 2542, 2544, as described in detail below.

The illustrated rotor assembly 2016 of FIGURE 23A rotatably connects the rotor 2020 to the cassette housing 2400 via a rotor axle boss 2426 which is fixed with respect to the cassette housing and pivotally holds a rotor axle 2430 and the rotor 2020 attached thereto. The rotor axle 2430 extends outwardly from the rotor axle boss 2426 and is fixedly attached to a rotor bracket 2436, which is preferably securely coupled to a rear face of the rotor 2020. Accordingly, the rotor assembly 2016 and the drive system 2030 cooperate to ensure that the rotor 2020 rotates about the axis 2024, even at high speeds. The illustrated cassette 820 has a single rotor assembly 2016. In other embodiments, the cassette 820 can have more than one rotor assembly 2016. Multiple rotor assemblies 2016 can be used to prepare (preferably simultaneously) and test multiple samples.

With reference again to FIGURES 25A, 25B, 25E and 25F, the sample element 2448 is coupled to the rotor 2020 and can hold a sample of body fluid for processing with the centrifuge. The sample element 2448 can, in certain embodiments, be generally similar to other sample elements or cuvettes disclosed herein (e.g., sample elements 1730, 2112) except as further detailed below.

The sample element 2448 comprises a sample chamber 2464 that holds a sample for centrifuging, and fluid channels 2466, 2468, which provide fluid communication between the chamber 2464 and the channels 2512, 2510, respectively, of the rotor 2020. Thus, the fluid channels 2512, 2466 define a first flow path between the port 2474 and the chamber 2464, and the channels 2510, 2468 define a second flow path between the port 2472 and the chamber 2464. Depending on the direction of fluid flow into the sample element 2448, either of the first or second flow paths can serve as an input flow path, and the other can serve as a return flow path.

A portion of the sample chamber 2464 can be considered an interrogation region 2091, which is the portion of the sample chamber through which electromagnetic radiation passes during analysis by the detection system 1700 of fluid contained in the chamber 2464. Accordingly, the interrogation region 2091 is aligned with the window 2460b when the sample element 2448 is coupled to the rotor 2020. The illustrated interrogation region 2091 comprises a radially inward portion (i.e., relatively close to the axis of rotation 2024 of the rotor 2020) of the chamber 2464, to facilitate spectroscopic analysis of the lower density portion(s) of the body fluid sample (e.g., the plasma of a whole blood sample) after centrifuging, as will be discussed in greater detail below. Where the higher-density portions of the body fluid sample are of interest for spectroscopic analysis, the interrogation region 2091 can be located in a radially outward (i.e., further from the axis of rotation 2024 of the rotor 2020) portion of the chamber 2464.

The rotor 2020 can temporarily or permanently hold the sample element 2448. As shown in FIGURE 25F, the rotor 2020 forms a recess 2502 which receives the sample element 2448. The sample element 2448 can be held in the recess 2502 by frictional interaction, adhesives, or any other suitable coupling means. The illustrated sample element 2448 is recessed in the rotor 2020. However, the sample element 2448 can alternatively overlie or protrude from the rotor 2020.

The sample element 2448 can be used for a predetermined length of time, to prepare a predetermined amount of sample fluid, to perform a number of analyses, etc. If desired, the sample element 2448 can be removed from the rotor 2020 and then discarded. Another sample element 2448 can then be placed into the recess 2502. Thus, even if the cassette 820 is disposable, a plurality of disposable sample elements 2448 can be used with a single cassette 820. Accordingly, a single cassette 820 can be used with any number of sample elements as desired. Alternatively, the cassette 820 can have a sample element 2448 that is permanently coupled to the rotor 2020. In some embodiments, at least a portion of the sample element 2448 is integrally or monolithically formed with the rotor body 2446. Additionally or alternatively, the rotor 2020 can comprise a plurality of sample elements (e.g., with a record sample element in place of the bypass 2452). In this embodiment, a plurality of samples (e.g., bodily fluid) can be prepared simultaneously to reduce sample preparation time.

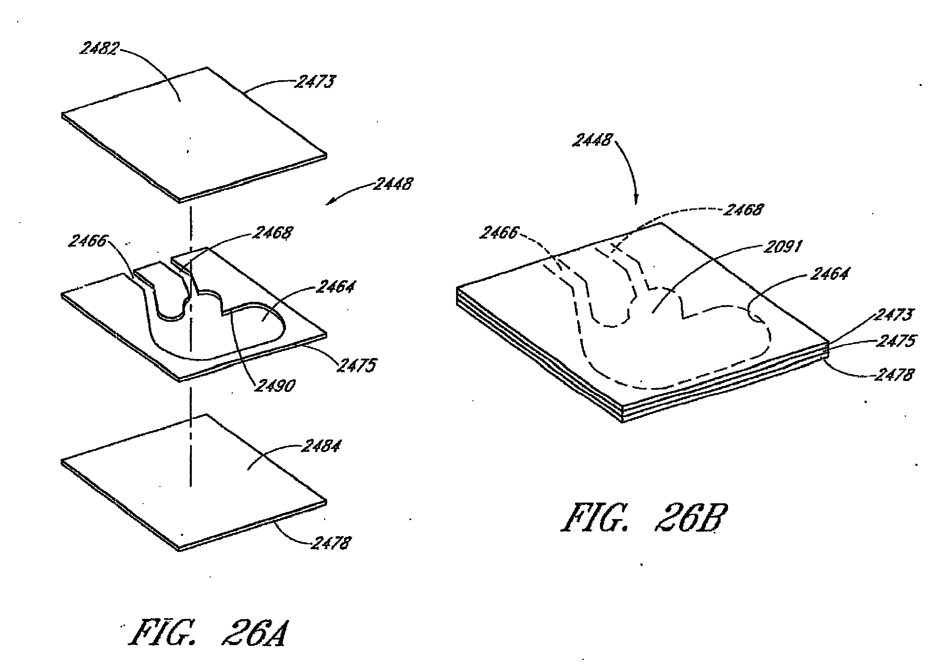

FIGURES 26A and 26B illustrate a layered construction technique which can be employed when forming certain embodiments of the sample element 2448. The depicted layered sample element 2448 comprises a first layer 2473, a second layer 2475, and a third layer 2478. The second layer 2475 is preferably positioned between the first layer 2473 and the third layer 2478. The first layer 2473 forms an upper chamber wall 2482, and the third layer 2478 forms a lower chamber wall 2484. A lateral wall 2490 of the second layer 2475 defines the sides of the chamber 2464 and the fluid channels 2466, 2468.

The second layer 2475 can be formed by die-cutting a substantially uniform-thickness sheet of a material to form the lateral wall pattern shown in FIGURE 26A. The second layer 2475 can comprise a layer of lightweight flexible material, such as a polymer material, with adhesive disposed on either side thereof to adhere the first and third layers 2473, 2478 to the second layer 2475 in "sandwich" fashion as shown in FIGURE 26B. Alternatively, the second layer 2475 can comprise an "adhesive-only" layer formed from a uniform-thickness sheet of adhesive which has been die-cut to form the depicted lateral wall pattern.

However constructed, the second layer 2475 is preferably of uniform thickness to define a substantially uniform thickness or path length of the sample chamber 2464 and/or interrogation region 2091. This path length (and therefore the thickness of the second layer 2475 as well) is preferably between 10 microns and 100 microns, or is 20, 40, 50, 60, or 80 microns, in various embodiments.

The upper chamber wall 2482, lower chamber wall 2484, and lateral wall 2490 cooperate to form the chamber 2464. The upper chamber wall 2482 and/or the lower chamber wall 2484 can permit the passage of electromagnetic energy therethrough. Accordingly, one or both of the first and third layers 2473, 2478 comprises a sheet or layer of material which is relatively or highly transmissive of electromagnetic radiation (preferably infrared radiation or mid-infrared radiation) such as barium fluoride, silicon, polyethylene or polypropylene. If only one of the layers 2473, 2478 is so transmissive, the other of the layers is preferably reflective, to back-reflect the incoming radiation beam for detection on the same side of the sample element 2448 as it was emitted. Thus the upper chamber wall 2482 and/or lower chamber wall 2484 can be considered optical window(s). These window(s) are disposed on one or both sides of the interrogation region 2091 of the sample element 2448.

In one embodiment, sample element 2448 has opposing sides that are transmissive of infrared radiation and suitable for making optical measurements as described, for example, in U.S. Patent Application Publication No. 2005/0036146, published February 17, 2005, titled SAMPLE ELEMENT QUALIFICATION, and hereby incorporated by reference and made a part of this specification. Except as further described herein, the embodiments, features, systems, devices, materials, methods and techniques described herein may, in some embodiments, be similar to any one or more of the embodiments, features, systems, devices, materials, methods and techniques described in U.S. Patent Application Publication No. 2003/0090649, published on May 15, 2003, titled REAGENT-LESS WHOLE-BLOOD GLUCOSE METER; or in U.S. Patent Application Publication No. 2003/0086075, published on May 8, 2003, titled DEVICE AND METHOD FOR IN VITRO DETERMINATION OF ANALYTE CONCENTRATIONS WITHIN BODY FLUIDS; or in U.S. Patent Application Publication No. 2004/0019431, published on January 29, 2004, titled METHOD OF DETERMINING AN ANALYTE CONCENTRATION IN A SAMPLE FROM AN ABSORPTION SPECTRUM, or in U.S. Patent No. 6,652,136, issued on November 25, 2003 to Marziali, titled METHOD OF SIMULTANEOUS MIXING OF SAMPLES. In addition, the embodiments, features, systems, devices, materials, methods and techniques described herein may, in certain embodiments, be applied to or used in connection with any one or more of the embodiments, features, systems, devices, materials, methods and techniques disclosed in the above-mentioned U.S. Patent Applications Publications Nos. 2003/0090649; 2003/0086075; 2004/0019431; or U.S. Patent No. 6,652,136. All of the above-mentioned publications and patent are hereby incorporated by reference herein and made a part of this specification.

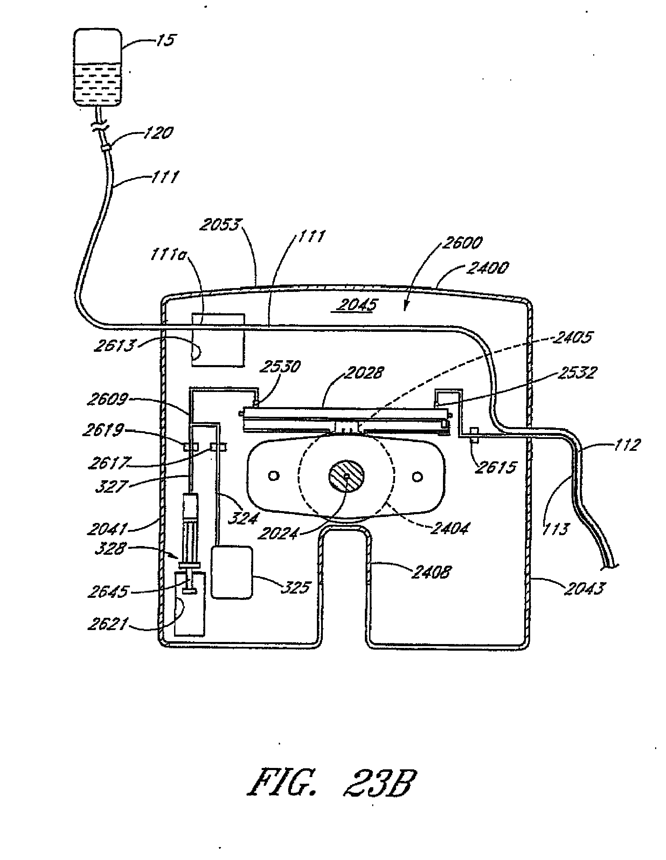

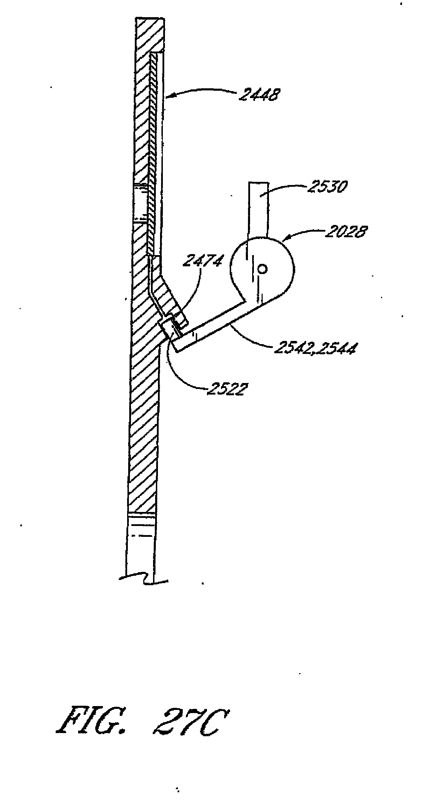

With reference to FIGURES 23B and 23C, the cassette 820 can further comprise the movable fluid interface 2028 for filling and/or removing sample liquid from the sample element 2448. In the depicted embodiment, the fluid interface 2028 is rotatably mounted to the housing 2400 of the cassette 820. The fluid interface 2028 can be actuated between a lowered position (FIGURE 22C) and a raised or filling position (FIGURE 27C). When the interface 2028 is in the lowered position, the rotor 2020 can freely rotate. To transfer sample fluid to the sample element 2448, the rotor 2020 can be held stationary and in a sample element loading position (see FIGURE 22C) the fluid interface 2028 can be actuated, as indicated by the arrow 2590, upwardly to the filling position. When the fluid interface 2028 is in the filling position, the fluid interface 2028 can deliver sample fluid into the sample element 2448 and/or remove sample fluid from the sample element 2448.

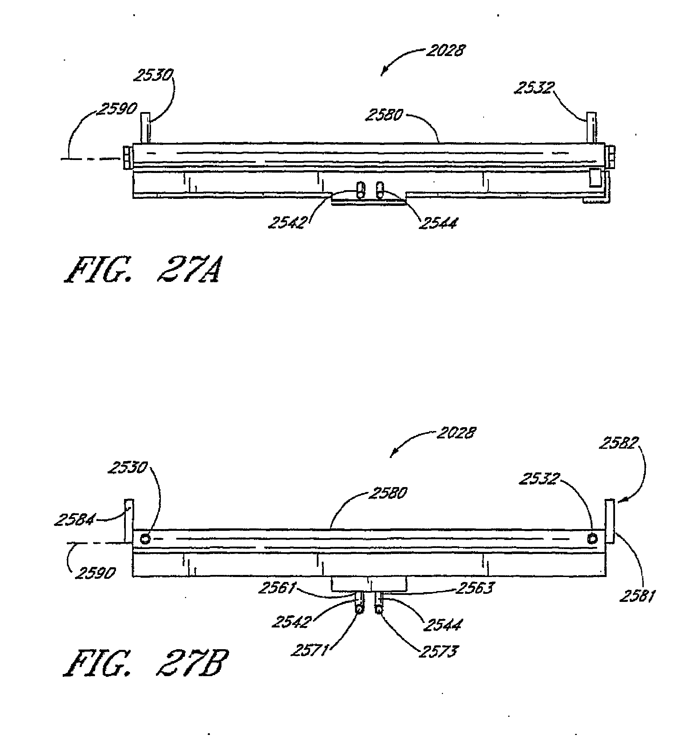

With continued reference to FIGURES 27A and 27B, the fluid interface 2028 has a main body 2580 that is rotatably mounted to the housing 2400 of the cassette 820. Opposing brackets 2581, 2584 can be employed to rotatably couple the main body 2580 to the housing 2400 of the cassette 820, and permit rotation of the main body 2580 and the pins 2542, 2544 about an axis of rotation 2590 between the lowered position and the filling position. The main instrument 810 can include a horizontally moveable actuator (not shown) in the form of a solenoid, pneumatic actuator, etc. which is extendible through an opening 2404 in the cassette housing 2400 (see FIG. 23B). Upon extension, the actuator strikes the main body 2580 of the fluid interface 2028, causing the body 2580 to rotate to the filling position shown in FIGURE 27C. The main body 2580 is preferably spring-biased towards the retracted position (shown in FIGURE 23A) so that retraction of the actuator allows the main body to return to the retracted position. The fluid interface 2028 can thus be actuated for periodically placing fluid passageways of the pins 2542, 2544 in fluid communication with a sample element 2448 located on the rotor 2020.

The fluid interface 2028 of FIGURES 27A and 23B includes fluid connectors 2530, 2532 that can provide fluid communication between the interface 2028 and one or more of the fluid passageways of the apparatus 140 and/or sampling system 100/800, as will be discussed in further detail below. The illustrated connectors 2530, 2532 are in an upwardly extending orientation and positioned at opposing ends of the main body 2580. The connectors 2530, 2532 can be situated in other orientations and/or positioned at other locations along the main body 2580. The main body 2580 includes a first inner passageway (not shown) which provides fluid communication between the connector 2530 and the pin 2542, and a second inner passageway (not shown) which provides fluid communication between the connector 2532 and the pin 2544.

The fluid pins 2542, 2544 extend outwardly from the main body 2580 and can engage the rotor 2020 to deliver and/or remove sample fluid to or from the rotor 2020. The fluid pins 2542, 2544 have respective pin bodies 2561, 2563 and pin ends 2571, 2573. The pin ends 2571, 2573 are sized to fit within corresponding ports 2472, 2474 of the fluid connector 2027 and/or the ports 2572, 2574 of the fluid connector 2029, of the rotor 2020. The pin ends 2571, 2573 can be slightly chamfered at their tips to enhance the sealing between the pin ends 2571, 2573 and rotor ports. In some embodiments, the outer diameters of the pin ends 2573, 2571 are slightly larger than the inner diameters of the ports of the rotor 2020 to ensure a tight seal, and the inner diameters of the pins 2542, 2544 are preferably identical or very close to the inner diameters of the channels 2510, 2512 leading from the ports. In other embodiments, the outer diameter of the pin ends 2571, 2573 are equal to or less than the inner diameters of the ports of the rotor 2020.

The connections between the pins 2542, 2544 and the corresponding portions of the rotor 2020, either the ports 2472, 2474 leading to the sample element 2448 or the ports 2572, 2574 leading to the bypass element 2452, can be relatively simple and inexpensive. At least a portion of the rotor 2020 can be somewhat compliant to help ensure a seal is formed with the pins 2542, 2544. Alternatively or additionally, sealing members (e.g., gaskets, O-rings, and the like) can be used to inhibit leaking between the pin ends 2571, 2573 and corresponding ports 2472, 2474, 2572, 2574.

FIGURES 23A and 23B illustrate the cassette housing 2400 enclosing the rotor assembly 2016 and the fluid interface 2028. The housing 2400 can be a modular body that defines an aperture or opening 2404 dimensioned to receive a drive system housing 2050 when the cassette 820 is operatively coupled to the main instrument 810. The housing 2400 can protect the rotor 2020 from external forces and can also limit contamination of samples delivered to a sample element in the rotor 2020, when the cassette 820 is mounted to the main instrument 810.

The illustrated cassette 820 has a pair of opposing side walls 2041, 2043, top 2053, and a notch 2408 for mating with the detection system 1700. A front wall 2045 and rear wall 2047 extend between the side walls 2041, 2043. The rotor assembly 2016 is mounted to the inner surface of the rear wall 2047. The front wall 2045 is configured to mate with the main instrument 810 while providing the drive system 2030 with access to the rotor assembly 2016.

The illustrated front wall 2045 has the opening 2404 that provides access to the rotor assembly 2016. The drive system 2030 can be passed through the opening 2404 into the interior of the cassette 820 until it operatively engages the rotor assembly 2016. The opening 2404 of FIGURE 23B is configured to mate and tightly surround the drive system 2030. The illustrated opening 2404 is generally circular and includes an upper notch 2405 to permit the fluid interface actuator of the main instrument 810 to access the fluid interface 2028, as discussed above. The opening 2404 can have other configurations suitable for admitting the drive system 2030 and actuator into the cassette 820.

The notch 2408 of the housing 2400 can at least partially surround the projecting portion of the analyte detection system 1700 when the cassette 820 is loaded onto the main instrument 810. The illustrated notch 2408 defines a cassette slot 2410 (FIGURE 23A) that is aligned with elongate slot 2074 shown in FIGURE 22C, upon loading of the cassette 820. The rotating rotor 2020 can thus pass through the aligned slots 2410, 2074. In some embodiments, the notch 2408 has a generally U-shaped axial cross section as shown. More generally, the configuration of the notch 2408 can be selected based on the design of the projecting portion of the detection system 1700.

Although not illustrated, fasteners, clips, mechanical fastening assemblies, snaps, or other coupling means can be used to ensure that the cassette 820 remains coupled to the main instrument 810 during operation. Alternatively, the interaction between the housing 2400 and the components of the main instrument 810 can secure the cassette 820 to the main instrument 810.

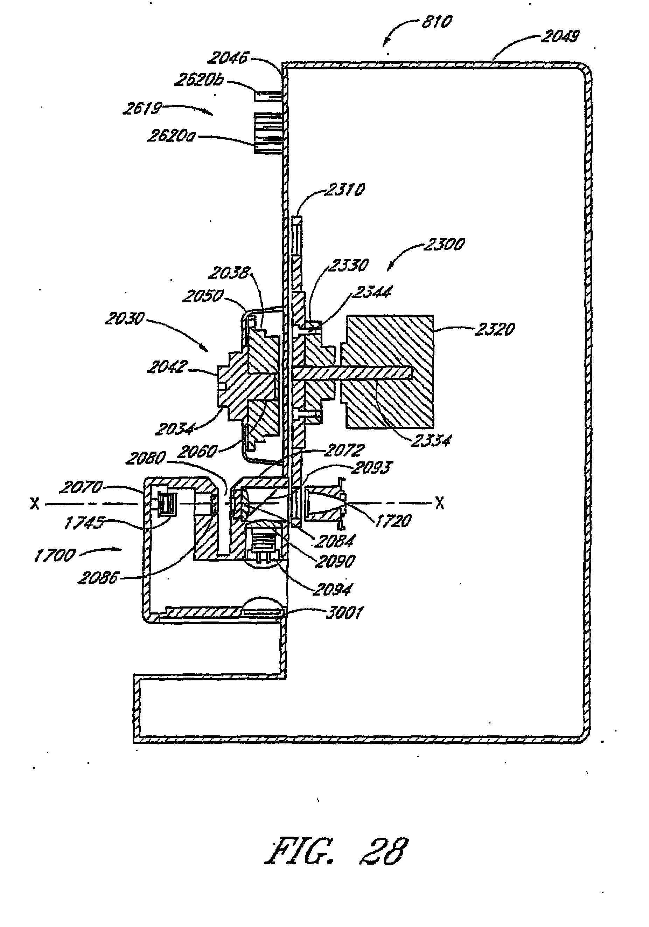

FIGURE 28 is a cross-sectional view of the main instrument 810. The illustrated centrifuge drive system 2030 extends outwardly from a front face 2046 of the main instrument 810 so that it can be easily mated with the rotor assembly 2016 of the cassette 820. When the centrifuge drive system 2030 is energized, the drive system 2030 can rotate the rotor 2020 at a desired rotational speed.

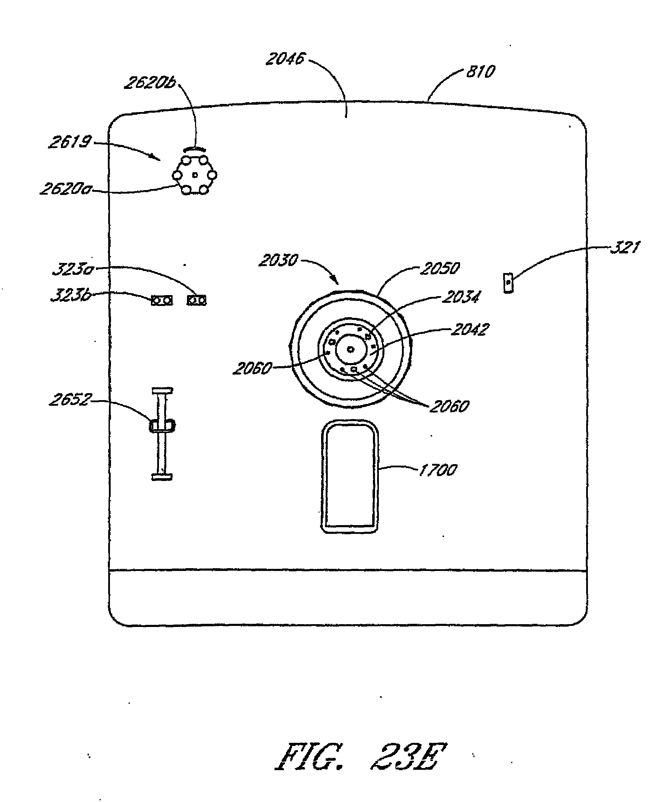

The illustrated centrifuge drive system 2030 of FIGURES 23E and 28 includes a centrifuge drive motor 2038 and a drive spindle 2034 that is drivingly connected to the drive motor 2038. The drive spindle 2034 extends outwardly from the drive motor 2038 and forms a centrifuge interface 2042. The centrifuge interface 2042 extends outwardly from the drive system housing 2050, which houses the drive motor 2038. To impart rotary motion to the rotor 2020, the centrifuge interface 2042 can have keying members, protrusions, notches, detents, recesses, pins, or other types of structures that can engage the rotor 2020 such that the drive spindle 2034 and rotor 2020 are coupled together.

The centrifuge drive motor 2038 of FIGURE 28 can be any suitable motor that can impart rotary motion to the rotor 2020. When the drive motor 2038 is energized, the drive motor 2038 can rotate the drive spindle 2034 at constant or varying speeds. Various types of motors, including, but not limited to, centrifuge motors, stepper motors, spindle motors, electric motors, or any other type of motor for outputting a torque can be utilized. The centrifuge drive motor 2038 is preferably fixedly secured to the drive system housing 2050 of the main instrument 810.

The drive motor 2038 can be the type of motor typically used in personal computer hard drives that is capable of rotating at about 7,200 RPM on precision bearings, such as a motor of a Seagate Model ST380011A hard drive (Seagate Technology, Scotts Valley, CA) or similar motor. In one embodiment, the drive spindle 2034 may be rotated at 6,000 rpm, which yields approximately 2,000 G's for a rotor having a 2.5 inch (64 millimeter) radius. In another embodiment, the drive spindle 2034 may be rotated at speeds of approximately 7,200 rpm. The rotational speed of the drive spindle 2034 can be selected to achieve the desired centrifugal force applied to a sample carried by the rotor 2020.

The main instrument 810 includes a main housing 2049 that defines a chamber sized to accommodate a filter wheel assembly 2300 including a filter drive motor 2320 and filter wheel 2310 of the analyte detection system 1700. The main housing 2049 defines a detection system opening 3001 configured to receive an analyte detection system housing 2070. The illustrated analyte detection system housing 2070 extends or projects outwardly from the housing 2049.

The main instrument 810 of FIGURES 23C and 23E includes a bubble sensor unit 321, a pump 2619 in the form of a peristaltic pump roller 2620a and a roller support 2620b, and valves 323a, 323b. The illustrated valves 323a, 323b are pincher pairs, although other types of valves can be used. When the cassette 820 is installed, these components can engage components of a fluid handling network 2600 of the cassette 820, as will be discussed in greater detail below.

With continued reference to FIGURE 28, the analyte detection system housing 2070 surrounds and houses some of the internal components of the analyte detection system 1700. The elongate slot 2074 extends downwardly from an upper face 2072 of the housing 2070. The elongated slot 2074 is sized and dimensioned so as to receive a portion of the rotor 2020. When the rotor 2020 rotates, the rotor 2020 passes periodically through the elongated slot 2074. When a sample element of the rotor 2020 is in the detection region 2080 defined by the slot 2074, the analyte detection system 1700 can analyze material in the sample element.

The analyte detection system 1700 can be a spectroscopic bodily fluid analyzer that preferably comprises an energy source 1720. The energy source 1720 can generate an energy beam directed along a major optical axis X that passes through the slot 2074 towards a sample detector 1745. The slot 2074 thus permits at least a portion of the rotor (e.g., the interrogation region 2091 or sample chamber 2464 of the sample element 2448) to be positioned on the optical axis X. To analyze a sample carried by the sample element 2448, the sample element and sample can be positioned in the detection region 2080 on the optical axis X such that light emitted from the source 1720 passes through the slot 2074 and the sample disposed within the sample element 2448.

The analyte detection system 1700 can also comprise one or more lenses positioned to transmit energy outputted from the energy source 1720. The illustrated analyte detection system 1700 of FIGURE 28 comprises a first lens 2084 and a second lens 2086. The first lens 2084 is configured to focus the energy from the source 1720 generally onto the sample element and material sample. The second lens 2086 is positioned between the sample element and the sample detector 1745. Energy from energy source 1720 passing through the sample element can subsequently pass through the second lens 2086. A third lens 2090 is preferably positioned between a beam splitter 2093 and a reference detector 2094. The reference detector 2094 is positioned to receive energy from the beam splitter 2093.

The analyte detection system 1700 can be used to determine the analyte concentration in the sample carried by the rotor 2020. Other types of detection or analysis systems can be used with the illustrated centrifuge apparatus or sample preparation unit. The fluid handling and analysis apparatus 140 is shown for illustrative purposes as being used in conjunction with the analyte detection system 1700, but neither the sample preparation unit nor analyte detection system are intended to be limited to the illustrated configuration, or to be limited to being used together.

To assemble the fluid handling and analysis apparatus 140, the cassette 820 can be moved towards and installed onto the main instrument 810, as indicated by the arrow 2007 in FIGURE 22A. As the cassette 820 is installed, the drive system 2030 passes through the aperture 2040 so that the spindle 2034 mates with the rotor 2020. Simultaneously, the projecting portion of the detection system 1700 is received in the notch 2408 of the cassette 820. When the cassette 820 is installed on the main instrument 810, the slot 2410 of the notch 2048 and the slot 2074 of the detection system 1700 are aligned as shown in FIGURE 22C. Accordingly, when the cassette 820 and main instrument 810 are assembled, the rotor 2020 can rotate about the axis 2024 and pass through the slots 2410, 2074.

After the cassette 820 is assembled with the main instrument 810, a sample can be added to the sample element 2448. The cassette 820 can be connected to an infusion source and a patient to place the system in fluid communication with a bodily fluid to be analyzed. Once the cassette 820 is connected to a patient, a bodily fluid may be drawn from the patient into the cassette 820. The rotor 2020 is rotated to a vertical loading position wherein the sample element 2448 is near the fluid interface 2028 and the bypass element 2452 is positioned within the slot 2074 of the detection system 1700. Once the rotor 2020 is in the vertical loading position, the pins 2542, 2544 of the fluid interface 2028 are positioned to mate with the ports 2472, 2474 of the rotor 2020. The fluid interface 2028 is then rotated upwardly until the ends 2571, 2573 of the pins 2542, 2544 are inserted into the ports 2472, 2474.

When the fluid interface 2028 and the sample element 2448 are thus engaged, sample fluid (e.g., whole blood) is pumped into the sample element 2448. The sample can flow through the pin 2544 into and through the rotor channel 2512 and the sample element channel 2466, and into the sample chamber 2464. As shown in FIGURE 25C, the sample chamber 2464 can be partially or completely filled with sample fluid. In some embodiments, the sample fills at least the sample chamber 2464 and the interrogation region 2091 of the sample element 2448. The sample can optionally fill at least a portion of the sample element channels 2466, 2468. The illustrated sample chamber 2464 is filled with whole blood, although the sample chamber 2464 can be filled with other substances. After the sample element 2448 is filled with a desired amount of fluid, the fluid interface 2028 can be moved to a lowered position to permit rotation of the rotor 2020.

The centrifuge drive system 2030 can then spin the rotor 2020 and associated sample element 2448 as needed to separate one or more components of the sample. The separated component(s) of the sample may collect or be segregated in a section of the sample element for analysis. In the illustrated embodiment, the sample element 2448 of FIGURE 25C is filled with whole blood prior to centrifuging. The centrifugal forces can be applied to the whole blood until plasma 2594 is separated from the blood cells 2592. After centrifuging, the plasma 2594 is preferably located in a radially inward portion of the sample element 2448, including the interrogation region 2091. The blood cells 2592 collect in a portion of the sample chamber 2464 which is radially outward of the plasma 2594 and interrogation region 2091.

The rotor 2020 can then be moved to a vertical analysis position wherein the sample element 2448 is disposed within the slot 2074 and aligned with the source 1720 and the sample detector 1745 on the major optical axis X. When the rotor 2020 is in the analysis position, the interrogation portion 2091 is preferably aligned with the major optical axis X of the detection system 1700. The analyte detection system 1700 can analyze the sample in the sample element 2448 using spectroscopic analysis techniques as discussed elsewhere herein.

After the sample has been analyzed, the sample can be removed from the sample element 2448. The sample may be transported to a waste receptacle so that the sample element 2448 can be reused for successive sample draws and analyses. The rotor 2020 is rotated from the analysis position back to the vertical loading position. To empty the sample element 2448, the fluid interface 2028 can again engage the sample element 2448 to flush the sample element 2448 with fresh fluid (either a new sample of body fluid, or infusion fluid). The fluid interface 2028 can be rotated to mate the pins 2542, 2544 with the ports 2472, 2474 of the rotor 2020. The fluid interface 2028 can pump a fluid through one of the pins 2542, 2544 until the sample is flushed from the sample element 2448. Various types of fluids, such as infusion liquid, air, water, and the like, can be used to flush the sample element 2448. After the sample element 2448 has been flushed, the sample element 2448 can once again be filled with another sample.

In an alternative embodiment, the sample element 2448 may be removed from the rotor 2020 and replaced after each separate analysis, or after a certain number of analyses. Once the patient care has terminated, the fluid passageways or conduits may be disconnected from the patient and the sample cassette 820 which has come into fluid contact with the patient's bodily fluid may be disposed of or sterilized for reuse. The main instrument 810, however, has not come into contact with the patient's bodily fluid at any point during the analysis and therefore can readily be connected to a new fluid handling cassette 820 and used for the analysis of a subsequent patient.

The rotor 2020 can be used to provide a fluid flow bypass. To facilitate a bypass flow, the rotor 2020 is first rotated to the vertical analysis/bypass position wherein the bypass element 2452 is near the fluid interface 2028 and the sample element 2448 is in the slot 2074 of the analyte detection system 1700. Once the rotor 2020 is in the vertical analysis/bypass position, the pins 2542, 2544 can mate with the ports 2572, 2574 of the rotor 2020. In the illustrated embodiment, the fluid interface 2028 is rotated upwardly until the ends 2571, 2573 of the pins 2542, 2544 are inserted into the ports 2572, 2574. The bypass element 2452 can then provide a completed fluid circuit so that fluid can flow through one of the pins 2542, 2544 into the bypass element 2452, through the bypass element 2452, and then through the other pin 2542, 2544. The bypass element 2452 can be utilized in this manner to facilitate the flushing or sterilizing of a fluid system connected to the cassette 820.

As shown in FIGURE 23B, the cassette 820 preferably includes the fluid handling network 2600 which can be employed to deliver fluid to the sample element 2448 in the rotor 2020 for analysis. The main instrument 810 has a number of components that can, upon installation of the cassette 820 on the main instrument 810, extend through openings in the front face 2045 of cassette 820 to engage and interact with components of the fluid handling network 2600, as detailed below.

The fluid handling network 2600 of the fluid handling and analysis apparatus 140 includes the passageway 111 which extends from the connector 120 toward and through the cassette 820 until it becomes the passageway 112, which extends from the cassette 820 to the patient connector 110. A portion 111a of the passageway 111 extends across an opening 2613 in the front face 2045 of the cassette 820. When the cassette 820 is installed on the main instrument 810, the roller pump 2619 engages the portion 111a, which becomes situated between the impeller 2620a and the impeller support 2620b (see FIGURE 23C).

The fluid handling network 2600 also includes passageway 113 which extends from the patient connector 110 towards and into the cassette 820. After entering the cassette 820, the passageway 113 extends across an opening 2615 in the front face 2045 to allow engagement of the passageway 113 with a bubble sensor 321 of the main instrument 810, when the cassette 820 is installed on the main instrument 810. The passageway 113 then proceeds to the connector 2532 of the fluid interface 2028, which extends the passageway 113 to the pin 2544. Fluid drawn from the patient into the passageway 113 can thus flow into and through the fluid interface 2028, to the pin 2544. The drawn body fluid can further flow from the pin 2544 and into the sample element 2448, as detailed above.

A passageway 2609 extends from the connector 2530 of the fluid interface 2028 and is thus in fluid communication with the pin 2542. The passageway 2609 branches to form the waste line 324 and the pump line 327. The waste line 324 passes across an opening 2617 in the front face 2045 and extends to the waste receptacle 325. The pump line 327 passes across an opening 2619 in the front face 2045 and extends to the pump 328. When the cassette 820 is installed on the main instrument 810, the pinch valves 323a, 323b extend through the openings 2617, 2619 to engage the lines 324, 327, respectively.

The waste receptacle 325 is mounted to the front face 2045. Waste fluid passing from the fluid interface 2028 can flow through the passageways 2609, 324 and into the waste receptacle 325. Once the waste receptacle 325 is filled, the cassette 820 can be removed from the main instrument 810 and discarded. Alternatively, the filled waste receptacle 325 can be replaced with an empty waste receptacle 325.

The pump 328 can be a displacement pump (e.g., a syringe pump). A piston control 2645 can extend over at least a portion of an opening 2621 in the cassette face 2045 to allow engagement with an actuator 2652 when the cassette 820 is installed on the main instrument 810. When the cassette 820 is installed, the actuator 2652 (FIGURE 23E) of the main instrument 810 engages the piston control 2645 of the pump 328 and can displace the piston control 2645 for a desired fluid flow.

It will be appreciated that, upon installing the cassette 820 of FIGURE 23A on the main instrument 810 of FIGURE 23E, there is formed (as shown in FIGURE 23E) a fluid circuit similar to that shown in the sampling unit 200 in FIGURE 3. This fluid circuit can be operated in a manner similar to that described above in connection with the apparatus of FIGURE 3 (e.g., in accordance with the methodology illustrated in FIGURES 7A-7J and Table 1).

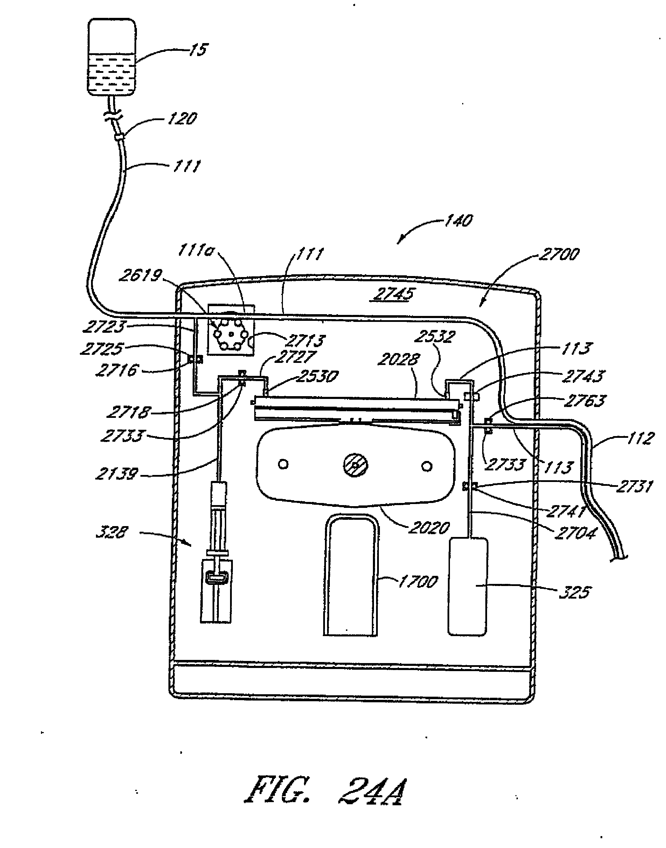

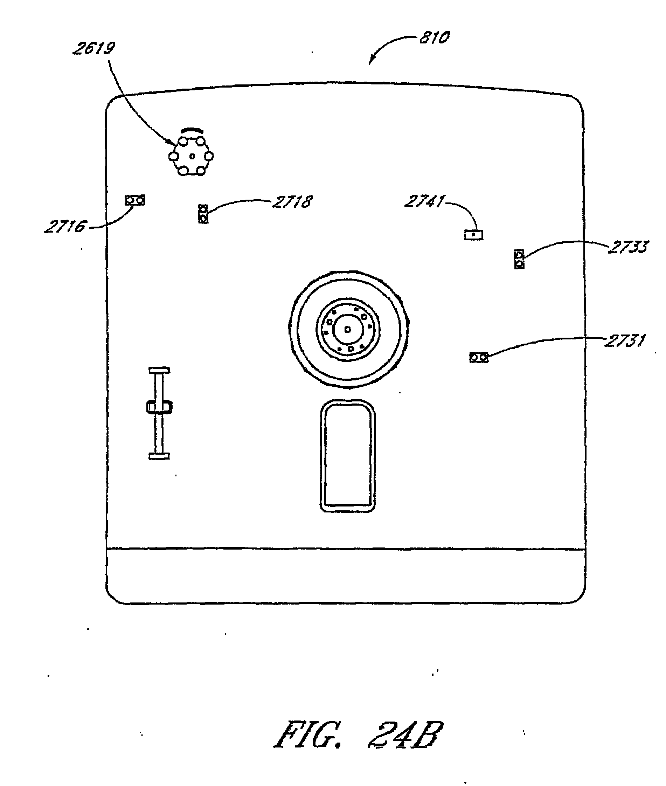

FIGURE 24A depicts another embodiment of a fluid handling network 2700 that can be employed in the cassette 820. The fluid handling network 2700 can be generally similar in structure and function to the network 2600 of FIGURE 23B, except as detailed below. The network 2700 includes the passageway 111 which extends from the connector 120 toward and through the cassette 820 until it becomes the passageway 112, which extends from the cassette 820 to the patient connector 110. A portion 111a of the passageway 111 extends across an opening 2713 in the front face 2745 of the cassette 820. When the cassette 820 is installed on the main instrument 810, a roller pump 2619 of the main instrument 810 of FIGURE 24B can engage the portion 111a in a manner similar to that described above with respect to FIGURES 23B-23C. The passageway 113 extends from the patient connector 110 towards and into the cassette 820. After entering the cassette 820, the passageway 113 extends across an opening 2763 in the front face 2745 to allow engagement with a valve 2733 of the main instrument 810. A waste line 2704 extends from the passageway 113 to the waste receptacle 325 and across an opening 2741 in the front face 2745. The passageway 113 proceeds to the connector 2532 of the fluid interface 2028, which extends the passageway 113 to the pin 2544. The passageway 113 crosses an opening 2743 in the front face 2745 to allow engagement of the passageway 113 with a bubble sensor 2741 of the main instrument 810 of FIGURE 24B. When the cassette 820 is installed on the main instrument 810, the pinch valves 2732, 2733 extend through the openings 2731, 2743 to engage the passageways 113, 2704, respectively.

The illustrated fluid handling network 2700 also includes a passageway 2723 which extends between the passageway 111 and a passageway 2727, which in turn extends between the passageway 2723 and the fluid interface 2028. The passageway 2727 extends across an opening 2733 in the front face 2745. A pump line 2139 extends from a pump 328 to the passageways 2723, 2727. When the cassette 820 is installed on the main instrument 810, the pinch valves 2716, 2718 extend through the openings 2725, 2733 in the front face 2745 to engage the passageways 2723, 2727, respectively.

It will be appreciated that, upon installing the cassette 820 on the main instrument 810 (as shown in FIGURE 24A), there is formed a fluid circuit that can be operated in a manner similar to that described above, in connection with the apparatus of FIGS. 9-10.

In view of the foregoing, it will be further appreciated that the various embodiments of the fluid handling and analysis apparatus 140 (comprising a main instrument 810 and cassette 820) depicted in FIGURES 22A-28 can serve as the fluid handling and analysis apparatus 140 of any of the sampling systems 100/300/500, or the fluid handling system 10, depicted in FIGURES 1-5 herein. In addition, the fluid handling and analysis apparatus 140 of FIGURES 22A-28 can, in certain embodiments, be similar to the apparatus 140 of FIGURES 1-2 or 8-10, except as further described above.

## SECTION V - METHODS FOR DETERMINING ANALYTE CONCENTRATIONS FROM SAMPLE SPECTRA

This section discusses a number of computational methods or algorithms which may be used to calculate the concentration of the analyte(s) of interest in the sample S, and/or to compute other measures that may be used in support of calculations of analyte concentrations. Any one or combination of the algorithms disclosed in this section may reside as program instructions stored in the memory 212 so as to be accessible for execution by the processor 210 of the fluid handling and analysis apparatus 140 or analyte detection system 334 to compute the concentration of the analyte(s) of interest in the sample, or other relevant measures.

Several disclosed embodiments are devices and methods for analyzing material sample measurements and for quantifying one or more analytes in the presence of interferents. Interferents can comprise components of a material sample being analyzed for an analyte, where the presence of the interferent affects the quantification of the analyte. Thus, for example, in the spectroscopic analysis of a sample to determine an analyte concentration, an interferent could be a compound having spectroscopic features that overlap with those of the analyte. The presence of such an interferent can introduce errors in the quantification of the analyte. More specifically, the presence of interferents can affect the sensitivity of a measurement technique to the concentration of analytes of interest in a material sample, especially when the system is calibrated in the absence of, or with an unknown amount of, the interferent.

Independently of or in combination with the attributes of interferents described above, interferents can be classified as being endogenous (i.e., originating within the body) or exogenous (i.e., introduced from or produced outside the body). As example of these classes of interferents, consider the analysis of a blood sample (or a blood component sample or a blood plasma sample) for the analyte glucose. Endogenous interferents include those blood components having origins within the body that affect the quantification of glucose, and may include water, hemoglobin, blood cells, and any other component that naturally occurs in blood. Exogenous interferents include those blood components having origins outside of the body that affect the quantification of glucose, and can include items administered to a person, such as medicaments, drugs, foods or herbs, whether administered orally, intravenously, topically, etc.

Independently of or in combination with the attributes of interferents described above, interferents can comprise components which are possibly but not necessarily present in the sample type under analysis. In the example of analyzing samples of blood or blood plasma drawn from patients who are receiving medical treatment, a medicament such as acetaminophen is possibly, but not necessarily present in this sample type. In contrast, water is necessarily present in such blood or plasma samples.

To facilitate an understanding of the inventions, embodiments are discussed herein where one or more analyte concentrations are obtained using spectroscopic measurements of a sample at wavelengths including one or more wavelengths that are identified with the analyte(s). The embodiments disclosed herein are not meant to limit, except as claimed, the scope of certain disclosed inventions which are directed to the analysis of measurements in general.

As an example, certain disclosed methods are used to quantitatively estimate the concentration of one specific compound (an analyte) in a mixture from a measurement, where the mixture contains compounds (interferents) that affect the measurement. Certain disclosed embodiments are particularly effective if each analyte and interferent component has a characteristic signature in the measurement, and if the measurement is approximately affine (i.e., includes a linear component and an offset) with respect to the concentration of each analyte and interferent. In one embodiment, a method includes a calibration process including an algorithm for estimating a set of coefficients and an offset value that permits the quantitative estimation of an analyte. In another embodiment, there is provided a method for modifying hybrid linear algorithm (HLA) methods to accommodate a random set of interferents, while retaining a high degree of sensitivity to the desired component. The data employed to accommodate the random set of interferents are (a) the signatures of each of the members of the family of potential additional components and (b) the typical quantitative level at which each additional component, if present, is likely to appear.

Certain methods disclosed herein are directed to the estimation of analyte concentrations in a material sample in the possible presence of an interferent. In certain embodiments, any one or combination of the methods disclosed herein may be accessible and executable processor 210 of system 334. Processor 210 may be connected to a computer network, and data obtained from system 334 can be transmitted over the network to one or more separate computers that implement the methods. The disclosed methods can include the manipulation of data related to sample measurements and other information supplied to the methods (including, but not limited to, interferent spectra, sample population models, and threshold values, as described subsequently). Any or all of this information, as well as specific algorithms, may be updated or changed to improve the method or provide additional information, such as additional analytes or interferents.

Certain disclosed methods generate a "calibration constant" that, when multiplied by a measurement, produces an estimate of an analyte concentration. Both the calibration constant and measurement can comprise arrays of numbers. The calibration constant is calculated to minimize or reduce the sensitivity of the calibration to the presence of interferents that are identified as possibly being present in the sample. Certain methods described herein generate a calibration constant by: 1) identifying the presence of possible interferents; and 2) using information related to the identified interferents to generate the calibration constant. These certain methods do not require that the information related to the interferents includes an estimate of the interferent concentration - they merely require that the interferents be identified as possibly present. In one embodiment, the method uses a set of training spectra each having known analyte concentration(s) and produces a calibration that minimizes the variation in estimated analyte concentration with interferent concentration. The resulting calibration constant is proportional to analyte concentration(s) and, on average, is not responsive to interferent concentrations.

In one embodiment, it is not required (though not prohibited either) that the training spectra include any spectrum from the individual whose analyte concentration is to be determined. That is, the term "training" when used in reference to the disclosed methods does not require training using measurements from the individual whose analyte concentration will be estimated (e.g., by analyzing a bodily fluid sample drawn from the individual).

Several terms are used herein to describe the estimation process. As used herein, the term "Sample Population" is a broad term and includes, without limitation, a large number of samples having measurements that are used in the computation of a calibration - in other words, used to train the method of generating a calibration. For an embodiment involving the spectroscopic determination of glucose concentration, the Sample Population measurements can each include a spectrum (analysis measurement) and a glucose concentration (analyte measurement). In one embodiment, the Sample Population measurements are stored in a database, referred to herein as a "Population Database."

The Sample Population may or may not be derived from measurements of material samples that contain interferents to the measurement of the analyte(s) of interest. One distinction made herein between different interferents is based on whether the interferent is present in both the Sample Population and the sample being measured, or only in the sample. As used herein, the term "Type-A interferent" refers to an interferent that is present in both the Sample Population and in the material sample being measured to determine an analyte concentration. In certain methods it is assumed that the Sample Population includes only interferents that are endogenous, and does not include any exogenous interferents, and thus Type-A interferents are endogenous. The number of Type-A interferents depends on the measurement and analyte(s) of interest, and may number, in general, from zero to a very large number. The material sample being measured, for example sample S, may also include interferents that are not present in the Sample Population. As used herein, the term "Type-B interferent" refers to an interferent that is either: 1) not found in the Sample Population but that is found in the material sample being measured (e.g., an exogenous interferent), or 2) is found naturally in the Sample Population, but is at abnormally high concentrations in the material sample (e.g., an endogenous interferent). Examples of a Type-B exogenous interferent may include medications, and examples of Type-B endogenous interferents may include urea in persons suffering from renal failure. In the example of mid-IR spectroscopic absorption measurement of glucose in blood, water is found in all blood samples, and is thus a Type-A interferent. For a Sample Population made up of individuals who are not taking intravenous drugs, and a material sample taken from a hospital patient who is being administered a selected intravenous drug, the selected drug is a Type-B interferent.

In one embodiment, a list of one or more possible Type-B Interferents is referred to herein as forming a "Library of Interferents," and each interferent in the library is referred to as a "Library Interferent." The Library Interferents include exogenous interferents and endogenous interferents that may be present in a material sample due, for example, to a medical condition causing abnormally high concentrations of the endogenous interferent.

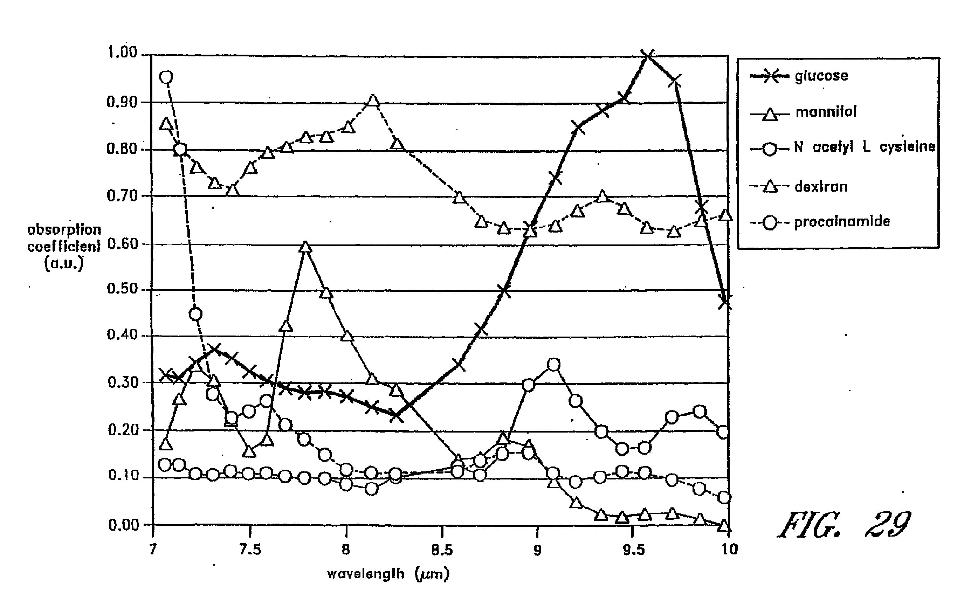

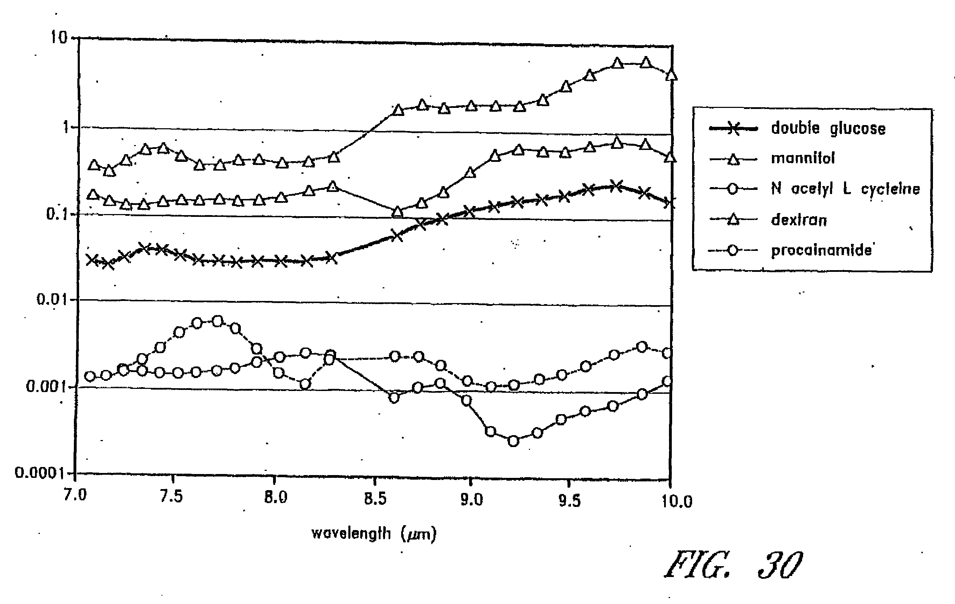

In addition to components naturally found in the blood, the ingestion or injection of some medicines or illicit drugs can result in very high and rapidly changing concentrations of exogenous interferents. This results in problems in measuring analytes in blood of hospital or emergency room patients. An example of overlapping spectra of blood components and medicines is illustrated in FIGURE 29 as the absorption coefficient at the same concentration and optical pathlength of pure glucose and three spectral interferents, specifically mannitol (chemical formula: hexane-1,2,3,4,5,6-hexaol), N acetyl L cysteine, dextran, and procainamide (chemical formula: 4-amino-N-(2-diethylaminoethyl)benzamid). FIGURE 30 shows the logarithm of the change in absorption spectra from a Sample Population blood composition as a function of wavelength for blood containing additional likely concentrations of components, specifically, twice the glucose concentration of the Sample Population and various amounts of mannitol, N acetyl L cysteine, dextran, and procainamide. The presence of these components is seen to affect absorption over a wide range of wavelengths. It can be appreciated that the determination of the concentration of one species without a priori knowledge or independent measurement of the concentration of other species is problematic.

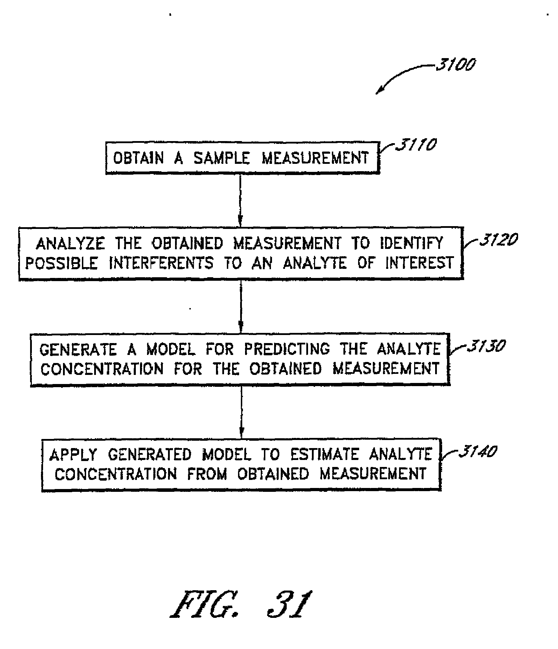

One method for estimating the concentration of an analyte in the presence of interferents is presented in flowchart 3100 of FIGURE 31 as a first step (Block 3110) where a measurement of a sample is obtained, a second step (Block 3120), where the obtained measurement data is analyzed to identify possible interferents to the analyte, a third step (Block 3130) where a model is generated for predicting the analyte concentration in the presence of the identified possible interferents, and a fourth step (Block 3140) where the model is used to estimate the analyte concentration in the sample from the measurement. Preferably the step of Block 3130 generates a model where the error is minimized for the presence of the identified interferents that are not present in a general population of which the sample is a member.

The method Blocks 3110, 3120, 3130, and 3140 may be repeatedly performed for each analyte whose concentration is required. If one measurement is sensitive to two or more analytes, then the methods of Blocks 3120, 3130, and 3140 may be repeated for each analyte. If each analyte has a separate measurement, then the methods of Blocks 3110, 3120, 3130, and 3140 may be repeated for each analyte.

An embodiment of the method of flowchart 3100 for the determination of an analyte from spectroscopic measurements will now be discussed. Further, this embodiment will estimate the amount of glucose concentration in blood sample S, without limit to the scope of the inventions disclosed herein. In one embodiment, the measurement of Block 3110 is an absorbance spectrum, $C_s(\lambda_i)$, of a measurement sample S that has, in general, one analyte of interest, glucose, and one or more interferents. In one embodiment, the methods include generating a calibration constant $\kappa(\lambda_i)$ that, when multiplied by the absorbance spectrum $C_s(\lambda_i)$, provides an estimate, $g_{est}$, of the glucose concentration $g_s$.

As described subsequently, one embodiment of Block 3120 includes a statistical comparison of the absorbance spectrum of sample S with a spectrum of the Sample Population and combinations of individual Library Interferent spectra. After the analysis of Block 3120, a list of Library Interferents that are possibly contained in sample S has been identified and includes, depending on the outcome of the analysis of Block 3120, either no Library Interferents, or one or more Library Interferents. Block 3130 then generates a large number of spectra using the large number of spectra of the Sample Population and their respective known analyte concentrations and known spectra of the identified Library Interferents. Block 3130 then uses the generated spectra to generate a calibration constant matrix to convert a measured spectrum to an analyte concentration that is the least sensitive to the presence of the identified Library Interferents. Block 3140 then applies the generated calibration constant to predict the glucose concentration in sample S.

As indicated in Block 3110, a measurement of a sample is obtained. For illustrative purposes, the measurement, $C_s(\lambda_i)$, is assumed to be a plurality of measurements at different wavelengths, or analyzed measurements, on a sample indicating the intensity of light that is absorbed by sample S. It is to be understood that spectroscopic measurements and computations may be performed in one or more domains including, but not limited to, the transmittance, absorbance and/or optical density domains. The measurement $C_s(\lambda_i)$ is an absorption, transmittance, optical density or other spectroscopic measurement of the sample at selected wavelength or wavelength bands. Such measurements may be obtained, for example, using analyte detection system 334. In general, sample S contains Type-A interferents, at concentrations preferably within the range of those found in the Sample Population.

In one embodiment, absorbance measurements are converted to pathlength normalized measurements. Thus, for example, the absorbance is converted to optical density by dividing the absorbance by the optical pathlength, L, of the measurement. In one embodiment, the pathlength L is measured from one or more absorption measurements on known compounds. Thus, in one embodiment, one or more measurements of the absorption through a sample S of water or saline solutions of known concentration are made and the pathlength, L, is computed from the resulting absorption measurement(s). In another embodiment, absorption measurements are also obtained at portions of the spectrum that are not appreciably affected by the analytes and interferents, and the analyte measurement is supplemented with an absorption measurement at those wavelengths.

Some methods are "pathlength insensitive," in that they can be used even when the precise pathlength is not known beforehand. The sample can be placed in the sample chamber 903 or 2464, sample element 1730 or 2448, or in a cuvette or other sample container. Electromagnetic radiation (in the mid-infrared range, for example) can be emitted from a radiation source so that the radiation travels through the sample chamber. A detector can be positioned where the radiation emerges, on the other side of the sample chamber from the radiation source, for example. The distance the radiation travels through the sample can be referred to as a "pathlength." In some embodiments, the radiation detector can be located on the same side of the sample chamber as the radiation source, and the radiation can reflect off one or more internal walls of the sample chamber before reaching the detector.

As discussed above, various substances can be inserted into the sample chamber. For example, a reference fluid such as water or saline solution can be inserted, in addition to a sample or samples containing an analyte or analytes. In some embodiments, a saline reference fluid is inserted into the sample chamber and radiation is emitted through that reference fluid. The detector measures the amount and/or characteristics of the radiation that passes through the sample chamber and reference fluid without being absorbed or reflected. The measurement taken using the reference fluid can provide information relating to the pathlength traveled by the radiation. For example, data may already exist from previous measurements that have been taken under similar circumstances. That is, radiation can be emitted previously through sample chambers with various known pathlengths to establish reference data that can be arranged in a "look-up table," for example. With reference fluid in the sample chamber, a one-to-one correspondence can be experimentally established between various detector readings and various pathlengths, respectively. This correspondence can be recorded in the look-up table, which can be recorded in a computer database or in electronic memory, for example.

One method of determining the radiation pathlength can be accomplished with a thin, empty sample chamber. In particular, this approach can determine the thickness of a narrow sample chamber or cell with two reflective walls. (Because the chamber will be filled with a sample, this same thickness corresponds to the "pathlength" radiation will travel through the sample). A range of radiation wavelengths can be emitted in a continuous manner through the cell or sample chamber. The radiation can enter the cell and reflect off the interior cell walls, bouncing back and forth between those walls one or multiple times before exiting the cell and passing into the radiation detector. This can create a periodic interference pattern or "fringe" with repeating maxima and minima. This periodic pattern can be plotted where the horizontal axis is a range of wavelengths and the vertical axis is a range of transmittance, measured as a percentage of total transmittance, for example. The maxima occur when the radiation reflected off of the two internal surfaces of the cell has traveled a distance that is an integral multiple N of the wavelength of the radiation that was transmitted without reflection. Constructive interference occurs whenever the wavelength is equal to 2b/N, where "b" is the thickness (or pathlength) of the cell. Thus, if $\Delta N$ is the number of maxima in this fringe pattern for a given range of wavelengths $\lambda_1$-$\lambda_2$, then the thickness of the cell b is provided by the following relation: $b = \Delta N / 2(\lambda_1 - \lambda_2)$. This approach can be especially useful when the refractive index of the material within the sample chamber or fluid cell is not the same as the refractive index of the walls of the cell, because this condition improves reflection.

Once the pathlength has been determined, it can be used to calculate or determine a reference value or a reference spectrum for the interferents (such as protein or water) that may be present in a sample. For example, both an analyte such as glucose and an interferent such as water may absorb radiation at a given wavelength. When the source emits radiation of that wavelength and the radiation passes through a sample containing both the analyte and the interferent, both the analyte and the interferent absorb the radiation. The total absorption reading of the detector is thus fully attributable to neither the analyte nor the interferent, but a combination of the two. However, if data exists relating to how much radiation of a given wavelength is absorbed by a given interferent when the radiation passes through a sample with a given pathlength, the contribution of the interferent can be subtracted from the total reading of the detector and the remaining value can provide information regarding concentration of the analyte in the sample. A similar approach can be taken for a whole spectrum of wavelengths. If data exists relating to how much radiation is absorbed by an interferent over a range of wavelengths when the radiation passes through a sample with a given pathlength, the interferent absorbance spectrum can be subtracted from the total absorbance spectrum, leaving only the analyte's absorbance spectrum for that range of wavelengths. If the interferent absorption data is taken for a range of possible pathlengths, it can be helpful to determine the pathlength of a particular sample chamber first so that the correct data can be found for samples measured in that sample chamber.

This same process can be applied iteratively or simultaneously for multiple interferents and/or multiple analytes. For example, the water absorbance spectrum and the protein absorbance spectrum can both be subtracted to leave behind the glucose absorbance spectrum.

The pathlength can also be calculated using an isosbestic wavelength. An isosbestic wavelength is one at which all components of a sample have the same absorbance. If the components (and their absorption coefficients) in a particular sample are known, and one or multiple isosbestic wavelengths are known for those particular components, the absorption data collected by the radiation detector at those isosbestic wavelengths can be used to calculate the pathlength. This can be advantageous because the needed information can be obtained from multiple readings of the absorption detector that are taken at approximately the same time, with the same sample in place in the sample chamber. The isosbestic wavelength readings are used to determine pathlength, and other selected wavelength readings are used to determine interferent and/or analyte concentration. Thus, this approach is efficient and does not require insertion of a reference fluid in the sample chamber.

In some embodiments, a method of determining concentration of an analyte in a sample can include inserting a fluid sample into a sample container, emitting radiation from a source through the container and the fluid sample, obtaining total sample absorbance data by measuring the amount of radiation that reaches the detector, subtracting the correct interferent absorbance value or spectrum from the total sample absorbance data, and using the remaining absorbance value or spectrum to determine concentration of an analyte in the fluid sample. The correct interferent absorbance value can be determined using the calculated pathlength.

The concentration of an analyte in a sample can be calculated using the Beer-Lambert law (or Beer's Law) as follows: If T is transmittance, A is absorbance, $P_0$ is initial radiant power directed toward a sample, and P is the power that emerges from the sample and reaches a detector, then $T = P / P_0$, and $A = -\log T = \log (P_0 / P)$. Absorbance is directly proportional to the concentration (c) of the light-absorbing species in the sample, also known as an analyte or an interferent. Thus, if e is the molar absorptivity (1/M 1/cm), b is the path length (cm), and c is the concentration (M), Beer's Law can be expressed as follows: $A = e\, b\, c$. Thus, $c = A/(e\, b)$.

Referring once again to flowchart 3100, the next step is to determine which Library Interferents are present in the sample. In particular, Block 3120 indicates that the measurements are analyzed to identify possible interferents. For spectroscopic measurements, it is preferred that the determination is made by comparing the obtained measurement to interferent spectra in the optical density domain. The results of this step provide a list of interferents that may, or are likely to, be present in the sample. In one embodiment, several input parameters are used to estimate a glucose concentration $g_{est}$ from a measured spectrum, $C_s$. The input parameters include previously gathered spectrum measurement of samples that, like the measurement sample, include the analyte and combinations of possible interferents from the interferent library; and spectrum and concentration ranges for each possible interferent. More specifically, the input parameters are:

Library of Interferent Data: Library of Interferent Data includes, for each of "M" interferents, the absorption spectrum of each interferent, $IF = \{IF_1, IF_2, ..., IF_M\}$, where $m = 1, 2, ..., M$; and a maximum concentration for each interferent, $Tmax = Tmax_1, Tmax_2, ..., Tmax_M$; and

Sample Population Data: Sample Population Data includes individual spectra of a statistically large population taken over the same wavelength range as the sample spectrum, $Cs_i$, and an analyte concentration corresponding to each spectrum. As an example, if there are N Sample Population spectra, then the spectra can be represented as $C = \{C_1, C_2, ..., C_N\}$, where $n = 1, 2, ..., N$, and the analyte concentration corresponding to each spectrum can be represented as $g = \{g_1, g_2, ..., g_N\}$.

Preferably, the Sample Population does not have any of the M interferents present, and the material sample has interferents contained in the Sample Population and none or more of the Library Interferents. Stated in terms of Type-A and Type-B interferents, the Sample Population has Type-A interferents and the material sample has Type-A and may have Type-B interferents. The Sample Population Data are used to statistically quantify an expected range of spectra and analyte concentrations. Thus, for example, for a system 10 or 334 used to determine glucose in blood of a person having unknown spectral characteristics, the spectral measurements are preferably obtained from a statistical sample of the population.

The following discussion, which is not meant to limit the scope of the present disclosure, illustrates embodiments for measuring more than one analyte using spectroscopic techniques. If two or more analytes have non-overlapping spectral features, then a first embodiment is to obtain a spectrum corresponding to each analyte. The measurements may then be analyzed for each analyte according to the method of flowchart 3100. An alternative embodiment for analytes having non-overlapping features, or an embodiment for analytes having overlapping features, is to make one measurement comprising the spectral features of the two or more analytes. The measurement may then be analyzed for each analyte according to the method of flowchart 3100. That is, the measurement is analyzed for each analyte, with the other analytes considered to be interferents to the analyte being analyzed for.

INTERFERENT DETERMINATION

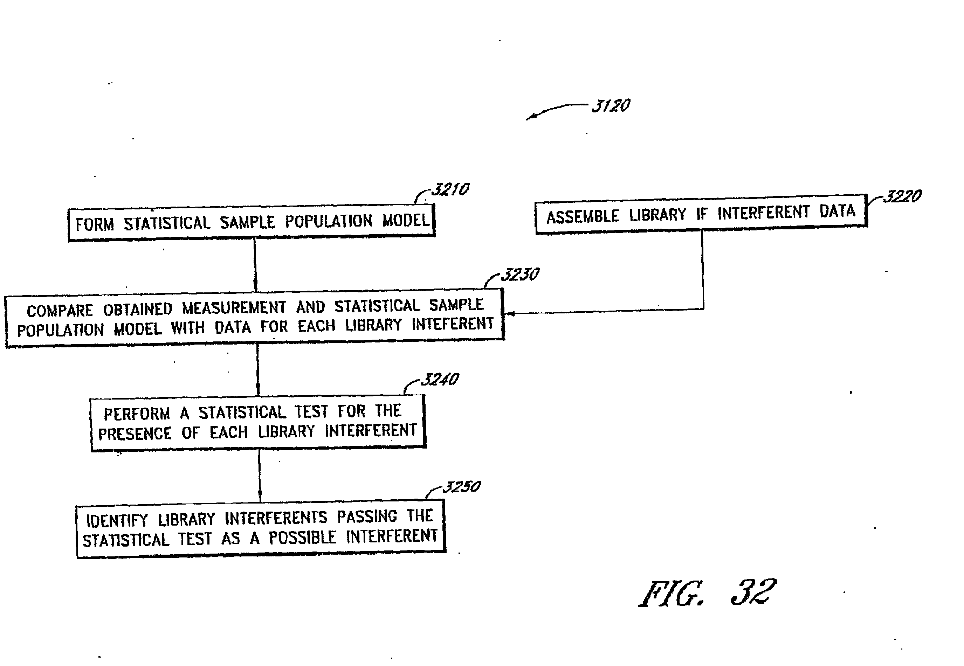

One embodiment of the method of Block 3120 is shown in greater detail with reference to the flowchart of FIGURE 32. The method includes forming a statistical Sample Population model (Block 3210), assembling a library of interferent data (Block 3220), comparing the obtained measurement and statistical Sample Population model with data for each interferent from an interferent library (Block 3230), performing a statistical test for the presence of each interferent from the interferent library (Block 3240), and identifying each interferent passing the statistical test as a possible Library Interferent (Block 3250). The steps of Block 3220 can be performed once or can be updated as necessary. The steps of Blocks 3230, 3240, and 3250 can either be performed sequentially for all interferents of the library, as shown, or alternatively, be repeated sequentially for each interferent.

One embodiment of each of the methods of Blocks 3210, 3220, 3230, 3240, and 3250 are now described for the example of identifying Library Interferents in a sample from a spectroscopic measurement using Sample Population Data and a Library of Interferent Data, as discussed previously. Each Sample Population spectrum includes measurements (e.g., of optical density) taken on a sample in the absence of any Library Interferents and has an associated known analyte concentration. A statistical Sample Population model is formed (Block 3210) for the range of analyte concentrations by combining all Sample Population spectra to obtain a mean matrix and a covariance matrix for the Sample Population. Thus, for example, if each spectrum at n different wavelengths is represented by an n x 1 matrix, C, then the mean spectrum, $\mu$, is a n x 1 matrix with the (e.g., optical density) value at each wavelength averaged over the range of spectra, and the covariance matrix, V, is the expected value of the deviation between C and $\mu$ as $V = E((C-\mu)(C-\mu)^T)$. The matrices $\mu$ and V are one model that describes the statistical distribution of the Sample Population spectra.

In another step, Library Interferent information is assembled (Block 3220). A number of possible interferents are identified, for example as a list of possible medications or foods that might be ingested by the population of patients at issue or measured by system 10 or 334, and their spectra (in the absorbance, optical density, or transmission domains) are obtained. In addition, a range of expected interferent concentrations in the blood, or other expected sample material, are estimated. Thus, each of M interferents has spectrum IF and maximum concentration Tmax. This information is preferably assembled once and is accessed as needed.

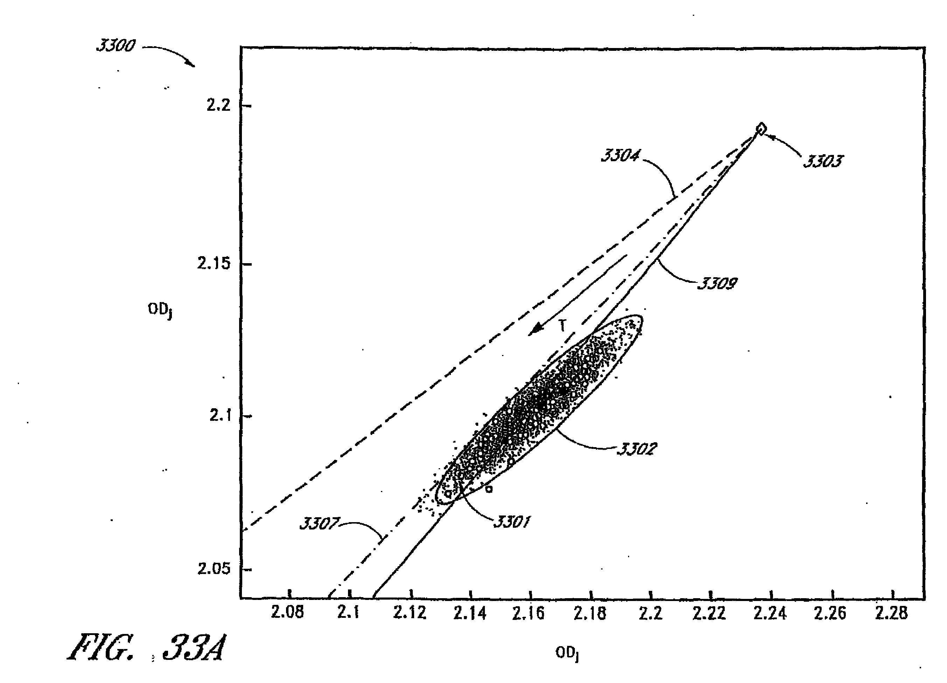

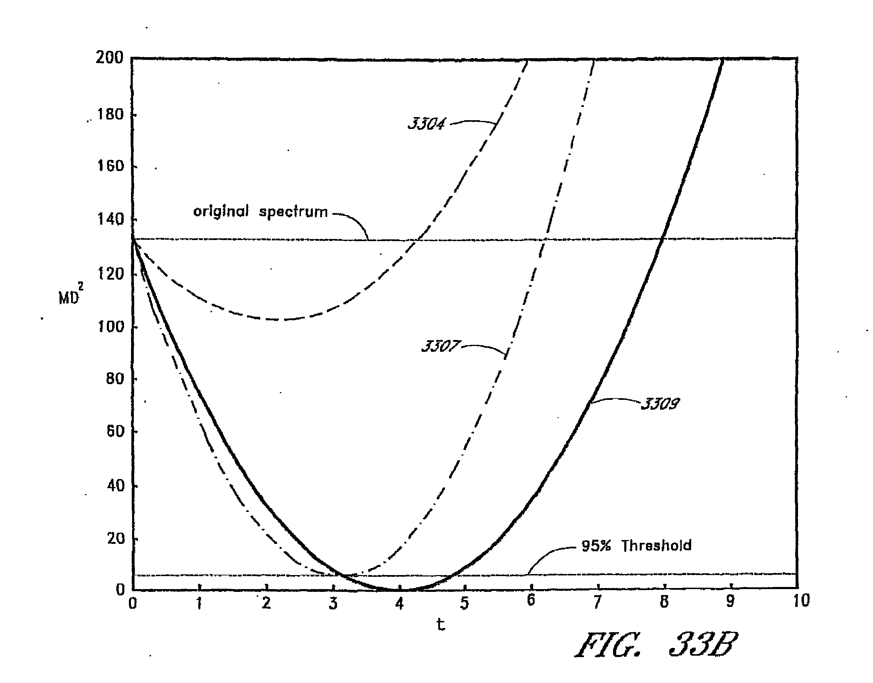

The obtained measurement data and statistical Sample Population model are next compared with data for each interferent from the interferent library (Block 3230) to perform a statistical test (Block 3240) to determine the identity of any interferent in the mixture (Block 3250). This interferent test will first be shown in a rigorous mathematical formulation, followed by a discussion of FIGURES 33A and 33B which illustrates the method.

Mathematically, the test of the presence of an interferent in a measurement proceeds as follows. The measured optical density spectrum, $C_s$, is modified for each interferent of the library by analytically subtracting the effect of the interferent, if present, on the measured spectrum. More specifically, the measured optical density spectrum, $C_s$, is modified, wavelength-by-wavelength, by subtracting an interferent optical density spectrum. For an interferent, M, having an absorption spectrum per unit of interferent concentration, $IF_M$, a modified spectrum is given by $C'_s(T) = C_s - IF_M T$, where T is the interferent concentration, which ranges from a minimum value, Tmin, to a maximum value Tmax. The value of Tmin may be zero or, alternatively, be a value between zero and Tmax, such as some fraction of Tmax.

Next, the Mahalanobis distance (MD) between the modified spectrum $C'_s(T)$ and the statistical model $(\mu, V)$ of the Sample Population spectra is calculated as:

$$\mathrm{MD}^2\, (C_s\text{-}(T\, t),\mu;\, \rho_s\, ) = (C_s - (T\, IF_m) - \mu)^T\, V^{-1}\, (C_s - (T\, IF_m) - \mu) \qquad \text{Eq. (1)}$$

The test for the presence of interferent IF is to vary T from Tmin to Tmax (i.e., evaluate $C'_s$ (T) over a range of values of T) and determine whether the minimum MD in this interval is in a predetermined range. Thus for example, one could determine whether the minimum MD in the interval is sufficiently small relative to the quantiles of a $\chi^2$ random variable with L degrees of freedom (L = number of wavelengths).

FIGURE 33A is a graph 3300 illustrating the steps of Blocks 3230 and 3240. The axes of graph 3300, $OD_i$ and $OD_j$, are used to plot optical densities at two of the many wavelengths at which measurements are obtained. The points 3301 are the measurements in the Sample Population distribution. Points 3301 are clustered within an ellipse that has been drawn to encircle the majority of points. Points 3301 inside ellipse 3302 represent measurements in the absence of Library Interferents. Point 3303 is the sample measurement. Presumably, point 3303 is outside of the spread of points 3301 due the presence of one or more Library Interferents. Lines 3304, 3307, and 3309 indicate the measurement of point 3303 as corrected for increasing concentration, T, of three different Library Interferents over the range from Tmin to Tmax. The three interferents of this example are referred to as interferent #1, interferent #2, and interferent #3. Specifically, lines 3304, 3307, and 3309 are obtained by subtracting from the sample measurement an amount T of a Library Interferent (interferent #1, interferent #2, and interferent #3, respectively), and plotting the corrected sample measurement for increasing T.

FIGURE 33B is a graph further illustrating the method of FIGURE 32. In the graph of FIGURE 33B, the squared Mahalanobis distance, $MD^2$ has been calculated and plotted as a function of t for lines 3304, 3307, and 3309. Referring to FIGURE 33A, line 3304 reflects decreasing concentrations of interferent #1 and only slightly approaches points 3301. The value of $MD^2$ of line 3304, as shown in FIGURE 33B, decreases slightly and then increases with decreasing interferent #1 concentration.

Referring to FIGURE 33A, line 3307 reflects decreasing concentrations of interferent #2 and approaches or passes through many points 3301. The value of $MD^2$ of line 3307, as shown in FIGURE 33B, shows a large decrease at some interferent #2 concentration, then increases. Referring to FIGURE 33A, line 3309 has decreasing concentrations of interferent #3 and approaches or passes through even more points 3303. The value of $MD^2$ of line 3309, as shown in FIGURE 33B, shows a still larger decrease at some interferent #3 concentration.

In one embodiment, a threshold level of $MD^2$ is set as an indication of the presence of a particular interferent. Thus, for example, FIGURE 33B shows a line labeled "original spectrum" indicating $MD^2$ when no interferents are subtracted from the spectrum, and a line labeled "95% Threshold", indicating the 95% quantile for the chi$^2$ distribution with L degrees of freedom (where L is the number of wavelengths represented in the spectra). This level is the value which should exceed 95% of the values of the $MD^2$ metric; in other words, values at this level are uncommon, and those far above it should be quite rare. Of the three interferents represented in FIGURES 33A and 33B, only interferent #3 has a value of $MD^2$ below the threshold. Thus, this analysis of the sample indicates that interferent #3 is the most likely interferent present in the sample. Interferent #1 has its minimum far above the threshold level and is extremely unlikely to be present; interferent #2 barely crosses the threshold, making its presence more likely than interferent #1, but still far less likely to be present than interferent #1.

As described subsequently, information related to the identified interferents is used in generating a calibration constant that is relatively insensitive to a likely range of concentration of the identified interferents. In addition to being used in certain methods described subsequently, the identification of the interferents may be of interest and may be provided in a manner that would be useful. Thus, for example, for a hospital based glucose monitor, identified interferents may be reported on display 141 or be transmitted to a hospital computer via communications link 216.

CALIBRATION CONSTANT GENERATION EMBODIMENTS

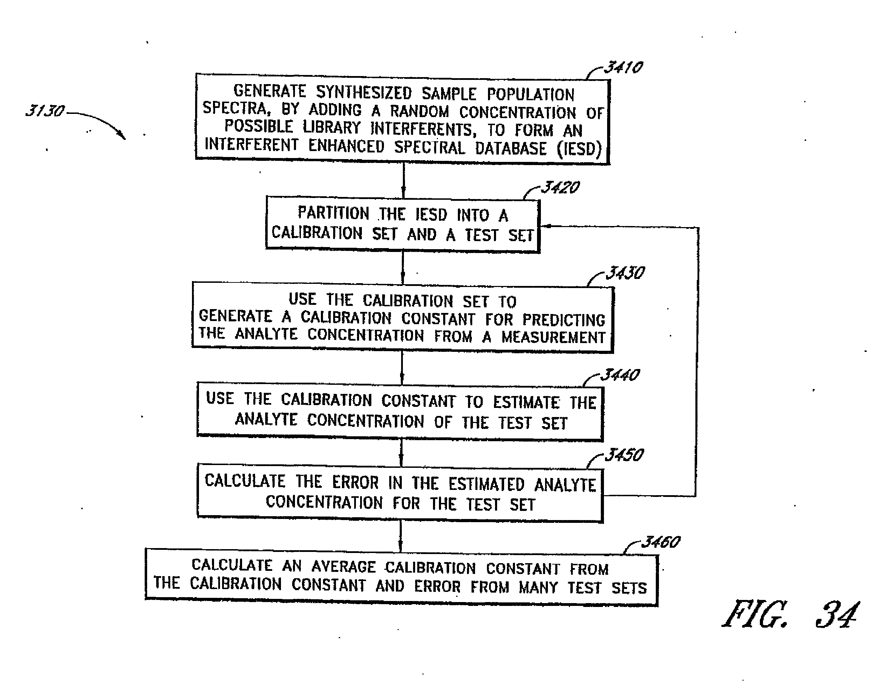

Once Library Interferents are identified as being possibly present in the sample under analysis, a calibration constant for estimating the concentration of analytes in the presence of the identified interferents is generated (Block 3130). More specifically, after Block 3120, a list of possible Library Interferents is identified as being present. One embodiment of the steps of Block 3120 are shown in the flowchart of FIGURE 34 as Block 3410, where synthesized Sample Population measurements are generated, Block 3420, where the synthesized Sample Population measurements are partitioned in to calibration and test sets, Block 3430, where the calibration are is used to generate a calibration constant, Block 3440, where the calibration set is used to estimate the analyte concentration of the test set, Block 3450 where the errors in the estimated analyte concentration of the test set is calculated, and Block 3460 where an average calibration constant is calculated.

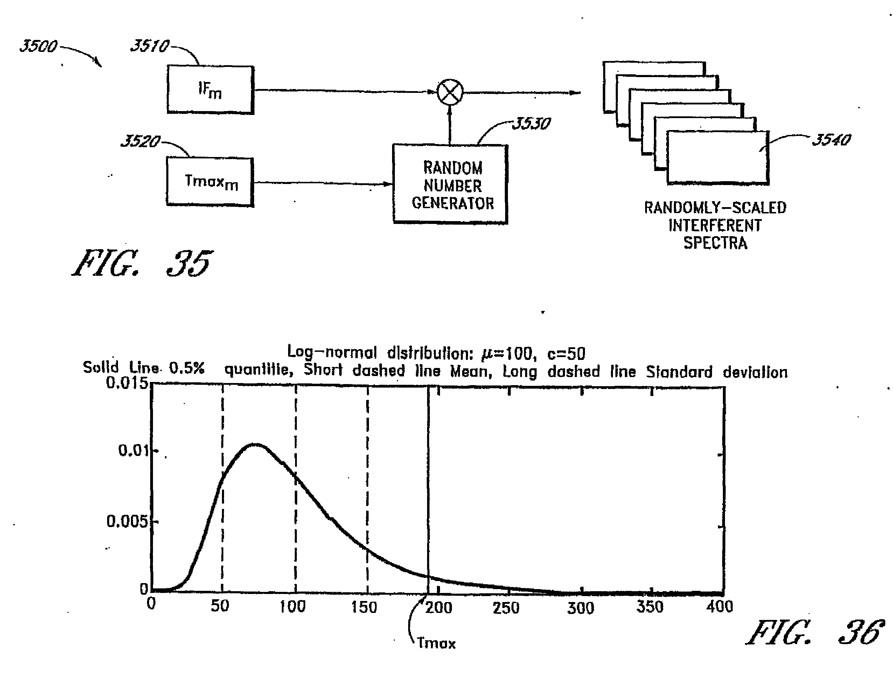

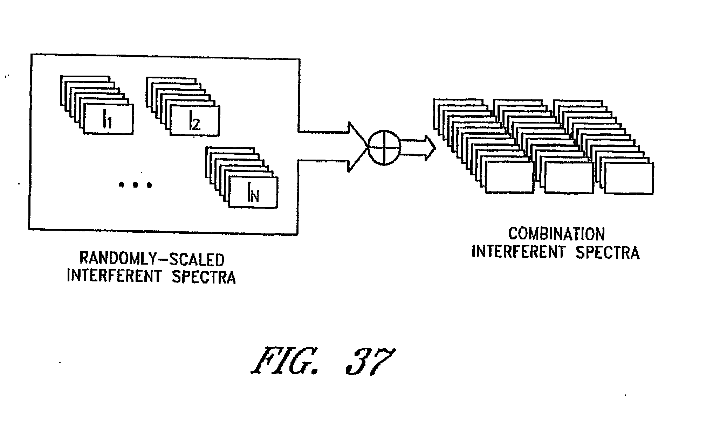

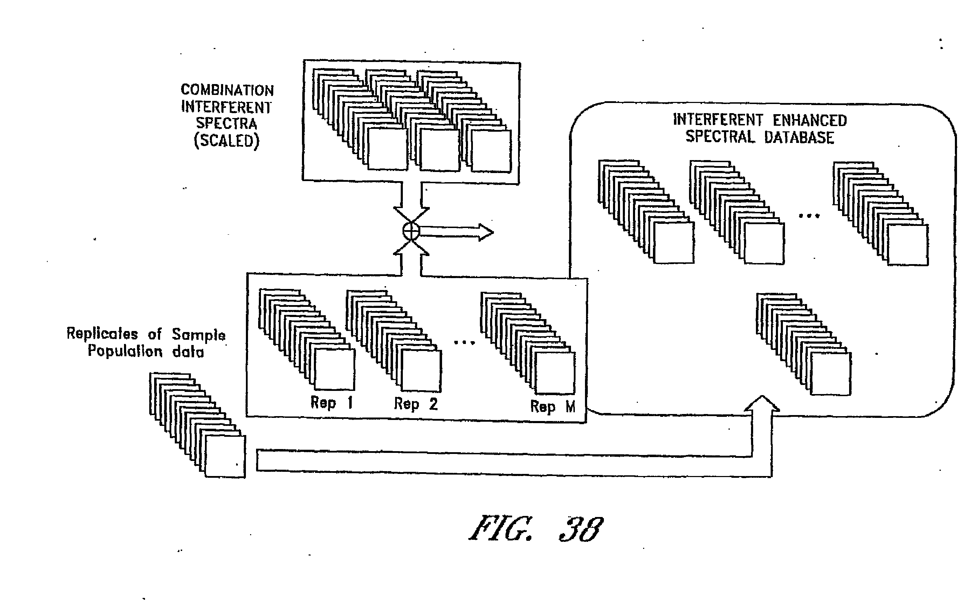

One embodiment of each of the methods of Blocks 3410, 3420, 3430, 3440, 3450, and 3460 are now described for the example of using identifying interferents in a sample for generating an average calibration constant. As indicated in Block 3410, one step is to generate synthesized Sample Population spectra, by adding a random concentration of possible Library Interferents to each Sample Population spectrum. The spectra generated by the method of Block 3410 are referred to herein as an Interferent-Enhanced Spectral Database, or IESD. The IESD can be formed by the steps illustrated in FIGURES 35-38, where FIGURE 35 is a schematic diagram 3500 illustrating the generation of Randomly-Scaled Single Interferent Spectra, or RSIS; FIGURE 36 is a graph 3600 of the interferent scaling; FIGURE 37 is a schematic diagram illustrating the combination of RSIS into Combination Interferent Spectra, or CIS; and FIGURE 38 is a schematic diagram illustrating the combination of CIS and the Sample Population spectra into an IESD.

The first step in Block 3410 is shown in FIGURES 35 and 36. As shown schematically in flowchart 3500 in FIGURE 35, and in graph 3600 in FIGURE 36, a plurality of RSIS (Block 3540) are formed by combinations of each previously identified Library Interferent having spectrum $IF_m$ (Block 3510), multiplied by the maximum concentration $Tmax_m$ (Block 3520) that is scaled by a random factor between zero and one (Block 3530), as indicated by the distribution of the random number indicated in graph 3600. In one embodiment, the scaling places the maximum concentration at the 95th percentile of a log-normal distribution to produce a wide range of concentrations with the distribution having a standard deviation equal to half of its mean value. The distribution of the random numbers in graph 3600 are a log-normal distribution of $\mu$=100, $\sigma$=50.

Once the individual Library Interferent spectra have been multiplied by the random concentrations to produce the RSIS, the RSIS are combined to produce a large population of interferent-only spectra, the CIS, as illustrated in FIGURE 37. The individual RSIS are combined independently and in random combinations, to produce a large family of CIS, with each spectrum within the CIS consisting of a random combination of RSIS, selected from the full set of identified Library Interferents. The method illustrated in FIGURE 37 produces adequate variability with respect to each interferent, independently across separate interferents.

The next step combines the CIS and replicates of the Sample Population spectra to form the IESD, as illustrated in FIGURE 38. Since the Interferent Data and Sample Population spectra may have been obtained at different pathlengths, the CIS are first scaled (i.e., multiplied) to the same pathlength. The Sample Population database is then replicated M times, where M depends on the size of the database, as well as the number of interferents to be treated. The IESD includes M copies of each of the Sample Population spectra, where one copy is the original Sample Population Data, and the remaining M-1 copies each have an added random one of the CIS spectra. Each of the IESD spectra has an associated analyte concentration from the Sample Population spectra used to form the particular IESD spectrum.

In one embodiment, a 10-fold replication of the Sample Population database is used for 130 Sample Population spectra obtained from 58 different individuals and 18 Library Interferents. Greater spectral variety among the Library Interferent spectra requires a smaller replication factor, and a greater number of Library Interferents requires a larger replication factor.

The steps of Blocks 3420, 3430, 3440, and 3450 are executed to repeatedly combine different ones of the spectra of the IESD to statistically average out the effect of the identified Library Interferents. First, as noted in Block 3420, the IESD is partitioned into two subsets: a calibration set and a test set. As described subsequently, the repeated partitioning of the IESD into different calibration and test sets improves the statistical significance of the calibration constant. In one embodiment, the calibration set is a random selection of some of the IESD spectra and the test set are the unselected IESD spectra. In a preferred embodiment, the calibration set includes approximately two-thirds of the IESD spectra.

In an alternative embodiment, the steps of Blocks 3420, 3430, 3440, and 3450 are replaced with a single calculation of an average calibration constant using all available data.

Next, as indicted in Block 3430, the calibration set is used to generate a calibration constant for predicting the analyte concentration from a sample measurement. First an analyte spectrum is obtained. For the embodiment of glucose determined from absorption measurements, a glucose absorption spectrum is indicated as $a_G$. The calibration constant is then generated as follows. Using the calibration set having calibration spectra $C = \{C_1, C_2, \ldots, C_n\}$ and corresponding glucose concentration values $G = \{g_1, g_2, \ldots, g_n\}$, then glucose-free spectra $C' = \{C'_1, C'_2, \ldots, C'_n\}$ can be calculated as: $C'_j = C_j - a_G g_j$. Next, the calibration constant, $\kappa$, is calculated from C' and $a_G$, according to the following 5 steps:

1) C' is decomposed into $C' = A_{c'} \Delta_{c'} B_{c'}$, that is, a singular value decomposition,
where the A-factor is an orthonormal basis of column space, or span, of C';
2) $A_{c'}$ is truncated to avoid overfitting to a particular column rank r, based on the sizes of the diagonal entries of $\Delta$ (the singular values of C' ). The selection of r involves a trade-off between the precision and stability of the calibration, with a larger r resulting in a more precise but less stable solution. In one embodiment, each spectrum C includes 25 wavelengths, and r ranges from 15 to 19;
3) The first r columns of $A_{c'}$ are taken as an orthonormal basis of span( C' );
4) The projection from the background is found as the product $P_{c'} = A_{c'} A_{c'}^T$, that is the orthogonal projection onto the span of C', and the complementary, or nulling projection $P_{c'}^\perp = 1 - P_{c'}$, which forms the projection onto the complementary subspace $C'^\perp$, is calculated; and
5) The calibration vector $\kappa$ is then found by applying the nulling projection to the absorption spectrum of the analyte of interest: $\kappa_{RAW} = P_{c'}^\perp a_G$ ,and normalizing: $\kappa = \kappa_{RAW} / K_{RAW}$ , $a_G \rangle$, where the angle brackets $\langle , \rangle$ denote the standard inner (or dot) product of vectors. The normalized calibration constant produces a unit response for a unit $a_G$ spectral input for one particular calibration set.

Next, the calibration constant is used to estimate the analyte concentration in the test set (Block 3440). Specifically, each spectrum of the test set (each spectrum having an associated glucose concentration from the Sample Population spectra used to generate the test set) is multiplied by the calibration vector $\kappa$ from Block 3430 to calculate an estimated glucose concentration. The error between the calculated and known glucose concentration is then calculated (Block 3450). Specifically, the measure of the error can include a weighted value averaged over the entire test set according to $1/rms^2$.

Blocks 3420, 3430, 3440, and 3450 are repeated for many different random combinations of calibration sets. Preferably, Blocks 3420, 3430, 3440, and 3450 are repeated are repeated hundreds to thousands of times. Finally, an average calibration constant is calculated from the calibration and error from the many calibration and test sets (Block 3460). Specifically, the average calibration is computed as weighted average calibration vector. In one embodiment the weighting is in proportion to a normalized rms, such as the $\kappa_{ave} = \kappa^* rms^2/\Sigma(rms^2)$ for all tests.

With the last of Block 3130 executed according to FIGURE 34, the average calibration constant $\kappa_{ave}$ is applied to the obtained spectrum (Block 3140).

Accordingly, one embodiment of a method of computing a calibration constant based on identified interferents can be summarized as follows:

> 1. Generate synthesized Sample Population spectra by adding the RSIS to raw (interferent-free) Sample Population spectra, thus forming an Interferent Enhanced Spectral Database (IESD) -- each spectrum of the IESD is synthesized from one spectrum of the Sample Population, and thus each spectrum of the IESD has at least one associated known analyte concentration
> 2. Separate the spectra of the IESD into a calibration set of spectra and a test set of spectra
> 3. Generate a calibration constant for the calibration set based on the calibration set spectra and their associated known correct analyte concentrations (e.g., using the matrix manipulation outlined in five steps above)
> 4. Use the calibration constant generated in step 3 to calculate the error in the corresponding test set as follows (repeat for each spectrum in the test set):
>
>> a. Multiply (the selected test set spectrum) x (average calibration constant generated in step 3) to generate an estimated glucose concentration
>> b. Evaluate the difference between this estimated glucose concentration and the known, correct glucose concentration associated with the selected test spectrum to generate an error associated with the selected test spectrum
>
> 5. Average the errors calculated in step 4 to arrive at a weighted or average error for the current calibration set - test set pair
> 6. Repeat steps 2 through 5 n times, resulting in n calibration constants and n average errors
> 7. Compute a "grand average" error from the n average errors and an average calibration constant from the n calibration constants (preferably weighted averages wherein the largest average errors and calibration constants are discounted), to arrive at a calibration constant which is minimally sensitive to the effect of the identified interferents

EXAMPLE 1

One example of certain methods disclosed herein is illustrated with reference to the detection of glucose in blood using mid-IR absorption spectroscopy. Table 2 lists 10 Library Interferents (each having absorption features that overlap with glucose) and the corresponding maximum concentration of each Library Interferent. Table 2 also lists a Glucose Sensitivity to Interferent without and with training. The Glucose Sensitivity to Interferent is the calculated change in estimated glucose concentration for a unit change in interferent concentration. For a highly glucose selective analyte detection technique, this value is zero. The Glucose Sensitivity to Interferent without training is the Glucose Sensitivity to Interferent where the calibration has been determined using the methods above without any identified interferents. The Glucose Sensitivity to Interferent with training is the Glucose Sensitivity to Interferent where the calibration has been determined using the methods above with the appropriately identified interferents. In this case, least improvement (in terms of reduction in sensitivity to an interferent) occurs for urea, seeing a factor of 6.4 lower sensitivity, followed by three with ratios from 60 to 80 in improvement. The remaining six all have seen sensitivity factors reduced by over 100, up to over 1600. The decreased Glucose Sensitivity to Interferent with training indicates that the methods are effective at producing a calibration constant that is selective to glucose in the presence of interferents.

Table 2. Rejection of 10 interfering substances

| Library Interferent | Maximum Concentration | Glucose Sensitivity to Interferent w/o training | Glucose Sensitivity to Interferent w/ training |
|---|---|---|---|
| Sodium Bicarbonate | 103 | 0.330 | 0.0002 |
| Urea | 100 | -0.132 | 0.0206 |
| Magnesium Sulfate | 0.7 | 1.056 | -0.0016 |
| Naproxen | 10 | 0.600 | -0.0091 |
| Uric Acid | 12 | -0.557 | 0.0108 |
| Salicylate | 10 | 0.411 | -0.0050 |
| Glutathione | 100 | 0.041 | 0.0003 |
| Niacin | 1.8 | 1.594 | -0.0086 |
| Nicotinamide | 12.2 | 0.452 | -0.0026 |
| Chlorpropamide | 18.3 | 0.334 | 0.0012 |

EXAMPLE 2

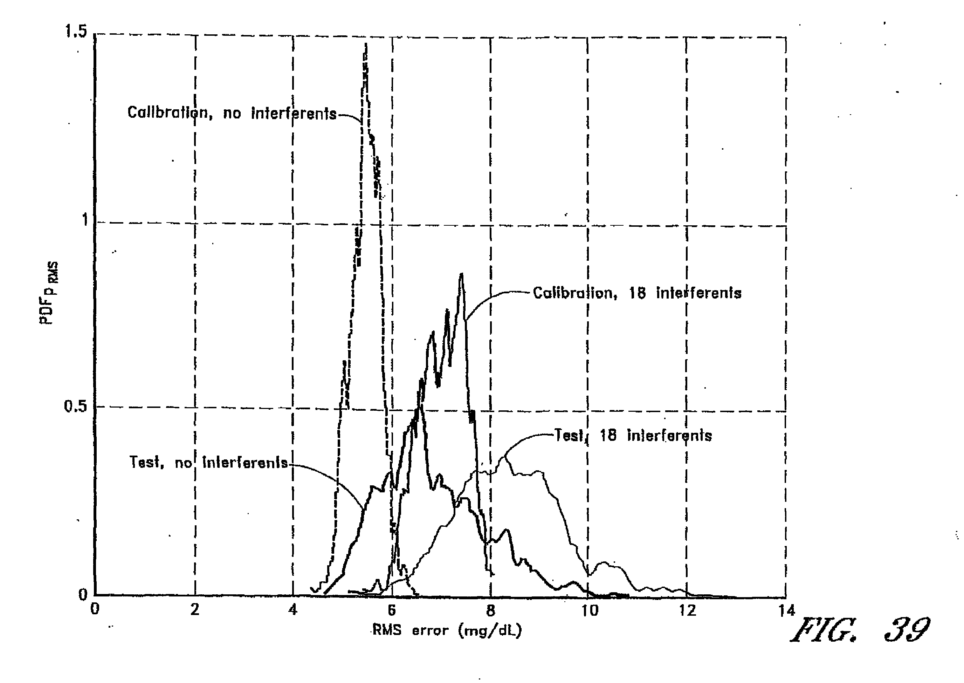

Another example illustrates the effect of the methods for 18 interferents. Table 3 lists of 18 interferents and maximum concentrations that were modeled for this example, and the glucose sensitivity to the interferent without and with training. The table summarizes the results of a series of 1000 calibration and test simulations that were performed both in the absence of the interferents, and with all interferents present. FIGURE 39 shows the distribution of the R.M.S. error in the glucose concentration estimation for 1000 trials. While a number of substances show significantly less sensitivity (sodium bicarbonate, magnesium sulfate, tolbutamide), others show increased sensitivity (ethanol, acetoacetate), as listed in Table 3. The curves in FIGURE 39 are for calibration set and the test set both without any interferents and with all 18 interferents. The interferent produces a degradation of performance, as can be seen by comparing the calibration or test curves of FIGURE 39. Thus, for example, the peaks appear to be shifted by about 2 mg/dL, and the width of the distributions is increased slightly. The reduction in height of the peaks is due to the spreading of the distributions, resulting in a modest degradation in performance.

Table 3. List of 18 Interfering Substances with maximum concentrations and Sensitivity with respect to interferents, with/without training

| | Library Interferent | Conc. (mg/dL) | Glucose Sensitivity to Interferent w/o training | Glucose Sensitivity to Interferent w/ training |
|---|---|---|---|---|
| 1 | Urea | 300 | -0.167 | -0.100 |
| 2 | Ethanol | 400.15 | -0.007 | -0.044 |
| 3 | Sodium Bicarbonate | 489 | 0.157 | -0.093 |
| 4 | Acetoacetate Li | 96 | 0.387 | 0.601 |
| 5 | Hydroxybutyric Acid | 465 | -0.252 | -0.101 |
| 6 | Magnesium Sulfate | 29.1 | 2.479 | 0.023 |
| 7 | Naproxen | 49.91 | 0.442 | 0.564 |
| 8 | Salicylate | 59.94 | 0.252 | 0.283 |
| 9 | Ticarcillin Disodium | 102 | -0.038 | -0.086 |
| 10 | Cefazolin | 119.99 | -0.087 | -0.006 |
| 11 | Chlorpropamide | 27.7 | 0.387 | 0.231 |
| 12 | Nicotinamide | 36.6 | 0.265 | 0.366 |
| 13 | Uric Acid | 36 | -0.641 | -0.712 |

(continued)

|  | Library Interferent | Conc. (mg/dL) | Glucose Sensitivity to Interferent w/o training | Glucose Sensitivity to Interferent w/ training |
|---|---|---|---|---|
| 14 | Ibuprofen | 49.96 | -0.172 | -0.125 |
| 15 | Tolbutamide | 63.99 | 0.132 | 0.004 |
| 16 | Tolazamide | 9.9 | 0.196 | 0.091 |
| 17 | Bilirubin | 3 | -0.391 | -0.266 |
| 18 | Acetaminophen | 25.07 | 0.169 | 0.126 |

EXAMPLE 3

In a third example, certain methods disclosed herein were tested for measuring glucose in blood using mid-IR absorption spectroscopy in the presence of four interferents not normally found in blood (Type-B interferents) and that may be common for patients in hospital intensive care units (ICUs). The four Type-B interferents are mannitol, dextran, n-acetyl L cysteine, and procainamide.

Of the four Type-B interferents, mannitol and dextran have the potential to interfere substantially with the estimation of glucose: both are spectrally similar to glucose (see FIGURE 1), and the dosages employed in ICUs are very large in comparison to typical glucose levels. Mannitol, for example, may be present in the blood at concentrations of 2500 mg/dL, and dextran may be present at concentrations in excess of 5000 mg/dL. For comparison, typical plasma glucose levels are on the order of 100 - 200 mg/dL. The other Type-B interferents, n-acetyl L cysteine and procainamide, have spectra that are quite unlike the glucose spectrum.

FIGURES 40A, 40B, 40C, and 40D each have a graph showing a comparison of the absorption spectrum of glucose with different interferents taken using two different techniques: a Fourier Transform Infrared (FTIR) spectrometer having an interpolated resolution of 1 cm$^{-1}$ (solid lines with triangles); and by 25 finite-bandwidth IR filters having a Gaussian profile and full-width half-maximum (FWHM) bandwidth of 28 cm$^{-1}$ corresponding to a bandwidth that varies from 140 nm at 7.08 $\mu$m, up to 279 nm at 10 $\mu$m (dashed lines with circles). Specifically, the figures show a comparison of glucose with mannitol (FIGURE 40A), with dextran (FIGURE 40B), with n-acetyl L cysteine (FIGURE 40C), and with procainamide (FIGURE 40D), at a concentration level of 1 mg/dL and path length of 1 $\mu$m. The horizontal axis in FIGURES 40A-40D has units of wavelength in microns ($\mu$m), ranging from 7 $\mu$m to 10 $\mu$m, and the vertical axis has arbitrary units.

The central wavelength of the data obtained using filter is indicated in FIGURES 40A, 40B, 40C, and 40D by the circles along each dashed curve, and corresponds to the following wavelengths, in microns: 7.082, 7.158, 7.241, 7.331, 7.424, 7.513, 7.605, 7.704, 7.800, 7.905, 8.019, 8.150, 8.271, 8.598, 8.718, 8.834, 8.969, 9.099, 9.217, 9.346, 9.461, 9.579, 9.718, 9.862, and 9.990. The effect of the bandwidth of the filters on the spectral features can be seen in FIGURES 40A-40D as the decrease in the sharpness of spectral features on the solid curves and the relative absence of sharp features on the dashed curves.

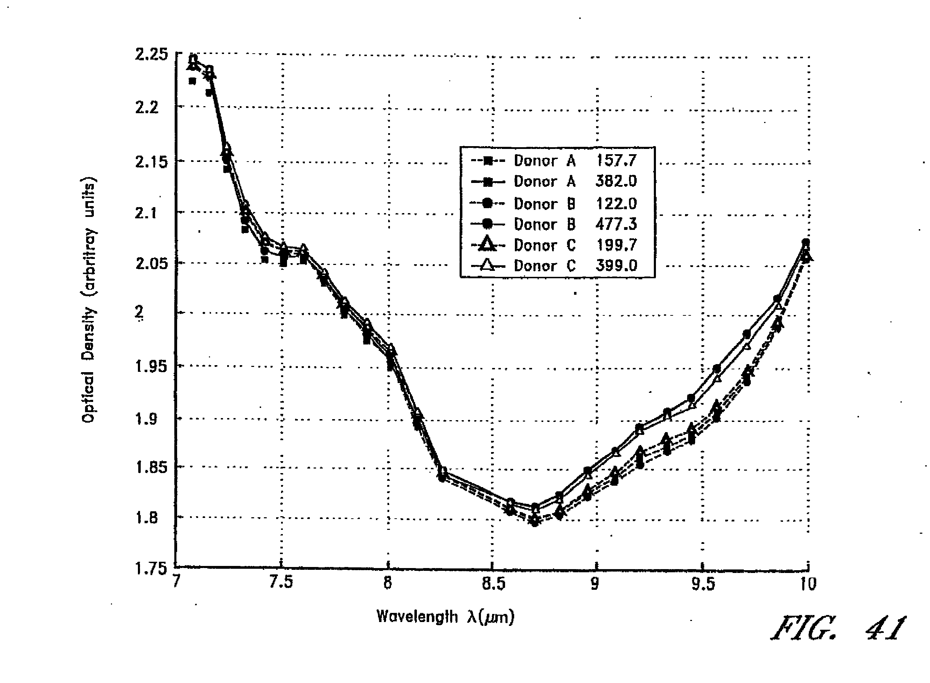

FIGURE 41 shows a graph of the blood plasma spectra for 6 blood samples taken from three donors in arbitrary units for a wavelength range from 7 $\mu$m to 10 $\mu$m, where the symbols on the curves indicate the central wavelengths of the 25 filters. The 6 blood samples do not contain any mannitol, dextran, n-acetyl L cysteine, and procainamide - the Type-B interferents of this Example, and are thus a Sample Population. Three donors (indicated as donor A, B, and C) provided blood at different times, resulting in different blood glucose levels, shown in the graph legend in mg/dL as measured using a YSI Biochemistry Analyzer (YSI Incorporated, Yellow Springs, OH). The path length of these samples, estimated at 36.3 $\mu$m by analysis of the spectrum of a reference scan of saline in the same cell immediately prior to each sample spectrum, was used to normalize these measurements. This quantity was taken into account in the computation of the calibration vectors provided, and the application of these vectors to spectra obtained from other equipment would require a similar pathlength estimation and normalization process to obtain valid results.

Next, random amounts of each Type-B interferent of this Example are added to the spectra to produce mixtures that, for example could make up an Interferent Enhanced Spectral. Each of the Sample Population spectra was combined with a random amount of a *single* interferent added, as indicated in Table 4, which lists an index number N, the Donor, the glucose concentration (GLU), interferent concentration (conc(IF)), and the interferent for each of 54 spectra. The conditions of Table 4 were used to form combined spectra including each of the 6 plasma spectra was combined with 2 levels of each of the 4 interferents.

Table 4. Interferent Enhanced Spectral Database for Example 3.

| N | Donor | GLU | conc(IF) | IF |
|---|---|---|---|---|
| 1 | A | 157.7 | | N/A |
| 2 | A | 382 | | N/A |
| 3 | B | 122 | | N/A |
| 4 | B | 477.3 | | N/A |
| 5 | C | 199.7 | | N/A |
| 6 | C | 399 | | N/A |
| 7 | A | 157.7 | 1001.2 | Mannitol |
| 8 | A | 382 | 2716.5 | Mannitol |
| 9 | A | 157.7 | 1107.7 | Mannitol |
| 10 | A | 382 | 1394.2 | Mannitol |
| 11 | B | 122 | 2280.6 | Mannitol |
| 12 | B | 477.3 | 1669.3 | Mannitol |
| 13 | B | 122 | 1710.2 | Mannitol |
| 14 | B | 477.3 | 1113.0 | Mannitol |
| 15 | C | 199.7 | 1316.4 | Mannitol |
| 16 | C | 399 | 399.1 | Mannitol |
| 17 | C | 199.7 | 969.8 | Mannitol |
| 18 | C | 399 | 2607.7 | Mannitol |
| 19 | A | 157.7 | 8.8 | N Acetyl L Cysteine |
| 20 | A | 382 | 2.3 | N Acetyl L Cysteine |
| 21 | A | 157.7 | 3.7 | N Acetyl L Cysteine |
| 22 | A | 382 | 8.0 | N Acetyl L Cysteine |
| 23 | B | 122 | 3.0 | N Acetyl L Cysteine |
| 24 | B | 477.3 | 4.3 | N Acetyl L Cysteine |
| 25 | B | 122 | 8.4 | N Acetyl L Cysteine |
| 26 | B | 477.3 | 5.8 | N Acetyl L Cysteine |
| 27 | C | 199.7 | 7.1 | N Acetyl L Cysteine |
| 28 | C | 399 | 8.5 | N Acetyl L Cysteine |
| 29 | C | 199.7 | 4.4 | N Acetyl L Cysteine |
| 30 | C | 399 | 4.3 | N Acetyl L Cysteine |
| 31 | A | 157.7 | 4089.2 | Dextran |
| 32 | A | 382 | 1023.7 | Dextran |
| 33 | A | 157.7 | 1171.8 | Dextran |
| 34 | A | 382 | 4436.9 | Dextran |
| 35 | B | 122 | 2050.6 | Dextran |
| 36 | B | 477.3 | 2093.3 | Dextran |
| 37 | B | 122 | 2183.3 | Dextran |
| 38 | B | 477.3 | 3750.4 | Dextran |

(continued)

| N | Donor | GLU | conc(IF) | IF |
|---|---|---|---|---|
| 39 | C | 199.7 | 2598.1 | Dextran |
| 40 | C | 399 | 2226.3 | Dextran |
| 41 | C | 199.7 | 2793.0 | Dextran |
| 42 | C | 399 | 2941.8 | Dextran |
| 43 | A | 157.7 | 22.5 | Procainamide |
| 44 | A | 382 | 35.3 | Procainamide |
| 45 | A | 157.7 | 5.5 | Procainamide |
| 46 | A | 382 | 7.7 | Procainamide |
| 47 | B | 122 | 18.5 | Procainamide |
| 48 | B | 477.3 | 5.6 | Procainamide |
| 49 | B | 122 | 31.8 | Procainamide |
| 50 | B | 477.3 | 8.2 | Procainamide |
| 51 | C | 199.7 | 22.0 | Procainamide |
| 52 | C | 399 | 9.3 | Procainamide |
| 53 | C | 199.7 | 19.7 | Procainamide |
| 54 | C | 399 | 12.5 | Procainamide |

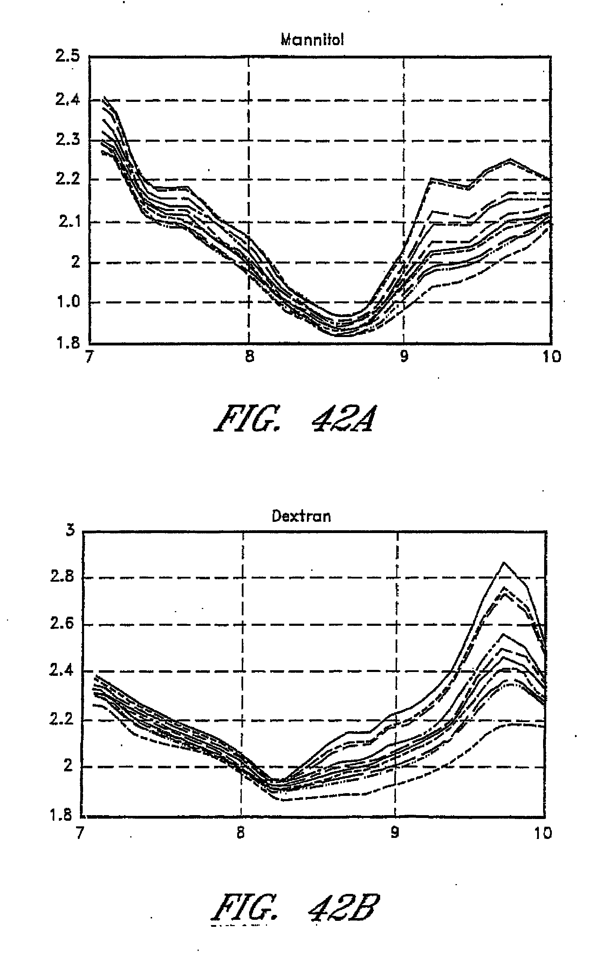

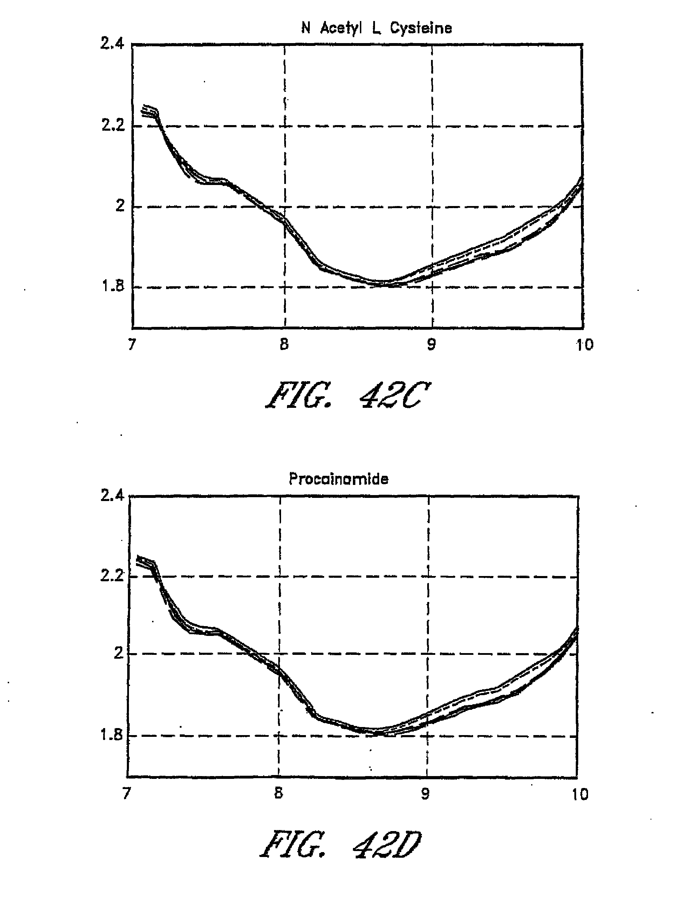

FIGURES 42A, 42B, 42C, and 42D contain spectra formed from the conditions of Table 4. Specifically, the figures show spectra of the Sample Population of 6 samples having random amounts of mannitol (FIGURE 42A), dextran (FIGURE 42B), n-acetyl L cysteine (FIGURE 42C), and procainamide (FIGURE 42D), at a concentration levels of 1 mg/dL and path lengths of 1 $\mu$m.

Next, calibration vectors were generated using the spectra of FIGURES 42A-42D, in effect reproducing the steps of Block 3120. The next step of this Example is the spectral subtraction of water that is present in the sample to produce water-free spectra. As discussed above, certain methods disclosed herein provide for the estimation of an analyte concentration in the presence of interferents that are present in both a sample population and the measurement sample (Type-A interferents), and it is not necessary to remove the spectra for interferents present in Sample Population and sample being measured. The step of removing water from the spectrum is thus an alternative embodiment of the disclosed methods.

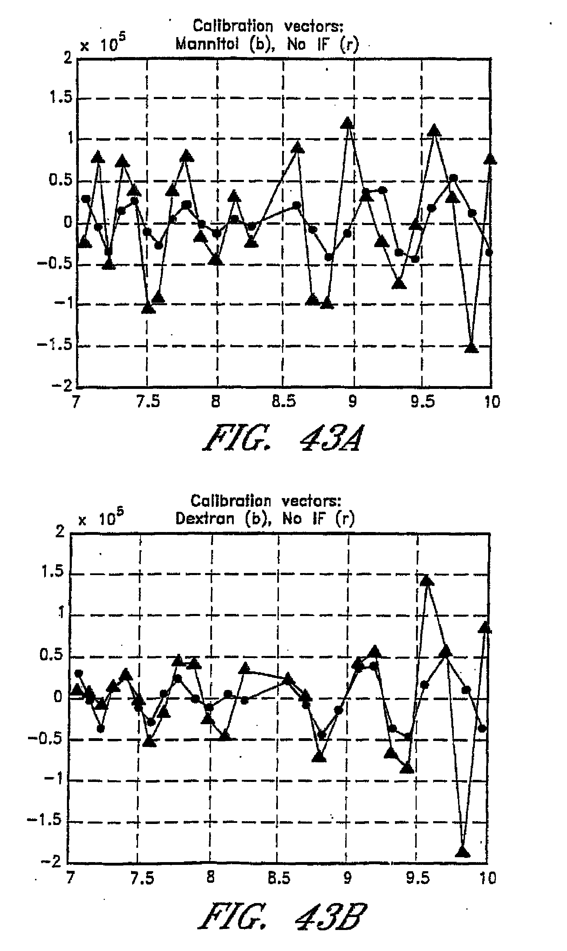

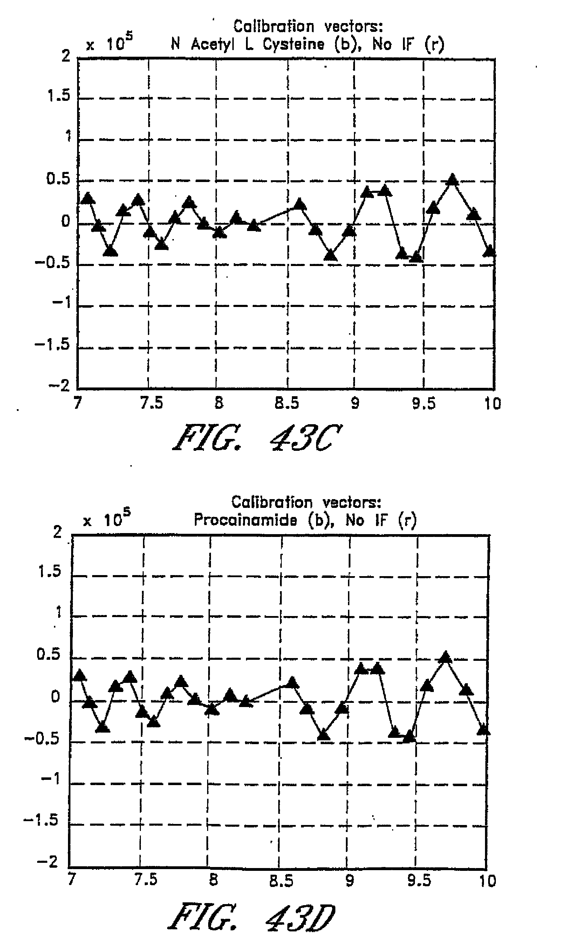

The calibration vectors are shown in FIGURES 43A-43D for mannitol (FIGURE 43A), dextran (FIGURE 43B), n-acetyl L cysteine (FIGURE 43C), and procainamide (FIGURE 43D) for water-free spectra. Specifically each one of FIGURES 43A-43D compares calibration vectors obtained by training in the presence of an interferent, to the calibration vector obtained by training on clean plasma spectra alone. The calibration vector is used by computing its dot-product with the vector representing (pathlength-normalized) spectral absorption values for the filters used in processing the reference spectra. Large values (whether positive or negative) typically represent wavelengths for which the corresponding spectral absorbance is sensitive to the presence of glucose, while small values generally represent wavelengths for which the spectral absorbance is insensitive to the presence of glucose. In the presence of an interfering substance, this correspondence is somewhat less transparent, being modified by the tendency of interfering substances to mask the presence of glucose.

The similarity of the calibration vectors obtained for minimizing the effects of the two interferents n-acetyl L cysteine and procainamide, to that obtained for pure plasma, is a reflection of the fact that these two interferents are spectrally quite distinct from the glucose spectrum; the large differences seen between the calibration vectors for minimizing the effects of dextran and mannitol, and the calibration obtained for pure plasma, are conversely representative of the large degree of similarity between the spectra of these substances and that of glucose. For those cases in which the interfering spectrum is similar to the glucose spectrum (that is, mannitol and dextran), the greatest change in the calibration vector. For those cases in which the interfering spectrum is different from the glucose spectrum (that is, n-acetyl L cysteine and procainamide), it is difficult to detect the difference between the calibration vectors obtained with and without the interferent.

It will be understood that the steps of methods discussed are performed in one embodiment by an appropriate processor (or processors) of a processing (i.e., computer) system executing instructions (code segments) stored in appropriate storage. It will also be understood that the disclosed methods and apparatus are not limited to any particular implementation or programming technique and that the methods and apparatus may be implemented using any appropriate techniques for implementing the functionality described herein. The methods and apparatus are not limited to any particular programming language or operating system. In addition, the various components of the apparatus may be included in a single housing or in multiple housings that communication by wire or wireless communication.

Further, the interferent, analyte, or population data used in the method may be updated, changed, added, removed, or otherwise modified as needed. Thus, for example, spectral information and/or concentrations of interferents that are accessible to the methods may be updated or changed by updating or changing a database of a program implementing the method. The updating may occur by providing new computer readable media or over a computer network. Other changes that may be made to the methods or apparatus include, but are not limited to, the adding of additional analytes or the changing of population spectral information.

One embodiment of each of the methods described herein may include a computer program accessible to and/or executable by a processing system, e.g., a one or more processors and memories that are part of an embedded system. Thus, as will be appreciated by those skilled in the art, embodiments of the disclosed inventions may be embodied as a method, an apparatus such as a special purpose apparatus, an apparatus such as a data processing system, or a carrier medium, e.g., a computer program product. The carrier medium carries one or more computer readable code segments for controlling a processing system to implement a method. Accordingly, various ones of the disclosed inventions may take the form of a method, an entirely hardware embodiment, an entirely software embodiment or an embodiment combining software and hardware aspects. Furthermore, any one or more of the disclosed methods (including but not limited to the disclosed methods of measurement analysis, interferent determination, and/or calibration constant generation) may be stored as one or more computer readable code segments or data compilations on a carrier medium. Any suitable computer readable carrier medium may be used including a magnetic storage device such as a diskette or a hard disk; a memory cartridge, module, card or chip (either alone or installed within a larger device); or an optical storage device such as a CD or DVD.

Reference throughout this specification to "one embodiment" or "an embodiment" means that a particular feature, structure or characteristic described in connection with the embodiment is included in at least one embodiment. Thus, appearances of the phrases "in one embodiment" or "in an embodiment" in various places throughout this specification are not necessarily all referring to the same embodiment. Furthermore, the particular features, structures or characteristics may be combined in any suitable manner, as would be apparent to one of ordinary skill in the art from this disclosure, in one or more embodiments.

Similarly, it should be appreciated that in the above description of embodiments, various features of the inventions are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Rather, as the following claims reflect, inventive aspects lie in a combination of fewer than all features of any single foregoing disclosed embodiment. Thus, the claims following the Detailed Description are hereby expressly incorporated into this Detailed Description, with each claim standing on its own as a separate embodiment.

Further information on analyte detection systems, sample elements, algorithms and methods for computing analyte concentrations, and other related apparatus and methods can be found in U.S. Patent Application Publication No. 2003/0090649, published May 15, 2003, titled REAGENT-LESS WHOLE BLOOD GLUCOSE METER; U.S. Patent Application Publication No. 2003/0178569, published September 25, 2003, titled PATHLENGTH-INDEPENDENT METHODS FOR OPTICALLY DETERMINING MATERIAL COMPOSITION; U.S. Patent Application Publication No. 2004/0019431, published January 29, 2004, titled METHOD OF DETERMINING AN ANALYTE CONCENTRATION IN A SAMPLE FROM AN ABSORPTION SPECTRUM; U.S. Patent Application Publication No. 2005/0036147, published February 17, 2005, titled METHOD OF DETERMINING ANALYTE CONCENTRATION IN A SAMPLE USING INFRARED TRANSMISSION DATA; and U.S. Patent Application Publication No. 2005/0038357, published on February 17, 2005, titled SAMPLE ELEMENT WITH BARRIER MATERIAL. The entire contents of each of the above-mentioned publications are hereby incorporated by reference herein and are made a part of this specification.

A number of applications, publications and external documents are incorporated by reference herein. Any conflict or contradiction between a statement in the bodily text of this specification and a statement in any of the incorporated documents is to be resolved in favor of the statement in the bodily text.

INFUSION AND MONITORING SYSTEM

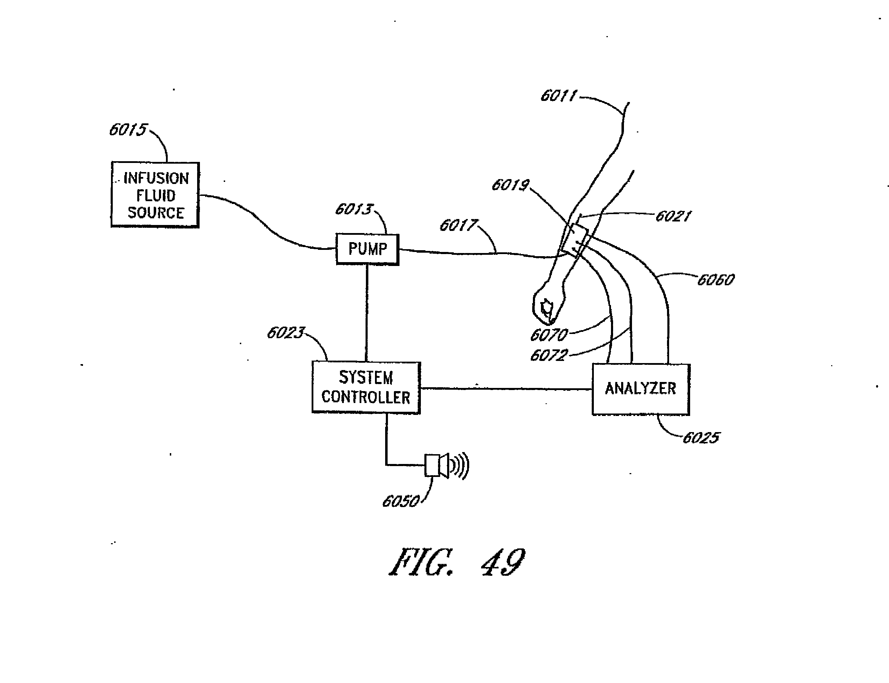

With reference to the drawings, for purposes of illustration, and particularly to FIG. 49, there is shown a system for infusing an infusion fluid into a patient 6011 while intermittently monitoring a number of parameters of the patient's blood. The infusion and monitoring system includes an infusion pump 6013 for pumping the infusion fluid in a forward direction from a source 6015 to the patient, via an infusion tube 6017, a blood chemistry sensor assembly 6019, and a catheter 6021. The infusion fluid preferably is a physiological isotonic saline solution of appropriate concentration, although the fluid also may incorporate selected nutrients or medications for delivery to the patient.

At appropriate times, a system controller 6023 causes the infusion pump 6013 to reverse its direction, and instead to draw blood from the patient 6011 through the catheter 6021 and into the sensor assembly 6019. This reversal of the pump's direction may occur at predetermined time intervals, or upon receipt by the controller of a manual command issued by a caregiver.

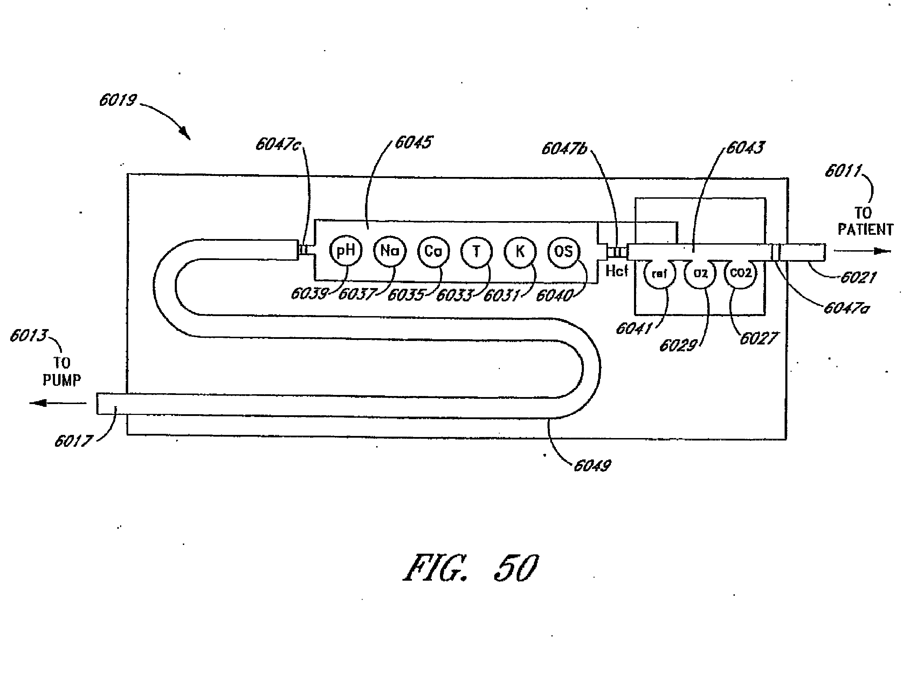

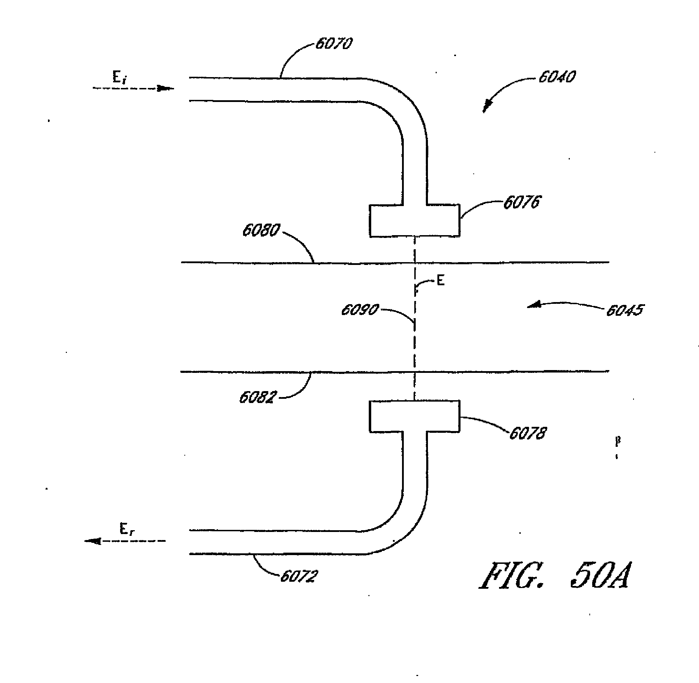

One suitable blood chemistry sensor assembly 6019 is depicted in FIG. 50. It includes a plurality of analytical sensors, each producing, modifying, or transmitting a signal indicative of one or more parameters or characteristics of the adjacent fluid. As used herein, the terms "analytical sensor" or "sensor" are used interchangeably and are broad terms and are used in their ordinary sense and include, without limitation, except as explicitly stated, devices, meters, modules, or units that produce, modify, or transmit a signal indicative of one or more parameters or characteristics of a sample material under analysis using electrical techniques, optical techniques, electrochemical techniques, or any combination of these techniques. Embodiments of the blood chemistry sensor assembly 6019 may include one or more electrical sensors, one or more optical sensors, one or more electrochemical sensors, or a plurality of electrical, optical, and/or electrochemical sensors. Examples of the parameters or characteristics of the adjacent fluid that may be sensed include concentrations of carbon dioxide, oxygen, potassium, calcium, and sodium. Other parameters that can be sensed include hematocrit, temperature, and pH. Additionally, the blood chemistry sensor assembly 6019 may include analytical sensors to measure the concentration of analytes in the presence of interferents, as described herein.

To perform the desired analysis, a sample of the patient's blood may be drawn into a position where it contacts or otherwise engages one or more of the analytical sensors of the sensor assembly 6019. In addition, sufficient additional blood preferably is drawn to minimize the effects of any dilution of the blood by the adjacent infusion fluid.

After the patient's blood sample has been drawn to the appropriate position, signals from the various analytical sensors are read and analyzed by an analyzer 6025 (FIG. 49). The signals produced, modified, or transmitted by the analytical sensors may be electrical and/or optical. For example, a temperature sensor 6033 disposed in the sensor assembly 6019 (FIG. 50) may comprise a thermistor that outputs an electrical signal that is in proportion to the temperature of the blood sample. Electrical signals may be carried by an electrical line 6060 extending from the sensor assembly 6019 to the analyzer 6025. Additionally, the electrical line 6060 may carry signals from the analyzer 6025 or the controller 6023 to the sensor assembly 6019. These signals may be used to monitor, control, or regulate the analytical sensors. Although one electrical line 6060 is shown in the embodiment depicted in FIG. 49, it is appreciated that more than one electrical line 6060 may be used. Optionally, the electrical line 6060 may be replaced by a wireless communications system.

In some embodiments, one or more of the analytical sensors comprises an optical sensor. The optical sensor may enable spectroscopic or photometric measurements of the properties or characteristics of the blood sample and may utilize the visible, near-infrared, mid-infrared, or other portions of the electromagnetic spectrum. FIG. 49 depicts two optical lines 6070 and 6072 extending between the sensor assembly 6019 and the analyzer 6025. In some embodiments, the optical lines 6070 and 6072 may comprise an optical fiber or a fiber optic bundle. The optical fibers may be single-mode or multi-mode. It is preferred that the optical lines 6070 and 6072 be substantially transmissive to the wavelengths of electromagnetic radiation used by the optical sensor. Although two optical lines 6070 and 6072 are shown in the embodiment depicted in FIG. 49, it is appreciated that other embodiments may use one, two, three, four, or more optical lines to interconnect the sensor assembly 6019 and the analyzer 6025. Optionally, the electrical line 6060 and the optical lines 6070 and 6072 may be collected together into one or more bundles passing between the sensor assembly 6019 and the analyzer 6025.

After a blood sample has been drawn into the sensor assembly 6019, it is preferred, but not necessary, that a brief period of time, such as about 8 seconds, be allowed to elapse before the sensors are read so that a stable sensor output is achieved. The analyzer 6025 converts electrical signals from one or more electrical sensors, if present, into corresponding indications of the presence or concentrations of one or more components, or of other parameters, of the patient's blood. Similarly, the analyzer 6025 converts optical signals from one or more optical sensors into corresponding indications of the presence or concentrations of one or more components, or other parameters, of the patient's blood. These indications can be read by a caregiver monitoring the patient.

Thereafter, after the analysis has been completed, the controller 6023 again operates the pump 6013 in its forward direction, to flush the blood sample out of the sensor assembly 6019 and back into the patient 6011. Pumping of the infusion fluid into the patient then resumes. This pumping can occur at a relatively low flow rate of about 1 to 10 milliliters per hour.

When the infusion pump 6013 is operated in its rearward direction, it draws the patient's blood at a substantially constant flow rate. However, because the length and internal volume of the catheter 6021 located between the sensor assembly 6019 and the patient 6011 can vary, merely drawing the blood for a fixed time duration cannot ensure that the blood will reach its desired position in the sensor assembly without a possibly large draw of blood from the patient. Some means for sensing the arrival of the blood sample at its desired position, therefore, is required. If necessary, a dedicated sensor may be provided within the sensor assembly 6019, for sensing the arrival of the blood sample at its desired position. For example, some embodiments may use a sensor that detects changes in the color of the fluid, such as a color sensor, e.g., the colorimetric sensor 311 (FIG. 3). However, such a dedicated sensor may be eliminated in other embodiments to avoid added expense and complexity to the sensor assembly 6019. Such embodiments may detect the arrival of the blood sample as further described herein.

In some embodiments, the need for such a dedicated sensor within the sensor assembly 6019, for detecting the arrival of the blood sample at its desired position within the assembly, may be obviated by configuring the controller 6023 to monitor the signal from one or more of the analytical sensors that already are present within the assembly. In response to detecting a predetermined signal or change in signal from the sensor or sensors being monitored, the controller ceases operating the pump 6013 in the rearward direction.

As mentioned above, an additional amount of blood is drawn from the patient 6011 after the sample first reaches the analytical sensor being monitored, to minimize the dilution effects of the adjacent infusion fluid. The amount of additional blood required to minimize dilution effects depends, in part, on the dimension of the passageway through which the blood is drawn. Although several milliliters would be required to reduce any such dilution effects in a passageway having an inside diameter of about one millimeter and a length of about 0.25 meters, for example, the effects can be reduced to an acceptably small, and repeatable, level by drawing merely about 0.1-1.0 milliliters of additional blood after the blood has been detected to have arrived at the sensor being monitored. The controller 6023, therefore, is programmed to continue drawing blood for whatever time duration is required after detecting the arrival of the blood sample at the sensor before switching off the pump 6013.

Additionally, the controller 6023 preferably is programmed to actuate an alarm and to switch off the infusion pump 6013 if the arrival of the patient's blood sample has not been detected within a predetermined maximum time duration following initiation of pump's reversal and also if the arrival is detected to have occurred before a predetermined minimum time duration. This ensures that the pump is not operated indefinitely to draw blood from the patient in case of a sensor failure or other system failure, and it also ensures that the caregiver is alerted to a possible sensor failure, a blockage in the infusion tube 6017 or catheter 6021, or other system failure.

With reference again to FIG. 50, there is shown a first embodiment of a blood chemistry sensor assembly 6019 that can be incorporated into the blood analysis system. In addition to the temperature (T) sensor 6033 discussed previously, the sensor assembly is depicted to include a number of analytical sensors, including a carbon dioxide (CO2) sensor 6027, an oxygen (02) sensor 6029, a potassium (K) sensor 6031, a calcium (Ca) sensor 6035, a sodium (Na) sensor 6037, a pH sensor 6039, and an optical (OS) sensor 6040. The temperature sensor 6033 may comprise a thermistor. Each sensor may produce, modify, or transmit a signal indicative of the presence or concentration of a particular component, analyte, or interferent, or of another parameter of whatever fluid is located adjacent to it. In some embodiments, an analytical sensor may produce, modify, or transmit a signal that is indicative of the presence or concentration of more than one component, analyte, or interferent in the adjacent fluid. The signal may be transmitted from the sensor assembly 6019 to the analyzer 6025 via the electrical line 6060 and/or the optical lines 6070 or 6072.

In the embodiment illustrated in FIG. 50, the carbon dioxide sensor 6027 and the oxygen sensor 6029, as well as a reference sensor 6041, are located adjacent to a first chamber 6043 of the sensor assembly 6019, while the remaining sensors 6031-6040 are located adjacent to a second chamber 6045. In some embodiments, the reference sensor 6041 comprises an enzymeless electrode. In other embodiments of the sensor assembly 6019, the selection, location, and arrangement of the analytical sensors may be different. The sensor assembly 6019 may include a greater or lesser number of sensors and chambers than illustrated in FIG. 50. The sensor assembly 6019 may include a selection of electrical and/or optical sensors. Further, some embodiments may measure or detect a property or characteristic of the blood sample using both an electrical sensor and an optical sensor so as to increase accuracy and precision or to increase signal to noise or for some other purpose.

One optical sensor 6040 is illustrated in the embodiment shown in FIG. 50, although more than one may be used in other embodiments. The optical sensor 6040 may be used to perform spectroscopic and/or photometric measurements of the adjacent fluid. The optical measurements may be via absorption, reflection, or transmission and may use different wavelength regions of the electromagnetic spectrum, for example, visible, near-infrared, mid-infrared, or some other wavelength region. The optical sensor 6040 is illustrated in FIG. 50 as located within the second chamber 6045; however, in other embodiments the optical sensor 6040 may be located in the first chamber 6043 or in another position within the sensor assembly 6019.

In one embodiment, the optical sensor 6040 may comprise a sensor similar to the colorimetric sensor 311 (see FIG. 3), which may be used to measure the color of the adjacent fluid. Examples of a colorimetric sensor include, for example, an Optical Blood Leak/Blood vs. Saline Detector available from Introtek International (Edgewood, NJ).

The optical sensor 6040 may be employed in addition to sensors 6027-6039, as shown in the embodiment depicted in FIG. 50, or it may replace or act in conjunction with one or a combination of the other sensors. The spectroscopic and non-spectroscopic methods disclosed herein may be used to determine the presence or concentration of blood oxygen, carbon dioxide, sodium, potassium, calcium, or hematocrit. Optical sensors may also be used to determine the presence or concentration of other analytes or interferents as described herein. In the embodiment shown in FIG. 50, one or more of the oxygen, carbon dioxide, potassium, calcium, sodium, and hematocrit sensors 6027-6039 and 6047a-c may comprise an optical sensor and/or an electrical sensor.

The optical sensor 6040 may be used to determine the presence and/or concentration of more than one analyte. For example, the optical sensor 6040 may transmit an optical signal to the analyzer 6025 that represents or otherwise conveys spectroscopic or non-spectroscopic characteristics of a group of analytes or interferents. Accordingly, one embodiment of the sensor assembly 6019 may comprise the temperature sensor 6033, the pH sensor 6039, and a single optical sensor 6040 configured to transmit information about the concentrations of a group of analytes, such as, for example, oxygen, carbon dioxide, sodium, potassium, calcium, and other suitable characteristics such as, for example, hematocrit.

Embodiments of the sensor assembly 6019 may include one or more non-optical sensors to measure fluid properties and characteristics such as, for example, temperature, pH, pressure, salinity, electrical conductivity, and the like. Some embodiments may omit such sensors. It will be apparent to one of ordinary skill in the art that embodiments of the sensor assembly 6019 may include a selection of sensors, including optical and non-optical sensors, such that the blood analysis system may measure desired properties and characteristics of the fluid in within the system.

FIG. 50A is a top view of the optical sensor 6040 shown in FIG. 50. An energy source (not shown) generates an energy beam $E_i$ that is directed into the optical line 6070. The energy source may be disposed in, for example, the analyzer 6025. The energy beam $E_i$ may comprise a portion of the electromagnetic spectrum, such as, for example, visible, near-infrared, mid-infrared, or other suitable wavelengths.

The incident energy beam $E_i$ propagates along the optical line 6070 until it reaches the second chamber 6045. An optical coupler 6076, which may comprise a lens, may be used to focus or direct the energy beam E into the chamber 6045. The energy beam E passes through a first window 6080 that forms at least a portion of a side of the chamber 6045. The energy beam E is transmitted through the fluid sample 6090 within the chamber 6045. In the embodiment shown in FIG. 50A, the energy beam E exits the chamber 6045 through a second window 6082, which also forms at least a portion of a side of the chamber 6045. The energy beam E may be directed into the optical line 6072 by an optical coupler 6078, which may be generally similar to the optical coupler 6076, and thereby returned to the analyzer 6025.

The returned energy beam $E_r$ comprises an optical signal that carries information about the properties and characteristics of fluid sample 6090 in the chamber 6045. The analyzer 6025 may measure spectroscopic or non-spectroscopic properties of the returned energy beam $E_r$ and may comprise a spectrometer, a photometer, a colorimeter, a filter, and/or other optical devices. The analyzer 6025 may comprise an analyte detection system similar to any of the embodiments of the analyte detection system 1700 depicted in FIGS. 17 and 44-48. The analyzer 6025 may be configured to determine absorbance, reflectance, transmittance, color characteristics, or any other optical properties of the fluid sample 6090. The analyzer 6025 may also determine the presence or concentrations of one or more analytes of interest in the fluid under analysis.

In some embodiments, the energy beam E may comprise different wavelength ranges of the electromagnetic spectrum at different times. For example, the energy source may be selectively tunable by the system controller 6023 so as to emit an energy beam $E_i$ comprising a desired wavelength range. Alternatively, the energy beam $E_i$ from the energy source may pass through a filter wheel similar to the embodiment shown in FIG. 45, a tunable filter, an interferometer, or a tunable monochrometer such that desired portions of the electromagnetic spectrum are transmitted to the optical sensor 6040. The system controller 6023 may be configured to monitor and control the energy beam $E_i$. Accordingly, the properties and characteristics of the fluid sample 6090 at a plurality of wavelengths may be measured by the optical sensor 6040.

In the embodiment shown in FIG. 50A, both the first and second windows 6080 and 6082 are substantially transmissive to the energy beam E. In other embodiments, the second window 6082 may be replaced by a wall that is substantially opaque to the energy beam E. In embodiments comprising an opaque wall, it is preferred, but not necessary, that a surface of the wall be made reflective so as to reflect the energy beam E back through the sample 6090. A desired reflectivity for a particular wavelength range may be achieved, for example, by manufacturing the wall from a reflective material such as a metal, by depositing a reflective coating on the wall, or by adhering a reflective film to the wall. In these embodiments, the optical line 6072 and optical coupler 6078 may be disposed on the windowed-side of the optical sensor 6040 so as to accept and to return the reflected energy beam E to the analyzer 6025. Alternatively, the optical line 6072 and the optical coupler 6078 may be eliminated, and the optical line 6070 may be used both to transmit and to return the energy beam E to the analyzer 6025.

The optical sensor 6040 may have additional features. For example, one or both of the windows 6080 or 6082 may comprise a filter to selectively pass only predetermined wavelength regions of the energy beam E. Additionally, one or both of the windows 6080 or 6082 may comprise etalons so that the optical sensor 6040 functions as a Fabry-Perot interferometer. In some embodiments, one or more piezoelectric devices may be used to vary the distance between the etalons.

In the embodiment shown in FIG. 50, three electrically conductive sleeves 6047a, 6047b and 6047c are positioned at the entrance to the first chamber 6043, between the first chamber 6043 and the second chamber 6045, and at the exit of the second chamber 6045, respectively. The conductive sleeves 6047a-6047c may comprise a metal, which preferably may be stainless steel. The sleeves 6047a-6047c are arranged to come into direct contact with the adjacent fluid, and the sleeves 6047a and 6047b are electrically shorted together. The sleeves 6047a-6047c serve several functions, including without limitation the following. First, the three sleeves form part of a hematocrit sensor, which operates by measuring the electrical conductivity of the fluid between the sleeve 6047b and the sleeve 6047c. Second, the sleeves 6047a and 6047b may be connected to an isolated electrical ground, to protect the patient 6011 from electrical shock. Third, the three sleeves may form part of a noise-reduction circuit (not shown in the drawings) that seeks to reduce electrical currents from traveling along the catheter 6021 and infusion tube 6017, which otherwise could lead to interference with the signals produced by the various analytical sensors 6027-6040. An example of a suitable noise reduction circuit is disclosed in U.S. Patent No. 5,220,920, titled "Electrochemical measurement system having interference reduction circuit," issued June 22, 1993.

In the embodiment shown in FIG. 50, as the reversible infusion pump 6013 draws a blood sample from the patient 6011, the blood first comes into sensing contact with the carbon dioxide sensor 6027 and, shortly thereafter, with the calcium sensor 6035. When the blood sample reaches the carbon dioxide sensor 6027, the signal that is produced begins to increase, because blood ordinarily carries substantially more dissolved carbon dioxide than does the saline solution infusion fluid. When the analyzer 6025 detects a rise in the signal from the carbon dioxide sensor above its baseline level, a timer is activated. In an embodiment in which the carbon dioxide sensor 6027 comprises an electrical sensor, a rise to a level in the range of 5 to 15 millivolts above the baseline voltage line may trigger the timer.

After activation of the timer, the analyzer 6025 begins monitoring the signal produced by the calcium sensor 6035. That signal should show a similar rise above its baseline level after activation of the timer. This is because blood ordinarily carries substantially more ionized calcium than does the infusion fluid being used. If the expected rise in the calcium sensor signal does in fact occur within this time period, it may be concluded that the blood sample has reached both the carbon dioxide sensor 6027 and the calcium sensor 6035. In some embodiments, the calcium sensor signal may show a rise above its baseline level within seven to ten seconds after activation of the timer.

After the analyzer has detected a rise in the level of the signal from the calcium sensor 6035, the controller 6023 continues to operate the infusion pump 6013 in the rearward direction for a time sufficient to draw about an additional 0.1 to 1.0 milliliters of blood from the patient 6011. The internal construction of the sensor assembly 6019 is such that the leading edge of the blood sample thereby is drawn nearly all of the way up the length of the tubing 6049 located within the assembly housing. In this position, the blood sample should be in sensing contact with the analytical sensors 6027-6040 included in the sensor assembly 6019. The leading edge of the drawn blood preferably remains within the assembly housing both for cosmetic reasons and also to avoid undue delays caused by drawing excessive amounts of blood.

At the point where the prescribed additional amount of blood has been drawn from the patient 6011, readings can be taken from any or all of the analytical sensors 6027-6040. The analyzer 6025 reads the various signals from these sensors and converts them into indications of conditions of the patient's blood chemistry. The analyzer 6025 may then communicate these blood conditions to the caregiver via a printed record, an optical display, digital data transmission, or any other suitable means.

The blood chemistry analysis system may also include safety/alarm features that alert the caregiver if a fault or other failure condition is detected. For example, if a predetermined maximum time period elapses after reversal of the infusion pump 6013 without the analyzer 6025 detecting the expected rise in the signals from the carbon dioxide sensor 6027 and/or the calcium sensor 6035, it may be determined that a failure condition is present. This could be due, for example, to an obstruction in the line or a failure of one or both of the sensors. When this occurs, the controller 6023 may cease operating the pump and may activate an alarm 6050 (FIG. 49), to alert the caregiver. Further, if after reversing the pump a substantial sudden change is noted in the signals from the carbon dioxide sensor 6027 and/or the calcium sensor 6035, it is determined that an air bubble might be present in the line. Again, when this occurs, the controller ceases operating the pump, and actuates the alarm, to alert the caregiver.

It is apparent to those of skill in the art that alternative embodiments of the sensor assembly 6019 may use sensors other than the carbon dioxide sensor 6027 and/or the calcium sensor 6035 to determine when the blood sample has reached a predetermined position within the sensor assembly 6019. For example, some embodiments may use the sodium 6037 or the hematocrit sensor 6047a-6047c instead of the carbon dioxide sensor 6027 or the calcium sensor 6035. In other embodiments, the optical sensor 6040 may comprise a colorimetric sensor, generally similar to the sensor 311 (FIG. 3), to determine the presence and/or concentration of blood in the fluid sample.

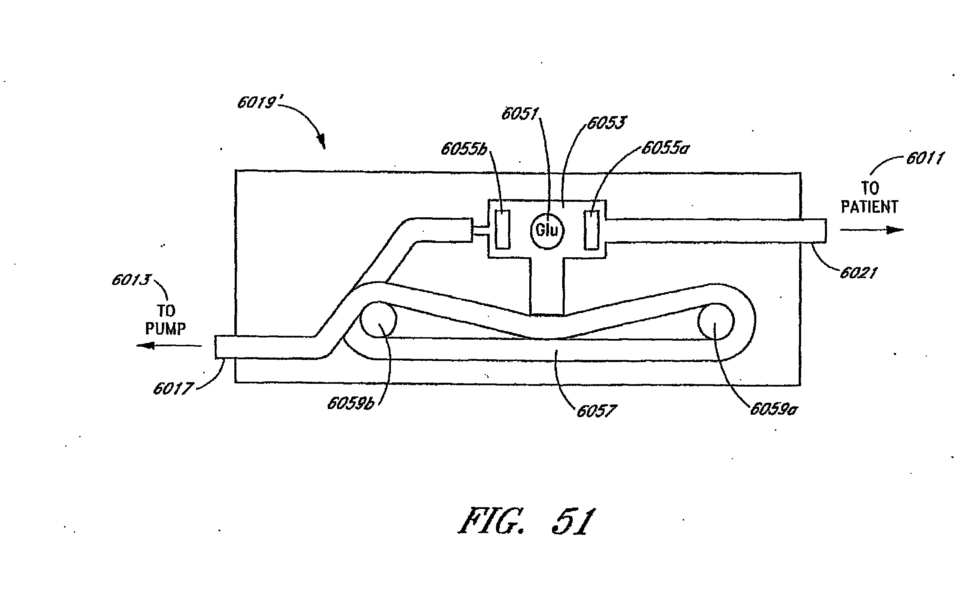

With reference now to FIG. 51, there is shown a second embodiment of a blood chemistry sensor assembly 6019' suitable for incorporation into the blood analysis system. The embodiment shown in FIG. 51 may be generally similar to the embodiment 6019 illustrated in FIGS. 50 and 50A except as further described herein. This sensor assembly 6019' is depicted to include just a single analytical sensor 6051, for example, a glucose sensor. The sensor 6051 is located adjacent to a chamber 6053 of the sensor assembly 6019'. Although FIG. 51 illustrates the use of a glucose sensor 6051, it is for ease of presentation alone, and it will be apparent to one of ordinary skill in the art that different embodiments may include sensors for different analytes or characteristics, or combinations of analytes and/or characteristics.

The glucose sensor 6051 may operate using electrochemical techniques and/or optical techniques and may transmit an electrical signal, an optical signal, or both indicating the concentration of glucose in the fluid sample adjacent to the sensor. An optical glucose sensor 6051 may in one embodiment be generally similar to the optical sensor 6040 depicted in FIG. 50A and further described herein. The optical glucose sensor 6051 may use spectroscopic or non-spectroscopic techniques. In embodiments using spectroscopic techniques, the concentration of analytes, such as glucose, in the presence of interferents may be determined from an optical signal transmitted by the optical glucose sensor 6051 to the analyzer 6025 using embodiments of the methods presented in the flowcharts illustrated in FIGS. 31, 32, and 34 and further described herein.

Electrically conductive sleeves 6055a and 6055b may be located at the inlet and outlet of the chamber 6053, respectively, and may be used to sense the arrival of a drawn blood sample. The conductive sleeves 6055a and 6055b may be comprised of a metal and preferably may be comprised of stainless steel. As was the case with the electrically conductive sleeves 6047a, 6047b and 6047c in the sensor assembly 6019 of FIG. 50, these sleeves 6055a and 6055b also may be used to provide an isolated electrical ground, for shock prevention, and to form electrodes used in a noise-reduction circuit. An example of a suitable noise reduction circuit is disclosed in U.S. Patent No. 5,220,920, titled "Electrochemical measurement system having interference reduction circuit," issued June 22, 1993.

In the sensor assembly 6019' of FIG. 51, the analyzer 6025 may detect the arrival of a drawn blood sample at the site of the chamber 6053 by monitoring the electrical conductivity of the fluid between the two conductive sleeves 6055a and 6055b. Such arrival is deduced when the conductivity is detected to exceed a predetermined threshold. An additional blood volume may be drawn to minimize the dilution effects of the adjacent infusion fluid. In some embodiments, an additional draw of about 0.4 milliliters is sufficient to minimize dilution effects. The leading edge of the blood sample thereby may be drawn nearly all of the way up the length of the tubing 6057 located within the assembly housing. Additional tubing length may be provided by wrapping the tubing around a pair of spaced spools 6059a and 6059b. Additionally and optionally, embodiments of the sensor assembly 6019' may include an optical sensor, such as, for example, the colorimetric sensor 311, to detect the arrival of the drawn blood sample at some point within the sensor assembly 6019'.

It should be appreciated from the foregoing description that the blood analysis system of FIGS. 49-54C provides an improved system for monitoring a patient's blood chemistry, which intermittently draws blood samples from the patient into a special sensor assembly having a number of sensors, each sensitive to a particular parameter or group of parameters. After signals produced by these various sensors have been read, the system reinfuses the blood samples back into the patient. Withdrawal of the successive samples into a desired, optimal position within the sensor assembly is achieved by monitoring signals produced by one or more of the analytical sensors, themselves. This allows the infusion tube and catheter to have variable lengths and internal volumes and obviates the need for a separate sensor for detecting the arrival of the blood sample at the desired position.

Preferred embodiments of a fluid infusion and blood testing system incorporating the invention have been described in detail for purposes of understanding and illustration. Various additions and modifications will no doubt occur to those skilled in the art. For example, the layout, number, and type of sensors used may be varied considerably. Other modifications may be made as well.

Certain embodiments disclosed herein include an improved method and apparatus for precisely and repeatably controlling the drawing of a patient's blood sample to a prescribed position within a blood chemistry sensor assembly. This method and apparatus ensures that the blood sample reaches all of the sensor assembly's individual sensors and that sufficient additional blood is drawn to minimize the dilution effects of an adjacent infusion fluid. The method and apparatus have particular use as part of an infusion system that substantially continuously infuses an infusion fluid into patient, while intermittently reversing itself and drawing blood samples from the patient, for chemical analysis.

More particularly, one embodiment of the apparatus can include a reversible infusion pump that, under the control of a controller, normally pumps an infusion fluid in a forward direction from a fluid source into the patient via an infusion tube and a catheter. Intermittently, the controller operates the pump in a rearward direction, to draw a blood sample from the patient into the blood chemistry sensor assembly, which is connected to the infusion tube. The controller monitors the signal produced by a sensor of the sensor assembly, to detect the arrival of the blood at the sensor, after which time it ceases operating the pump in the rearward direction. The sensor signal then is monitored, to provide an indication of a predetermined parameter of the patient's blood.

In another optional feature, the sensor whose signal is monitored in the detecting of the arrival of the patient's blood sample is selected from the group consisting of carbon dioxide sensors, oxygen sensors, potassium sensors, calcium sensors, sodium sensors, hematocrit sensors, temperature sensors, glucose sensors, and pH sensors. In a preferred form of the apparatus, the controller detects the arrival of the blood sample in response to a predetermined combination of signals generated by both a carbon dioxide sensor and a calcium sensor.

In another optional feature, the apparatus actuates an alarm in response to a predetermined signal received from one or more of the sensor assembly's sensors. An alarm also is actuated in response to a failure to detect the arrival of the blood sample within a predetermined time period after operation of the infusion pump in the rearward direction is initiated and in response to detecting the arrival of the blood sample at a time too soon after operation of the infusion pump in the rearward direction is initiated.

In yet another optional feature, the controller ceases operation of the infusion pump in the rearward direction a prescribed time after the arrival of the blood sample has been detected. Preferably, this occurs after a prescribed additional volume of blood has been drawn from the patient.

Additional information related to a method and apparatus for monitoring blood chemistry may be found in U.S. Patent No. 5,758,643, titled "METHOD AND APPARATUS FOR MONITORING BLOOD CHEMISTRY," issued June 2, 1998, which is hereby incorporated by reference herein and made a part of this specification in its entirety.

PATIENT SPECIFIC PLASMA SEPARATION

In certain embodiments, the extraction and analysis of a patient's bodily fluid, for example blood plasma, may be performed entirely at the patient's point of care or bedside, and/or with a device attached or connected to a patient. Prior art methods of analyzing bodily fluid from a hospital patient involved taking a sample of a bodily fluid, transporting the sample to a central processing and analysis lab and periodically batch processing a group of samples collected from several patients using a common, central device, for example a centrifuge and bodily fluid analyzer. Here, methods of analysis are disclosed wherein a fluid handling system or sampling system is attached to a single patient, for example at the patients bedside or point of care, and is capable of extracting a bodily fluid sample from the patient, preparing the sample for analysis and analyzing the sample all at the patient's bedside.

At a first step, a fluid handling system, sampling system, analyte detection system or other suitable apparatus is connected to a patient so that the system is placed in fluid communication with a bodily fluid of the patient. Since the system is only associated with a single patient, the connector between the system and patient may be of a type to establish a sustained connection to the patient such as through an IV tube or a catheter inserted into the patient's vasculature.

At a second step, once fluid communication has been established with the patient's bodily fluid, a sample of the bodily fluid may be drawn into the system. The sample may then be transported through one or more passageways in the system to a sample preparation unit located with in the system. At a third step, the sample preparation unit prepares the sample for analysis. Depending on the bodily fluid to be analyzed, the preparation of the sample may involve diverting or isolating of a fraction of the drawn portion of fluid for analysis, filtering the sample through a filter or membrane to remove impurities, or separating a first component from the whole sample, for example separating plasma from a sample of whole blood, to analyze only the first component. Since the sample preparation unit is co-located with the sample draw apparatus, the sample may be analyzed almost immediately after it has been drawn. Once the sample has been prepared, it may be transferred to a chamber, a sample cell or any other location accessible by an analyte detection system for analysis. Alternatively, the sample preparation unit itself may be configured to hold the sample of component for analysis by the analyte detection system.

At a fourth step, after the sample has been prepared, the analyte detection system which is preferably located within the fluid handling system or sampling system connected to the patient determines the concentration of one or more analytes based on or within the prepared sample. The concentration of the measured analyte(s) and/or values of other suitable characteristics may then be reported to a display or operator's console located at the patient's bedside or point of care, and/or uploaded to a data network such as a Hospital Information System (HIS), shortly after the sample was drawn from the patient.

At a fifth step, once the sample has been drawn, prepared, and analyzed the fluid handling system or sampling system may shift to infusing the patient with an infusion fluid, such as saline, lactated Ringer's solution, water or any other suitable infusion liquid. In shifting to the infusion mode, the system may return at least a portion of the drawn portion or sample of bodily fluid to the patient. In addition, since the system is dedicated to a single patient use and continuously connected to the patient, the system may further be automated to periodically draw, prepare, and measure a sample of bodily fluid from the patient. In an alternative embodiment where the fluid handling system or sampling system includes an alarm system, the determined analyte concentration(s) may then be compared to a predetermined range of acceptable concentrations and if the determined concentration(s) fall outside the range, an indicator may be triggered, for example an alarm may be sounded, to alert the hospital staff.

Embodiments of the above described method and apparatus as used to prepare a plasma sample from a patient's whole blood and analyze the plasma sample at the patient's bedside or point of care are further described herein in reference to FIGS. 1-3 and 49. However, it is envisioned that the presently-described methods and apparatus could be used to prepare and analyze a sample of any one of a number of bodily fluids extracted from the patient at the point of care, for example interstitial fluid, intercellular fluid, saliva, urine, sweat and/or other organic or inorganic materials.

In use, the patient sampling system 100 of FIG. 1 may be connected to a patient via the patient connector 110 and passageway 112. Since the sampling system is associated with only a single patient, the patient connector 110 may be configured to allow a sustained connection to the patient, for example through IV tubing or the catheter 11 inserted into the patient's vasculature. The sampling system further includes a fluid handling and analysis apparatus 140 which is connected to the patient in part via passageway 112. The fluid handling and analysis apparatus 140 is thus also located at the patient's bedside or point of care and dedicated to a single patient via connector 110 and passageway 112. As shown in FIG. 3, the fluid handling system or sampling system 300 may further include a fluid component separator, such as the sample preparation unit 332, and an analyte detection system 334 for preparing the sample for analysis and determining the concentration of an analyte based on analysis of the prepared sample. In an alternative embodiment, the fluid handling system or sampling system 100 may be further associated to the patient for example, via manual input of patient data or a patient code into the sampling system.

Once the system 100 is connected to a patient, a sample of whole blood from the patient may be periodically withdrawn from the patient's vasculature through connector 110 and passageway 112. The whole blood sample may then be transported to the co-located fluid handling and analysis apparatus 140 where it may be processed and analyzed. Such a system and method of analysis is advantageous over the prior methods because it permits the sample to be processed in a much shorter timeframe. Since the sample does not have to be transported to a central facility and is not batch processed with a group of samples from other hospital patients, but rather is drawn and analyzed at the patient's bedside via a dedicated machine, the sample can be processed and analyzed almost without delay. In addition, such a system and method of analysis permits the system to use a smaller sample size to perform the analysis, since multiple transfers (and the associated incidental fluid loss) from a separate sampling device to a separate processing device to a separate analysis device are no longer necessary.

Once the sample of whole blood has been drawn from the patient, at least a portion of the sample may be transported through passageway 112 to the fluid component separator or sample preparation unit 332, for example a centrifuge or filter membrane, located in the fluid handling and analysis apparatus 140. Here, the sample may be separated into at least one component for analysis and a remainder portion, for example a whole blood sample may be separated into a plasma sample and a remainder. Again, because the fluid component separator is co-located with the sampling system at the patient's bedside, the sample may be separated almost without delay, for example in less than 5 minutes from drawing, alternatively less than 2 minutes from drawing, alternatively immediately after drawing from the patient. In an alternative embodiment, for example analysis of whole blood, separation into components may not be required and the sample may simply be filtered to remove impurities. Once the sample has been processed into a first component, the first component may then be almost immediately analyzed by the analyte detection system 334 co-located in the fluid handling and analysis apparatus 140.

This is especially advantageous when the sample is whole blood and the component desired is blood plasma. For example, the glucose levels in plasma are an important indicator of patient health. However, since blood typically clots in less than two minutes, the delay in prior art systems where the samples are transported to a central lab for batch processing often complicate separation of plasma from whole blood. Under certain prior art methods, an anticoagulant is added to the whole blood sample to prevent clotting prior to processing and separation of the plasma. Under other prior art methods, a coagulant is added to the whole blood sample, serum is separated from the sample and analyzed in lieu of the plasma, and finally blood glucose level in the plasma is extrapolated from the levels in the serum. With regard to certain embodiments of the presently disclosed method and apparatus, because the samples are processed shortly after they are drawn, it is possible to separate the plasma from the whole blood without the addition of anti-coagulants and thus it is possible to get an accurate measurement of the plasma glucose level.

In addition, as shown in FIG. 1, the sampling system may further include a connector 120 for attaching an infusion source 15 containing an infusion liquid to 14 to the system. In use, connector 120 may connect the infusion source 15 to a passageway 111 that is in fluid communication with the patient via passageway 112 and patient connector 110. In use, the infusion liquid may then be delivered to the patient in between periodic draws of a sample of bodily fluid. Infusing the patient's vasculature with a fluid such as saline, lactated Ringer's solution, water or any other suitable infusion fluid, may keep the patient's vascular line from becoming occluded and preventing periodic future extraction of additional samples of bodily fluid. To keep the patient's vascular line open between extractions of bodily fluid samples, the infusion fluid may be delivered at a rate ranging from 1-5 ml/hr. Here, the system may alternate between drawing a bodily fluid sample from the patient's vasculature through passageway 112 and into the fluid handling and analysis apparatus 140 and delivering an infusion liquid via passageways 111 and 112 to the patient's vasculature. Since the system is dedicated to the patient and is continuously attached to the patient, this process may be automatically cycled according to a preset schedule to periodically sample a patient's bodily fluid, measure the levels of an analyte in the sample and update the results on a display 141 at the patient's bedside. In addition, in an alternate embodiment, the system may further include an indicator which may be set to sound an alarm if the levels of the analyte fall outside a preset range.

Certain alternative embodiments, shown in FIGS. 5 and 8, are generally similar to the sampling systems 100 and 300 as described herein. For example, FIG. 5 depicts a sampling system 500, configured to perform the methods described herein and further including a return line 503 connected to the sample analysis device 330 and passageway 111. Here, once the sample has been prepared and analyzed, as described above, the remainder of the sample may be transported to passageway 111 where it may be reintroduced to the patient's vasculature along with the infusion liquid. FIG. 8 depicts an alternative embodiment of a sampling system 800 wherein a fluid handling and analysis apparatus 140 comprises two modules, a main instrument 810 and a disposable cassette 820, that have been configured to be connected at a patient's bedside or point of care and interface to perform the fluid handling and analysis functions described herein. Thus, it should be understood that sampling systems 100, 300, 500 and 800 as shown in FIGS. 1-8 each represent variations of an apparatus configured to carry out the above described method for extracting and analyzing a bodily fluid from a hospital patient at the patient's bedside or point of care.

The methods for extracting and analyzing a bodily fluid from a hospital patient at the patient's bedside or point of care described herein may also be used with embodiments of the patient infusion and monitoring system illustrated in FIG. 49. The catheter 6021 is connected to the vasculature of the patient 6011 and to one of the embodiments of the sensor assembly 6019. Various embodiments of the sensor assembly 6019 may be used, for example, any of the embodiments of any of the sensor assemblies illustrated in FIGS. 50-51, 52B, and 54A-54C. The sensor assembly 6019 is connected to the pump 6013 and to a source of infusion fluid 6015 by the infusion tube 6017. During normal operation, the controller 6023 directs the pump 6013 to operate in the forward direction so as to infuse the patient 6011 with the infusion fluid.

A sample of whole blood from the patient 6011 may be drawn at various times. The sampling times may be spaced periodically or they may be intermittent or subject to the control of an attending health care provider. To draw a sample of whole blood, the controller 6023 directs the pump 6013 to operate in the reverse direction so as to draw whole blood from the patient 6011. An advantage of the system shown in FIG. 49 is that it permits the blood sample to be processed in a much shorter timeframe. Since the sample does not have to be transported to a central facility and is not batch processed with a group of samples from other hospital patients, but rather is drawn and analyzed at the patient's bedside via the dedicated sensor assembly 6019, the sample can be processed and analyzed almost without delay. In addition, such a system and method of analysis permits the system to use a smaller sample size to perform the analysis, since multiple transfers (and the associated incidental fluid loss) from a separate sampling device to a separate processing device to a separate analysis device are no longer necessary.

Once the sample of whole blood has been drawn from the patient 6011, at least a portion of the sample may be transported into the sensor assembly 6019 through the tubing 6049. In certain embodiments, the sensor assembly 6019 comprises a fluid component separator such as a filter, which may be generally similar to the blood filter 1500 or membrane 1509 shown in FIGS. 15 and 16. For example, in some embodiments, the filter is disposed next to the reference sensor 6041, or in the position of one of the other sensors 6027-6040, or in some other suitable location. In certain embodiments, the filter is positioned where the catheter 6021 junctions with the sensor assembly 6019, or otherwise between the patient end of the catheter 6021 and the sensor 6040. The filter may comprise a filter membrane (generally similar to the filter membrane 1509) that blocks the passage of red blood cells and permits blood plasma to flow into one or both of the sensing chambers 6043, 6045 of the sensor assembly 6019 and into sensing contact with some or all of the sensors 6027-6040.

Again, because the fluid component separator is co-located with the sampling system at the patient's bedside, the sample may be separated almost without delay, for example in less than 5 minutes from drawing, alternatively less than 2 minutes from drawing, alternatively immediately after drawing from the patient. In an alternative embodiment, for example analysis of whole blood, separation into components may not be required and the sample may simply be filtered to remove impurities. Once the sample has been processed into a blood plasma component, the blood plasma component may then be almost immediately analyzed by any or all of the sensors 6031-6040 disposed within the sensor assembly 6019. For example, in certain embodiments, the blood plasma component is analyzed by the optical sensor 6040 (as shown, e.g., in FIG. 50A), for instance, by using spectroscopic or non-spectroscopic techniques as further described herein. In particular, the optical sensor 6040 may determine, for example, the glucose concentration of the blood plasma component in the presence of one or more interferents using the spectroscopic methods described with reference to FIGS. 31-34. After the blood sample has been drawn, separated, and analyzed, the controller 6023 may direct the pump 6013 to operate in the forward direction to resume infusing the patient 6011.

In other embodiments, the fluid component separator comprises a centrifuge in place of, or in addition to, a filter. The centrifuge may be generally similar to the centrifuge 2110 illustrated in FIG. 21. In some of these embodiments, a portion of a whole blood sample drawn from the patient 601 through the catheter 6021 is passed into the centrifuge 2110. As is further shown in FIG. 21, the sample is transferred into the sample element 2112 via a fluid injection probe 2121 having a first needle 2122 and is removed from the sample element 2112 via a fluid removal probe 2123 having a second needle 2124. When the sample element 2112 is properly oriented relative to the catheter 6120, a portion of the whole blood sample is dispensed into or passes through the sample element 2112. More specifically, fluid injection probe 2121 includes a passageway to receive the sample from the catheter 6021. The whole blood sample can be passed through the fluid injection probe 2121 and the first needle 2122 into the sample element 2112. After centrifuging, the sample element 2112 can be aligned with the second needle 2124, as illustrated in FIG. 21. Blood plasma can be passed through the second needle 2124 into the fluid removal probe 2123. The blood plasma can then pass through a passageway of the removal probe 2123 away from the sample element 2112 and into the sensor assembly 6019.

Inside the sensor assembly 6019, the blood plasma component can be analyzed by any or all of the sensors 6031-6040. In particular, the blood plasma component can be promptly analyzed via the spectroscopic methods of FIGS. 31-34. As described above, because the centrifuge 2110 is co-located with the sampling system at the patient's bedside, the whole blood sample may be separated almost without delay, for example in less than 5 minutes from drawing, alternatively less than 2 minutes from drawing, alternatively immediately after drawing from the patient. After the blood sample has been drawn, separated, and analyzed, the controller 6023 may direct the pump 6013 to operate in the forward direction to resume infusing the patient 6011.

In other embodiments, the fluid component separator may be disposed in other locations in the infusion and monitoring system. For example, in some embodiments, the analyzer 6025 comprises the fluid component separator. In such embodiments, additional passageways, valves, and pumps may direct a portion of a drawn whole blood sample into the analyzer 6025 wherein the whole blood sample is separated into a blood plasma component. The separation may be performed by one or more filters and/or one or more centrifuges.

Variations of the infusion and monitoring system shown in FIG. 49 may be utilized to carry out the methods for extracting and analyzing a bodily fluid from a hospital patient at the patient's bedside or point of care. For example, embodiments of the sensor assembly 6019 shown in FIGS. 50-51, 52B, and 54A-54C generally may be substituted for the sensor assembly 6019 shown in FIG. 49. In other embodiments, the fluid handling system may be configured differently such as, for example, by including a separate bodily fluid passageway from the sensor assembly 6019 to the analyzer 6025. Many other variations are possible. Further, it is envisioned that the presently-described methods and apparatus could be used to prepare and analyze a sample of any one of a number of bodily fluids extracted from the patient at the point of care, for example interstitial fluid, intercellular fluid, saliva, urine, sweat and/or other organic or inorganic materials.

CLOT INHIBITION

The coagulation of blood may affect the operation of extracorporeal blood systems. In general, coagulation proceeds according to a series of complex chemical reactions within the blood. In extracorporeal systems, coagulation may begin upon the contact of blood with most types of surfaces, and may collect on surfaces or within crevices or changes in surface type or flow conditions. Thus, for example, blood flowing through passageways may build up on the passageway walls or may form clots that restrict or block the flow of blood, hindering the operation of the system. Accordingly, it is advantageous for embodiments of the fluid handling system 10 illustrated in FIG. 1 or the infusion and monitoring system shown in FIG. 49 to include methods and apparatus for inhibiting blood clot formation.

One method for inhibiting and/or removing blood clots comprises intermittently providing one or more anti-clotting agents and/or clot disrupting or dissolving agents to a passageway of the extracorporeal blood flow system. In embodiments of the system 10 shown in FIG. 1, the agents may be provided in the flow passageways 20. In embodiments of the infusion and monitoring system shown in FIG. 49, the agents may be provided in the infusion tube 6017 and/or in tubing 6049 disposed within the sensor assembly 6019 (see FIGS. 50-51, 52B, and 54A-54C). The agents may inhibit and/or prevent clots from forming, and/or the agents may disrupt, dissolve, or remove clots after they have formed.

In some embodiments of the blood clot inhibition method and apparatus, the agents may comprise a cleaning solution S, such as a detergent, which may be delivered through the flow passageways. In one embodiment, the cleaning solution S is effective in removing blood, blood components, and/or clotted blood from the surfaces of the passageways, sample elements, or other blood contacting surfaces. In one embodiment, the solution S may be thermally stable at room temperatures. Suitable solutions S include solutions commonly used for cleaning hospital and laboratory instruments. In one embodiment, the solution S may include nonspecific protease enzymes for digesting blood. In another embodiment, the cleaning solution S may comprise a mixture of approximately 1% TERGAZYME™ (manufactured by Alconox, Inc., White Planes, NY) in saline. In yet another embodiment, a mixture of more than one cleaning solution S may be utilized.

The system 10 in FIG. 1 or the infusion and monitoring system in FIG. 49 may include an anti-clotting or clot disrupting device. In some embodiments, the anti-clotting or clot disrupting device comprises a container configured to store the cleaning solution S that is in fluid communication with the flow passageways. The anti-clotting device may comprise one or more valves and/or pumps that may be configured to transport the cleaning solution S into selected flow passageways. For example, in the system 10 (FIG. 1), the cleaning solution S may be delivered into the passageway 113 and the sample analysis device 330.

In some embodiments of the system 10 in FIG. 1, the anti-clotting or clot disrupting device may be connected to and controlled by the controller 210, which may intermittently operate the anti-clotting device so as to flush cleaning solution S through the passageway 113 and the sample analysis device 330. The controller 210 may utilize one or more pumps and/or valves in the flushing operation. In one embodiment, the flushing action may be a backflow - that is the flow may be in the reverse direction of the normal flow of the system. Some embodiments may permit the cleaning solution S to remain in the flow passageways for a time period sufficient to allow effective cleaning of the passageways. Other embodiments may provide a waste receptacle, generally similar to the waste receptacle 325 (FIG. 3), into which residual blood, saline, or other fluids may be pumped. Fluid handling methods generally similar to those described with reference to FIGS. 7A-7J may be utilized in the flushing process.

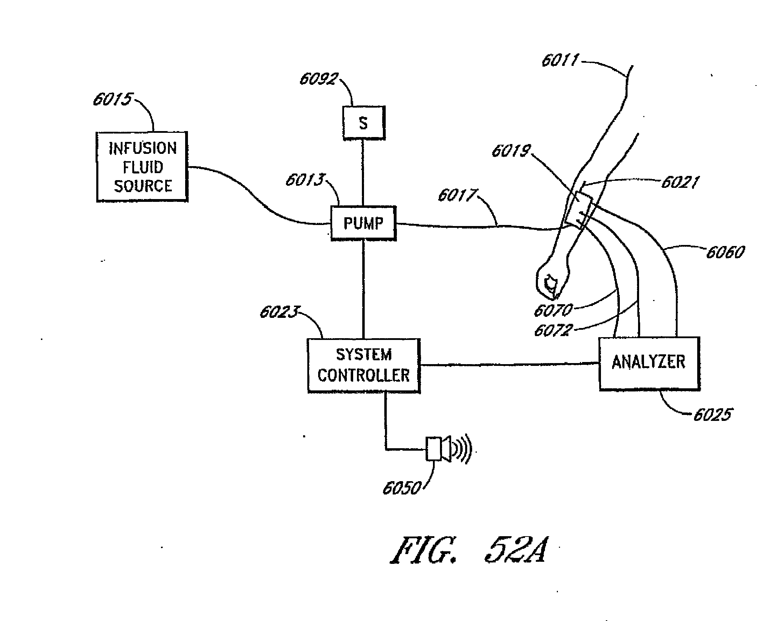

In embodiments of the infusion and monitoring system shown in FIG. 52A, the anti-clotting or clot disrupting device comprises a container 6092 that is configured to be in fluid communication with the flow passageways and is configured to store the cleaning solution S. The anti-clotting device may comprise one or more valves and/or pumps that may be configured to transport the cleaning solution S into selected flow passageways. For example, the cleaning solution S may be delivered into the infusion tube 6017, the sensor assembly 6019, and/or the catheter 6021. In one embodiment shown in FIG. 52A, the container 6092 is in fluid communication with the pump 6013. Additional valves may be disposed within the pump 6013 (or other suitable locations) to control the fluid flow such that the cleaning solution S can be delivered into the flow passageways.

In one embodiment of an anti-clotting procedure, the system controller 6023 directs the pump 6013 to close valves permitting the infusion fluid to flow from the infusion source 6015 and to open valves permitting the cleaning solution S to flow through the pump 6013, into the infusion line 6017, and thereby into the sensor assembly 6019 and/or the catheter 6021. After a time sufficient for the cleaning solution S to clean the passageways, the system controller 6023 directs the pump 6013 to close the valves permitting the cleaning solution S to flow into the infusion tube 6017 and to re-open the valves permitting infusion fluid to be pumped into the patient 6011.

In other embodiments of the anti-clotting or clot disrupting device, the container 6092 is fluidly coupled to the infusion tube 6017 at a "T" junction. Additional pumps and/or valves may be included in the anti-clotting device in order to transfer the cleaning solution S through the "T" junction into the infusion tube 6017 and thereby into the sensor assembly 6019. In certain embodiments, the anti-clotting device is disposed within the sensor assembly 6019. For example, in certain such embodiments, the container 6092 storing the cleaning solution S is disposed within the sensor assembly 6019 so that it is in fluid communication with the tubing 6049 and/or the sensing chambers 6043 and 6045. In other embodiments, more than one anti-clotting device may be used. For example, a first anti-clotting device can be disposed within the sensor assembly 6019, and a second anti-clotting device can be put into fluid communication with the pump 6013 as shown in FIG. 52A. Many variations are possible.

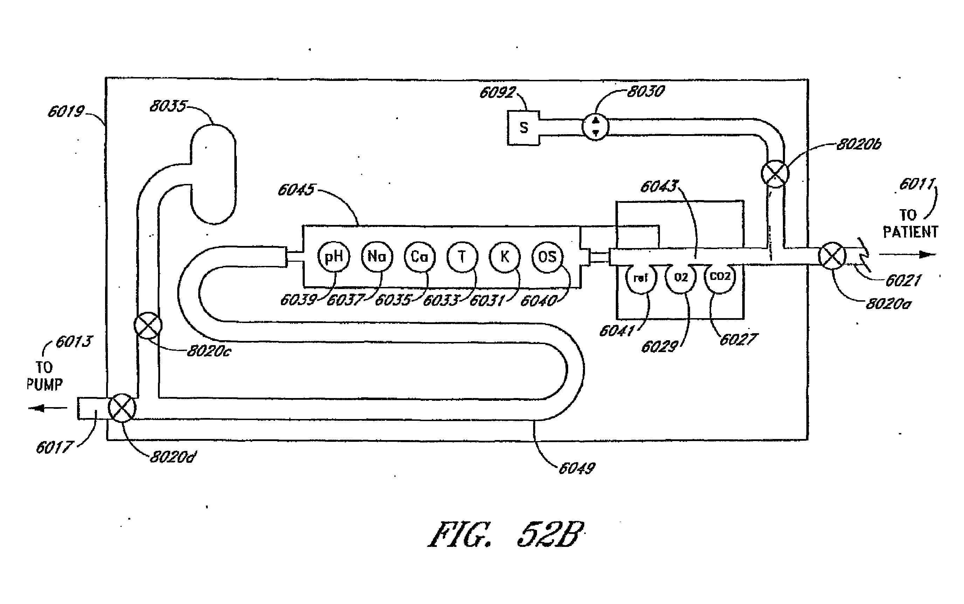

FIG. 52B illustrates an embodiment of the sensor assembly 6019 that comprises the cleaning solution container 6092, a pump 8030, valves 8020a-8020d, and a cleaning solution waste receptacle 8035. During either infusion or sampling, the valves 8020a and 8020d are open to permit flow of either infusion fluid or blood, while the valves 8020b and 8020c are closed to prevent the cleaning solution S from entering the sensing chambers 6043 and 6045. In one embodiment of an anti-clotting procedure to clean the sensor assembly 6019, the valve 8020a is closed to prevent cleaning solution S from being pumped into the patient 6011. The valves 8020b and 8020d are opened, and the valve 8020c is closed. The pump 8030 operates in a forward direction to pump the cleaning solution S through the chambers 6043, 6045 and the tubing 6049. Optionally, the pump 6013 may operate in the reverse direction to assist the pump 8030 and to further draw the cleaning fluid S into the infusion tube 6017. After a time sufficient for the cleaning solution S to clean the passageways 6017, 6049 and the chambers 6043, 6045 of the sensor assembly 6019, the valve 8020d is closed and the valve 8020c is opened to permit the cleaning solution S to be pumped into the cleaning solution waste receptacle 8035. In some embodiments, the valve 8020d remains open, and the pump 6013 operates in the forward-direction to assist transferring the cleaning solution S into the waste receptacle 8035. Other embodiments may utilize additional pumps, valves, and/or passageways. Many variations are possible.

In some embodiments, the pump 8030 and the valves 8020a-8020d are operated by a controller disposed within the sensor assembly 6019, while in other embodiments, these devices are controlled by the system controller 6023. The anti-clotting device may be intermittently operated so as to flush the system with cleaning solution S in order to keep the system free from clots. In one embodiment, the flushing action may be a backflow - that is the flow may be in the reverse direction of the normal flow of the system. Some embodiments permit the cleaning solution S to remain in the flow passageways for a time period sufficient to allow effective cleaning of the passageways. In certain embodiments, the system is configured to permit an attending health care professional to actuate the anti-clotting device so as to advantageously clean the system if the health care professional visually observes signs of clot formation.

In certain embodiments, residual blood, infusion fluid, cleaning solution S, or other fluids may be pumped into the waste receptacle 8035 or into other waste receptacles disposed within the system (e.g., the waste receptacle 325 shown in FIG. 3). In some embodiments, the waste receptacle is disposed within the anti-clotting device or along the infusion line 6017, for example, at a "T" junction. Fluid handling methods generally similar to those described with reference to FIGS. 7A-7J may be utilized in the flushing process.

Additional embodiments of the anti-clotting or clot disrupting agents are possible. For example, it has been found by the inventors that the application of vibrations to an extracorporeal system inhibits the formation of blood clots within the system and disrupts clots already formed in the system and facilitates flushing of the passageways and chambers. The vibrations may be at frequencies above the range of human hearing such as, for example, greater than 15 kHz, and are referred to herein and without limitation as ultrasonic vibrations or ultrasonic waves, or as "ultrasound." Accordingly, certain embodiments of the anti-clotting or clot disrupting system comprise ultrasound units or devices. The ultrasonic unit may be positioned adjacent to or otherwise in sonic transmission engagement with one or more fluid flow passageways and/or the sensor assembly 6019.

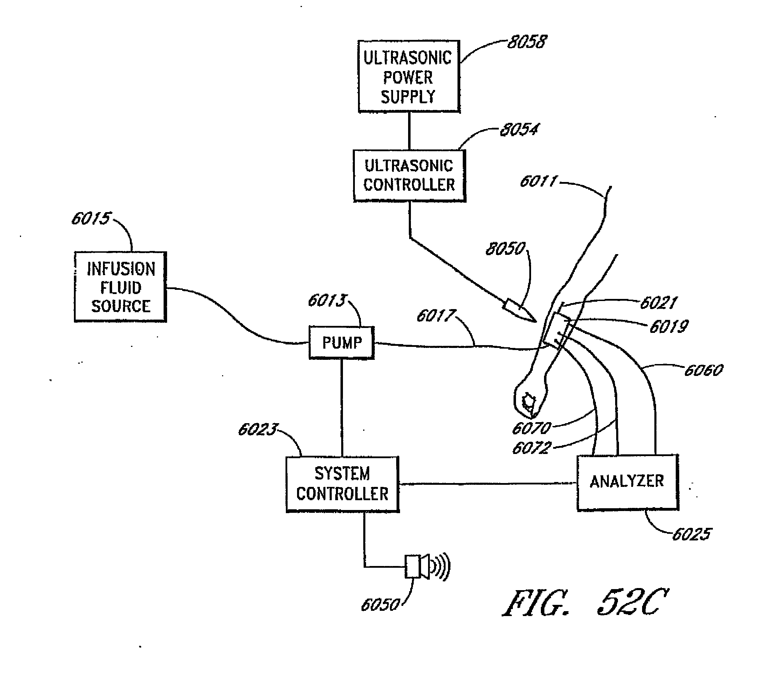

FIG. 52C schematically shows an embodiment of the anti-clotting or clot disrupting system comprising an ultrasonic transducer 8050, an ultrasonic controller 8054, and an ultrasonic power supply 8058. In some embodiments, the ultrasonic transducer 8050 comprises an ultrasonic horn and an ultrasonic generator. As shown in FIG. 52C, the ultrasonic transducer 8050 can be positioned near the sensor assembly 6019 in order to transmit ultrasonic vibrations into the passageways 6049 and the chambers 6043, 6045 within the assembly 6019. In other embodiments, the ultrasonic transducer 8050 is disposed near a portion of the infusion tube 6017 and/or the catheter 6021.

The ultrasonic controller 8054 is coupled to the ultrasonic transducer 8050 and controls characteristics of the ultrasonic vibrations transmitted by the transducer 8050 into portions of the infusion and sampling system. The ultrasound characteristics include, for example, amplitude, frequency, power, and duration of the ultrasonic vibrations. In some embodiments, the ultrasound may be transmitted continuously, or it may be pulsed, or a combination of continuous and pulsed ultrasound may be used. A single frequency or a range of frequencies may be used in various embodiments.

In the embodiment shown in FIG. 52C, the ultrasonic controller 8054 and the ultrasonic transducer 8050 are electrically coupled to an ultrasonic power supply 8058. In certain embodiments, the ultrasonic power supply 8058 provides sufficient power for the ultrasonic transducer 8050 to produce from 1 Watt to 1000 Watts of ultrasound. The ultrasonic power supply 8058 may be electrically coupled to the controller 8054 and the transducer 8050 by one or more electrical connectors. In certain embodiments, the ultrasonic power supply 8058 is electrically grounded and configured to prevent the patient 6011 from receiving electrical shocks.

In some preferred embodiments, the ultrasonic transducer 8050 is disposed adjacent to a chamber or cell holding a fluid sample such as, for example, the sample element or cuvette 1730 (FIG. 17) or the chambers 6043, 6045 (FIG. 50) in the sensor assembly 6019. In other preferred embodiments, the ultrasonic transducer is disposed adjacent to the analyzer 6025. In other embodiments, the ultrasonic transducer 8050 is adapted to be movable and may be positioned near to or in contact with a blood-containing portion of an extracorporeal system such as, for example, one or more flow passageways (e.g., the infusion tube 6017, the catheter 6021, or the tubing 6049). It is preferred, although not necessary, that the vibrations from the ultrasonic horn be directed towards a location where clots are known or expected to form.

In certain embodiments, the frequency transmitted through the ultrasonic transducer is from about 15 kHz to about 60 kHz, and the power transmitted through the transducer is from about 2 Watts to 200 Watts. In one preferred embodiment, a model VC24 ultrasonic system obtained from Sonics & Materials, Inc (Newtown, CT) is operated at a frequency of 40 kHz and 25 Watts of power. The ultrasonic transducer may be operated to provide one or more pulses of ultrasonic energy to the system. In one preferred embodiment, the pulse duration is about 10 seconds and is delivered between fluid fillings of a sample chamber or a passageway. In this preferred embodiment, the fluid filling and providing of ultrasound is repeated every 30 minutes over a time period of 69 hours, after which there is typically very little evidence of clotting, as observed either visually or by measuring the inhibition of blood flowing through the passageway.

Some embodiments of the ultrasound anti-clotting or clot disrupting system comprise ultrasound comprising different frequencies, powers, pulse durations, and/or application times. In other embodiments, the ultrasound generator may drive two or more ultrasound transducers, which may be suitably disposed within the fluid handling system. In still other embodiments, the anti-clotting agent may comprise additional ultrasound generators. Other variations of ultrasound units or devices are possible.

Additionally, in certain preferred embodiments, the anti-clotting or clot disrupting system may comprise one or more cleaning solutions and one or more ultrasound units. Other variations are possible.

NETWORK CONNECTED DATA FOR VERIFICATION/CALIBRATION

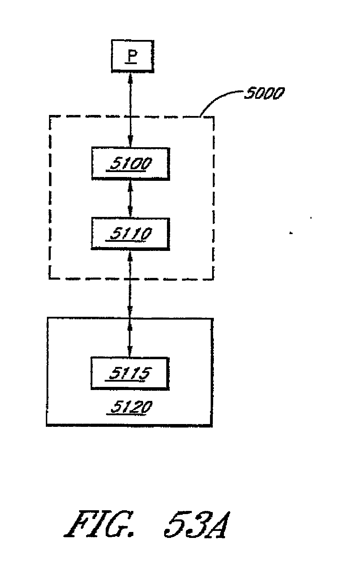

FIG. 53A is a schematic of one embodiment of a body fluid analyzing system 5000. Body fluid analyzing system 5000 includes a body fluid analyzer 5100 in fluid communication with a body fluid in a patient P and further includes a communication interface 5110. The body fluid analyzer 5100 can comprise, for example, any of the embodiments of the sampling system 100, the fluid handling and analysis apparatus 140, the analyte detection system 334/1700, the sensor assembly 6019, or the analyzer 6025 disclosed herein. The body fluid analyzer 5100 is configured to communicate with a data system 5120 via the communication interface 5110. The communication interface 5110 can comprise any suitable networking interface, such as a wired, wireless, or fiber optic interface. The data system 5120 includes at least one data file 5115. The body fluid analyzer 5100 is configured to access the at least one data file 5115. In certain embodiments, the at least one data file 5115 is stored in one or more components of data system 5120.

In certain embodiments, the body fluid analyzing system 5000 comprises the infusion and monitoring system shown in, for example, FIGS. 49, 52A, or 52C, and which is in fluid communication with the patient P (e.g., the patient 6011) through the catheter 6021. In some embodiments, the body fluid analyzer 5100 comprises the sensor assembly 6019 or the analyzer 6025. In various embodiments, the body fluid analyzing system 5000 provides an infusion mode and a sampling or draw mode. In an embodiment of the infusion mode, an infusion fluid is transferred by the pump 6013 from the infusion fluid source 6015, through the tubing 6017, the sensor assembly 6019, and the catheter 6021 to the patient 6011. In an embodiment of the sampling or draw mode, the pump 6013 is operated in reverse to draw a body fluid from the patient 6011 into the sensor assembly 6019 for monitoring and analysis. The communication interface 5110 permits the body fluid analyzer 5100 (e.g., the sensor assembly 6019 or the analyzer 6025) to communicate monitoring and analysis information with the data system 5120.

The data system 5120 may comprise one or more data-containing devices (e.g., computers, servers, storage devices) in communication with the system 5000 via one or more data links. In certain embodiments, the data system 5120 comprises a single data-containing device in communication with the analyzer 6025 or the sensor assembly 6019 via one or more data links. In certain such embodiments, the analyzer 6025 or the sensor assembly 6019 communicates directly with the data-containing device via the communication interface 5110. In certain other embodiments, the data system 5120 comprises a plurality of data-containing devices in communication with the analyzer 6025 or the sensor assembly 6019, and in certain embodiments with one another, via a network comprising one or more data links. In certain embodiments, the one or more data links can comprise wireless data links (e.g., electromagnetic radiation such as radiofrequency or infrared), hardwired data links, fiber optic data links, or any other suitable data links, or the one or more data links can comprise a combination of wireless, hardwired, fiber optic, or other suitable links, and/or the Internet.

In some configurations, the data system 5120 comprises one or more storage devices, processing devices, memory devices, input and/or output devices, computers, servers, and/or medical devices, which are interlinked by the one or more data links. Additional devices other than those listed above can be connected to the data system. In some embodiments, the data system 5120 comprises a local network and covers a limited region, such as a hospital building or a portion thereof, such as a floor, unit, or ward. In other configurations, the data system 5120 is more wide-ranging, and, in some instances, is connected to additional networks such as the Internet. In some embodiments, the data system 5120 can comprise a Hospital Information System (HIS). In certain embodiments, the communication interface 5110 comprises a CAT5 connection and communicates with the data system using the POCT-1A communication protocol standard.

In certain embodiments, the at least one data file 5115 is stored in one or more components of the data system 5120. The at least one data file 5115 can be stored in any suitable medium, such as, for example, a hard drive, a flash drive, a DVD drive, a CD drive, or RAM. In certain embodiments, the at least one data file 5115 contains calibration information for calibrating the analyzer 6025 or the sensor assembly 6019. In other embodiments, the data file 5115 comprises an electronic medical record pertaining to the patient 6011. Various programs for creating and updating electronic medical records are available, such as, for example, Enterprise Medical Record™ available from Meditech (Westwood, MA) and RALS® available from Medical Automated Systems (Charlottesville, VA).

In certain embodiments, the analyzer 6025 or the sensor assembly 6019 may be in continuous or selective communication with the data system 5120 via the communication interface 5110 and can thereby access the electronic medical record pertaining to the patient 6011. The electronic medical record can contain such information as the name, age, height, weight, blood type, medicine dosing history, other treatment dosing history, analyte level history, treatment response history, general medical history, etc. of the patient 6011. In certain embodiments, a single electronic medical record contains all the relevant information regarding the patient 6011, while in other embodiments, the relevant information is parsed among a plurality of electronic medical records corresponding to the patient 6011.

After determining one or more levels or concentrations of desired analytes, drugs, or compounds, the analyzer 6025, or in some embodiments, the sensor assembly 6019, can upload and record the determined one or more levels or concentrations onto the electronic medical record via the data system 5120. These recorded levels/concentrations can include a time stamp to indicate the time at which the analysis was performed. Because the body fluid analyzer 6025 (or the sensor assembly 6019) is simultaneously in communication with the patient 6011 and the data system 5120, it can provide a real-time flow of one or more current analyte levels and other data for recording in electronic medical record. The analyzer 6025 or the sensor assembly 6019 can also use information obtained on the one or more levels of analytes, drugs, or compounds as inputs or data to assist in determining the one or more levels of other analytes, drugs, or compounds. The analyzer 6025 or the sensor assembly 6019 can obtain this information directly (e.g., by analyzing a body fluid sample), or by accessing data stored in the electronic medical record.

For example, in one implementation of the patient infusion and monitoring system shown in FIG. 49, the electronic medical record can indicate that the patient 6011 is being administered a particular drug A (or particular nutritional compound, electrolyte, or any other analyte) which is a known interferent to estimating the level of one or more analytes sought to be measured in connection with the treatment of the patient 6011. Data which indicates that drug A is being administered to the patient 6011 can be added to the electronic medical record, for example, by a computer or other device in communication with the data system 5120 through which data is entered manually by clinical staff. The clinical staff can enter this patient treatment information or drug administration information based on prescriptions or other instructions from a physician, or based on analysis of the body fluid of the patient 6011 performed with equipment which is not in communication with the data system. In some embodiments, the computer or other data-entering device in communication with the data system 5120 can automatically update the electronic medical record. For example, data which indicates that drug A is being administered to the patient 6011 can be recorded in the electronic medical record by the analyzer 6025, the sensor assembly 6019, or by a separate measurement device (e.g., an additional analyzer) which is in communication with the data system 5120 and which analyzes a sample of the body fluid of the patient 6011 and determines that the drug A is present in the body fluid. One example of such an additional analyzer is a body fluid analyzer which is remote from the patient 6011 and which requires transporting a body fluid sample from the patient to the additional analyzer.

Based on such patient treatment information in the electronic medical record (e.g., an indication that drug A is being administered to the patient 6011), the analyzer 6025 or the sensor assembly 6019 can be prompted to estimate the level of drug A in addition to estimating the level of the "usual" one or more analytes of interest. This prompting can take the form of a command issued to the analyzer 6025 or the sensor assembly 6019 from another device on the data system 5120 (such as a computer, server or processor, based on a review or analysis of the patient's electronic medical record 5130), or self-prompting by the analyzer 6025 (or the sensor assembly 6019) itself, after receiving or accessing the patient's electronic medical record. However prompted, the analyzer 6025 or the sensor assembly 6019 analyzes a sample of the patient's body fluid, estimates based on this analysis the level of the drug A (or other interfering compound, as explained above), and makes an initial estimate of the level of one or more analytes of interest. The analyzer 6025 (and/or other appropriate device on the data system 5120) can then correct or adjust the initial estimate according to the known interfering tendency of drug A. For example, if a given level of drug A is known to affect the estimation of an analyte level by a given amount, the initial estimate is corrected or adjusted to eliminate or account for this effect. Alternatively, an indication in the electronic medical record (or a determination by the analyzer 6025 or the sensor assembly 6019 based on a body fluid analysis) that an interferent, such as drug A, is present can trigger execution of an algorithm by the analyzer 6025 (and/or other appropriate device on the data system 5120) for determining the desired analyte concentration in the presence of interferents. (See, e.g., any of the methods discussed with reference to the flowcharts illustrated in FIGS. 31, 32, and 34.)

Other variations are possible. In one embodiment, the sensor assembly 6019 communicates with the analyzer 6025, and the analyzer 6025 communicates with the data system 5120. The communication interface 5110 may be disposed in the analyzer 6025, or in the sensor assembly 6019, or in both. In other embodiments of the system, the communications interface 5110 may be disposed within the system controller 6023 instead of or in addition to a communications interface 5110 disposed within the analyzer 6025 or the sensor assembly 6019.

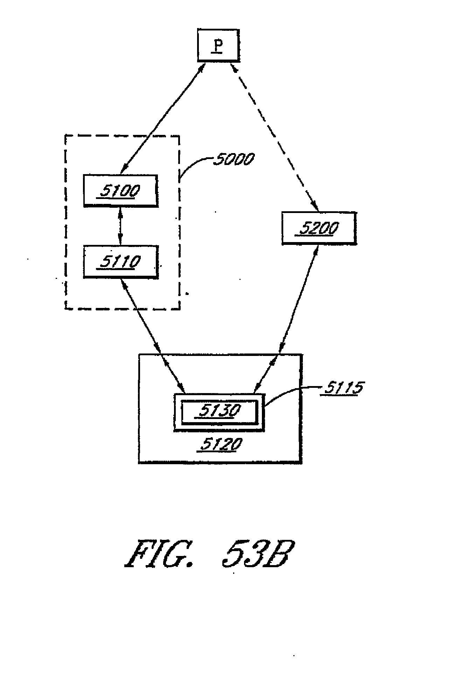

FIGURE 53B schematically illustrates another embodiment of the body fluid analyzing system 5000. As illustrated, a second body fluid analyzer 5200 separate from the body fluid analyzer 5100 is connected to the data system 5120 (e.g., a network) and can estimate one or more levels of analytes, drugs, or compounds by analyzing a sample of blood or other body fluid taken from the patient P. For example, the second analyzer 5200 can be a laboratory-based analyzer or a hand-held or portable analyzer. In these embodiments a sample of blood or other body fluid may be drawn or otherwise obtained from patient P and inserted into a hand-held or portable analyzer or transported to a laboratory analyzer which is located in a central lab for analysis of the fluid. After determining one or more levels or concentrations of the desired analytes, drugs, or compounds, the second body fluid analyzer 5200 can upload and record the determined one or more levels or concentrations onto the data file 5115 via the data system 5120. In some embodiments, the data file 5115 comprises an electronic medical record 5130. These recorded levels/concentrations can include a time stamp to indicate the time at which the analysis was performed. A data link enables continuous or selective data communication between the second analyzer 5200 and the data system 5120.

In certain embodiments, the body fluid analyzing system 5000 comprises the infusion and monitoring system shown in, for example, FIGS. 49, 52A, or 52C, and which is in fluid communication with the patient P (e.g., the patient 6011) through the catheter 6021. In some embodiments, the body fluid analyzer 5100 comprises the sensor assembly 6019 or the analyzer 6025. In various embodiments, the body fluid analyzing system 5000 provides an infusion mode and a sampling or draw mode. In an embodiment of the infusion mode, an infusion fluid is transferred by the pump 6013 from the infusion fluid source 6015, through the tubing 6017, the sensor assembly 6019, and the catheter 6021 to the patient 6011. In an embodiment of the sampling or draw mode, the pump 6013 is operated in reverse to draw a body fluid from the patient 6011 into the sensor assembly 6019 for monitoring and analysis. The communication interface 5110 permits the body fluid analyzer 5100 (e.g., the sensor assembly 6019 or the analyzer 6025) to communicate monitoring and analysis information with the data system 5120.

Where the second analyzer 5200 comprises a hand-held analyzer, a sample of body fluid may be taken from the patient via a finger-stick or other percutaneous sampling techniques, or by accessing a port provided in the I.V. tubing connected to the patient. Suitable hand-held analyzers include electrochemical test-strip-based meters, such as the Accu-Chek™ available from Roche Diagnostics Corp. (Indianapolis, IN), or other hand-held electrochemical analyzers such as the I-STAT™ available from Abbott Laboratories (Abbott Park, IL). Suitable laboratory meters include "bench-top" devices like the 2300 STAT Plus™ available from Yellow Springs Instruments (YSI, Inc., Yellow Springs, OH), and the BioProfile™ available from Nova Biomedical Corp. (Waltham, MA).

The patient monitoring system 5000 advantageously permits comparison of the analyte-concentration estimates made by the body fluid analyzer 5100 (e.g., the analyzer 6025 or the sensor assembly 6019) with the analyte-concentration estimates made by the second analyzer 5200 (e.g., a handheld or bench-top device), to facilitate, for example, verification that analyzer 5100 or second analyzer 5200 is functioning properly. After the body fluid analyzer 5100 analyzes a sample of the body fluid of the patient P and makes an estimate of analyte concentration, the analyzer 5100 (and/or any other suitable device of the data system 5120, such as the second analyzer 5200 or a computer or server) can compare that estimate to one or more (preferably recent) analyte-concentration estimates made by the second analyzer 5200 based on its analysis of the body fluid of the patient P. Performing the comparison can involve accessing one or more of the (subsequently) compared analyte-concentration estimates from the electronic medical record 5130 of the patient P. These accessed estimates can include, e.g., periodic laboratory-based measurements of one or more analytes of interest in the body fluid of the patient P, which are stored in the electronic medical record 5130.

In some preferred embodiments, the estimates made by second analyzer 5200 and used in this comparison must be made within a short time before or after the compared estimates are made by body fluid analyzer 5100. For example, estimates made by the second analyzer 5200 that serve as a basis for comparison can be made within a maximum of 1 day, 12 hours, 6 hours, 3 hours, 2 hours, 1 hour, or 30 minutes before or after the time of the corresponding estimate made by body fluid analyzer 5100.

If the comparison of estimates indicates that estimates made by body fluid analyzer 5100 unacceptably differ from estimates made by second analyzer 5200, an alert can be issued by the body fluid analyzer 5100, the second analyzer 5200, and/or other devices of data system 5120. Various forms of alerts can be issued; for example, an audible and/or visible alarm can be issued by the body fluid analyzer 5100 itself or by a hospital public address system or messaging system, a notation can be made in the electronic medical record 5130, and/or a message such as electronic mail can be sent to one or more devices of the data system 5120, such as a computer or other device discussed above. For example, in embodiments in which the body fluid analyzing system 5000 comprises the infusion and monitoring system shown, e.g., in FIG. 49, the alert can be issued by the alarm 6050.

Instead of or in addition to an alert, the body fluid analyzing system 5000 can activate a stop based on the comparison (e.g., a lock out or shutdown of body fluid analyzer 5100 can be triggered by body fluid analyzer 5100 itself, or second analyzer 5200, and/or other devices of the data system 5120).

The electronic medical record 5130 can include treatment information pertaining to patient P, such as analytes, drugs, or compounds that have been prescribed to the patient. The body fluid analyzer 5100, the second analyzer 5200, or other devices of the data system 5120 can issue one or more alerts when a comparison between treatment information and data from the body fluid analyzer 5100 indicates an unexpected result. For example, one or more alerts can signal when analytes, drugs, or compounds that have not been prescribed to patient P or are otherwise undesirable are present in the body fluid of patient P, or when a drug that has been prescribed for patient P (e.g., as shown in the medical record 5130) is not found in the body fluid of patient P. The one or more alerts can take any of the forms mentioned above. In response to the one or more alerts, hospital staff can take appropriate steps, such as administering particular compounds to patient P, to reestablish a desirable composition of the body fluid.

In a clinical or hospital environment, there may be several patients each fluidly coupled to a body fluid analyzing system. In some embodiments, each of the body fluid analyzing systems comprises an infusion and monitoring system as shown, for example, in FIGS. 49, 52A, or 52C. In other embodiments, some of the body fluid analyzing systems comprise additional or different analysis systems. Each of the body fluid analyzing systems may comprise a body fluid analyzer (e.g., the analyzer 6025 or the sensor assembly 6019) and a communication interface in communication with a data system. In addition, a laboratory analysis system comprising a laboratory analyzer and a laboratory communication interface in communication with the data system may be provided. The data system comprises electronic medical records for each of the patients.

The body fluid analyzing systems fluidly coupled to each of the patients may be configured to access and/or update the electronic medical records of the patients, respectively. In certain embodiments, the laboratory analyzer is used to analyze a body fluid or other sample from the patients and to update their respective electronic medical records via communication interface. In some embodiments, there may be two patients, while in further embodiments, additional patients and body fluid analyzing systems may be used to communicate with the data system and to access and/or update an electronic medical record for each patient that resides on data system.

MEASUREMENT OF MULTIPLE ANALYTES

In some of the embodiments of the infusion and monitoring system disclosed herein, an optical sensor 6040 may be disposed within the sensor assembly 6019 (FIGS. 50 and 50A) so as to enable the infusion and monitoring system to perform an optical analysis of the body fluid sample 6090 (FIG. 50A). The optical analysis may utilize spectroscopic or non-spectroscopic techniques. As described with reference to FIG. 50A, the system may be configured to pass an energy beam Ei into or through a portion of the body fluid sample 6090 disposed within the optical sensor 6040. The returned energy beam $E_r$ comprises an optical signal that carries information about the properties and characteristics of fluid sample 6090 in the chamber 6045. The analyzer 6025 may measure spectroscopic or non-spectroscopic properties of the returned energy beam $E_r$ and may comprise a spectrometer, a photometer, a colorimeter, a filter, and/or other optical devices. The analyzer 6025 may comprise an analyte detection system similar to any of the embodiments of the analyte detection system 1700 depicted in FIGS. 17 and 44-48. The analyzer 6025 may be configured to determine absorbance, reflectance, transmittance, color characteristics, or any other optical properties of the fluid sample 6090.

In some embodiments, the analyzer 6025 may also determine the presence or concentrations of one or more analytes of interest in the fluid under analysis. For example, the analyzer 6025 may be configured to quantify one or more analytes in the presence of interferents using embodiments of the spectroscopic methods disclosed herein. In certain embodiments, some of the disclosed spectroscopic methods are used to quantitatively estimate the concentration of one or more analyte in a mixture from a measurement, where the mixture contains one or more interferents that affect the measurement. In certain preferred embodiments, the measurement comprises one or more spectroscopic measurements of the mixture. Certain disclosed embodiments are particularly effective if each analyte and interferent component has a characteristic signature in the measurement, and if the measurement is approximately affine (i.e., includes a linear component and an offset) with respect to the concentration of each analyte and interferent. In one embodiment, a method includes a calibration process including an algorithm for estimating a set of coefficients and an offset value that permits the quantitative estimation of an analyte.

One method for estimating the concentration of an analyte in the presence of interferents is presented in flowchart 3100 of FIGURE 31 as a first step (Block 3110) where a measurement of a sample is obtained, a second step (Block 3120), where the obtained measurement data is analyzed to identify possible interferents to the analyte, a third step (Block 3130) where a model is generated for predicting the analyte concentration in the presence of the identified possible interferents, and a fourth step (Block 3140) where the model is used to estimate the analyte concentration in the sample from the measurement. Preferably the step of Block 3130 generates a model where the error is minimized for the presence of the identified interferents that are not present in a general population of which the sample is a member.

An embodiment of the method of flowchart 3100 for the determination of an analyte from spectroscopic measurements is further discussed herein with reference to FIGS. 31, 32, and 34. In some embodiments of the third step 3120 of the flowchart 3100, a statistical method generally similar to those discussed with reference to FIG. 32 may be used to identify possible interferents in the sample. For example, the statistical method may include forming a statistical Sample Population model (Block 3210), assembling a library of interferent data (Block 3220), comparing the obtained measurement and statistical Sample Population model with data for each interferent from an interferent library (Block 3230), performing a statistical test for the presence of each interferent from the interferent library (Block 3240), and identifying each interferent passing the statistical test as a possible interferent (Block 3250). In one preferred embodiment, the Mahalanobis Distance defined in Eq. (1) may be calculated to determine the statistical likelihood that a particular interferent from the Library is present in the sample (see the discussion with reference to FIG. 33B).

Once interferents are identified as being possibly present in the sample under analysis, a calibration constant, $\kappa$, for estimating the concentration of analytes in the presence of the identified interferents is generated (Block 3130 in flowchart 3100 of FIG. 31). FIG. 34 illustrates a flowchart showing a method to calculate the calibration constant. The calibration constant is applied to the obtained spectrum to derive an estimated concentration for the analyte of interest (Block 3140 of FIG. 31).

For example, in one embodiment of the method of flowchart 3100 (FIG. 31), the glucose concentration in a body fluid sample, such as a blood sample or a blood plasma sample, may be estimated by mid-IR absorption spectroscopy. Examples 1-3 (and Tables 1-3) describe further aspects of the measurement of the glucose concentration in the presence of one or more interferents.

The methods described with reference to FIGS. 31, 32, and 34 may be applied to determine the concentration of more than one analyte in the presence of interferents. For example, in one embodiment, the method of flowchart 3100 (FIG. 31) may be repeated for each analyte of interest so as to generate a status report on the chemistry of the body fluid sample. In one embodiment, the sensor assembly 6019 (FIGS. 50 and 50A) may comprise one optical sensor 6040, and the method of flowchart 3100 may be performed serially for each analyte of interest that may be in the fluid sample 6090 within the sensor 6040. In another embodiment, the sensor assembly 6019 may comprise more than one optical sensor 6040, and the method of flowchart 3100 may be performed on each fluid sample 6090 in each optical sensor 6040. In other embodiments, the method of flowchart 3100 may be performed to determine the concentrations of a selected group of analytes rather than a single analyte. Other variations are possible to generate the multi-analyte body fluid status report.

Additional embodiments of methods for measuring more than one analyte using spectroscopic techniques may be used. If two or more analytes have non-overlapping spectral features, then a first embodiment of the method is to obtain a spectrum corresponding to each analyte. The spectra may be analyzed serially for each analyte concentration according to the method of flowchart 3100 (FIG. 31). After the concentration of a first analyte is determined at Block 3140, the method may be resumed at Block 3110 for a second analyte, and so forth.

A second embodiment of the method may be suitable for analytes having non-overlapping features or for two or more analytes having overlapping features. In the second embodiment, one measurement is made that comprises the spectral features of the two or more analytes. The measurement may then be analyzed for each analyte according to the method of flowchart 3100 (FIG. 31) wherein the measurement is analyzed for each analyte, while treating the other analytes as interferents to the analyte being analyzed for. For example, after the concentration of a first analyte is determined at Block 3140, the method may be resumed at Block 3120 using the concentration of the first analyte as an interferent to the analysis of a second analyte and so forth. In some embodiments, this process is iterated so that a subsequent iteration may use the concentrations derived in earlier iterations as estimates in Block 3120.

An alternative embodiment of a method for calculating analyte concentrations includes obtaining a sequence of measurements of an analyte and mathematically manipulating these measurements. The measurements may be combined to yield an analyte concentration parameter such as, for example, a best estimate of an analyte concentration, or an average analyte concentration, or a trend in analyte concentration, or some combination thereof. An example where this alternative embodiment may be advantageous includes, but is not limited to, conditions where there are temporal variations in the fluid being analyzed such as, for example, variations in analyte or interferent concentration.

The determination of an average concentration from several previous measurements may provide a more useful indication of the analyte concentration. In cases where there is a variation in the average concentration, the measurements may be analyzed for the determination of a temporal trend in a concentration - for example a rate of change of a concentration of an analyte. Another example of a situation where this alterative embodiment may be useful is in systems where averaging measurements results in a more accurate estimate as, for example, where the measurement is subject to random noise. One illustrative example is a spectroscopic analysis system having random noise. There are other situations where the alternative embodiment may be useful.

In one embodiment of a method for calculating analyte concentrations from a sequence of measurements, a sample may be provided, for example, to sample analysis device 330 (FIG. 3), the sensors 6027-6040 (FIG. 49), or the analyzer 6025 (FIG. 49). The sample may comprise a series or sequence of drawn fluid samples. The drawn samples may be provided using fluid handling techniques generally similar to those described, for example, with reference to FIGS. 7A-7J. In other embodiments, a single fluid sample may be analyzed for a sequence of analytes.

In some embodiments, the controller 210 (FIG. 2) or 6023 (FIGS. 49, 52A, 52C) or the analyzer 6025 (FIGS. 49, 52A, 52C) may determine an estimate of an analyte concentration for each sample using, for example, the method described with reference to the flowchart shown in FIG. 31. The controller 210 or 6023 and/or the analyzer 6025 may store a sequence of estimated analyte concentrations for each of the drawn samples. The sequence of estimates may be further analyzed, and the results of the analysis may be communicated to a user, to other devices, to a data network, or to other components.

The sequence of estimates may be analyzed according to any one of a number of techniques for averaging measurements. In an embodiment involving performing a sequence of measurements on a single sample, an average of the measurements may be determined. In an embodiment involving performing a sequence of measurements obtained on a series of different samples, the sequence may be analyzed to determine an average. Averaging methods include, but are not limited to, providing an average of several of the most recent measurements, providing a weighted average of the most recent measurements that favors the most recent over the more distant measurements in time, or a statistical analysis that factors in the most statistically relevant measurements. Many other averaging methods may be used. Other embodiments may analyze the measurements for quantities in addition to an average such as, for example, a standard deviation, a variance, a median, a mode, or any other statistical parameter of interest. Still other embodiments may perform additional mathematical operations on the sequence of measurements.

Embodiments of the system disclosed herein that measure the concentration of a broader range of analytes may give a more complete status report of the parameters, characteristics, and chemistry of the body fluid sample. A further benefit is that a more accurate status report can be generated by using all the measured analytes to correct for interferences among them.

It will be understood that the steps of methods discussed herein are performed in one embodiment by an appropriate processor (or processors) of a processing (i.e., computer) system executing instructions (code segments) stored in appropriate storage. It will also be understood that the disclosed methods and apparatus are not limited to any particular implementation or programming technique and that the methods and apparatus may be implemented using any appropriate techniques for implementing the functionality described herein. The methods and apparatus are not limited to any particular programming language or operating system. In addition, the various components of the apparatus may be included in a single housing or in multiple housings that communication by wire or wireless communication.

Further, the interferent, analyte, or population data used in the method may be updated, changed, added, removed, or otherwise modified as needed. Thus, for example, spectral information and/or concentrations of interferents that are accessible to the methods may be updated or changed by updating or changing a database of a program implementing the method. The updating may occur by providing new computer readable media or over a computer network. Other changes that may be made to the methods or apparatus include, but are not limited to, the adding of additional analytes or the changing of population spectral information.

One embodiment of each of the methods described herein may include a computer program accessible to and/or executable by a processing system, e.g., a one or more processors and memories that are part of an embedded system. Thus, as will be appreciated by those skilled in the art, embodiments of the disclosed inventions may be embodied as a method, an apparatus such as a special purpose apparatus, an apparatus such as a data processing system, or a carrier medium, e.g., a computer program product. The carrier medium carries one or more computer readable code segments for controlling a processing system to implement a method. Accordingly, various ones of the disclosed inventions may take the form of a method, an entirely hardware embodiment, an entirely software embodiment or an embodiment combining software and hardware aspects. Furthermore, any one or more of the disclosed methods (including but not limited to the disclosed methods of measurement analysis, interferent determination, and/or calibration constant generation) may be stored as one or more computer readable code segments or data compilations on a carrier medium. Any suitable computer readable carrier medium may be used including a magnetic storage device such as a diskette or a hard disk; a memory cartridge, module, card or chip (either alone or installed within a larger device); or an optical storage device such as a CD or DVD.

APPARATUS AND METHODS FOR DISTRIBUTED SENSING

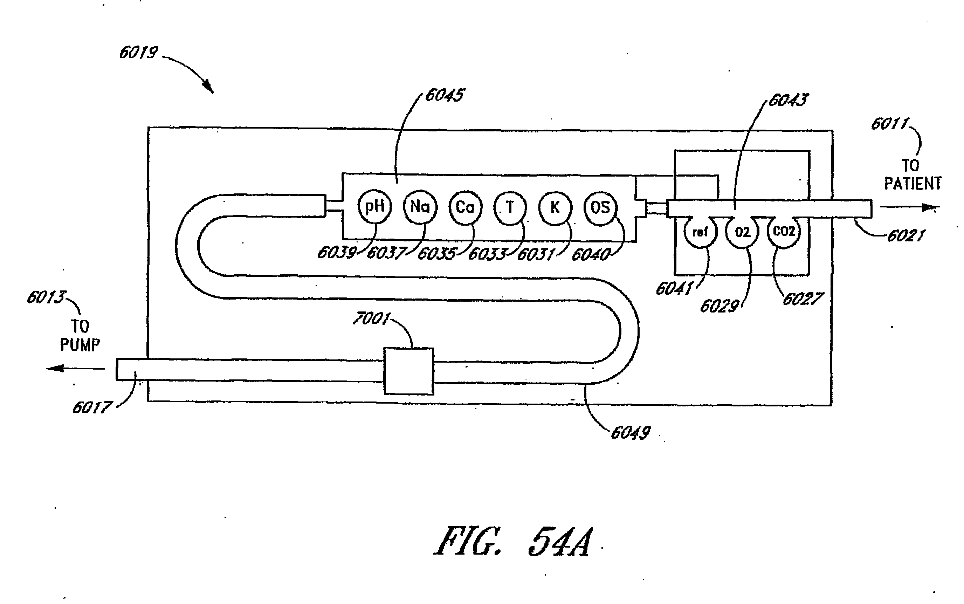

FIG. 54A illustrates an embodiment of the sensor assembly 6019 that may be generally similar to the embodiment illustrated in FIG. 50 except as further described below. The conductive sleeves 6047a-6047c are preferably not utilized in this embodiment. The sensor assembly 6019 includes a color sensor 7001 that may be used to detect changes in the color or the rate of change of the color in the fluid within the tubing 6049. The color sensor 7001 may be disposed at, or otherwise in sensing contact with, a position along the tubing 6049, or between the first and second chambers 6043 and 6045, or forward of the first chamber 6043. In other embodiments, the color sensor 7001 may be disposed along, or otherwise in sensing contact with, the catheter 6021 or the infusion line 6017 or at some other suitable position. The color sensor 7001 may be used to sense the color of the fluid within a passageway of choice and differentiate between a fluid such as, for example, saline, and blood within the passageway. The color sensor 7001 may be configured to transmit a signal to the system controller 6023.

In one embodiment, the color sensor 7001 comprises an optical colorimetric sensor, such as, for example, an Optical Blood Leak/Blood vs. Saline Detector available from Introtek International (Edgewood, NJ). Other devices may be used as well. For example, in some embodiments, the color sensor 7001 may detect the concentration of hemoglobin or the hematocrit in the fluid sample.

The color sensor 7001 advantageously may be used in a procedure in which a blood sample is drawn from the patient 6011. In an example of this procedure utilizing the embodiment shown in FIG. 54A, the infusion pump 6013 may operate in the rearward direction to draw blood into the chambers 6043 and 6045 and into tubing 6043. The color sensor 7001 is disposed such that when the leading edge of the blood sample has reached the position of the color sensor 7001, a sufficient volume of drawn blood has entered the chambers 6043 and 6045 so that the analytical sensors 6027-6040 are in sensing contact with the blood sample. In one embodiment, a blood sample comprising a range 0.1 - 25 milliliters may be drawn.

When the leading edge of the blood sample reaches the position of the color sensor 7001, the color sensor 7001 may detect the presence of blood by measuring the fluid color or a rate of change thereof. If the color sensor 7001 detects a sufficient change in the color properties of the fluid or a stable "plateau" of a color of reference (e.g., red), the sensor 7001 may signal the system controller 6023 to cease operation of the infusion pump 6013. The analytical sensors 6027-6040 may then be read to determine the desired properties and characteristics of the blood sample. Accordingly, by monitoring the color sensor 7011 to ascertain the presence of an undiluted or minimally diluted blood sample, the blood analysis system may reduce the effects of dilution of the blood by adjacent infusion fluid.

One or more color sensors 7001 may be included in the sensor assembly 6019, or positioned along the catheter 6021 or the infusion tube 6017. For example, in addition to the color sensor 7001 shown in FIG. 54A, some embodiments may include color sensors positioned in the chambers 6043 and 6045 or at a junction where the catheter 6021 couples to the assembly 6019. Additional color sensors may be advantageously used to monitor the presence of blood within the chambers 6043 and 6045 so as to increase the likelihood that a sufficient blood sample contacts the analytical sensors 6027-6040. Further, an optical sensor positioned at the junction with the catheter 6021 may be used during an operation to return the drawn blood sample to the patient 6011 so as to signal the system controller 6023 that the blood sample has exited the assembly 6019. By suitably monitoring color sensors, the system controller 6023 may operate the infusion pump 6013 at a different flow rate when blood is not being drawn or returned to the patient 6011, for example, at a rate of 0.1-10 milliliters per hour to beneficially keep open the catheterized blood vessel.

In the preferred embodiment shown and described with reference to FIG. 54A, the sensor 7001 comprises a color sensor. This is not intended to be a limitation and in other embodiments of the sensor assembly 6019, the sensor 7001 may comprise, for example, a hemoglobin sensor, a hematocrit sensor, a pressure sensor, a bubble sensor, a dilution sensor, or a combination thereof. Other sensors may be used as well. Examples of such sensors are discussed herein, including hemoglobin sensor 1003 (FIG. 10), pressure sensors and sensor units 317, 507, 1011, and 1102 (FIGS. 3, 5, 10-11), and bubble sensors and sensor units 314a, 314b, 321, 505, 1001a, 1001b, and 1001c (FIGS. 3, 5, 10). A dilution sensor compatible with certain embodiments described herein provides a signal indicative of the dilution of the fluid within a fluid passageway. In certain embodiments, the signal from any one or any combination of the aforementioned sensors may be used by the controller 6023 to indicate that the pump 6013 can discontinue drawing body fluid from the patient 6011.

Accordingly, the sensor 7001 in FIG. 54A may comprise various sensors or sensor units that are configured to sense a property of the fluid in a passageway. FIG. 54A shows the sensor 7001 disposed along the tubing 6049, however, in other embodiments the sensor 7001 may be disposed along or in other passageways such as, for example, the infusion line 6017, the catheter 6021, the chambers 6043, 6045, the analyzer 6025, or at some other suitable position. In various embodiments, sensing a property of the fluid within a fluid passageway comprises sensing the color, the hemoglobin content, the hematocrit, the dilution, the pressure of the fluid in the passageway, the presence of one or more bubbles in the passageway, detecting the arrival of a body fluid in the passageway, or a combination thereof. In certain embodiments, sensing a property of the fluid within the fluid passageway comprises sensing a property of the infusion fluid or the body fluid within the fluid passageway. In certain embodiments, sensing a property of the fluid with the fluid passageway may include determining the concentration of at least one analyte in at least one component of the fluid within the fluid passageway.

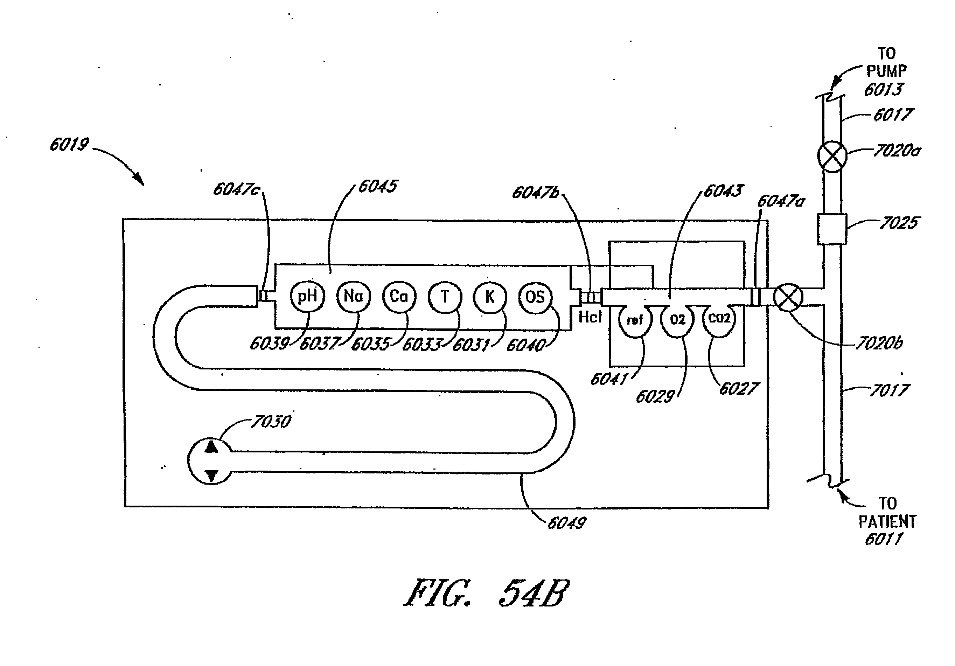

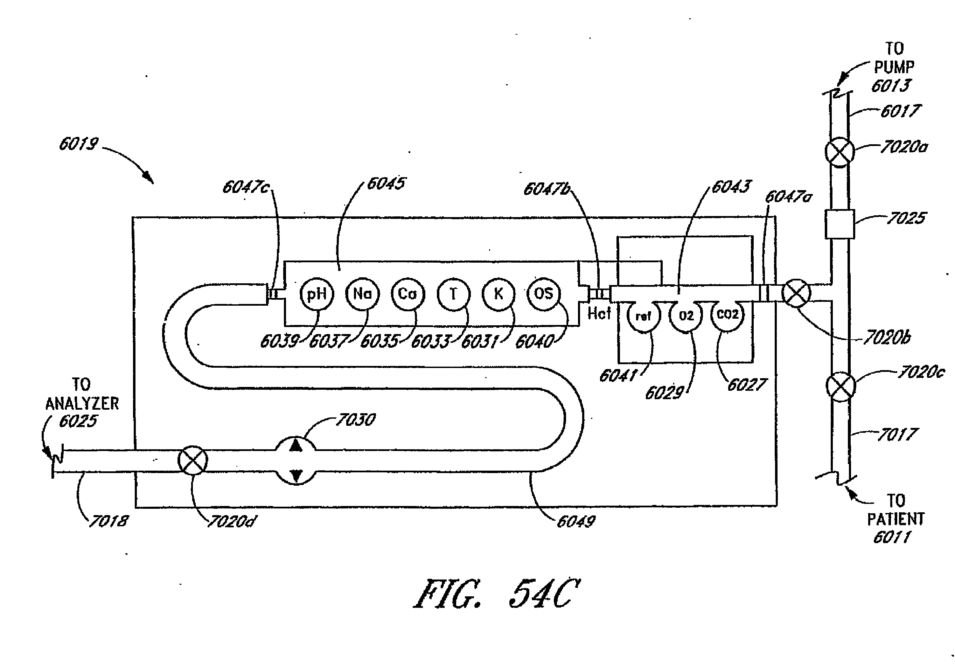

FIGS. 54B and 54C illustrate embodiments of the blood analysis system wherein a sensor 7025 is disposed along (or otherwise in sensing contact with) a portion of infusion tubing 7017. Similarly as described above with reference to the sensor 7001 in FIG. 54A, in the embodiments shown in FIGS. 54B and 54C, the sensor 7025 may comprise, for example, a color sensor, a hemoglobin sensor, a hematocrit sensor, a pressure sensor, a bubble sensor, a dilution sensor, a combination thereof, or some other suitable sensor.

In certain preferred embodiments of the systems illustrated in FIGS. 54B and 54C, the sensor 7025 comprises a color sensor that is used to detect changes in the color or the rate of change of the color in the fluid in the infusion tubing 7017. The color sensor 7025 may be used to sense the color of the fluid within a passageway of choice and differentiate between fluid and blood within the passageway. In other embodiments, the color sensor 7025 may be disposed along, or otherwise in sensing contact with, the catheter 6021 or at some other suitable position. The color sensor 7025 may be configured to transmit a signal to the system controller 6023 indicative of the color or the rate of change of the color of the fluid.

The color sensor 7025 advantageously may be used in a procedure in which a blood sample is drawn from the patient 6011. In an example of this procedure utilizing the embodiments shown in FIGS. 54B or 54C (and which is described further below), the infusion pump 6013 operates in the rearward direction to draw blood from the patient 6011 into the infusion tubing 7017. During this procedure, the system controller 6023 directs a valve 7027a to remain open and a valve 7020b to remain closed. The color sensor 7025 is disposed along (or in sensing contact with) the infusion tubing 7017 to detect when a leading edge of the blood sample has reached the position of the color sensor 7025. For example, when the leading edge of the blood sample reaches the position of the color sensor 7025, the color sensor 7025 may detect the presence of blood by measuring the fluid color or a rate of change thereof. If the color sensor 7025 detects a sufficient change in the color properties of the fluid or a stable "plateau" of a color of reference (e.g., red), the sensor 7025 transmits a suitable signal to the system controller 6023, which directs the infusion pump 6013 to cease operation and the valve 7020a to close. The system controller 6023 then signals the valve 7020b to open and signals a pump 7030 to operate in the rearward direction so as to draw a portion of the blood sample from the passageway 7017, through the open valve 7020b, into the sensor assembly 6019. When a suitable volume of blood has entered the sensor assembly 6019 (as determined by any of the techniques discussed herein, e.g., monitoring the $CO_2$ and Ca sensors 6027, 6035), the analytical sensors 6027-6040 may be used to determine the desired properties and characteristics of the blood sample. Accordingly, the color sensor 7025 is advantageously used in the embodiments shown in FIGS. 54B and 54C to ascertain the presence of an undiluted or minimally diluted blood sample at the position of the sensor 7025, which reduces the effects of dilution of the blood sample by adjacent infusion fluid being drawn into the sensor assembly 6019.

BLOOD SAMPLE SEPARATION

FIG. 54B illustrates an embodiment that enables the blood analysis system to separate a drawn blood sample into a portion that is sent to the sensor assembly 6019 and a portion that is returned to the patient 6011. The sensor assembly 6019 may be generally similar to the embodiments illustrated in FIGS. 50-51, 52B, and 54A-54C, except as further described below. The system may include a passageway 7017, a first valve 7020a, a second valve 7020b, a color sensor 7025, and a pump 7030. The infusion pump 6013 is in fluid communication with the infusion tubing 6017. The first valve 7020a and the color sensor 7025 are disposed along the infusion tubing 6017. The infusion tubing 6017 forms a "T" with the second valve 7020b at a junction with the passageway 7017, which is in fluid communication with the catheter 6021 connected to the patient 6011. The valves 7020a and 7020b, the color sensor 7025, and the pumps 6013 and 7030 may be operably connected to the system controller 6023.

The valves 7020a and 7020b may comprise "pinch valves," in which one or more movable surfaces compress the tube to restrict or stop flow therethrough. In one embodiment, the pinch valves include one or more moving surfaces that are actuated to move together and "pinch" a flexible passageway to stop flow therethrough. Examples of a pinch valve include, for example, Model PV256 Low Power Pinch Valve (Instech Laboratories, Inc., Plymouth Meeting, PA). Embodiments of suitable pinch valves are illustrated in FIGS. 13A-13C and 14A-14B. Alternatively, one or more of valves 7020a or 7020b may be other valve types for controlling the flow through their respective passageways.

In one embodiment, pump 7030 is a directionally controllable pump that acts on a flexible portion of passageway 6049. Examples of a single, directionally controllable pump include, but are not limited to a reversible peristaltic pump or two unidirectional pumps that work in concert with valves to provide flow in two directions. In an alternative embodiment, pump 7030 includes a combination of pumps, including but not limited to displacement pumps, such as a syringe, and/or valve to provide bi-directional flow control through passageway 6049.

The embodiment illustrated in FIG. 54B provides for infusion and monitoring of patient blood. The infusion process advantageously may be used to reopen or keep open a blood vessel that has been catheterized. During an infusion process, infusion fluid from the infusion fluid source 6015 may be directed to the patient 6011. In one embodiment, the system controller 6023 operates the infusion pump 6013 in the forward direction to direct the infusion fluid into the infusion tubing 6017. The system controller 6023 signals the first valve 7020a to remain open and the second valve 7020b to remain closed to permit the infusion fluid to flow through the passageway 7017 and into the patient 6011 via the catheter 6021. During the infusion process, infusion fluid is prevented from entering the sensor assembly 6019, because the second valve 7020b is closed. The pump 7030 does not typically operate during the infusion process. The system controller 6023 may control the infusion pump 6013 so as to regulate the rate of flow of the infusion fluid into the patient 6011. Suitable rates of flow may be in the range 1-10 milliliters per hour.

During a blood sampling process, the fluid handling system in FIG. 54B may be used to draw blood from or to return blood to the patient 6011. In one embodiment of a method to draw blood from the patient 6011, the second valve 7020b is closed and the first valve 7020a is open. The system controller 6023 signals the infusion pump 6013 to operate in the rearward direction so as to draw blood from the patient into tubing 7017. When the color sensor 7025 signals that blood has reached the position of the color sensor 7025, the system controller 6023 signals the infusion pump 6013 to cease operation and signals the first valve 7020a to close. The system controller 6023 then signals the second valve 7020b to open and signals pump 7030 to operate in the rearward direction so as to draw a first portion of the blood sample from the passageway 7017, through the valve 7020b, and into the sensor assembly 6019. A second portion of the blood sample remains in tubing 7017. The pump 7030 continues to operate until a sufficient volume of blood is drawn into the chambers 6043 and 6045 so that the analytical sensors 6029-6040 are in sensing contact with the blood sample, after which the pump 7030 then ceases operation. As described herein with respect to FIGS. 50-54C, the system controller 6023 may monitor one or more of the analytical sensors 6029-6040 or a dedicated color sensor (not shown in FIG. 54B) to determine when to halt the pump 7030. Further processing of the first portion of the blood sample using the analytical sensors 6029-6040 may proceed using embodiments of any of the methods disclosed herein such as, for example, the spectroscopic methods for determining analyte concentrations discussed with reference to FIGS. 31, 32, and 34 or other optical, electrical, or electrochemical methods.

Some embodiments of the blood sampling system shown in FIG. 54B may be employed to implement a "draw and return" procedure in which the second portion of the blood sample remaining in the tubing 7017 after the first portion has been separated and moved into the sensor assembly 6019 is returned to the patient 6011. To return the second portion to the patient 6011, the system controller 6023 signals the second valve 7020b to close, the first valve 7020a to open, and the infusion pump 6013 to operate in the forward direction so as to return the second portion of the blood sample from the tubing 7017 back into the patient 6011. A draw and return procedure beneficially reduces the volume of blood drawn from the patient 6011 and used for sampling and analysis.

Additionally, in some embodiments, the volume of the drawn blood used for sampling and analysis (e.g., the first portion moved into the sensor assembly 6019) is reduced, for example, by decreasing the volume of the passageway between the infusion tubing 7017 and the sensor assembly 6019, and by decreasing the fluid volume within the sensor assembly 6019. In some embodiments, the internal diameters of the passageway 6049 and the passageway between the tubing 7017 and the sensor assembly 6019 are smaller than the internal diameter of the infusion tubing 7017. In other embodiments, the lengths of the passageway 6049 within the sensor assembly 6019 and the passageway between the tubing 7017 and the sensor assembly 6019 are reduced. In certain embodiments, the chambers 6043, 6045 are configured to have a volume large enough to provide a sufficient blood sample for the analytical sensors 6027-6041 to take measurements but without additional, unnecessary volume. Certain preferred embodiments are configured to use some or all of these techniques for reducing the volume of blood drawn from the patient 6011 and used for sampling and analysis.

After sampling and analysis is complete, some embodiments may return the first portion of the blood sample from the sensor assembly 6019 to the patient 6011. In these embodiments, the system controller 6023 signals the first valve 7020a to close, the second valve 7020b to open, and the pump 7030 to operate in the forward direction to return the first portion back into the patient 6011. These embodiments may use an additional color sensor positioned along tubing 7017 (not shown) to signal the system controller 6023 when the first portion has reached the patient 6011.

In other embodiments, the sensor assembly 6019 may include additional pumps, valves, and sensors configured to direct the first portion of the blood sample into a waste receptacle (not shown), which may be generally similar to the waste receptacle 325 illustrated and described with reference to FIG. 3. In these embodiments, the first portion of the blood sample is not returned to the patient 6011, which may be beneficial if the analytical sensors 6029-6040 cause the first portion of the blood sample to become contaminated, such as by mixing with a reagent.

FIG. 54C illustrates an embodiment that is generally similar to the embodiment illustrated in FIG. 54B, except as further described below. In this embodiment, the sensor assembly 6019 is configured to be coupled to the analyzer 6025 by a passageway 7018 so that a blood sample may be drawn from the patient 6011 and directed through the sensor assembly 6019 and into the analyzer 6025. As shown in FIG. 54C, this embodiment may include a third valve 7020c, a fourth valve 7020d, and the additional passageway 7018, which extends between the sensor assembly 6019 and the analyzer 6025 so as to maintain fluid contact therebetween.

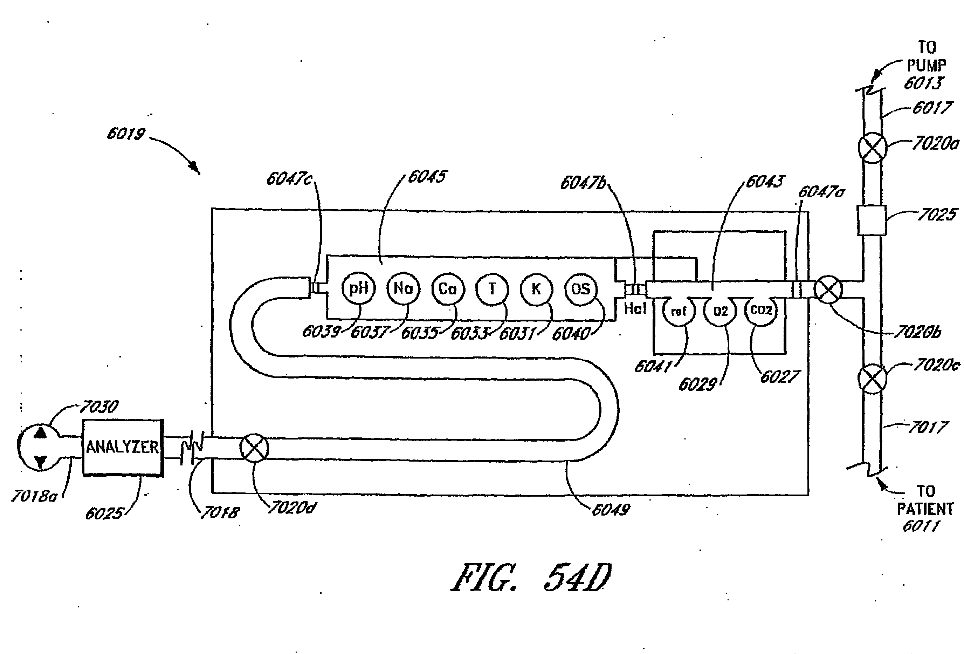

In different embodiments, the passageways, valves, and pumps may be configured differently. For example, FIG. 54D illustrates an embodiment that is generally similar to the embodiments illustrated in FIGS. 54B and 54C, except as further described below. In this embodiment, rather than being disposed within the sensor assembly 6019, the pump 7030 is disposed along the passageway 7018 or elsewhere in the system. For example, the pump 7030 may be disposed along the passageway 7018 coupling the analyzer 6025 and the sensor assembly 6019, or the pump 7030 may be disposed along a passageway 7018a that couples the pump 7030 and the analyzer 6025 (FIG. 54D). The lengths of the passageways 7018 and 7018a may be different from the lengths shown in FIG. 54D, which is intended to be a schematic representation rather than a scale drawing. In various alternate embodiments, different numbers, locations, arrangements, and configurations of valves, pumps, passageways, and sensors than illustrated in FIGS. 49-54D may be utilized. Further, the sizes, shapes, configurations, and orientations of the components schematically illustrated in FIGS. 49-54D can be different in different embodiments. Many variations are possible without departing from the scope of the principles disclosed herein.

In one embodiment of a procedure to draw a blood sample into the analyzer 6025, the system controller 6023 signals the first valve 7020a to close, the second, third, and fourth valves 7020b-7020d to open, and the pump 7030 to operate in the rearward direction. After a sufficient blood sample has been drawn from the patient 6011, the system controller 6023 signals the third valve 7020c to close. The controller 6023 may then signal the first valve 7020a to open such that infusion fluid may flow behind a trailing edge of the drawn blood sample. The pump 7030 may then direct the blood sample through the passageway 7018 into the analyzer 6025. Optionally, the infusion pump 6013 may be operated in the forward direction to assist the pump 7030 in directing the fluid sample into the analyzer 6025.

Sensors may be included in the analyzer 6025 to signal the system controller 6023 when the blood sample has reached the analyzer 6025 at which time the pump 7030 may cease operation. Suitable sensors include, for example, color sensors generally similar to color sensor 7001. In one embodiment, the system may restart the infusion process by closing the second valve 7020b, opening the third valve 7020c, and signaling the infusion pump 6013 to operate in the forward direction to direct infusion fluid into the patient 6011.

Analysis of the blood sample within the analyzer 6025 may proceed using any of the methods and apparatuses disclosed herein. In one embodiment, after reaching the analyzer 6025, the blood sample may pass through the sample preparation unit 332 for analysis by the analyte detection system 334 as illustrated in FIG. 3. For example, the concentration of analytes in the presence of interferents in the blood sample may be determined using embodiments of the methods presented in reference to FIGS. 31-34. In another embodiment, the drawn blood sample may be analyzed by the analytical sensors 6029-6040 in the sensor assembly 6019 before the blood sample is passed to the analyzer 6025 for further analysis.

Other embodiments of the blood analysis and fluid handling system illustrated in FIGS. 54B and 54C may include additional valves, pumps, tubing, passageways, devices, or sensors to achieve additional benefits as described herein. For example, an embodiment may additionally include pressure sensors, gas injectors, and bubble detectors as described herein with respect to FIGS. 3 and 6A-6B. Embodiments of the blood analysis and fluid handling system of FIGS. 54B and 54C also may utilize fluid handling methods generally similar to those described with respect to FIGS. 7A-7J.

TEMPERATURE MONITORING AND REGULATION

Some of the parameters and characteristics of the fluid sample 6090 sensed by the optical sensor 6040 may depend on the temperature of the fluid within the chamber 6045 (FIGS. 50 and 50A). For example, the absorbance, reflectance, or transmittance of the fluid sample 6090 in certain wavelength regions may be a function of fluid temperature. Accordingly, the temperature of the fluid sample 6090 may be monitored by the temperature sensor 6033 and transmitted to the analyzer 6025. The temperature sensor 6033 may comprise a thermocouple, a resistance temperature detector, a thermistor, a solid state temperature sensor, or any other suitable temperature detector. In one embodiment, the analyzer 6025 may use a temperature reading from the temperature sensor 6033 to normalize or adjust the sample measurements to a suitable reference or baseline temperature level, for example 95 degrees Fahrenheit, or to a suitable body temperature of the patient, or to a temperature suitable for performing the analyte detections, or to any other appropriate temperature. The analyzer 6025 may perform this normalization procedure by, for example, incorporating a table of differentials to be applied to the signal from the optical sensor 6040. Alternatively, the analyzer 6025 may include software or hardware processors that incorporate a temperature-dependent signal analysis model.

The temperature sensitivity of some fluid parameters may depend on the wavelengths being probed by the optical sensor 6040, and thus the measurement and/or control of the sample temperature may provide for improved fluid parameter estimation. For example, analyte concentrations determined using near-infrared optical methods may need correction based on the fluid temperature at the time of measurement of the fluid sample 6090. Accordingly, it may be preferred, although not necessary, for the sensor assembly 6019 to regulate the temperature of the fluid sample in one or both of the chambers 6043, 6045. In some embodiments, the sensor assembly 6019 includes a suitable thermal device disposed within the sensor assembly 6019. The thermal device may comprise, for example, a thermoelectric heating/cooling device coupled to a proportional-plus-integral-plus-derivative (PID) controller. In one embodiment, the thermoelectric device may comprise a Peltier thermoelectric device. The thermal device may be used to regulate the temperature of the fluid sample 6090 to a suitable reference or baseline value such as, for example, 95 degrees Fahrenheit, or to a suitable body temperature of the patient, or to a temperature suitable for performing the analyte detections, or to any other appropriate temperature. In some embodiments, the thermal device may be controlled by the system controller 6023. In other embodiments, the thermal device may be disposed elsewhere in the system, for example, along the infusion tube 6017. Still other embodiments may include additional thermal devices or temperature sensors.

Although the invention(s) presented herein have been disclosed in the context of certain preferred embodiments and examples, it will be understood by those skilled in the art that the invention(s) extend beyond the specifically disclosed embodiments to other alternative embodiments and/or uses of the invention(s) and obvious modifications and equivalents thereof. Thus, it is intended that the scope of the invention(s) herein disclosed should not be limited by the particular embodiments described above, but should be determined only by a fair reading of the claims that follow.

FURTHER EMBODIMENTS

1. Apparatus for monitoring a predetermined parameter of a patient's body fluid and infusing the patient, comprising:

an infusion line having a patient end configured for fluid communication with a blood vessel of the patient;
a source of an infusion fluid, said source in fluid communication with said infusion line;
a reversible infusion pump coupled to the infusion line;
a body fluid sensor assembly mounted in fluid communication with the infusion line and including a first sensor, said first sensor comprising an optical sensor configured to provide a signal indicative of a predetermined parameter of fluid present in the infusion line near said first sensor; and
a controller that is configured to operate the infusion pump in a forward direction, to pump the infusion fluid through the infusion line toward the patient end, and that is configured to intermittently interrupt its operation of the infusion pump in the forward direction to operate the infusion pump in a rearward direction, to draw a body fluid sample from the patient through the patient end of the infusion line.

2. The apparatus of embodiment 1, wherein said optical sensor comprises a sample cell that is substantially transmissive to light comprising the wavelength $\lambda$.

3. The apparatus of embodiment 2, wherein said sample cell comprises one or more windows that are substantially transmissive to light comprising the wavelength λ.

4. The apparatus of embodiment 2 further comprising:

a light source configured to produce light comprising the wavelength λ;
a light detector configured to be responsive to light comprising the wavelength λ;
an optical path defined from the light source to the sample cell and from the sample cell to the light detector;

wherein the light source, the light detector, and the sample cell are configured to be in optical communication along the optical path.

5. The apparatus of embodiment 4, wherein the optical path comprises one or more optical fibers.

6. The apparatus of embodiment 2, wherein the wavelength λ is in the infrared.

7. The apparatus of embodiment 2, wherein the wavelength λ is in the range from 1 micron to 20 microns.

8. The apparatus of embodiment 2, wherein the wavelength λ is in the range from 4 microns to 10 microns.

9. The apparatus of embodiment 4, wherein the light detector is a spectrometer.

10. The apparatus of embodiment 4, wherein light comprising the wavelength λ is directed from the light source to the sample cell along the optical path such that the light interacts with a portion of the body fluid sample in the sample cell and a portion of the light is directed to the light detector along the optical path.

11. The apparatus of embodiment 10, wherein the light is transmitted through the portion of the body fluid sample in the sample cell.

12. The apparatus of embodiment 10, wherein the light is reflected from the portion of the body fluid sample in the sample cell.

13. The apparatus of embodiment 1, wherein the controller is configured to monitor the signal provided by the first sensor of the body fluid sensor assembly and to detect a change in the signal indicative of an arrival of the body fluid sample at the first sensor.

14. The apparatus of embodiment 13, wherein the controller is configured to cease its operating of the infusion pump in the rearward direction in response to detecting the arrival of the body fluid sample at the first sensor.

15. A method of extracting and analyzing bodily fluids from a patient at the point of care for said patient comprising:

establishing fluid communication between an analyte detection system, a sensor assembly, and a bodily fluid in said patient, wherein said sensor assembly comprises a first sensor;
drawing from said patient a portion of said bodily fluid;
separating from said drawn portion a first component of said bodily fluid, while said analyte detection system and said sensor assembly remain in fluid communication with said patient; and
with said analyte detection system, analyzing said first component to measure a concentration of an analyte.

16. The method of embodiment 15, wherein said first sensor comprises an optical sensor.

17. The method of embodiment 15, wherein said separating comprises filtering said first component from said drawn portion of bodily fluid.

18. The method of embodiment 15, wherein said separating comprises centrifuging said first component from said drawn portion of bodily fluid.

19. The method of embodiment 15, said method further comprising:

monitoring a signal produced by said first sensor of the sensor assembly; and
detecting a change in said signal indicative of an arrival of said bodily fluid at said first sensor.

20. The method of embodiment 19, said method further comprising ceasing to draw said bodily fluid from said patient in response to detecting said arrival of said bodily fluid at said first sensor.

21. Apparatus for extracting and analyzing bodily fluids from a patient at the point of care for said patient comprising:

an infusion line having a patient end configured for fluid communication with a blood vessel of the patient;
a source of an infusion fluid, said source in fluid communication with said infusion line;
a reversible infusion pump coupled to the infusion line;
a body fluid sensor assembly mounted in fluid communication with the infusion line and including a first sensor, said first sensor configured to provide a signal indicative of a predetermined parameter of fluid present in the infusion line near said first sensor;
a controller that is configured to operate the infusion pump in a forward direction, to pump the infusion fluid through the infusion line toward the patient end, and that is configured to intermittently interrupt its operation of the infusion pump in the forward direction to operate the infusion pump in a rearward direction, to draw a body fluid sample from the patient through the patient end of the infusion line;
a fluid component separator mounted in fluid communication with said infusion line and configured to separate from said body fluid sample a first component of said body fluid sample.

22. The apparatus of embodiment 21, wherein said first sensor comprises an optical sensor.

23. The apparatus of embodiment 21, wherein the fluid component separator comprises a filter or a membrane.

24. The apparatus of embodiment 21, wherein the fluid component separator comprises a centrifuge.

25. The apparatus of embodiment 21, wherein the first component of the body fluid sample comprises blood plasma.

26. The apparatus of embodiment 21, wherein the first component of the body fluid sample has fewer impurities than the body fluid sample.

27. The apparatus of embodiment 21, wherein the predetermined parameter of the patient's body fluid comprises glucose concentration.

28. The apparatus of embodiment 21, wherein the controller is configured to monitor the signal provided by the first sensor of the body fluid sensor assembly and to detect a change in the signal indicative of the arrival of the first component of the body fluid sample at the first sensor.

29. The apparatus of embodiment 28, wherein the controller is configured to cease its operating of the infusion pump in the rearward direction in response to detecting the arrival of the first component of the body fluid sample at the first sensor.

30. Apparatus for monitoring a predetermined parameter of a patient's blood while infusing an infusion fluid into the patient, comprising:

an infusion line having a patient end configured for insertion into a blood vessel of the patient;
a source of an infusion fluid, said source in fluid communication with said infusion line;
a reversible infusion pump coupled to the infusion line;
a blood chemistry sensor assembly mounted in fluid communication with the infusion line and including a first sensor that provides a signal indicative of a predetermined parameter of fluid present in the infusion line;
a device operatively connected to the apparatus to provide one or more anti-clotting agents to at least a portion of said infusion line;
a controller configured to operate the infusion pump in a forward direction, to pump the infusion fluid through the patient end of the infusion line for infusion into the patient, and configured to intermittently interrupt its operation of the infusion pump in the forward direction to operate the infusion pump in a rearward direction, to draw a blood sample from the patient through the patient end of the infusion line into sensing contact with the first sensor of the blood chemistry sensor assembly.

31. The apparatus of embodiment 30, wherein the first sensor comprises an optical sensor.

32. The apparatus of embodiment 30, wherein said one or more anti-clotting agents comprises a detergent and wherein said device provides said detergent within at least a portion of said infusion line.

33. The apparatus of embodiment 32, wherein said detergent comprises a protease enzyme.

34. The apparatus of embodiment 30, wherein said device comprises an ultrasound transducer positionable to transmit ultrasound to said infusion line.

35. The apparatus of embodiment 34, wherein said ultrasound comprises a frequency within a range from about 15 kHz to about 60 kHz.

36. The apparatus of embodiment 34, wherein said ultrasonic transducer produces ultrasound having an ultrasonic power within a range from about 2 Watts to about 200 Watts.

37. The apparatus of embodiment 30, wherein the controller is configured to monitor the signal provided by the first sensor of the blood chemistry sensor assembly and to detect a change in the signal indicative of the arrival of the blood sample at the first sensor.

38. The apparatus of embodiment 37, wherein the controller is configured to cease its operating of the infusion pump in the rearward direction in response to detecting the arrival of the blood sample at the first sensor.

39. A patient status monitoring system, said system comprising:

a first body fluid analyzer;
a body fluid sensor assembly including a first sensor that provides a signal indicative of a predetermined parameter of fluid present in the body fluid sensor assembly;
a controller configured to monitor said signal from said body fluid sensor assembly and to detect a change in said signal indicative of an arrival of said fluid present in the body fluid sensor assembly at said first sensor;
a fluid passageway configured to provide fluid communication among said first body fluid analyzer, said body fluid sensor assembly, and a body fluid in a patient;
said first body fluid analyzer configured to be in communication with a data network, said data network including at least one data file; and
said first body fluid analyzer configured to access said at least one data file via said data network.

40. The patient status monitoring system of embodiment 39, wherein said first body fluid analyzer is configured to update said at least one data file.

41. The patient status monitoring system of embodiment 39, wherein said first sensor comprises an optical sensor.

42. The patient status monitoring system of embodiment 39, wherein said at least one data file contains calibration information for calibrating said first body fluid analyzer or said body fluid sensor assembly.

43. The patient status monitoring system of embodiment 39, wherein said at least one data file contains calibration information for calibrating said signal from said body fluid sensor assembly.

44. The patient status monitoring system of embodiment 39, said system further comprising:

a separate body fluid analyzer spaced apart from said first body fluid analyzer, said separate body fluid analyzer configured to be in communication with said data network; and

wherein said at least one data file contains separate measurement information relating to a measurement performed by said separate body fluid analyzer.

45. The patient status monitoring system of embodiment 44, wherein said first body fluid analyzer is configured to perform a first measurement on said body fluid sample and to compare information related to said first measurement with said separate measurement information from said separate body fluid analyzer.

46. The patient status monitoring system of embodiment 45, wherein said first body fluid analyzer is configured to use said compared information to verify the proper functioning of the first body fluid analyzer or the separate body fluid analyzer.

47. The patient status monitoring system of embodiment 46, wherein said first body fluid analyzer or said separate body fluid analyzer are configured to issue an alert if said compared information indicates improper functioning of said first body fluid analyzer or said separate body fluid analyzer.

48. The patient status monitoring system of embodiment 39, further comprising:

an infusion line having a patient end configured for insertion into a blood vessel of said patient;
a source of an infusion fluid, said source in fluid communication with said infusion line;
a reversible infusion pump coupled to said infusion line;

wherein said controller is configured to operate said infusion pump in a forward direction to pump said infusion fluid through said infusion line for infusion into said patient, and configured to intermittently interrupt its operation of said infusion pump in said forward direction to operate said infusion pump in a rearward direction, to draw a body fluid sample from said patient through said patient end of said infusion line into sensing contact with said first sensor of said body fluid sensor assembly; and

wherein said signal produced by said first sensor provides an indication of a predetermined parameter of said patient's body fluid when said body fluid sample is in sensing contact with said first sensor.

49. The patient status monitoring system of embodiment 48, wherein said controller is configured to cease its operating of said infusion pump in said rearward direction in response to detecting said arrival of said body fluid sample at said first sensor.

50. The patient status monitoring system of embodiment 48, wherein said first sensor comprises an optical sensor.

51. The patient status monitoring system of embodiment 48, wherein said controller is configured to communicate said indication of said predetermined parameter to said data network.

52. The patient status monitoring system of embodiment 51, wherein said controller is configured to update said data file with said indication of said predetermined parameter.

53. An analyte detection system comprising:

a body fluid sensor assembly including a first sensor, the first sensor configured to provide information relating to a measurement of at least one analyte in a body fluid sample that is in sensing contact with the first sensor;
a processor; and
stored program instructions executable by said processor such that said system:

(a) identifies, based on said measurement, one or more possible interferents to the measurement of said at least one analyte in the body fluid sample;
(b) calculates a calibration which reduces error attributable to said one or more possible interferents;
(c) applies the calibration to the measurement; and
(d) estimates, based on the calibrated measurement, a concentration of said at least one analyte in the body fluid sample.

54. The analyte detection system of embodiment 53, wherein said at least one analyte comprises a first analyte and a second analyte.

55. The analyte detection system of embodiment 53, wherein said first sensor comprises an electrochemical sensor.

56. The analyte detection system of embodiment 53, wherein said first sensor comprises an optical sensor.

57. The analyte detection system of embodiment 53, said system further comprising:

an infusion line having a patient end configured for insertion into a blood vessel of a patient;

a source of an infusion fluid, said source in fluid communication with said infusion line;

a reversible infusion pump coupled to the infusion line;

a controller configured to operate the infusion pump in a forward direction, to pump the infusion fluid through the infusion line for infusion into the patient, and configured to intermittently interrupt its operation of the infusion pump in the forward direction to operate the infusion pump in a rearward direction, to draw the body fluid sample from the patient through the patient end of the infusion line into sensing contact with the first sensor of body fluid sensor assembly;

wherein the controller further is configured to monitor the signal provided by the first sensor of the body fluid sensor assembly and to detect a change in the signal indicative of the arrival of the body fluid sample at the first sensor.

58. The analyte detection system of embodiment 57, wherein the controller is configured to cease its operating of the infusion pump in the rearward direction in response to detecting the arrival of the body fluid sample at the first sensor.

59. The analyte detection system of embodiment 57, wherein the first sensor comprises an electrochemical sensor.

60. The analyte detection system of embodiment 57, wherein the first sensor comprises an optical sensor.

61. The analyte detection system of embodiment 60, further comprising a source of electromagnetic radiation, said source configured such that said radiation is directed to said optical sensor.

62. The analyte detection system of embodiment 61, further comprising a detector configured to detect radiation from said optical sensor.

63. The analyte detection system of embodiment 62, wherein said detector comprises a spectroscopic analyzer.

64. The analyte detection system of embodiment 63, wherein said spectroscopic analyzer comprises an infrared spectroscope.

65. Apparatus for analyzing the composition of bodily fluid, said apparatus comprising:

a first fluid passageway having a patient end which is configured to provide fluid communication with a bodily fluid within a patient;

at least one pump coupled to said first fluid passageway, said at least one pump configured to have an infusion mode in which said pump is operable to deliver infusion fluid to said patient through said patient end and a sample draw mode in which said pump is operable to draw a sample of said bodily fluid from said patient through said patient end;

a controller that is configured to operate said at least one pump in said infusion mode and in said sample draw mode;

a spectroscopic analyte detection system accessible via said first fluid passageway such that said analyte detection system can receive at least one component of said drawn sample of bodily fluid, and determine a concentration of at least one analyte, said analyte detection system being spaced from said patient end of said first fluid passageway;

a body fluid sensor assembly mounted in fluid communication with the first fluid passageway at or near said patient end and spaced from said analyte detection system, said body fluid sensor assembly including a first sensor and a second sensor;

wherein the first sensor is configured to sense a first property indicative of the fluid within said first fluid passageway and to provide a first signal indicative of said first property;

wherein the second sensor is configured to sense a second property indicative of the fluid within said first fluid passageway and to provide a second signal indicative of said second property; and

wherein the controller is configured to monitor the first signal provided by the first sensor and to detect a change in the first signal indicative of an arrival of the drawn sample of body fluid at the first sensor.

66. The apparatus of embodiment 65, wherein the controller is configured to signal said at least one pump to cease said sample draw mode in response to the change in the first signal indicative of the arrival of the drawn sample of body fluid at the first sensor.

67. The apparatus of embodiment 65, wherein said first sensor is selected from the group consisting of a colorimetric sensor, a hemoglobin sensor, a hematocrit sensor, a pressure sensor, a dilution sensor, and a bubble sensor.

68. The apparatus of embodiment 65, wherein the first property comprises a color or a rate of change of a color of the fluid within said first fluid passageway.

69. The apparatus of embodiment 65, wherein the second sensor comprises an electrochemical sensor.

70. The apparatus of embodiment 65, wherein the second sensor comprises an optical sensor.

71. A fluid handling and analysis system, said system comprising:

a fluid transport network comprising at least a first fluid passageway, said fluid transport network including a patient end configured to maintain fluid communication with a bodily fluid in a patient;
a sample analysis chamber and waste container, each accessible by said fluid transport network; a bodily fluid sensor assembly in fluid communication with said fluid transport network and including a first sensor that provides a signal indicative of a predetermined parameter of fluid present in said first fluid passageway;
a pump unit in operative engagement with said fluid transport network, said pump unit configured to have an infusion mode in which said pump unit delivers an infusion fluid to said patient through said patient end and a sample draw mode in which said pump unit draws a volume of said bodily fluid from said patient through said patient end, toward said sample analysis chamber;

wherein said fluid transport network and said pump unit are configured to draw a volume of said bodily fluid from said patient, isolate a fraction of said bodily fluid from said volume, and pass said fraction to said sample analysis chamber and then to said waste container.

72. The system of embodiment 71, wherein said first sensor comprises an optical sensor.

73. The system of embodiment 71, further comprising a spectroscopic fluid analyzer configured to analyze said fraction of said bodily fluid in said sample analysis chamber and to determine a concentration of at least one analyte in said fraction.

74. The system of embodiment 71, wherein said pump unit comprises a first pump operable in said infusion mode and a second pump operable in said sample draw mode.

75. The system of embodiment 71, wherein said pump unit is directionally controllable.

76. The system of embodiment 71, wherein said pump unit is further configured to return a remainder of said drawn volume of bodily fluid to said patient via said fluid transport network.

77. The system of embodiment 76, further comprising one or more valves configured to assist the pump unit to selectively draw a volume of said bodily fluid from said patient, isolate a fraction of said bodily fluid from said volume, pass said fraction to said sample analysis chamber and then to said waste container, and to return a remainder of said drawn volume of bodily fluid to said patient via said fluid transport network.

78. The system of embodiment 71, further comprising a controller configured to monitor the signal provided by the first sensor and to detect a change in the signal indicative of an arrival of the drawn bodily fluid at the first sensor.

79. The system of embodiment 78, wherein the controller is configured to operate said pump unit in said infusion mode and said sample draw mode.

80. The system of embodiment 79, wherein the controller is configured to signal said pump unit to cease said sample draw mode in response to said change in said signal indicative of the arrival of said drawn bodily fluid at the first sensor.

81. Apparatus for monitoring a predetermined parameter of a patient's body fluid while infusing an infusion fluid into the patient, comprising:

an infusion line having a patient end configured for insertion into a blood vessel of the patient;

a source of an infusion fluid, said source in fluid communication with said infusion line;

a reversible infusion pump coupled to the infusion line;

a body fluid sensor assembly mounted in fluid communication with the infusion line, said sensor assembly including a temperature sensor that provides temperature information relating to fluid present in the body fluid sensor assembly, and a first sensor that provides a signal indicative of a parameter of fluid present in the body fluid sensor assembly;

a controller configured to operate the infusion pump in a forward direction, to pump the infusion fluid through the infusion line for infusion into the patient, and configured to intermittently interrupt its operation of the infusion pump in the forward direction to operate the infusion pump in a rearward direction, to draw a body fluid sample from the patient through the patient end of the infusion line into sensing contact with the first sensor and the temperature sensor of the body fluid sensor assembly;

wherein the controller further is configured to monitor the signal provided by the first sensor of the body fluid sensor assembly and to detect a change in the first signal indicative of an arrival of the body fluid sample at the first sensor;

wherein the signal produced by the first sensor provides an indication of a predetermined parameter of the patient's body fluid when the body fluid sample is in sensing contact with the first sensor; and

wherein the controller is configured to acquire the temperature information and to use the temperature information when determining said predetermined parameter of the patient's body fluid.

82. The apparatus of embodiment 81, wherein the first sensor comprises an optical sensor.

83. The apparatus of embodiment 81, wherein the temperature sensor comprises a thermistor.

84. The apparatus of embodiment 81, wherein the controller is configured to cease its operation of the infusion pump in the rearward direction in response to detecting the arrival of the body fluid sample at the first sensor.

85. The apparatus of embodiment 81, wherein the body fluid sensor assembly further comprises a thermal device configured to regulate a temperature of the drawn body fluid sample.

86. The apparatus of embodiment 85, wherein the thermal device comprises a thermoelectric device.

87. The apparatus of embodiment 86, wherein the thermoelectric device comprises a resistor.

88. The apparatus of embodiment 86, wherein the thermoelectric device comprises a Peltier device.

89. The apparatus of embodiment 86, wherein the thermoelectric device is operable in a heating mode and in a cooling mode.

90. The apparatus of embodiment 85, wherein the thermal device is configured to regulate the temperature to be substantially equal to a body temperature of the patient.

91. The apparatus of embodiment 85, wherein the thermal device further comprises a temperature control system.

92. The apparatus of embodiment 91, wherein the temperature control system comprises a proportional, integral, derivative (PID) control system.

**Claims**

1. Apparatus for monitoring a predetermined parameter of a patient's body fluid while infusing an infusion fluid (14) into the patient, comprising:

an infusion line (6017) having a patient end configured for insertion into a blood vessel of the patient;

a source of an infusion fluid (6015), said source (6015) in fluid communication with said infusion line (6017);

a reversible infusion pump (6013) coupled to the infusion line (6017);

a body fluid sensor assembly (6019) mounted in fluid communication with the infusion line (6017), said sensor assembly (6019) including a temperature sensor (6033) that provides temperature information relating to fluid present in the body fluid sensor assembly (6019), and a first sensor (6029 - 6040) that provides a signal indicative of a parameter of fluid present in the body fluid sensor assembly (6019);

a controller (6023) configured to operate the infusion pump (6013) in a forward direction, to pump the infusion fluid (14) through the infusion line (6017) for infusion into the patient, and configured to intermittently interrupt its operation of the infusion pump (6013) in the forward direction to operate the infusion pump (6013) in a rearward direction, to draw a body fluid sample (6090) from the patient through the patient end of the infusion line (6017) into sensing contact with the first sensor (6029 - 6040) and the temperature sensor (6033) of the body fluid sensor assembly (6019);

wherein the controller (6023) further is configured to monitor the signal provided by the first sensor (6029 - 6040) of the body fluid sensor assembly (6019) and to detect a change in the first signal indicative of the arrival of the body fluid sample (6090) at the first sensor (6029 - 6040);

wherein the signal is produced by the first sensor (6029 - 6040) provides an indication of a predetermined parameter of the patient's body fluid when the body fluid sample (6090) is in sensing contact with the first sensor; and

wherein the controller (6023) is configured to acquire the temperature information and to use the temperature information when determining said predetermined parameter of the patient's body fluid.

2. The apparatus of Claim 1, wherein the first sensor (6029 - 6040) comprises an optical sensor (6040).

3. The apparatus of Claim 1, wherein the temperature sensor (6033) comprises a thermistor.

4. The apparatus of Claim 1, wherein the controller (6023) is configured to cease its operation of the infusion pump (6013) in the rearward direction in response to detecting the arrival of the body fluid sample (6090) at the first sensor (6029 - 6040)

5. The apparatus of Claim 1, wherein the controller (6023) is further configured to normalize a measurement of the body fluid sample (6090) to a reference temperature.

6. The apparatus of Claim 5, wherein the controller (6023) is configured to normalize the measurement of the body fluid sample (6090) based on a temperature-dependent signal analysis model.

7. The apparatus of Claim 5, wherein the first sensor (6029 - 6040) comprises an optical sensor (6040), and the controller (6023) is configured to normalize the measurement of the body fluid sample (6090) based on differentials applied to the signal provided by the optical sensor (6040).

8. The apparatus of Claim 1, wherein the body fluid sensor assembly (6019) further comprises a thermal device configured to regulate a temperature of the drawn body fluid sample (6090).

9. The apparatus of Claim 8, wherein the thermal device comprises a thermoelectric device.

10. The apparatus of Claim 9, wherein the thermoelectric device comprises a resistor.

11. The apparatus of Claim 9, wherein the thermoelectric device comprises a Peltier device.

12. The apparatus of Claim 9, wherein the thermoelectric device is operable in a heating mode and in a cooling mode.

13. The apparatus of Claim 8, wherein the thermal device is configured to regulate the temperature of the body fluid sample (6090) to a reference temperature.

14. The apparatus of Claim 8, wherein the thermal device further comprises a temperature control system.

15. The apparatus of Claim 14, wherein the temperature control system comprises a proportional, integral, derivative (PID) control system.

FIG. 1

EP 2 138 097 A2

FIG. 1A

FIG. 1B

FIG. 2

*FIG. 3*

EP 2 138 097 A2

*FIG. 4*

FIG. 5

_315_

_610_

_610a_    _610b_    _610c_

_615a_    _615b_    _615c_

_613a_    _613b_    _613c_

_114_

_112_

_611a_    _611b_    _611c_

_318_    _113_

# FIG. 6A

_315'_

_113_

_316_

_312_

_112_    _112_

_313'_    _318_

_613_

_615_

# FIG. 6B

FIG. 7A

FIG. 7B

FIG. 7C

FIG. 7D

FIG. 7E

FIG. 7F

FIG. 7G

FIG. 7H

*FIG. 7I*

*FIG. 7J*

FIG. 8

FIG. 9

FIG. 10

FIG. 11

FIG. 12

FIG. 13A

FIG. 13B

FIG. 13C

*FIG. 14B*

*FIG. 14A*

**FIG. 15**

**FIG. 16**

*FIG. 17*

FIG. 18

FIG. 19

FIG. 20

FIG. 21

FIG. 22A

FIG. 22B

FIG. 22C

FIG. 23A

FIG. 23B

FIG. 23C

FIG. 23D

FIG. 23E

FIG. 24A

FIG. 24B

FIG. 25B

FIG. 25A

2091

2594

2592

*FIG. 25D*

2464

2091

2594,2592

2464

*FIG. 25C*

FIG. 25E

FIG. 25F

FIG. 26B

FIG. 26A

FIG. 27A

FIG. 27B

*FIG. 27C*

FIG. 28

FIG. 29

EP 2 138 097 A2

*FIG. 30*

wavelength (µm)

double glucose
mannitol
N acetyl L cyclelne
dextran
procainamide

*3100*

OBTAIN A SAMPLE MEASUREMENT *3110*

ANALYZE THE OBTAINED MEASUREMENT TO IDENTIFY POSSIBLE INTERFERENTS TO AN ANALYTE OF INTEREST *3120*

GENERATE A MODEL FOR PREDICTING THE ANALYTE CONCENTRATION FOR THE OBTAINED MEASUREMENT *3130*

APPLY GENERATED MODEL TO ESTIMATE ANALYTE CONCENTRATION FROM OBTAINED MEASUREMENT *3140*

*FIG. 31*

*3120*

FORM STATISTICAL SAMPLE POPULATION MODEL *3210*

ASSEMBLE LIBRARY IF INTERFERENT DATA *3220*

COMPARE OBTAINED MEASUREMENT AND STATISTICAL SAMPLE
POPULATION MODEL WITH DATA FOR EACH LIBRARY INTEFERENT *3230*

PERFORM A STATISTICAL TEST FOR THE
PRESENCE OF EACH LIBRARY INTERFERENT *3240*

IDENTIFY LIBRARY INTERFERENTS PASSING THE
STATISTICAL TEST AS A POSSIBLE INTERFERENT *3250*

*FIG. 32*

FIG. 33A

FIG. 33B

EP 2 138 097 A2

EP 2 138 097 A2

3130

```
                                                    ┌─3410
┌─────────────────────────────────────────────┐
│        GENERATE SYNTHESIZED SAMPLE POPULATION │
│  SPECTRA, BY ADDING A RANDOM CONCENTRATION OF │
│   POSSIBLE LIBRARY INTERFERENTS, TO FORM AN   │
│  INTERFERENT ENHANCED SPECTRAL DATABASE (IESD)│
└─────────────────────────────────────────────┘
                      │
                      ▼                ┌─3420
          ┌──────────────────────────┐
          │   PARTITION THE IESD INTO A │
          │  CALIBRATION SET AND A TEST SET │
          └──────────────────────────┘
                      │
                      ▼                    ┌─3430
      ┌──────────────────────────────────┐
      │          USE THE CALIBRATION SET TO │
      │  GENERATE A CALIBRATION CONSTANT FOR PREDICTING │
      │  THE ANALYTE CONCENTRATION FROM A MEASUREMENT │
      └──────────────────────────────────┘
                      │
                      ▼                  ┌─3440
      ┌──────────────────────────────────┐
      │  USE THE CALIBRATION CONSTANT TO ESTIMATE THE │
      │    ANALYTE CONCENTRATION OF THE TEST SET │
      └──────────────────────────────────┘
                      │
                      ▼                ┌─3450
      ┌──────────────────────────────────┐
      │  CALCULATE THE ERROR IN THE ESTIMATED ANALYTE │
      │      CONCENTRATION FOR THE TEST SET │
      └──────────────────────────────────┘
                      │
                      ▼              ┌─3460
  ┌──────────────────────────────────────┐
  │   CALCULATE AN AVERAGE CALIBRATION CONSTANT FROM │
  │ THE CALIBRATION CONSTANT AND ERROR FROM MANY TEST SETS │
  └──────────────────────────────────────┘
```

*FIG. 34*

**FIG. 35**

Log-normal distribution: $\mu=100$, c=50
Solid Line 0.5% quantile, Short dashed line Mean, Long dashed line Standard deviation

**FIG. 36**

COMBINATION
INTERFERENT SPECTRA

RANDOMLY-SCALED
INTERFERENT SPECTRA

$I_1$ $I_2$ $I_N$

FIG. 37

COMBINATION
INTERFERENT
SPECTRA
(SCALED)

INTERFERENT ENHANCED
SPECTRAL DATABASE

Replicates of Sample
Population data

Rep 1        Rep 2        Rep M

*FIG. 38*

EP 2 138 097 A2

FIG. 39

*FIG. 40A*

*FIG. 40B*

*FIG. 40C*

*FIG. 40D*

FIG. 41

EP 2 138 097 A2

Mannitol

FIG. 42A

Dextran

FIG. 42B

N Acetyl L Cysteine

FIG. 42C

Procainamide

FIG. 42D

FIG. 43A

FIG. 43B

FIG. 43C

FIG. 43D

FIG. 44

EP 2 138 097 A2

FIG. 45

FIG. 46

EP 2 138 097 A2

FIG. 47

FIG. 48

EP 2 138 097 A2

FIG. 49

EP 2 138 097 A2

FIG. 50

EP 2 138 097 A2

EP 2 138 097 A2

$E_i$

6070

6040

6076

6080

E

6090

6045

6082

6078

$E_r$

6072

*FIG. 50A*

*FIG. 51*

EP 2 138 097 A2

FIG. 52A

EP 2 138 097 A2

*FIG. 52B*

FIG. 52C

P

5000

5100

5110

5115

5120

FIG. 53A

FIG. 53B

FIG. 54A

EP 2 138 097 A2

FIG. 54B

FIG. 54C

EP 2 138 097 A2

FIG. 54D

**REFERENCES CITED IN THE DESCRIPTION**

**Patent documents cited in the description**

- US 20050038357 A
- US 122794 A
- US 20050036146 A
- US 20030090649 A
- US 20030086075 A
- US 20040019431 A
- US 6652136 B
- US 20030178569 A
- US 20050036147 A
- US 5220920 A
- US 5758643 A

**Non-patent literature cited in the description**

- *SAMPLE ELEMENT WITH BARRIER MATERIAL,* 17 February 2005
- *SAMPLE ELEMENT WITH SEPARATOR,* 05 May 2005
- *SAMPLE ELEMENT QUALIFICATION,* 17 February 2005
- *REAGENT-LESS WHOLE-BLOOD GLUCOSE METER,* 15 May 2003
- *DEVICE AND METHOD FOR IN VITRO DETERMINATION OF ANALYTE CONCENTRATIONS WITHIN BODY FLUIDS,* 08 May 2003
- *METHOD OF DETERMINING AN ANALYTE CONCENTRATION IN A SAMPLE FROM AN ABSORPTION SPECTRUM,* 29 January 2004
- *METHOD OF SIMULTANEOUS MIXING OF SAMPLES,* 25 November 2003
- *REAGENT-LESS WHOLE BLOOD GLUCOSE METER,* 15 May 2003
- *PATHLENGTH-INDEPENDENT METHODS FOR OPTICALLY DETERMINING MATERIAL COMPOSITION,* 25 September 2003
- *METHOD OF DETERMINING ANALYTE CONCENTRATION IN A SAMPLE USING INFRARED TRANSMISSION DATA,* 17 February 2005
- *Electrochemical measurement system having interference reduction circuit,* 22 June 1993
- *Electrochemical measurement system having interference reduction circui,* 22 June 1993
- *METHOD AND APPARATUS FOR MONITORING BLOOD CHEMISTRY,* 02 June 1998